# EUROPEAN PATENT SPECIFICATION

(11) **EP 3 856 154 B1**
(45) Date of publication and mention of the grant of the patent: **27.08.2025**
(21) Application number: 19794758.3
(22) Date of filing: 27.09.2019
(51) Int. Cl.: A61K 9/50, A61K 35/12, A61K 38/36, A61K 38/37, C12N 15/85

(54) **IMPLANTABLE DEVICES FOR CELL THERAPY AND RELATED METHODS**
IMPLANTIERBARE VORRICHTUNGEN FÜR DIE ZELLTHERAPIE UND ZUGEHÖRIGE VERFAHREN
DISPOSITIFS IMPLANTABLES POUR THÉRAPIE CELLULAIRE ET PROCÉDÉS ASSOCIÉS

(30) Priority: 27.09.2018 US 201862737835 P; 27.03.2019 US 201962824768 P
(43) Date of publication of application: 04.08.2021
(73) Proprietor: Sigilon Therapeutics, Inc., Cambridge, MA 02142 (US)
(72) Inventor: BARNEY, Lauren, Emily, Cambridge, MA 02142 (US); HEIDEBRECHT, Richard, Cambridge, MA 02142 (US); MILLER, Robert, James, Cambridge, MA 02142 (US); OBERLI, Matthias, Alexander, Cambridge, MA 02142 (US); YIN, Zoe, Cambridge, MA 02142 (US); BEAUREGARD, Michael, Cambridge, MA 02142 (US); JOHNSTON, Erika Ellen, Cambridge, MA 02142 (US); O'CONNOR, Owen, Cambridge, MA 02142 (US); VEISEH, Omid, Cambridge, MA 02142 (US); CARMONA, Guillaume, Cambridge, MA 02142 (US); GONZALEZ, Francisco Caballero, Cambridge, MA 02142 (US); PERITT, David, Cambridge, MA 02142 (US); SMITH, Devyn McKinley, Cambridge, MA 02142 (US); WOTTON, Paul Kevin, Cambridge, MA 02142 (US); SEWELL, Jared A., Cambridge, MA 02142 (US)
(74) Representative: Kilburn & Strode LLP
(86) International application number: PCT/US2019/053637
(87) International publication number: WO 2020/069429

(56) References cited:
- US-A1- 2014 271 843
- US-A1- 2016 030 360
- BOCHENEK MATTHEW A ET AL: "Alginate encapsulation as long-term immune protection of allogeneic pancreatic islet cells transplanted into the omental bursa of macaques", NATURE BIOMEDICAL ENGINEERING, NATURE PUBLISHING GROUP UK, LONDON, vol. 2, no. 11, 13 August 2018 (2018-08-13), pages 810 - 821, XP036629632, DOI: 10.1038/S41551-018-0275-1
- MARWA BELHAJ: "Enhancements in Alginate Microencapsulation Technology & Impacts on Cell Therapy Development", DISSERTATION, 1 January 2018 (2018-01-01), XP055660732, Retrieved from the Internet <URL:https://scholarcommons.sc.edu/cgi/viewcontent.cgi?article=5489&context=etd> [retrieved on 20200122]
- WEBER LANEY M ET AL: "Multifunctional pancreatic islet encapsulation barriers achieved via multilayer PEG hydrogels", CELL TRANSPLANTATION, SAGE, US, vol. 16, no. 10, 1 January 2007 (2007-01-01), pages 1049 - 1057, XP009180672, ISSN: 0963-6897, DOI: 10.3727/000000007783472336
- OJU JEON ET AL: "Biodegradable, Photocrosslinked Alginate Hydrogels with Independently Tailorable Physical Properties and Cell Adhesivity", TISSUE ENGINEERING PART A, vol. 16, no. 9, 21 June 2010 (2010-06-21), US, pages 2915 - 2925, XP055660767, ISSN: 1937-3341, DOI: 10.1089/ten.tea.2010.0096
- ANONYMOUS: "Paper: Correcting Rare Blood Disorders Using Coagulation Factors Produced <em>In Vivo</Em> By Shielded Living Therapeutics<sup>TM</Sup> Products", 7 December 2019 (2019-12-07), XP055660747, Retrieved from the Internet <URL:https://ash.confex.com/ash/2019/webprogram/Paper127050.html> [retrieved on 20200122]

## Description

### BACKGROUND

Treating chronic and genetic diseases by implanting cells engineered to produce a therapeutic substance capable of treating such diseases has exciting potential to improve the health of patients with such diseases. To fully achieve this potential, the implanted cells must be protected from the patient's immune response, so that they remain viable, and the implanted cells must also be capable of producing therapeutic levels of the desired therapeutic substance for several weeks, months or even longer. One exploratory approach for delivering such cellular therapies is to encapsulate the engineered cells into semi-permeable devices (e.g., hydrogel capsules), with the objective that the device structure isolates the cells from immune system cells, while allowing entry of nutrients for the cells and exit of the produced therapeutic substances. A need exists to improve upon this general approach to achieve increased viability and/or productivity of living cells contained in implantable devices. US2014/0271843 discloses biocompatible hydrogel capsules. US2016/0030360 discloses covalently modified alginate polymers. Bochenek et al (2018). Nature Biomedical Engineering discloses single-compartment, covalently modified alginate hydrogel capsules. Belhaj (2018), Enhancements in Alginate Microenscapsulation Technology & Impacts on Cell Therapy Development (Doctoral dissertation) discloses single-compartment, covalently modified alginate hydrogel capsules. Weber et al. (2008) discloses encapsulation of islet cells in PEG-based hydrogel capsules. Jeon et al. (2010), Tissue Engineering discloses covalently modified photocrosslinkable alginates.

### SUMMARY

The invention is defined in the appended set of claims. The description may contain additional technical information, which although not part of the claimed invention, is provided to place the invention in a broader technical context and to illustrate possible related technical developments. The references to the methods of treatment by therapy or surgery or in vivo diagnosis methods in this description are to be interpreted as references to compounds, pharmaceutical compositions and medicaments of the present invention for use in those methods.

Described herein is a device comprising (i) at least one cell-containing compartment which comprises a polymer composition comprising a first cell-binding substance (CBS) and a plurality of cells. In an embodiment, the polymer composition comprises one or more additional cell-binding substances that are the same or different than the first CBS. In an embodiment, the amount of cell-binding substance(s) in the cell-containing compartment is an amount effective to increase viability of the cells and/or increase productivity of the cells *in vitro* (e.g., when the device is incubated in a cell culture medium) or *in vivo* (e.g., when the device is implanted in or otherwise administered to a test subject).

In an embodiment, the CBS is a cell-binding peptide (CBP) or cell-binding polypeptide (CBPP). The CBS recited in the present claims comprises a CBP covalently attached to a polymer molecule via a linker ("CBP-polymer"). In an embodiment not recited in the present claims, the CBS comprises a CBP covalently attached to a polymer molecule without a linker. In an embodiment, the polymer composition in the cell-containing compartment comprises one or more additional CBP-polymers which comprise different cell-binding peptides that are covalently attached to the same or different polymer type that is present in the first CBP-polymer. In an embodiment, cells encapsulated in a device described herein express at least a 1.25-fold, 1.5-fold, 2-fold, 5-fold, 8-fold, or 10-fold greater amount of the therapeutic agent at one or more time points after implant than when encapsulated in a substantially similar device lacking a cell-binding peptide, i.e., a CBS-null reference capsule or particle, as defined herein.

Various cell-binding peptides for use in the devices of the disclosure are described herein. The cell-binding peptide recited in the present claims is 6 amino acids or less in length and comprises the cell binding sequence of a ligand for a cell-adhesion molecule (CAM). In an embodiment not recited in the present claims, the cell-binding peptide consists essentially of a cell binding sequence shown in Table 1 herein. In an embodiment, the cell binding sequence is RGD (SEQ ID NO: 43) or RGDSP (SEQ ID NO: 59). The amino terminus of the cell-binding peptide is covalently attached to the polymer via an amino acid linker. In an embodiment, the amino acid in the linker is achiral. The amino acid linker consists essentially of one to three glycine residues or one to three beta-alanine residues. In an embodiment, the cell-binding peptide consists essentially of RGD (SEQ ID NO: 43) or RGDSP (SEQ ID NO: 59) and the linker consists essentially of a single glycine residue or a single beta-alanine residue.

The polymer in the first CBP-polymer is a polysaccharide. In an aspect not recited in the present claims, the polymer in the first CBP-polymer as another hydrogel-forming polymer. In an embodiment, the polymer in a second CBP-polymer and any other CBP-polymers that are present is also a hydrogel-forming polymer. In some embodiments, the hydrogel-forming polymer in each CBP-polymer in the cell-containing compartment is a polysaccharide. In some embodiments, the hydrogel-forming polymer in each CBP-polymer in the cell-containing compartment is an alginate. In some embodiments, the alginate in each CBP-polymer has a molecular weight of greater than 75 kDa (e.g., 75 kDa to 250 kDa) and a G:M ratio of greater than or equal to 1.5.

In an embodiment, each CBP-polymer present in the first compartment (e.g., in the polymer solution used to form the first compartment) has a cell-binding peptide density (% nitrogen as determined by combustion analysis, e.g., as described in Example 1 herein) of at least about 0.05% nitrogen (e.g., about 0.1%, 0.2% and 0.3% nitrogen) or within a range of between about any of 0.05%, 0.1%, 0.2% and 0.3% nitrogen as the lower value and any of 4%, 3%, 2% and 1% nitrogen as the upper value. In an embodiment, the CBP-polymer in the first compartment is an alginate with an approximate molecular weight of 75 kDa to 150 kD, a G:M ratio ≥ 1.5, that is covalently modified with GRGDSP (SEQ ID NO: 60).

In other embodiments, the density of a linker-RGD moiety (e.g., GRGDSP (SEQ ID NO: 60)) in each CBP-polymer (e.g., GRGDSP-alginate) in the first compartment (e.g., in the polymer solution used to form the first compartment) is determined by a quantitative peptide conjugation assay, e.g., by a quantitative peptide conjugation assay described herein. In an embodiment, the cells are engineered ARPE-19 cells, the linker-RGD moiety in the CBP-polymer is GRGDSP (SEQ ID NO: 60), the polymer is an alginate (e.g., sodium alginate) with an approximate molecular weight of 75 kDa to 150 kDa and a G:M ratio ≥ 1.5, and the determined conjugation density is expressed as micromoles of GRGDSP (SEQ ID NO: 60) / g of GRGDSP-alginate in solution (e.g., saline solution) with a viscosity of 80-120 cP. In an embodiment, the conjugation density for such a GRGDSP-alginate solution (MMW-Alg, G:M ratio ≥ 1.5) is about 0.1 to 1.0 micromoles/g or any of about 0.2 to about 0.8, about 0.3 to about 0.7 or about 0.3 to about 0.6 micromoles /g. In an embodiment, the engineered ARPE-19 cells comprise an exogenous coding sequence for a FVIII BDD protein, e.g., SEQ ID NO:15. In an embodiment, the exogenous coding sequence is operably linked to a promoter sequence, e.g., nucleotides 337-2069 of SEQ ID NO:26.

In an embodiment, the cell-containing compartment also comprises an unmodified hydrogel-forming polymer which is the same or different than the polymer in the CBP-polymer. In an embodiment, both the CBP-polymer and the unmodified polymer are alginate with a molecular weight of 75 kDa (e.g., 75 kDa to 150 kDa) and a G:M ratio of greater than or equal to 1.5.

The plurality of cells (e.g., live cells) (e.g., viable cells) in the cell-containing compartment may be present as single cells, cell clusters (e.g., as spheroids), or attached to a microcarrier. In some embodiments, the plurality of live cells in the first compartment is at least 100, 250, 500, 750, 1,000, 2,500, 5,000, 10,000, 25,000, or 50,000 cells. In an embodiment, the plurality comprises cells derived from a cell line and engineered to express the therapeutic agent. In an embodiment, the cell line is an endothelial, epithelial, fibroblast, keratinocyte or mesenchymal cell line, e.g., a commercially available cell line listed in Table 5 herein. In an embodiment, the plurality of cells are engineered retinal pigment epithelial (RPE cells), e.g. cells engineered from the ARPE-19 cell line. In some embodiments, the plurality of cells is capable of expressing a therapeutic agent, for example, when the device is implanted into a subject.

In an aspect, the therapeutic agent expressed by the plurality of cells is a biological or chemical molecule (e.g., a nucleic acid, a polypeptide, a lipid, sugar or a small molecule) that is capable of directly or indirectly conferring a therapeutic benefit when the device implanted into a subject (e.g., a human). In an embodiment, the therapeutic agent is a protein that is secreted by the cells and which replaces or augments an endogenous protein that is missing or deficient in the subject, e.g., a blood clotting protein that improves blood clotting in a hemophilia patient or an enzyme that mediates the removal of a toxic metabolite which would otherwise build up in the body. In another embodiment, the therapeutic agent is a substance that is secreted by the cells and which performs or mediates a function that results in a therapeutic benefit to the subject. In an embodiment not recited in the present claims, the therapeutic agent is an inhibitory RNA molecule that inhibits the expression of one or more proteins by endogenous cells in the subject to generate a therapeutic benefit. In some embodiments, the therapeutic agent is a replacement therapy or a replacement protein, e.g., useful for the treatment of a blood clotting disorder or a lysosomal storage disease in a subject. In some embodiments, the therapeutic agent is a polypeptide useful for treating a blood clotting disorder, e.g., a Factor VIII protein or variant thereof, a Factor IX protein or variant thereof or a Factor VII protein or variant thereof.

In some embodiments, the device further comprises at least one means for mitigating the foreign body response (FBR), for example, mitigating the FBR when the device is implanted into or onto a subject. Various means for mitigating the FBR of the devices are described herein. In an embodiment, the means for mitigating the FBR comprises a combination of a spherical shape and a linear dimension, e.g., diameter, of at least 1 millimeter (mm), e.g., about 1.25 mm, about 1.5 mm, about 2.0 mm, about 2.5 mm, about 3.0 mm. In an embodiment, the means comprises a barrier compartment formed of a biocompatible polymer that surrounds the cell-containing compartment, and optionally a third compartment formed of a biocompatible polymer that surrounds the barrier compartment, to provide an additional biocompatible physical barrier between the cells in the first compartment and the subject's immune system. In an embodiment, the biocompatible polymer is an alginate.

In another embodiment, the means for mitigating the FBR comprises a compound that mitigates the FBR, e.g., an afibrotic compound as defined herein. In an embodiment, the afibrotic compound is disposed on an exterior surface of the device surface. The means comprises a barrier compartment that surrounds the first compartment and comprises a polymer that is covalently modified with an afibrotic compound (an "afibrotic polymer", as defined herein). In some embodiments, an afibrotic compound is covalently attached to the exterior device surface and to a polymer within the barrier compartment, and optionally to a polymer within the cell-containing compartment. In an embodiment, the barrier compartment comprises an unmodified polymer which is the same or different than the polymer in the afibrotic polymer.

In an embodiment, the afibrotic compound is a compound of Formula (I): 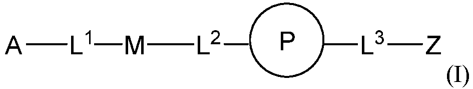 or a pharmaceutically acceptable salt thereof, wherein the variables A, L¹, M, L², P, L³, and Z, as well as related subvariables, are defined herein. In some embodiments, the compound of Formula (I) or a pharmaceutically acceptable salt thereof (e.g., Formulas (I-a), (II), (II-a), (II-b), (III), (III-a), (III-b), (III-c), (III-d), or (III-e)) is a compound described herein, including for example, one of the compounds shown in Table 4 herein. In an embodiment, the afibrotic compound is Compound 100, Compound 101 or Compound 102 shown in Table 4.

In one aspect, a device of the disclosure is a 2-compartment hydrogel capsule (e.g., a microcapsule (less than 1 mm in diameter) or a millicapsule (at least 1 mm in diameter)) in which a cell-containing compartment (e.g., the inner compartment) comprising a plurality of cells (e.g., live cells) and one or more CBP-polymer(s) is surrounded by a barrier compartment (e.g., the outer compartment) comprising an afibrotic polymer. In an embodiment, the afibrotic compound is a compound of Formula (I). In an embodiment, the barrier compartment comprises two or more afibrotic polymers modified with different Formula (I) compounds and comprising the same or different hydrogel-forming polymers. In some embodiments, the inner cell-containing compartment comprises an afibrotic polymer comprising (i) a compound of Formula (I) which is the same or different than the afibrotic compound(s) in the outer barrier compartment and (ii) a polymer which is the same or different than in the CBP-polymer in the inner compartment or in the afibrotic-polymer(s) in the barrier compartment. In an embodiment, the barrier compartment comprises a mixture of the afibrotic polymer and an unmodified hydrogel-forming polymer. In an embodiment, the unmodified hydrogel-forming polymer is an alginate with a molecular weight of 150 kDa to 250 kDa and a G:M ratio of greater than or equal to 1.5.

In an embodiment, the compartments in a multi-compartment hydrogel capsule of the disclosure have approximately equal volumes, e.g., a variance of less than 20%, 10% or 5%. In an embodiment, the hydrogel capsule of the disclosure has two compartments and is spherical in shape, has a diameter of about 0.5 mm to about 1.5 mm, about 0.8 mm to about 1.2 mm, or about 1.0 mm to about 1.5 mm. In an embodiment, the thickness of the second compartment is about 10 to about 500 microns, 10 to about 300 microns, about 20 to about 150 microns, or about 40 to about 75 microns.

In another aspect, a device of the disclosure comprises a second plurality of cells which are encapsulated in the first cell-containing compartment or in a second cell-containing compartment. In an embodiment, the second plurality of cells are engineered from the same cell line as in the first plurality, but express a different therapeutic agent. In another embodiment, the second plurality of cells are engineered from a different cell line than in the first plurality, but express the same therapeutic agent as the first plurality.

In another aspect, the present disclosure features a preparation (e.g., a composition) comprising a plurality (at least any of 3, 6, 12, 25, 50 or more) of cell-containing devices described herein. In one embodiment, one or more of the devices in the plurality is a device comprising (i) a cell-containing compartment which comprises a plurality of cells (e.g., live cells) encapsulated in a polymer composition comprising a first polymer covalently modified with a first cell-binding peptide (first CBP-polymer), wherein the cells are capable of expressing a therapeutic agent when the device is implanted into a subject; and means for mitigating the foreign body response (FBR) when the device is implanted into the subject. In some embodiments, at least 75%, 80%, 85%, 90%, 95%, 99%, or more of the devices in the plurality are spherical hydrogel capsules. In some embodiments, the preparation is a pharmaceutically acceptable composition.

In another aspect, the present disclosure features a method of implanting into a subject a device or a device preparation described herein. In another aspect, the present disclosure features a method of providing a therapeutic agent to a subject comprising administering to the subject one or more of the devices described herein, wherein the plurality of cells in the administered device(s) has the ability to produce the therapeutic agent after administration. In another aspect, the present disclosure features a method of treating a subject in need of a therapeutic agent comprising administering to the subject one or more of the devices described herein, wherein the administered device(s) has the ability to produce the therapeutic agent. In some embodiments, the administering step comprises implanting in the subject a pharmaceutically acceptable preparation comprising a plurality of devices, each of which has the ability to produce the therapeutic agent. In some embodiments, the subject is a mammal (e.g., a human).

In another aspect, the present disclosure features a method of evaluating a device described herein. In some embodiments, the method comprises providing a device described herein and evaluating a structural or functional parameter of the device. In some embodiments, the method comprises evaluating the device or devices within a preparation described herein for one or more of a) cell viability and b) amount of the therapeutic agent produced. In some embodiments, the evaluation is performed at least 1, 5, 10, 20, 30, 60, 90 or 120 days after (i) formation of the device (or preparation of devices) or (ii) administration of the device (or preparation of devices) to a subject. In an embodiment, the evaluation further comprises assessing the amount of fibrosis and /or structural integrity of the device (or devices within a preparation) at least 10, 20, 30, 60, 90 or 120 days after implant into the subject. In some embodiments, the subject is a mammal (e.g., a mouse (e.g., an immune-competent mouse), a non-human primate, a human).

The details of one or more embodiments of the disclosure are set forth herein. Other features, objects, and advantages of the disclosure will be apparent from the Detailed Description, the Figures, the Examples, and the Claims.

### BRIEF DESCRIPTION OF THE DRAWINGS

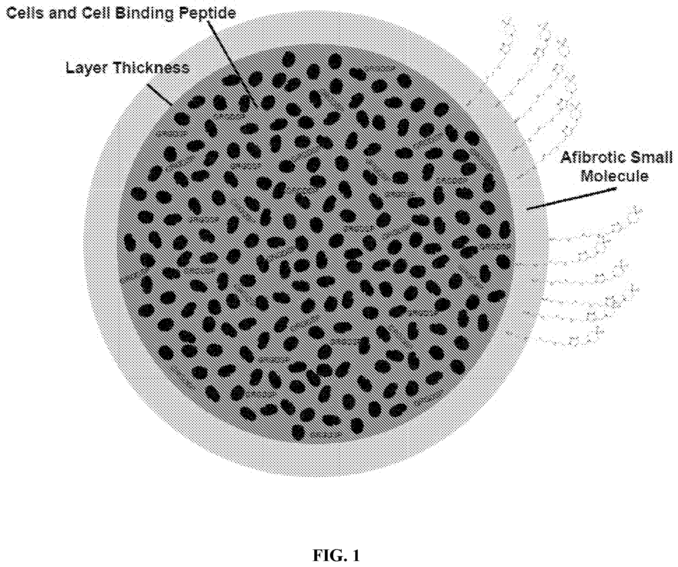

**FIG. 1** illustrates an exemplary spherical hydrogel two-compartment capsule of the disclosure, with lines indicating: a first, inner compartment formed from a polymer covalently attached to a cell binding peptide and cells encapsulated therein; a second compartment; and an afibrotic compound disposed both within the second compartment and on the surface of the capsule. FIG. 1 discloses "GRGDSP" as SEQ ID NO: 60.

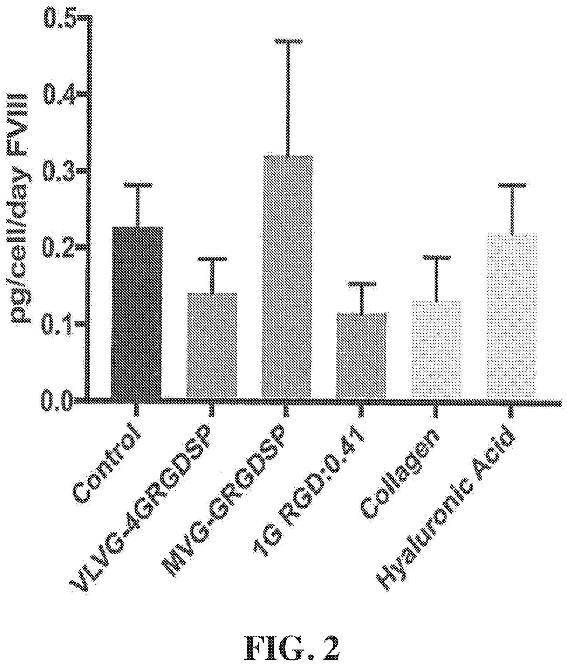

**FIG. 2** is a bar graph showing the *in vitro* productivity of ARPE19 cells engineered to express a FVIII-BDD protein (ARPE-19:FVIII-BDD cells) and then encapsulated in single-compartment hydrogel capsules formed from alginate solutions comprising no cell-binding site (control) or a cell-binding site as described in Example 2 below.

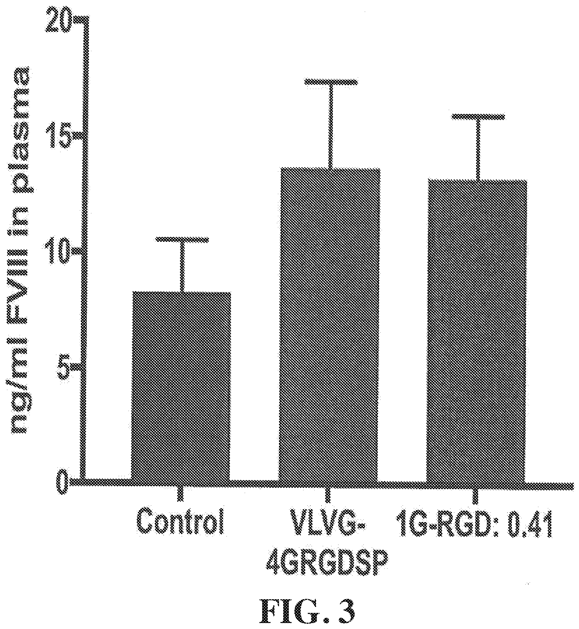

**FIG. 3** is a bar graph showing plasma FVIIII-BDD levels at 2 weeks after implantation into nude mice of two-compartment hydrogel capsules in which the second compartment was formed from a mixture of an afibrotic alginate and an unmodified alginate and the first compartment was formed from a suspension of ARPE-19:FVIII-BDD cells in a test CBP-alginate solution described in the Examples herein (VLVG-4GRGDSP, 1G-RGD: 0.41) or the afibrotic alginate + unmodified alginate mixture (Control).

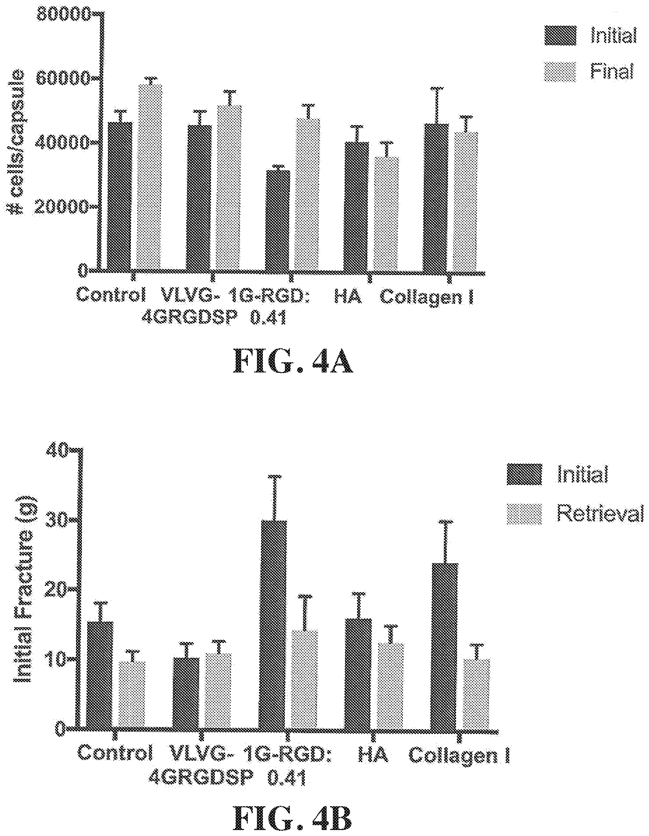

**FIGS. 4A and 4B** are bar graphs showing the number of cells/capsule (FIG. 4A) and mechanical strength (FIG. 4B) before implantation (Initial) and 1 month after IP implantation into mice (Final) of two-compartment hydrogel capsules in which the second compartment was formed from a mixture of an afibrotic alginate and an unmodified alginate and the first compartment was formed from a suspension of ARPE-19:FVIII-BDD cells in a test alginate solution comprising a CBP-alginate (VLVG-4GRGDSP, 1G-RGD: 0.41), HA, or Collagen I or the afibrotic alginate + unmodified alginate mixture (Control).

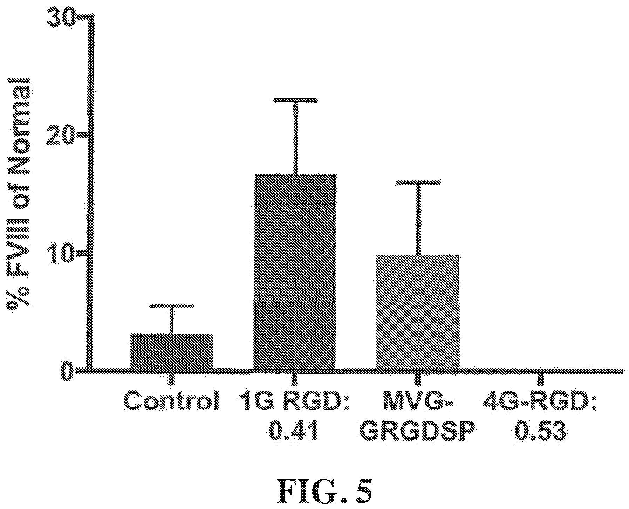

**FIG. 5** is a bar graph showing the plasma FVIIII-BDD levels at 2 weeks after implantation into nude mice of two-compartment hydrogel capsules in which the second compartment was formed from a mixture of an afibrotic alginate and an unmodified alginate and the first compartment was formed from a suspension of ARPE-19:FVIII-BDD cells in a test CBP-alginate solutions described in the Examples herein (1G RGD: 0.41, MVG-GRGDSP, 4G-RGD: 0.53) or the afibrotic alginate + unmodified alginate mixture (Control).

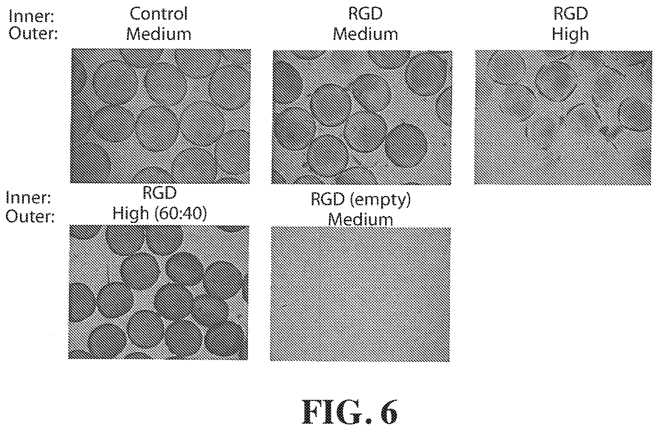

**FIG. 6** shows brightfield images of two-compartment hydrogel millicapsules with a 50:50 volume ratio of inner:outer compartments. Second (outer) compartments comprise a medium or high conjugation afibrotic alginate. Inner compartments contain either a medium conjugation afibrotic alginate (Control) or a CBP-alginate (RGD) with or without (RGD, Empty) exemplary RPE cells engineered to express an exogenous protein. The capsules were imaged after 11 day implantation in C57/BL6 mice for the presence of fibrosis.

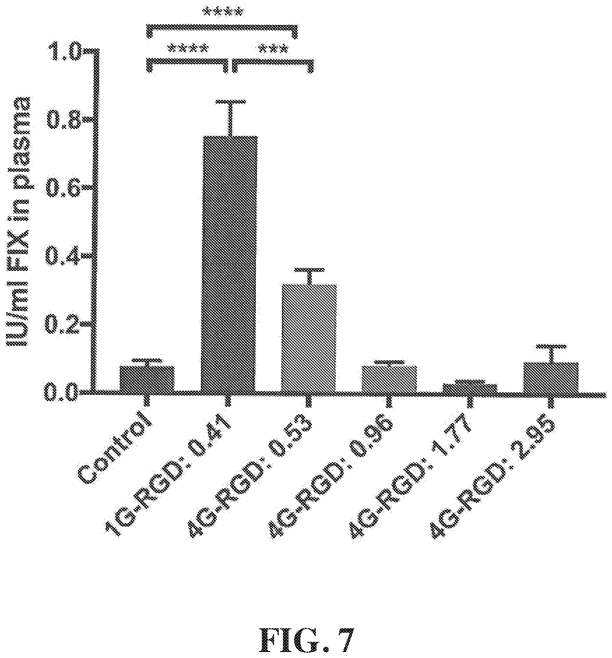

**FIG. 7** is a bar graph showing the plasma levels of FIX in nude mice after 2 week implantation of 2-compartment hydrogel capsules containing RPE cells engineered to express a FIX protein. The RPE cells were encapsulated in the first compartment with varying alginate compositions: no cell binding peptide (control), alginate modified with varying densities of an RGD peptide via a linker with a single glycine residue (1G-RGD: 0.41 or a 4G linker (SEQ ID NO: 61) (4G-RGD: 0.53, 0.96, 1.77 and 2.95).

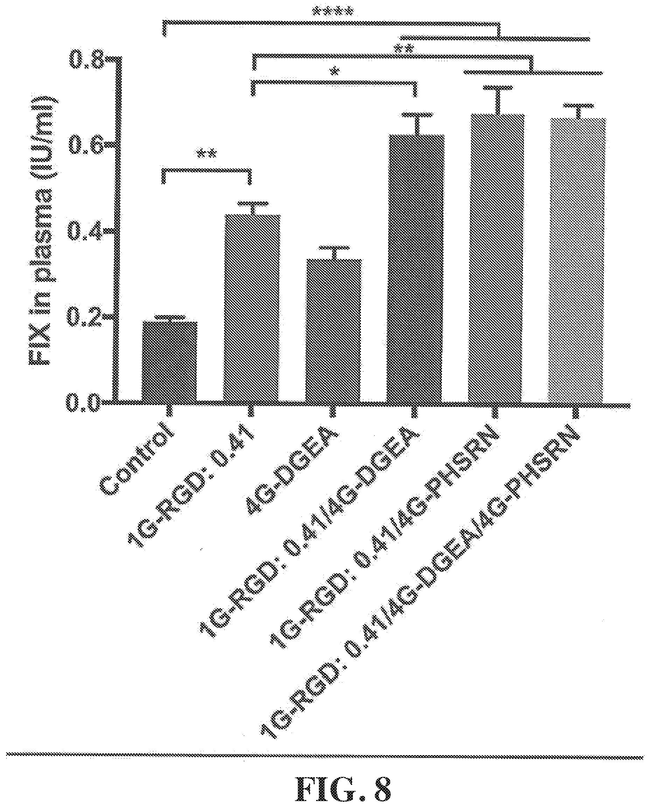

**FIG. 8** is a bar graph showing the plasma levels of FIX in nude mice after 2 week implantation of 2-compartment hydrogel capsules containing RPE cells engineered to express a FIX protein. The RPE cells were encapsulated in the first compartment with varying alginate compositions: no cell binding peptide (control), alginate modified with 1G-RGD, 4G-DGEA or 4G-PHSRN or combinations of 1G-RGD blended with other CBP alginates. (1G-RGD: 0.41, 4G-DGEA, 1:1 1G-RGD: 0.41/4G-DGEA, 1:1 1G-RGD: 0.41/4G-PHSRN, 1:1:1 1G-RGD: 0.41/4G-DGEA/4G-PHSRN).

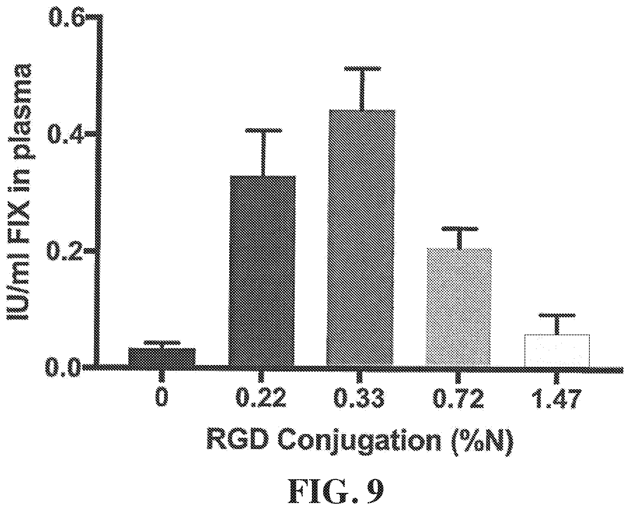

**FIG. 9** is a bar graph showing the plasma levels of FIX in nude mice after 2 week implantation of 2-compartment hydrogel capsules containing RPE cells engineered to express a FIX protein. The RPE cells were encapsulated in the first compartment with varying 1G-RGD density (0, 0.22, 0.33, 0.72 and 1.47% N).

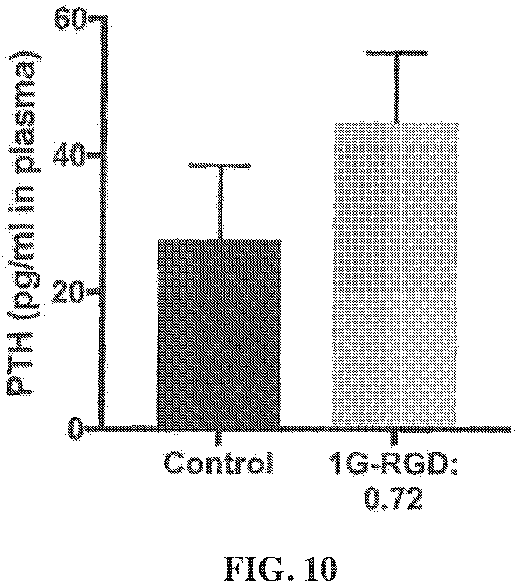

**FIG. 10** is a bar graph showing the plasma levels of PTH in nude mice after 2 week implantation of 2-compartment hydrogel capsules containing RPE cells engineered to express a PTH protein. The RPE cells were encapsulated in the first compartment with varying either control or 1G-RGD: 0.72 polymer.

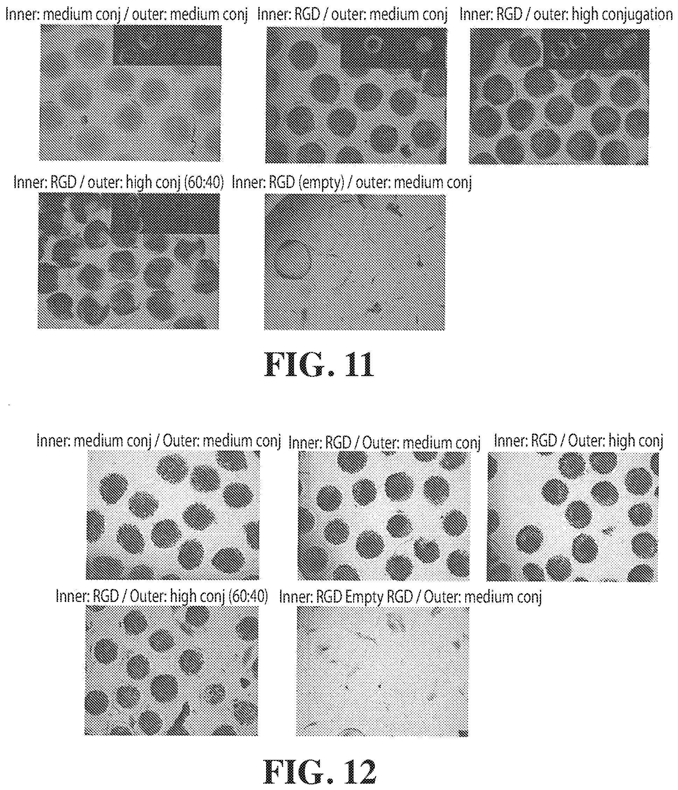

**FIG. 11** shows brightfield images of two-compartment hydrogel millicapsules where second (outer) compartments comprise a medium or high conjugation afibrotic alginate. Inner compartments contain either a medium conjugation afibrotic alginate (Control) or a CBP-alginate (RGD) with or without (RGD, Empty) exemplary RPE cells engineered to express an exogenous protein.
**FIG. 12** shows brightfield images of two-compartment hydrogel millicapsules where second (outer) compartments comprise a medium or high conjugation afibrotic alginate. Inner compartments contain either a medium conjugation afibrotic alginate (Control) or a CBP-alginate (RGD) with or without (RGD, Empty) mouse-derived Balb/3T3 cells.

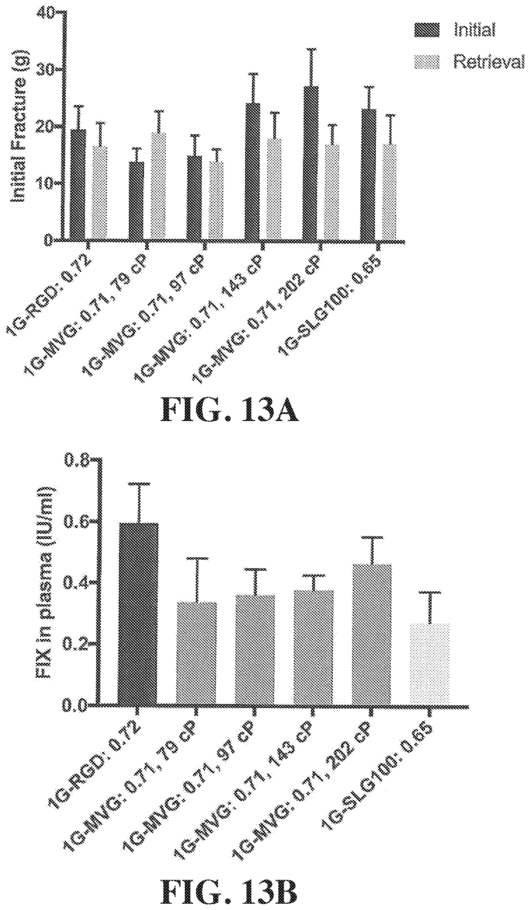

**FIGS. 13A and 13B** are bar graphs showing the initial fracture (FIG 13A) and FIX levels in the plasma (FIG. 13B) for capsules prepared with 1G-RGD polymers with varying polymer identity and solution viscosity.

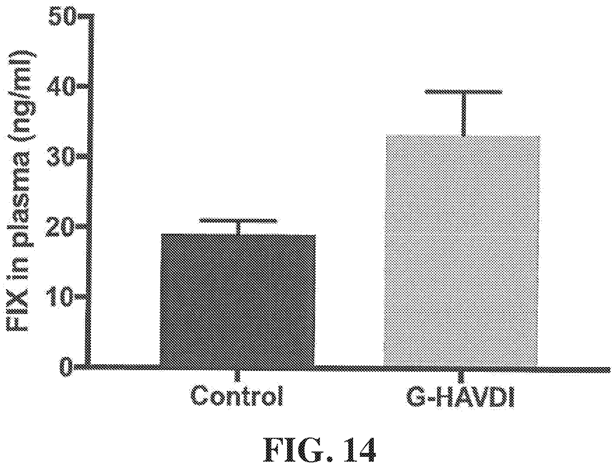

**FIG. 14** is a bar graph showing the plasma levels of FIX in nude mice after 2 week implantation of 2-compartment hydrogel capsules containing RPE cells engineered to express a FIX protein. The RPE cells were encapsulated in the first compartment with varying either control or 1G-HAVDI polymer.

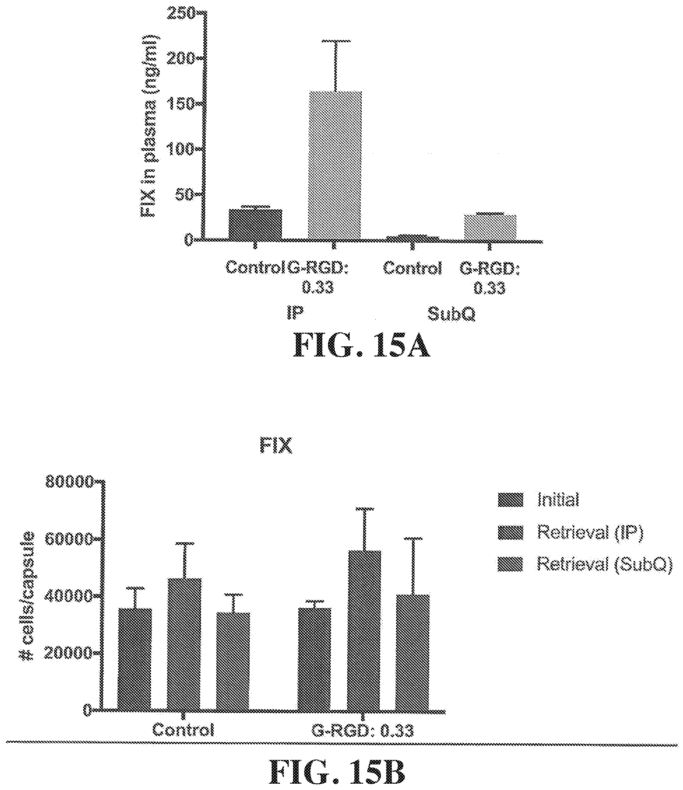

**FIGS. 15A and 15B** are bar graphs showing the FIX-padua levels and number of viable cells per capsule for capsules prepared with control or 1G-RGD polymers in the first compartment implanted into either the IP or SubQ site.

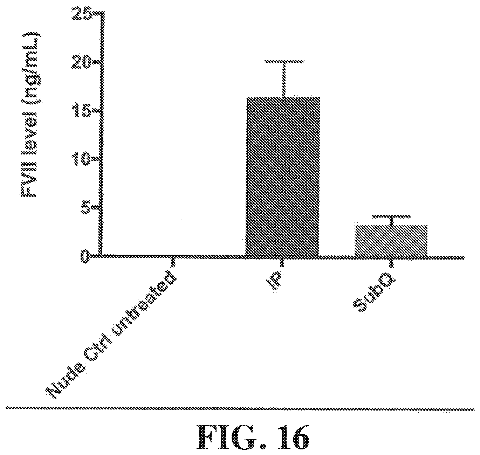

**FIG. 16** is a bar graph showing the plasma levels of FVII-padua for untreated nude mice, or mice with capsules containing ARPE19:FVII with 1G-RGD: 0.33 in the first compartment.

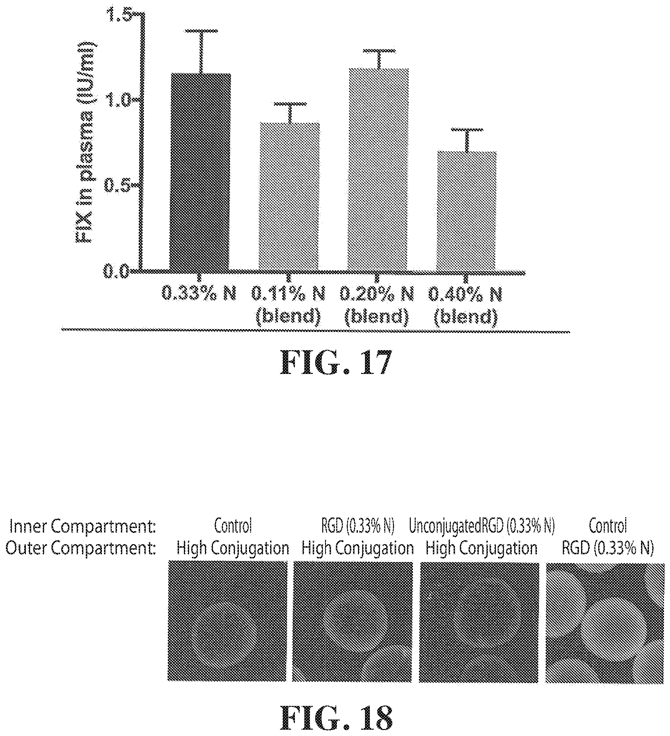

**FIG. 17** is a bar graph showing the plasma levels of FIX in nude mice after 2 week implantation of 2-compartment hydrogel capsules containing RPE cells engineered to express a FIX protein. The RPE cells were encapsulated in the first compartment with varying either 1G-RGD: 0.33 or 1:55, 1:30 or 1:50 blends of 1G-RGD: 6.04 with unmodified SLG20 polymer.
**FIG. 18** are black and white photocopies of fluorescent images of two-compartment capsules with either control or RGD-conjugated alginate on the first or second compartment. The capsules are stained for F4/80 to identify mouse macrophages adhered to the capsule surface, which normally appear as green fluorescence, but shown in white in these photocopies.

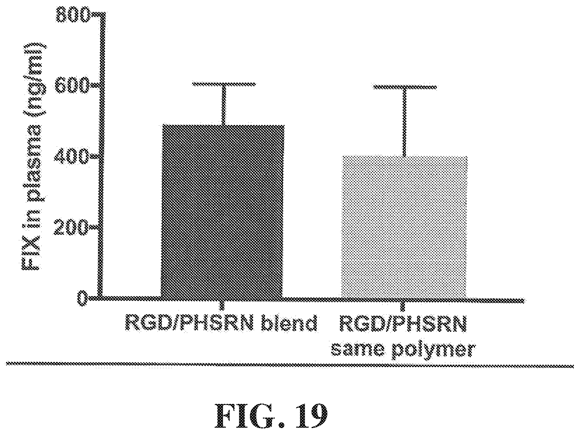

**FIG. 19** is a bar graph showing the plasma levels of FIX-padua in nude mice after 2 week implantation of 2-compartment hydrogel capsules containing RPE cells engineered to express a FIX protein. The RPE cells were encapsulated in the first compartment that contains both 1G-RGD and G-PHSRN peptides either on different polymers that were blended together or both conjugated to the same polymer.

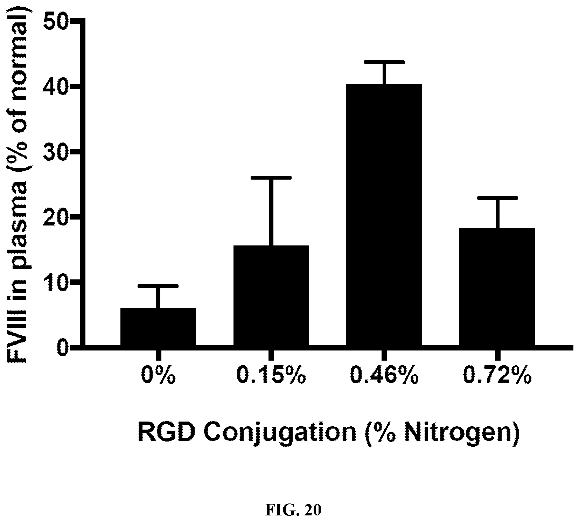

**FIG. 20** is a bar graph showing the plasma levels of FVIII-BDD in nude mice (5 mice per group) after 11 days implantation of 2-compartment hydrogel capsules containing RPE cells engineered to express the FVIII protein (SEQ ID NO:1 encoded by SEQ ID NO:8).

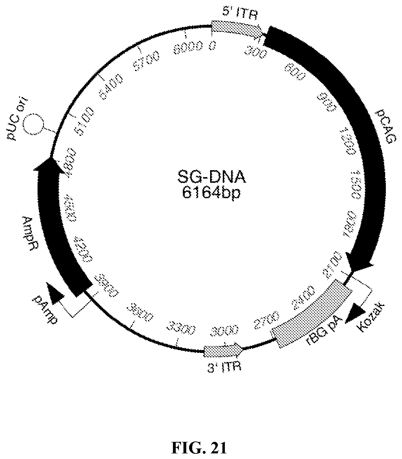

**FIG. 21** illustrates a PiggyBac transposon expression vector useful for generating engineered RPE cells.

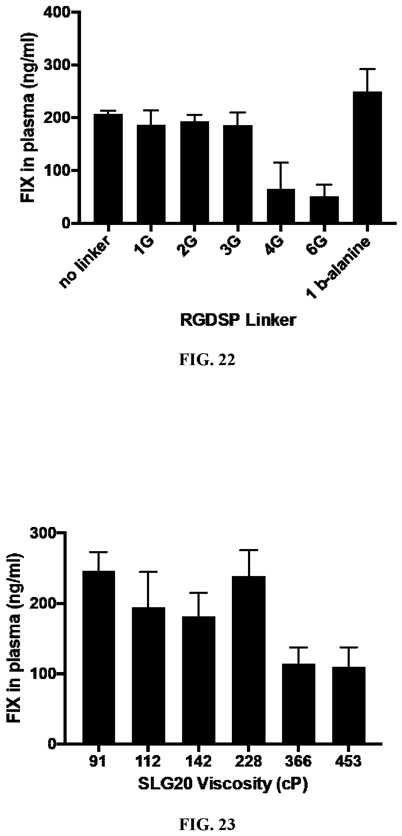

**FIG. 22** is a bar graph depicting the plasma levels of FIX protein in nude mice (3 mice per group) after 12 days implantation of 2-compartment hydrogel capsules containing exemplary RPE cells engineered to express the FIX protein. The engineered RPE cells were encapsulated in the first compartment of capsules modified with CBP-polymers comprising various linker lengths and sequences. FIG. 22 discloses "RDGSP" as SEQ ID NO: 59.
**FIG. 23** is a bar graph depicting the plasma levels of FIX protein in nude mice (4 mice per group) after 18 days implantation of 2-compartment hydrogel capsules containing exemplary RPE cells engineered to express the FIX protein. The engineered RPE cells were encapsulated in the first compartment of capsules prepared with CBP-modified alginate of different solution viscosities.

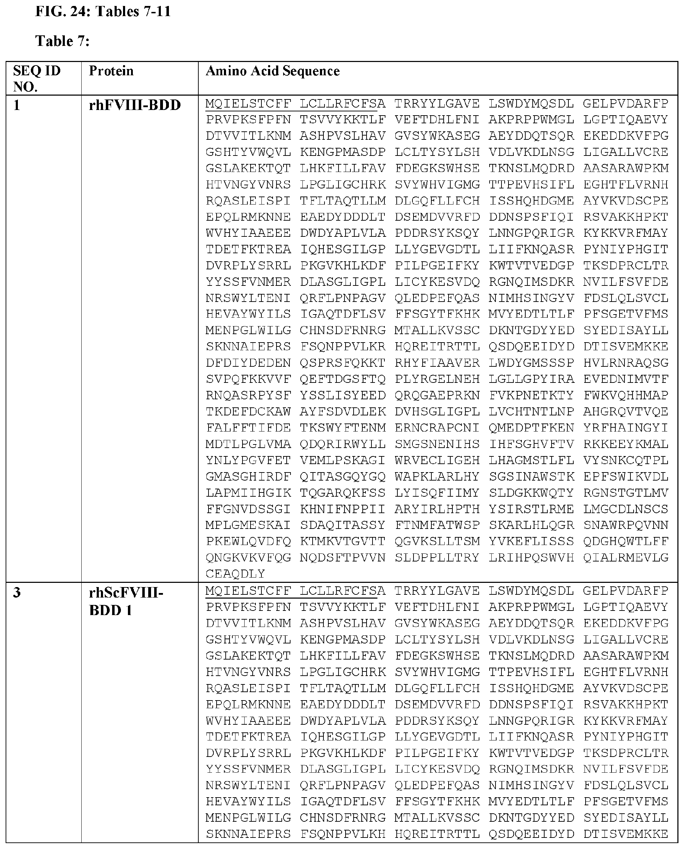

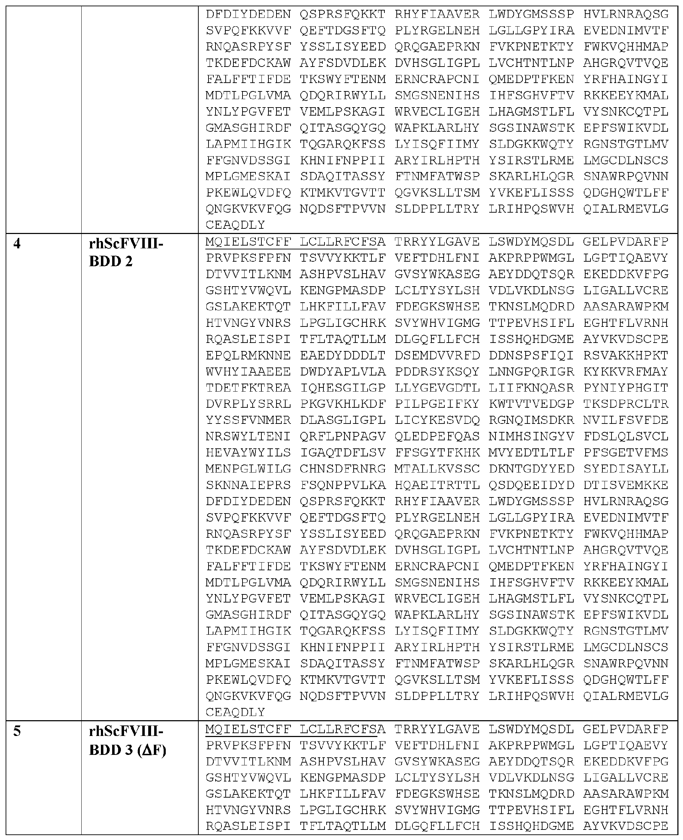

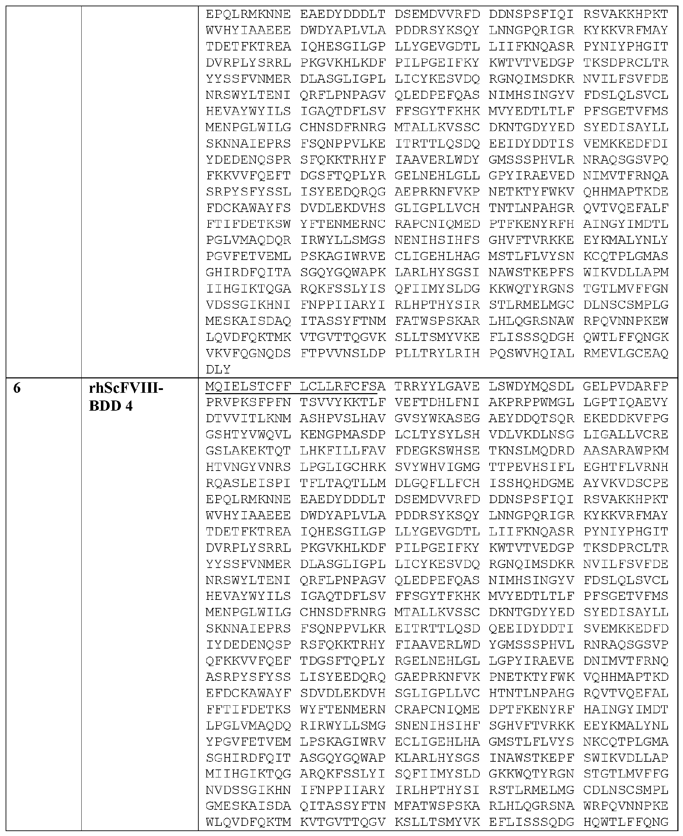

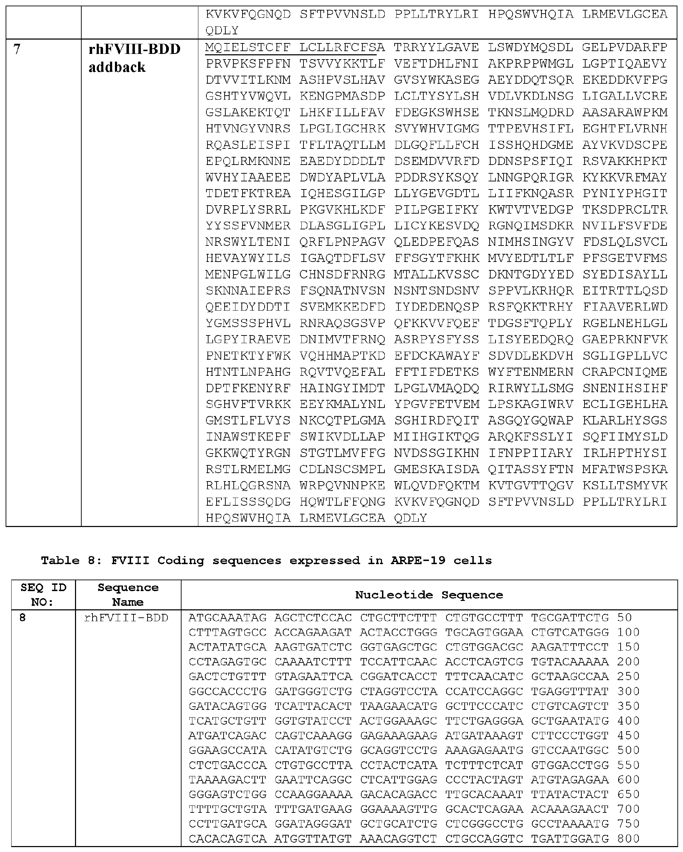

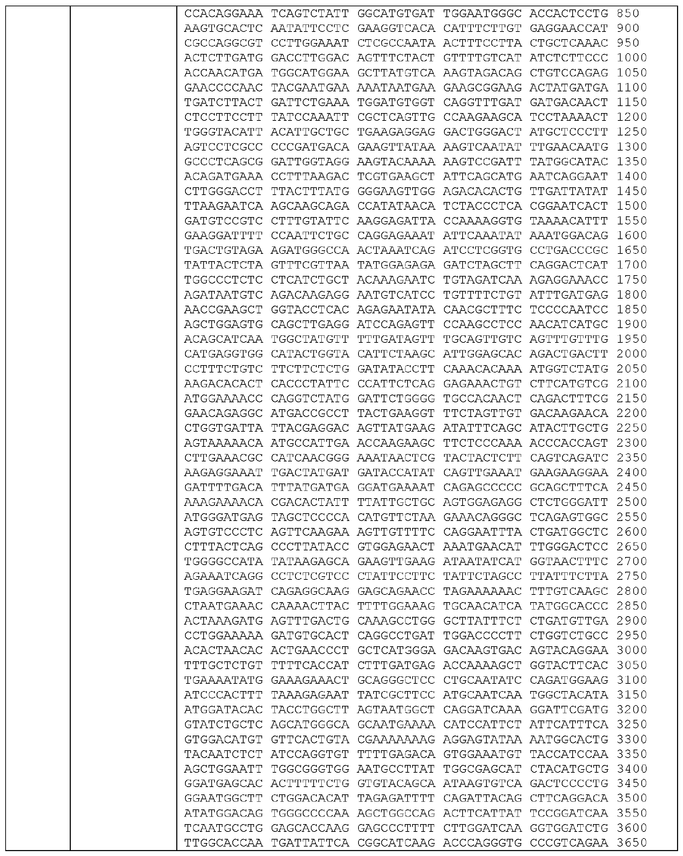

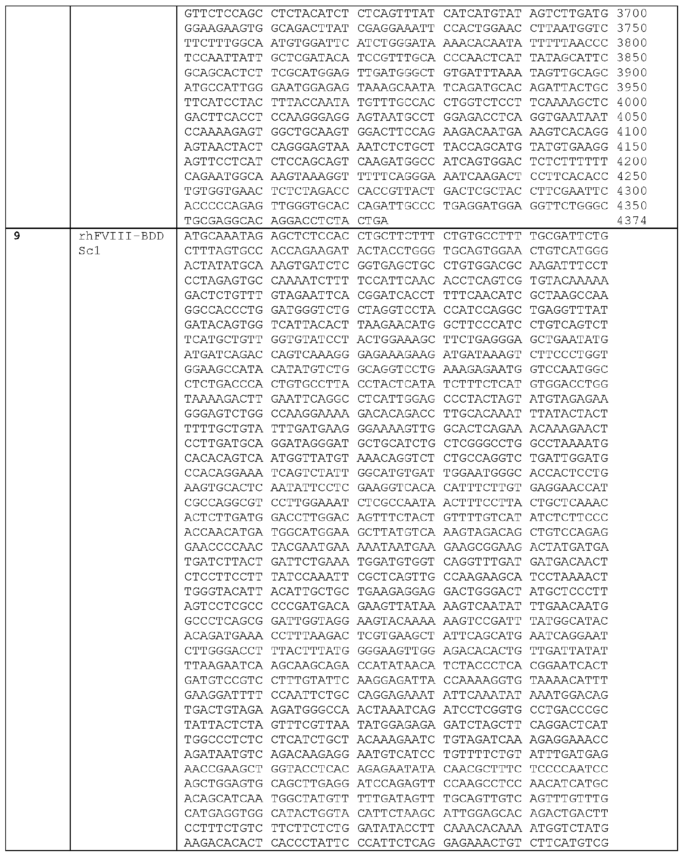

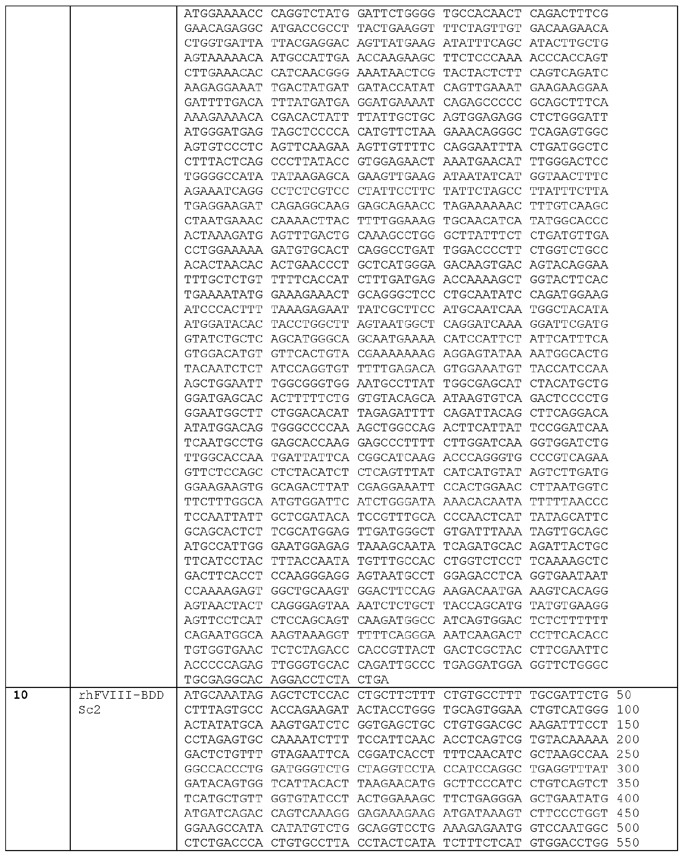

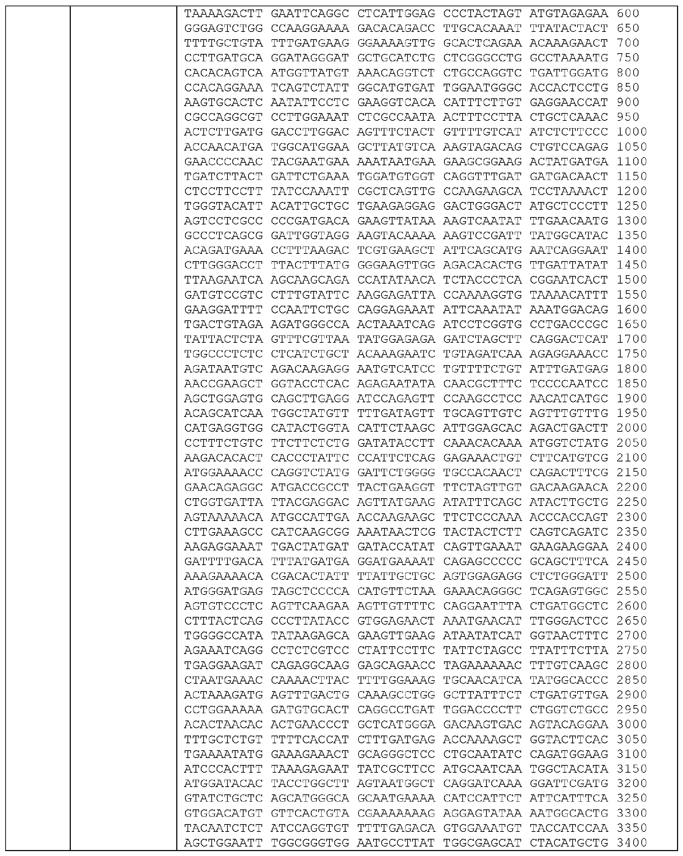

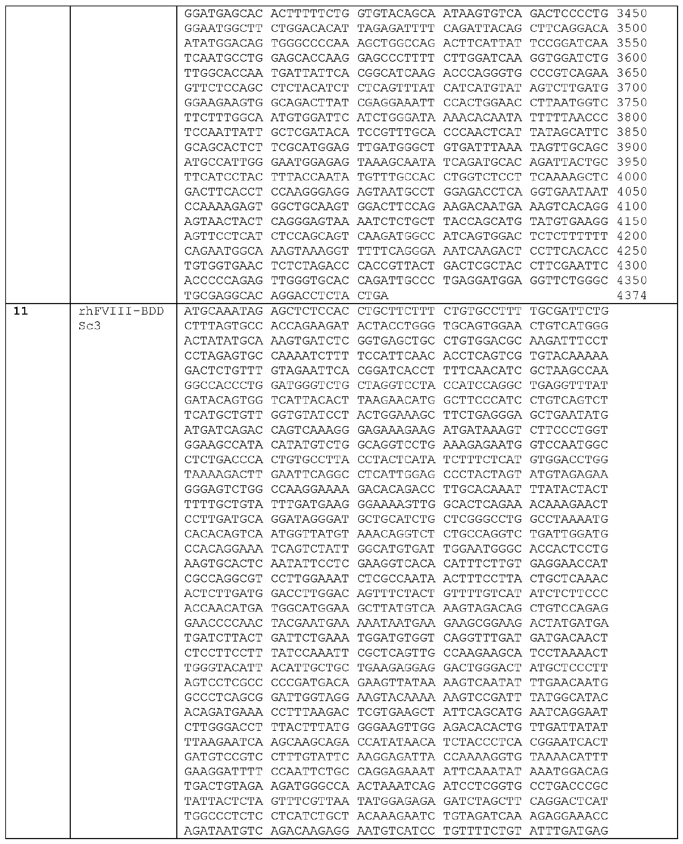

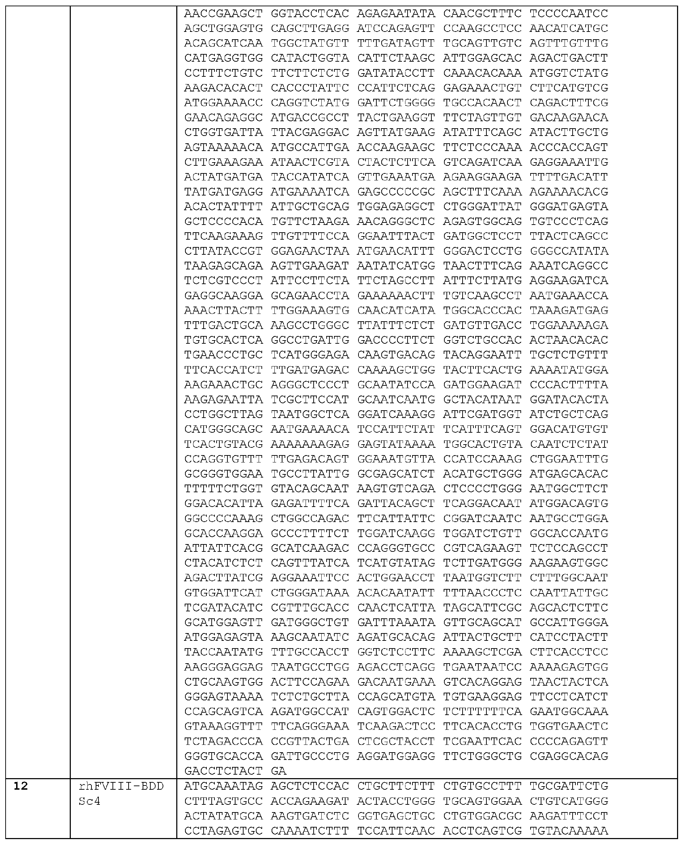

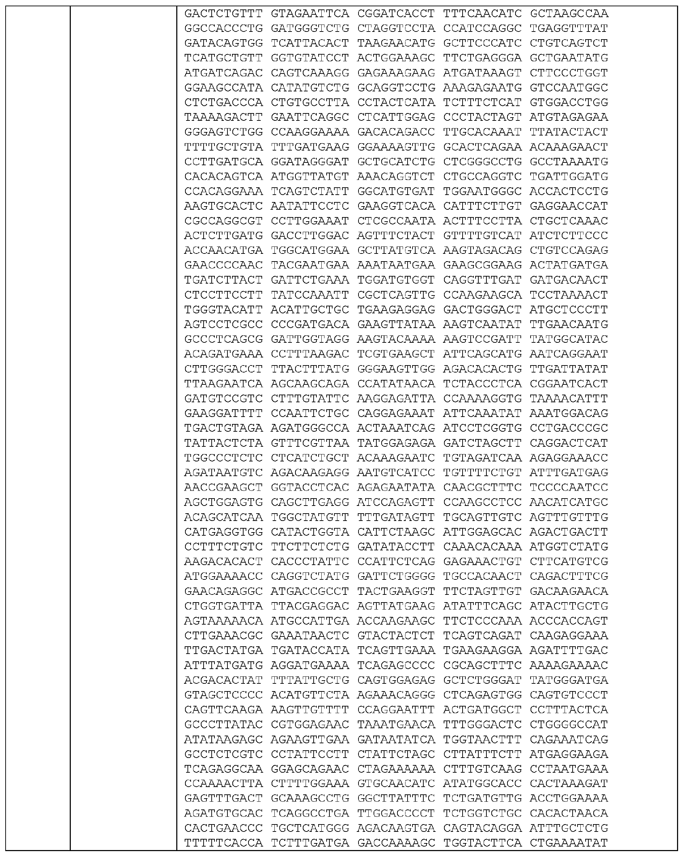

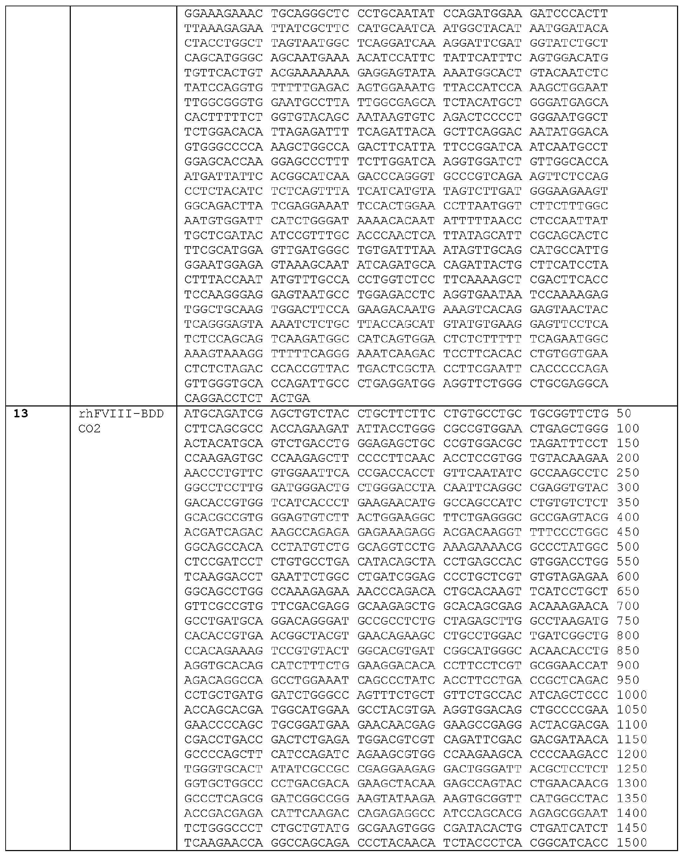

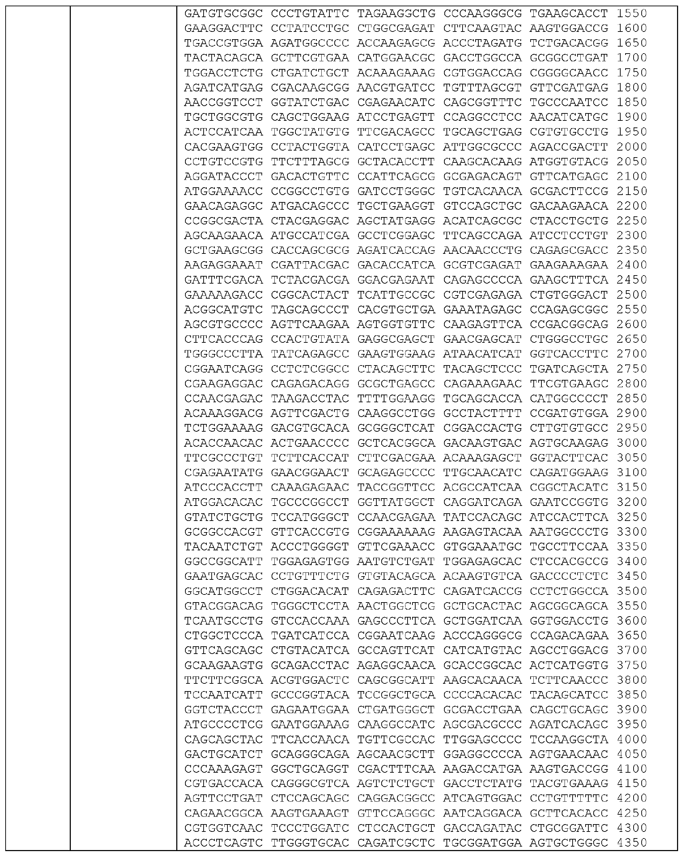

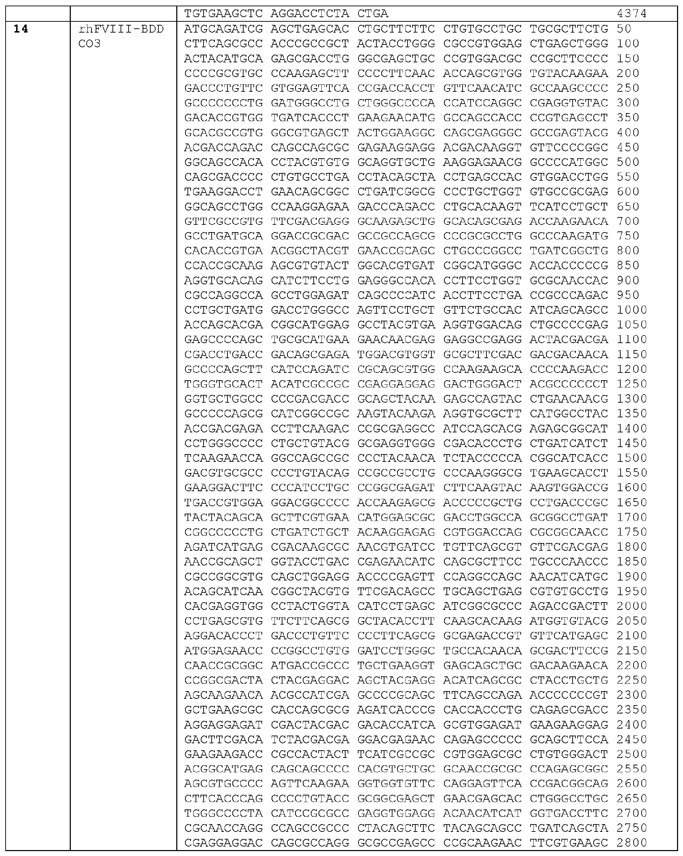

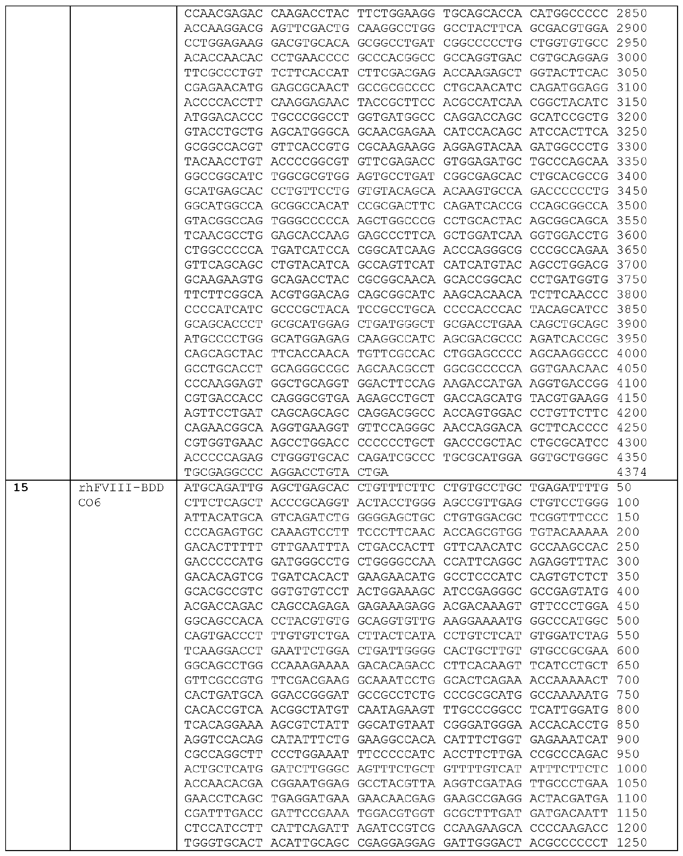

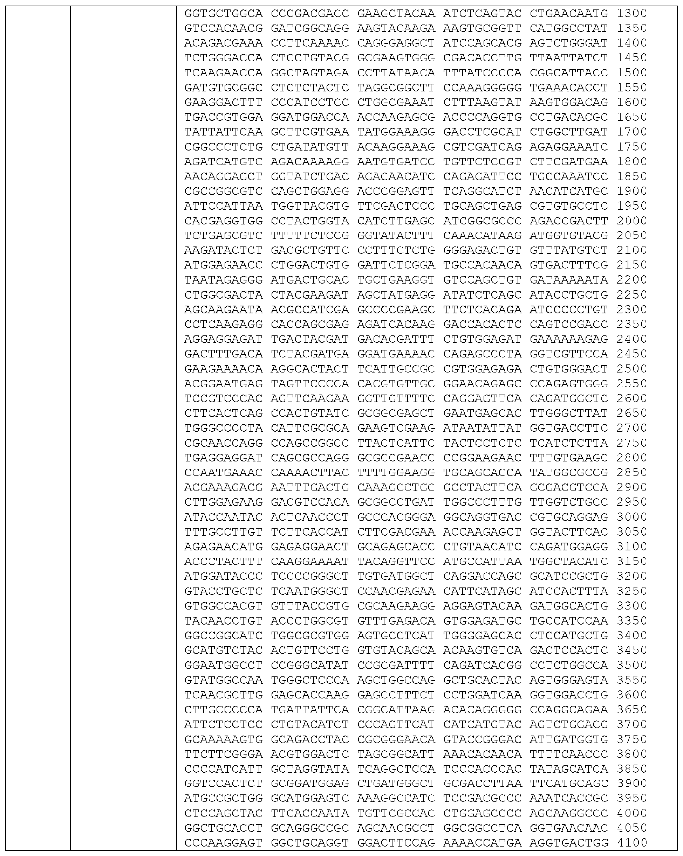

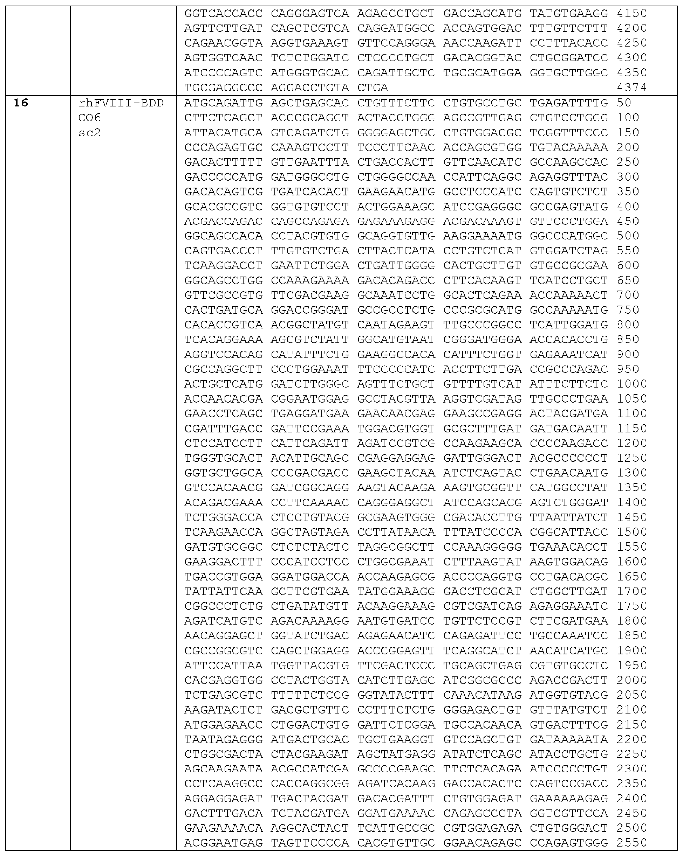

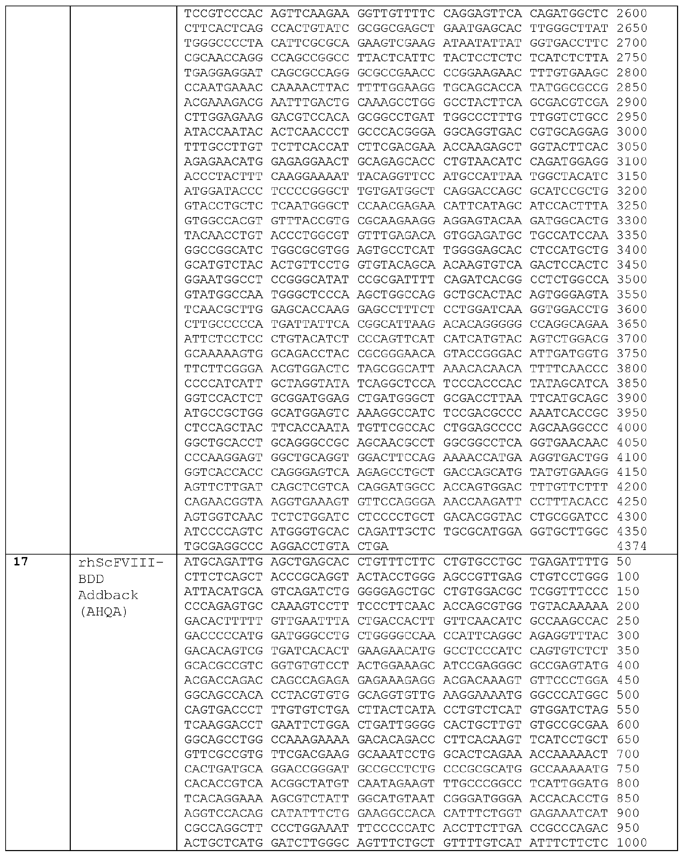

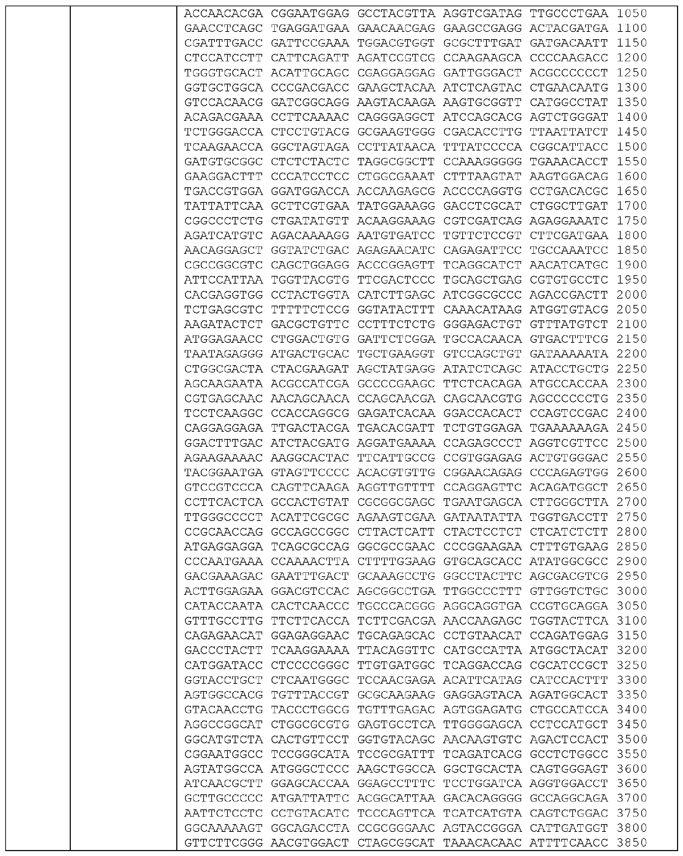

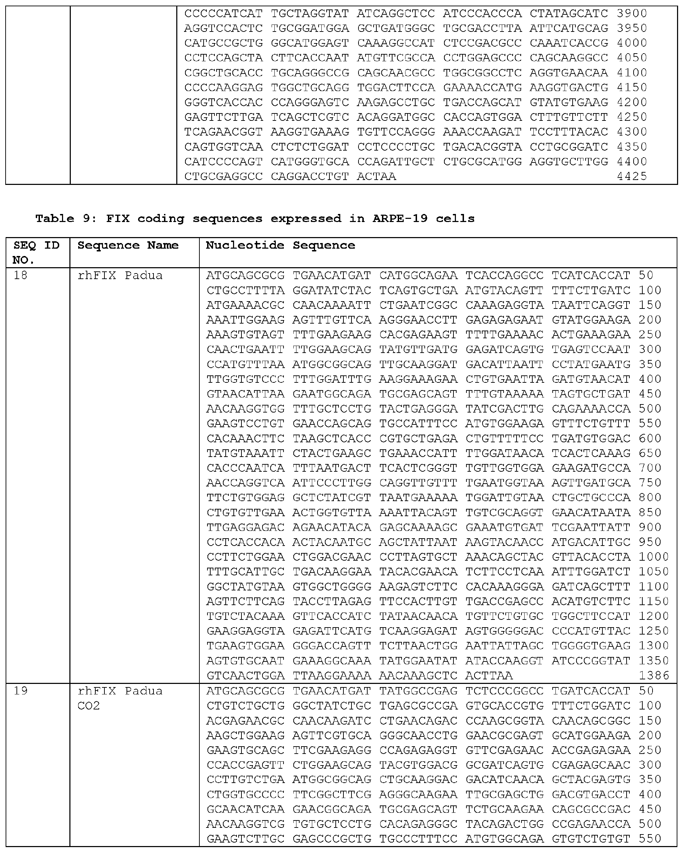

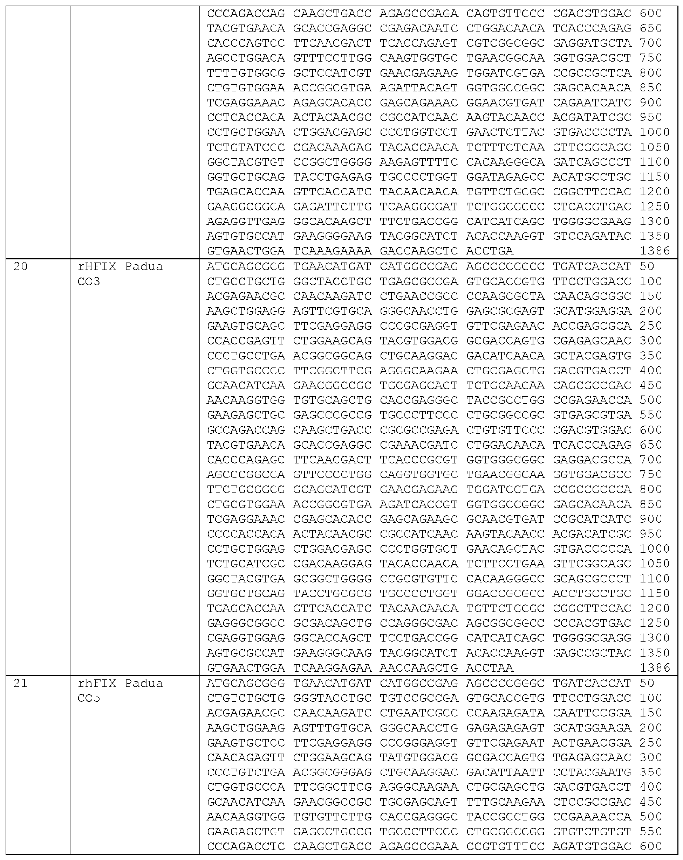

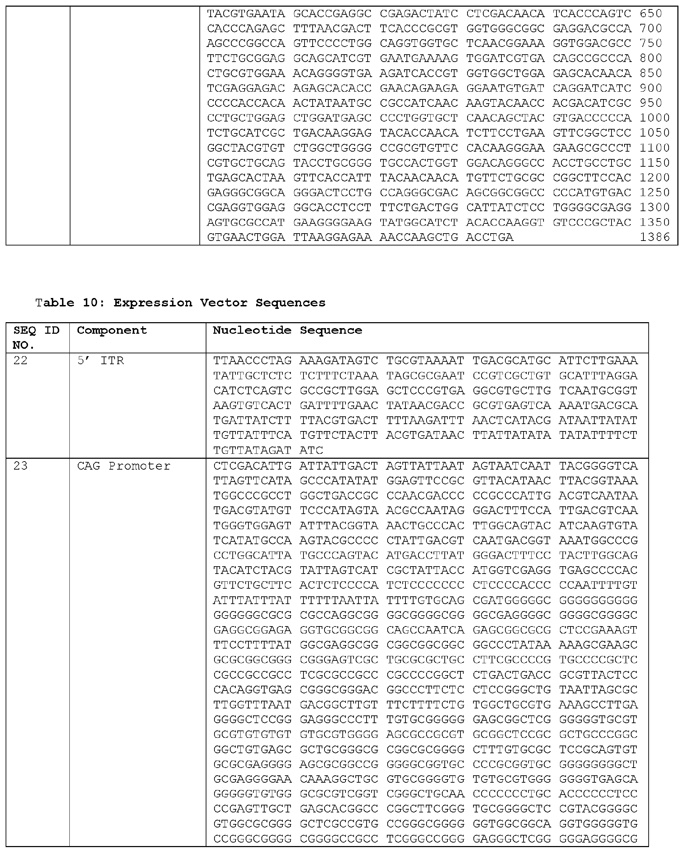

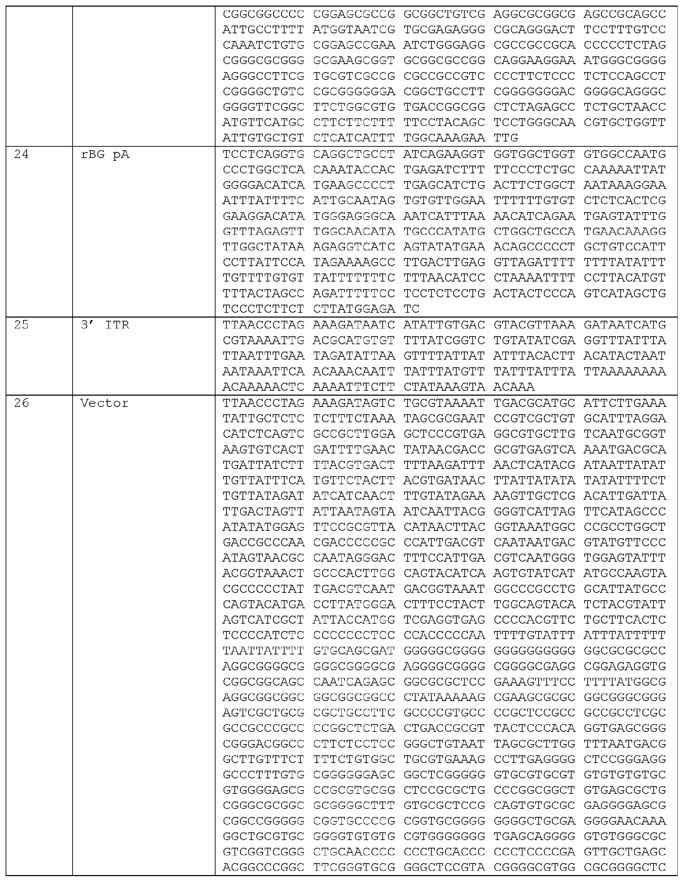

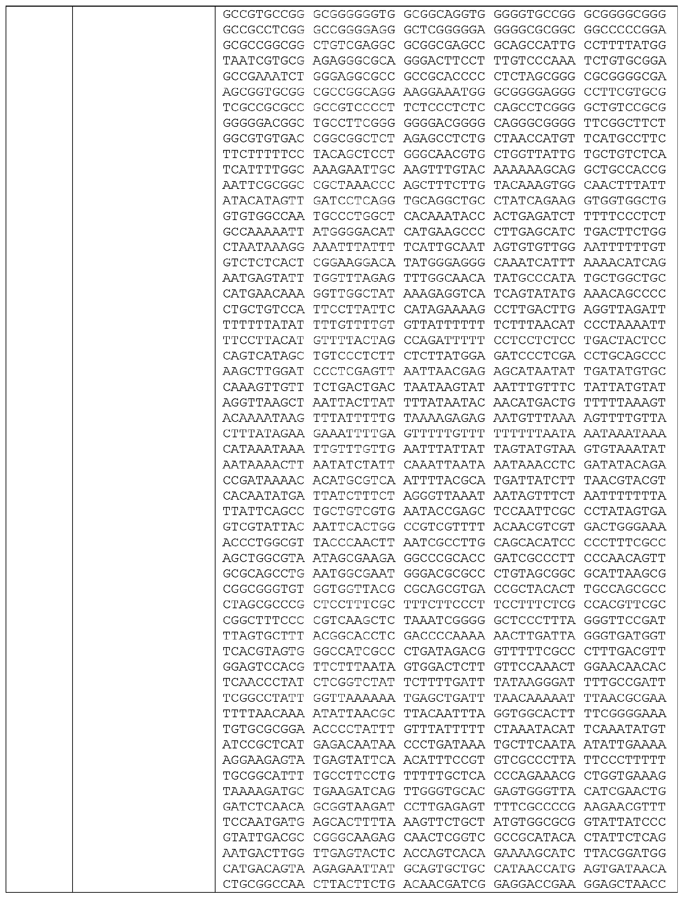

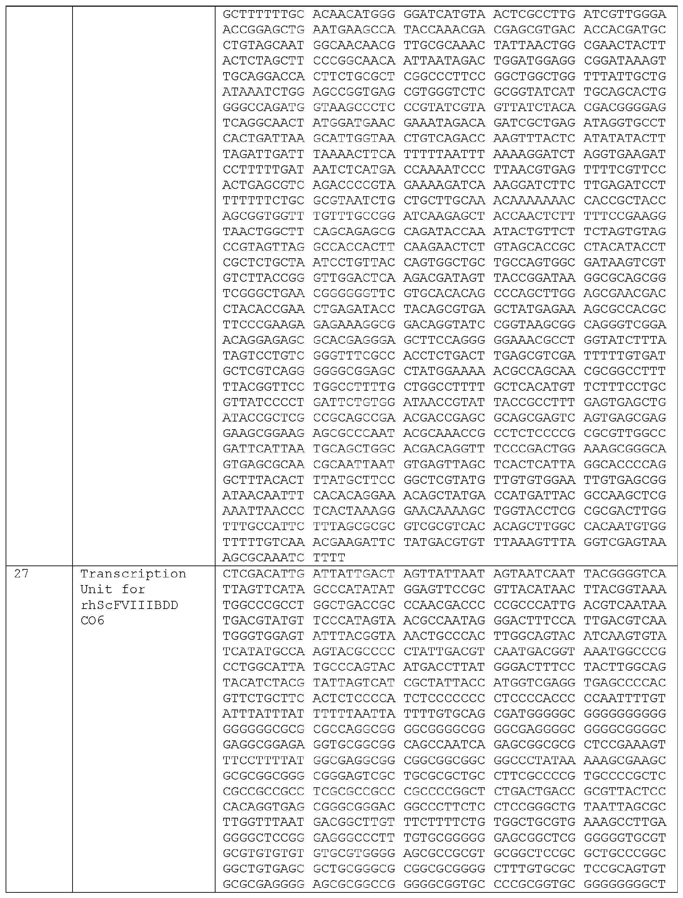

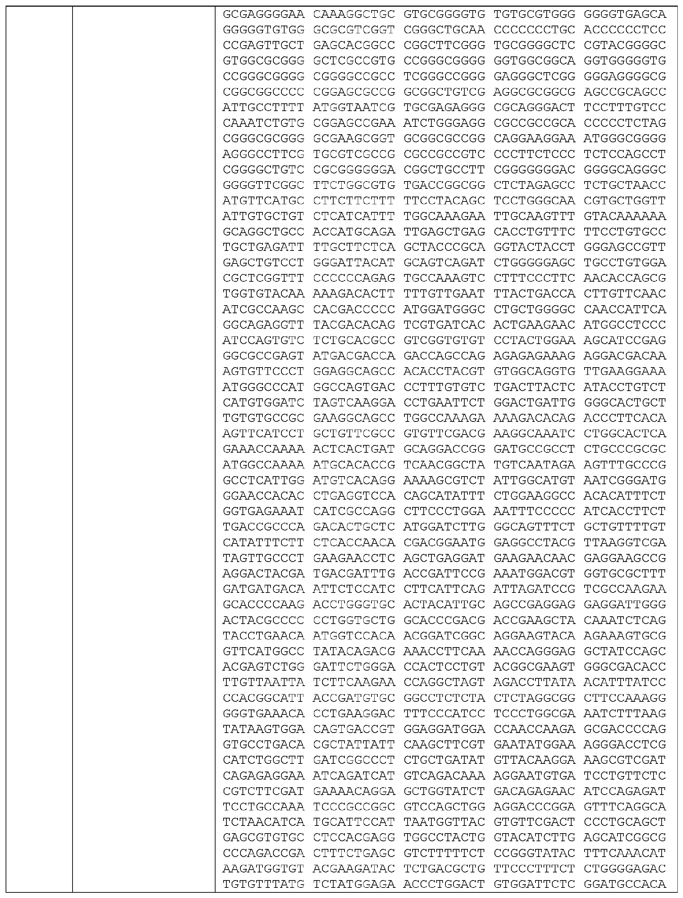

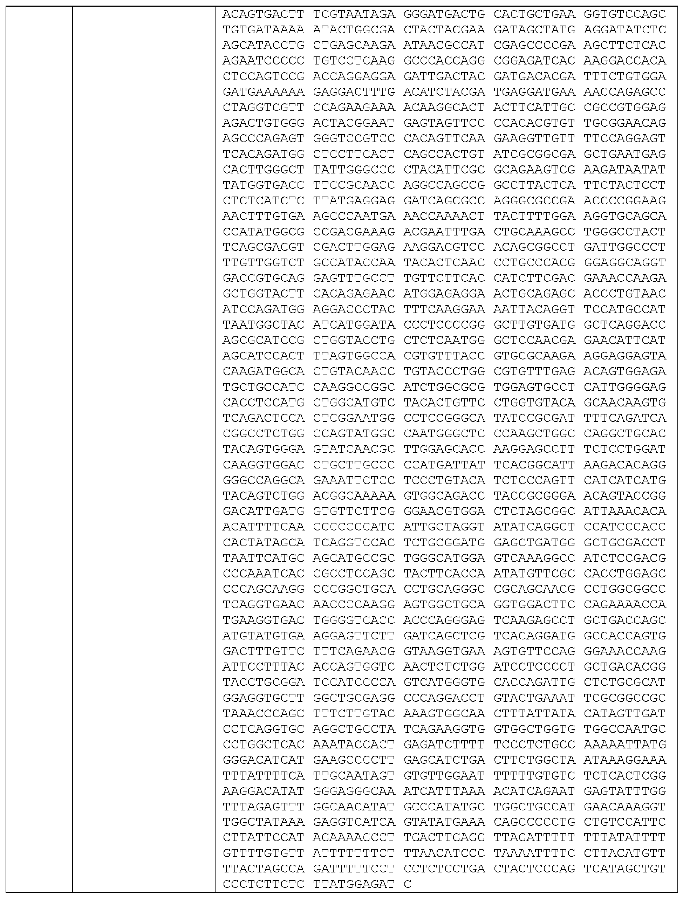

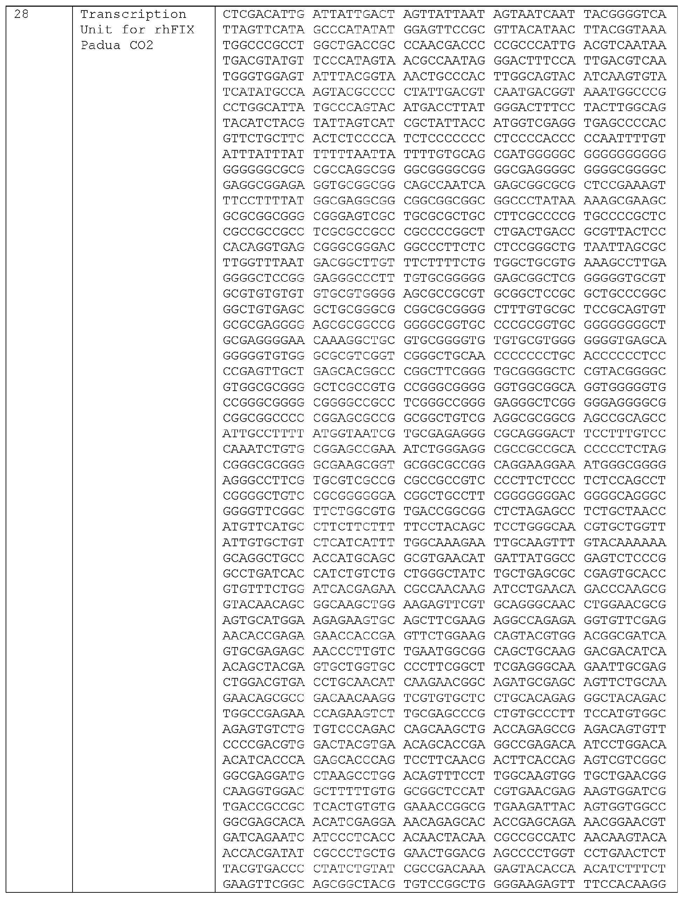

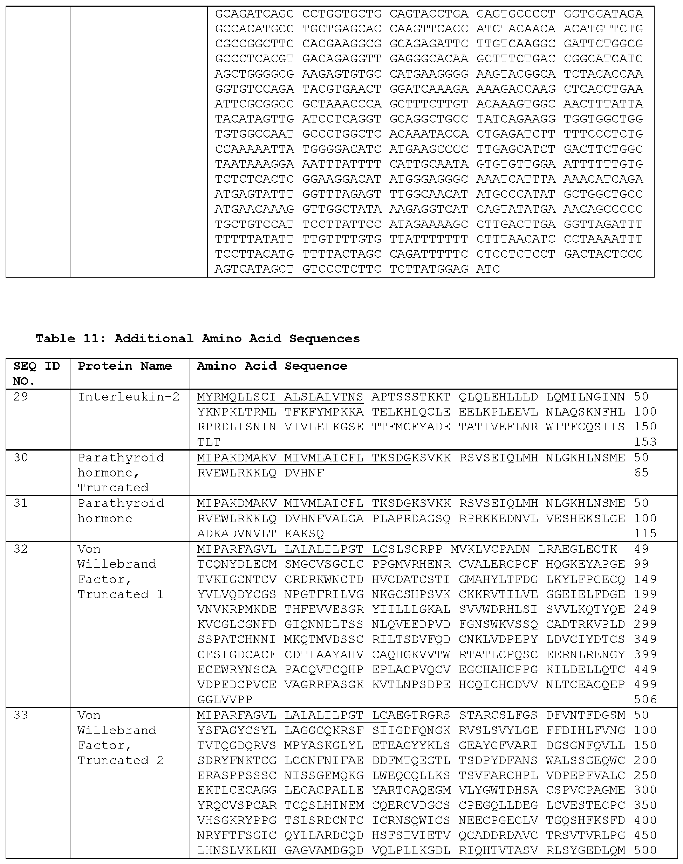

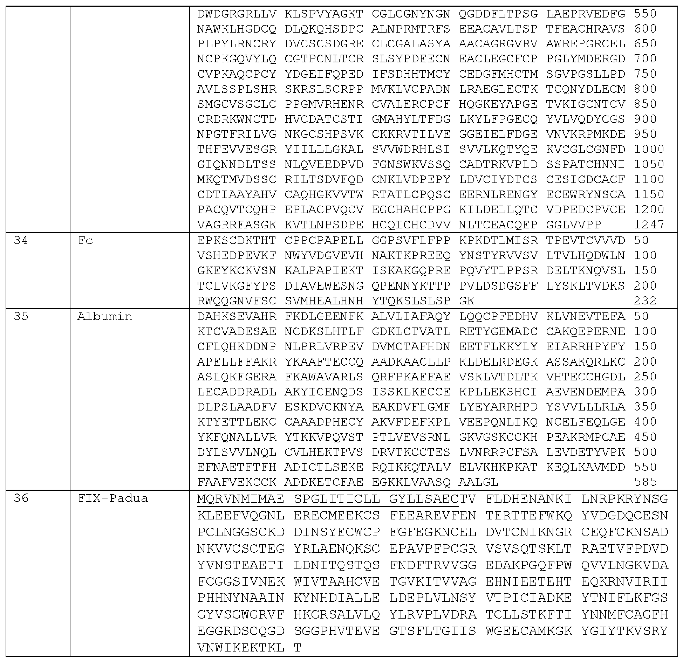

**FIG. 24** shows in Tables 7-11 exemplary amino acid sequences (SEQ ID NOs: 1, 3-7, 29-36) and coding sequences (SEQ ID NOs: 8-21) for therapeutic polypeptides and nucleotide sequences within an exemplary expression vector (SEQ ID NOs: 22-28) useful for engineering RPE cells.

### DETAILED DESCRIPTION

The present disclosure features a device comprising at least one compartment comprising a plurality of cells (e.g., live cells) encapsulated by a polymer composition. The encapsulating polymer composition comprises at least one cell-binding substance (CBS), e.g., a cell-binding peptide (CBP) or cell-binding polypeptide (CBPP). The cells are capable of expressing a therapeutic agent when the device is implanted into a subject (e.g., a human) and the device further comprises means for mitigating the foreign body response (FBR) when the device is implanted into the subject. In an embodiment, the means for mitigating the FBR comprises a compound of Formula (I) disposed on the surface of the device. The present disclosure also provides a preparation comprising a plurality of devices described herein and methods of making and using the same. In some embodiments, the devices described herein and preparations or compositions thereof are useful for the prevention or treatment of a disease, disorder, or condition which can be prevented or treatment by *in vivo* expression of the therapeutic agent. In some embodiments, the devices and preparations have advantageous properties, e.g., the encapsulated cells have higher *in vivo* productivity and/or viability than substantially the same cells implanted in substantially the same device without the CBS, e.g. a CBS-null reference device.

### Abbreviations and Definitions

Throughout the detailed description and examples of the disclosure the following abbreviations will be used.
- CBP: cell-binding peptide
- CBPP: cell-binding polypeptide
- CBP-polymer: polymer covalently modified with a CBP via a linker
- CBS: cell-binding substance
- CM-Alg: chemically modified alginate
- CM-LMW-Alg: chemically modified, low molecular weight alginate
- CM-LMW-Alg-101: low molecular weight alginate, chemically modified with Compound 101 shown in Table 4
- CM-HMW-Alg: chemically modified, high molecular weight alginate
- CM-HMW-Alg-101: high molecular weight alginate, chemically modified with Compound 101 shown in Table 4
- CM-MMW-Alg: chemically modified, medium molecular weight alginate
- CM-MMW-Alg-101: medium molecular weight alginate, chemically modified with Compound 101 shown in Table 4
- HMW-Alg: high molecular weight alginate
- MMW-Alg: medium molecular weight alginate
- RGD-alginate: an alginate covalently modified with a peptide comprising the amino acid sequence RGD (SEQ ID NO: 43).
- U-Alg: unmodified alginate
- U-HMW-Alg: unmodified high molecular weight alginate
- U-LMW-Alg: unmodified low molecular weight alginate
- U-MMW-Alg: unmodified medium molecular weight alginate
- 70:30 CM-Alg:U-Alg: 70:30 mixture (V:V) of a chemically modified alginate and an unmodified alginate, e.g., as described in Example 2

So that the disclosure may be more readily understood, certain technical and scientific terms used herein are specifically defined below. Unless specifically defined elsewhere in this document, all other technical and scientific terms used herein have the meaning commonly understood by one of ordinary skill in the art to which this disclosure belongs.

As used herein, including the appended claims, the singular forms of words such as "a," "an," and "the," include their corresponding plural references unless the context clearly dictates otherwise.

"About" or "approximately" means when used herein to modify a numerically defined parameter (e.g., a physical description of a hydrogel capsule such as diameter, sphericity, number of cells encapsulated therein, the number of capsules in a preparation), means that the recited numerical value is within an acceptable functional range for the defined parameter as determined by one of ordinary skill in the art, which will depend in part on how the numerical value is measured or determined, e.g., the limitations of the measurement system, including the acceptable error range for that measurement system. For example, "about" can mean a range of 20% above and below the recited numerical value. As a non-limiting example, a hydrogel capsule defined as having a diameter of about 1.5 millimeters (mm) and encapsulating about 5 million (M) cells may have a diameter of 1.2 to 1.8 mm and may encapsulate 4 M to 6 M cells. As another non-limiting example, a preparation of about 100 devices (e.g., hydrogel capsules) includes preparations having 80 to 120 devices. In some embodiments, the term "about" means that the modified parameter may vary by as much as 15%, 10% or 5% above and below the stated numerical value for that parameter. Alternatively, particularly with respect to certain properties of the devices described herein, such as cell productivity, or density of the CBP or the afibrotic compound, the term "about" can mean within an order of magnitude above and below the recited value, e.g., within 5-fold, 4-fold, 3-fold, 2-fold or 1-fold.

"Acquire" or "acquiring", as used herein, refer to obtaining possession of a value, e.g., a numerical value, or image, or a physical entity (e.g., a sample), by "directly acquiring" or "indirectly acquiring" the value or physical entity. "Directly acquiring" means performing a process (e.g., performing an analytical method or protocol) to obtain the value or physical entity. "Indirectly acquiring" refers to receiving the value or physical entity from another party or source (e.g., a third-party laboratory that directly acquired the physical entity or value). Directly acquiring a value or physical entity includes performing a process that includes a physical change in a physical substance or the use of a machine or device. Examples of directly acquiring a value include obtaining a sample from a human subject. Directly acquiring a value includes performing a process that uses a machine or device, e.g., using a fluorescence microscope to acquire fluorescence microscopy data.

"Administer", "administering", or "administration", as used herein, refer to implanting, absorbing, ingesting, injecting or otherwise introducing into a subject, an entity described herein (e.g., a device or a preparation of devices), or providing such an entity to a subject for administration.

"Afibrotic", as used herein, means a compound or material that mitigates the foreign body response (FBR). For example, the amount of FBR in a biological tissue that is induced by implant into that tissue of a device (e.g., hydrogel capsule) comprising an afibrotic compound (e.g., a hydrogel capsule comprising a polymer covalently modified with a compound listed in Table 4) is lower than the FBR induced by implantation of an afibrotic-null reference device, i.e., a device that lacks any afibrotic compound, but is of substantially the same composition (e.g., same CBP-polymer, same cell type(s)) and structure (e.g., size, shape, no. of compartments). In an embodiment, the degree of the FBR is assessed by the immunological response in the tissue containing the implanted device (e.g., hydrogel capsule), which may include, for example, protein adsorption, macrophages, multinucleated foreign body giant cells, fibroblasts, and angiogenesis, using assays known in the art, e.g., as described in WO 2017/075630, or using one or more of the assays / methods described Vegas, A., et al., Nature Biotechnol *(supra),* (e.g., subcutaneous cathepsin measurement of implanted capsules, Masson's trichrome (MT), hematoxylin or eosin staining of tissue sections, quantification of collagen density, cellular staining and confocal microscopy for macrophages (CD68 or F4/80), myofibroblasts (alpha-muscle actin, SMA) or general cellular deposition, quantification of 79 RNA sequences of known inflammation factors and immune cell markers, or FACS analysis for macrophage and neutrophil cells on retrieved devices (e.g., capsules) after 14 days in the intraperitoneal space of a suitable test subject, e.g., an immunocompetent mouse. In an embodiment, the FBR is assessed by measuring the levels in the tissue containing the implant of one or more biomarkers of immune response, e.g., cathepsin, TNF-α, IL-13, IL-6, G-CSF, GM-CSF, IL-4, CCL2, or CCL4. In some embodiments, the FBR induced by a device of the invention (e.g., a hydrogel capsule comprising an afibrotic compound disposed on its outer surface), is at least about 80%, about 85%, about 90%, about 95%, about 99%, or about 100% lower than the FBR induced by an FBR-null reference device, e.g., a device that is substantially identical to the test or claimed device except for lacking the means for mitigating the FBR (e.g., a hydrogel capsule that does not comprise an afibrotic compound but is otherwise substantially identical to the claimed capsule. In some embodiments, the FBR (e.g., level of a biomarker(s)) is measured after about 30 minutes, about 1 hour, about 6 hours, about 12 hours, about 1 day, about 2 days, about 3 days, about 4 days, about 1 week, about 2 weeks, about 1 month, about 2 months, about 3 months, about 6 months, or longer.

"Cell," as used herein, refers to an engineered cell or a cell that is not engineered. In an embodiment, a cell is an immortalized cell or an engineered cell derived from an immortalized cell. In an embodiment, the cell is a live cell, e.g., is viable as measured by any technique described herein or known in the art.

"Cell-binding peptide (CBP)", as used herein, means a linear or cyclic peptide that comprises an amino acid sequence that is derived from the cell binding domain of a ligand for a cell-adhesion molecule (CAM) (e.g., that mediates cell-matrix junctions or cell-cell junctions). The CBP is less than 50, 40 30, 25, 20, 15 or 10 amino acids in length. In an embodiment, the CBP is between 3 and 12 amino acids in length, 4 and 10 amino acids in length, or is 3, 4, 5, 6, 7 8, 9 or 10 amino acids in length. The CBP amino acid sequence may be identical to the naturally-occurring binding domain sequence or may be a conservatively substituted variant thereof. In an embodiment, the CAM ligand is a mammalian protein. In an embodiment, the CAM ligand is a human protein selected from the group of proteins listed in Table 1 below. In an embodiment, the CBP comprises a cell binding sequence listed in Table 1 below or a conservatively substituted variant thereof. In an embodiment, the CBP comprises at least one of the cell binding sequences listed in Table 1 below. In an embodiment, the CBP consists essentially of a cell binding sequence listed in Table 1 below. In an embodiment, the CBP is an RGD peptide, which means the peptide comprises the amino acid sequence RGD (SEQ ID NO: 43) and optionally comprises one or more additional amino acids located at one or both of the N-terminus and C-terminus. In an embodiment, the CBP is a cyclic peptide comprising RGD (SEQ ID NO: 43), e.g., one of the cyclic RGD peptides described in Vilaca, H. et al., Tetrahedron 70 (35):5420-5427 (2014). In an embodiment, the CBP is a linear peptide comprising RGD (SEQ ID NO: 43) and is less than 6 amino acids in length. In an embodiment, the CBP is a linear peptide that consists essentially of RGD (SEQ ID NO: 43) or RGDSP (SEQ ID NO: 59).

**Table 1: Exemplary CAM Ligand Proteins and Cell Binding Sequences**

| **Protein** | **Cell Binding Sequence** |
|---|---|
| E-cadherin | SWELYYPLRANL (SEQ ID NO: 37) |
| N-cadherin | HAVDI (SEQ ID NO:38) |
| Collagen I | DGEA (SEQ ID NO:39) |
| Collagen IV | FYFDLR (SEQ ID NO:40) |
| | GFOGER (SEQ ID NO:41) |
| | P(GPP)₅GFOGER(GPP)₅ (SEQ ID NO:54) |
| | (O in SEQ ID NO:41 and 54 is 4-hydroxyproline) |
| Elastin | VAPG (SEQ ID NO:42) |
| Fibrinogen | RGD (SEQ ID NO:43) |
| | GPR (SEQ ID NO:44) |
| Fibronectin | RGD (SEQ ID NO:43) |
| | KQAGDV (SEQ ID NO:45) |
| | PHSRN (SEQ ID NO:46) |
| | PHSRNGGGGGGRGDS (SEQ ID NO:55) |
| | REDV (SEQ ID NO:47) |
| Laminin | IKVAV (SEQ ID NO:48) |
| | SRARKQAASIKVAVADR (SEQ ID NO:56) |
| | LRE (SEQ ID NO:49) |
| | KQLREQ (SEQ ID NO:57) |
| | YIGSR (SEQ ID NO:50) |
| Nidogen-1 | RGD (SEQ ID NO: 43) |
| Osteopontin | SVVYGLR (SEQ ID NO:51) |
| Tenascin C (TN-C) | AEIDGIEL (SEQ ID NO:52) |
| Tenascin-R | RGD (SEQ ID NO:43) |
| Tenascin-X | RGD (SEQ ID NO: 43) |
| Thrombospondin | VTCG (SEQ ID NO:53) |
| | SVTCG (SEQ ID NO:58) |
| Vitronectin | RGD (SEQ ID NO:43) |
| Von Willebrand Factor | RGD (SEQ ID NO:43) |

"CBP-polymer", as used herein, means a polymer comprising at least one cell-binding peptide molecule covalently attached to the polymer via a linker. In an embodiment, the polymer is not a peptide or a polypeptide. In an embodiment, the polymer in a CBP-polymer does not contain any amino acids. In an embodiment, the polymer in a CBP-polymer is a synthetic or naturally occurring polysaccharide, e.g., an alginate, e.g., a sodium alginate. In an embodiment, the linker is an amino acid linker (i.e., consists essentially of a single amino acid, or a peptide of several identical or different amino acids), which is joined via a peptide bond to the N-terminus or C-terminus of the CBP. In an embodiment, the C-terminus of an amino acid linker is joined to the N-terminus of the CBP and the N-terminus of the amino acid linker is joined to at least one pendant carboxyl group in the polysaccharide via an amide bond. In an embodiment, the structure of the linker-CBP is expressed as G₍₁₋₄₎-CBP, meaning that the linker has one, two, three or four glycine residues ("G₍₁₋₄₎" is disclosed as SEQ ID NO: 70). In an embodiment, one or more of the monosaccharide moieties in a CBP-polysaccharide, e.g., a CBP-alginate) is not modified with the CBP, e.g, the unmodified moiety has a free carboxyl group or lacks a modifiable pendant carboxyl group. In an embodiment, the number of polysaccharide moieties with a covalently attached CBP is less than any of the following values: 99%, 95%, 90%, 80%, 70%, 60%, 50%, 40% 30%, 20%, 10%, 5%, 1%.

In an embodiment, the density of CBP modification in the CBP-polymer is estimated by combustion analysis for percent nitrogen, e.g., as described in the Examples below. In an embodiment, the CBP-polymer is an RGD-polymer (e.g., an RGD-alginate), which is a polymer (e.g., an alginate) covalently modified with a linker-RGD molecule (e.g., a peptide consisting essentially of GRGD (SEQ ID NO: 62) or GRGDSP (SEQ ID NO: 60)) and the density of linker-RGD molecule modification (e.g., conjugation density) is about 0.05 % nitrogen (N) to 1.00 % N, about 0.10 % N to about 0.75 % N, about 0.20 % N to about 0.50% N, or about 0.30 % N to about 0.40 % N, as determined using an assay described herein. In an embodiment, the conjugation density of the linker-RGD modification in an RGD-alginate (e.g., a MMW alginate covalently modified with GRGDSP (SEQ ID NO: 60)) is 0.1 to 1.0, 0.2 to 0.8, 0.3 to 0.7, 0.3 to 0.6, 0.4 to 0.6 micromoles of the linker-RGD moiety per g of the RGD-polymer in solution (e.g., saline solution) with a viscosity of 80-120cP, as determined by any assay that is capable of quantitating the amount of a peptide conjugated to a polymer, e.g., a quantitative peptide conjugation assay described herein. Unless otherwise explicitly stated or readily apparent from the context, a specifically recited numerical concentration, concentration range, density or density range for a CBP in a CBP-polymer refers to the concentration or density of conjugated CBP molecules, i.e., it does not include any residual free (e.g., unconjugated) CBP that may be present in the CBP-polymer.

"Cell-binding polypeptide (CBPP)", as used herein, means a polypeptide of at least 50, at least 75, or at least 100 amino acids in length and comprising the amino acid sequence of a cell binding domain of a CAM ligand, or a conservatively substituted variant thereof. In an embodiment, the CAM ligand is a mammalian protein. In an embodiment, the CBPP amino acid comprises the naturally occurring amino acid sequence of a full-length CAM ligand, e.g., one of the proteins listed in Table 1, or a conservatively substituted variant thereof.

"CBP-density", as used herein, refers to the amount or concentration of a linker-CBP moiety in a CBP-polymer, e.g., an alginate modified with G₁₋₃RGD (SEQ ID NO: 63) or G₁₋₃RGDSP (SEQ ID NO: 64), unless otherwise explicitly stated herein.

"Cell-binding substance (CBS)", as used herein, means any chemical, biological or other type of substance (e.g., a small organic compound, a peptide, a polypeptide) that is capable of mimicking at least one activity of a ligand for a cell-adhesion molecule (CAM) or other cell-surface molecule that mediates cell-matrix junctions or cell-cell junctions or other receptor-mediated signaling. In an embodiment, when present in a polymer composition encapsulating cells, the CBS is capable of forming a transient or permanent bond or contact with one or more of the cells. In an embodiment, the CBS facilitates interactions between two or more live cells encapsulated in the polymer composition. In an embodiment, the presence of a CBS in a polymer composition encapsulating a plurality of cells, (e.g., live cells) is correlated with one or both of increased cell productivity (e.g., expression of a therapeutic agent) and increased cell viability when the encapsulated cells are implanted into a test subject, e.g., a mouse. In an embodiment, the CBS is physically attached to one or more polymer molecules in the polymer composition. In an embodiment, the CBS is a cell-binding peptide or cell-binding polypeptide, as defined herein.

"Conservatively modified variants" or conservative substitution", as used herein, refers to a variant of a reference peptide or polypeptide that is identical to the reference molecule, except for having one or more conservative amino acid substitutions in its amino acid sequence. In an embodiment, a conservatively modified variant consists of an amino acid sequence that is at least 70%, 80%, 85%, 90%, 95%, 97%, 98% or 99% identical to the reference amino acid sequence. A conservative amino acid substitution refers to substitution of an amino acid with an amino acid having similar characteristics (e.g., charge, side-chain size, hydrophobicity/hydrophilicity, backbone conformation and rigidity, etc.) and which has minimal impact on the biological activity of the resulting substituted peptide or polypeptide. Conservative substitution tables of functionally similar amino acids are well known in the art, and exemplary substitutions grouped by functional features are set forth in Table 2 below.

**Table 2. Exemplary conservative amino acid substitution groups.**

| Feature | Conservative Amino Group |
|---|---|
| Charge/Polarity | His, Arg, Lys |
| | Asp, Glu |
| | Cys, Thr, Ser, Gly, Asn, Gln, Tyr |
| | Ala, Pro, Met, Leu, Ile, Val, Phe, Trp |
| Hydrophobicity | Asp, Glu, Asn, Gln, Arg, Lys |
| | Cys, Ser, Thr, Pro, Gly, His, Tyr |
| | Ala, Met, Ile Leu, Val, Phe, Trp |
| Structural/Surface Exposure | Asp, Glu, Asn, Aln, His, Arg, Lys |
| | Cys, Ser, Tyr, Pro, Ala, Gly, Trp, Tyr |
| | Met, Ile, Leu, Val, Phe |
| Secondary Structure Propensity | Ala, Glu, Aln, His, Lys, Met, Leu, Arg |
| | Cys, Thr, Ile, Val, Phe, Tyr, Trp |
| | Ser, Gly, Pro, Asp, Asn |
| Evolutionary Conservation | Asp, Glu |
| | His, Lys, Arg |
| | Asn, Gln |
| | Ser, Thr |
| | Leu, Ile, Val |
| | Phe, Tyr, Trp |
| | Ala, Gly |
| | Met, Cys |

"Consists essentially of", and variations such as "consist essentially of" or "consisting essentially of" as used throughout the specification and claims, indicate the inclusion of any recited elements or group of elements, and the optional inclusion of other elements, of similar or different nature than the recited elements, that do not materially change the basic or novel properties of the specified molecule, composition, device, or method. As a non-limiting example, a cell-binding peptide or a therapeutic protein that consists essentially of a recited amino acid sequence may also include one or more amino acids, including substitutions in the recited amino acid sequence, of one or more amino acid residues, which do not materially affect the relevant biological activity of the cell-binding peptide or the therapeutic protein, respectively. As another non-limiting example, a cell-binding peptide that consists essentially of a recited amino acid sequence may contain one or more covalently attached moieties (e.g., a radioactive or fluorescent label) that do not materially change the relevant biological activity of the cell-binding peptide, e.g., its ability to increase the viability or productivity of encapsulated cells as described herein.

"Derived from", as used herein with respect to cells, refers to cells obtained from tissue, cell lines, or cells, which optionally are then cultured, passaged, differentiated, induced, etc. to produce the derived cells. For example, mesenchymal stem cells can be derived from mesenchymal tissue and then differentiated into a variety of cell types.

"Device", as used herein, refers to any implantable object (e.g., a particle, a hydrogel capsule, an implant, a medical device), which contains cells (e.g., live cells) capable of expressing a therapeutic agent following implant of the device, and has a configuration that supports the viability of the cells by allowing cell nutrients to enter the device. In some embodiments, the device allows release from the device of metabolic byproducts and / or the therapeutic agent generated by the live cells.

"Differential volume," as used herein, refers to a volume of one compartment within a device described herein that excludes the space occupied by another compartment(s). For example, the differential volume of the second (e.g., outer) compartment in a 2-compartment device with inner and outer compartments, refers to a volume within the second compartment that excludes space occupied by the first (inner) compartment.

"Effective amount", as used herein, refers to an amount of a device, a device composition, or a component of the device or device composition, e.g, a plurality of hydrogel capsules comprising a cell, e.g., an engineered cell, or an agent, e.g., a therapeutic agent, produced by a cell, e.g., an engineered RPE cell, sufficient to elicit a biological response, e.g., to treat a disease, disorder, or condition. In some embodiments, the term "effective amount" refers to the amount of a component of the device, e.g., number of cells in the device, the density of an afibrotic compound disposed on the surface and/or in a barrier compartment of the device, the density of a CBS in the cell-containing compartment. As will be appreciated by those of ordinary skill in this art, the effective amount may vary depending on such factors as the desired biological endpoint, the pharmacokinetics of the therapeutic agent, composition or device (e.g., capsule, particle), the condition being treated, the mode of administration, and the age and health of the subject. An effective amount encompasses therapeutic and prophylactic treatment. For example, to mitigate the FBR, an effective amount of a compound of Formula (I) may reduce the fibrosis or stop the growth or spread of fibrotic tissue on or near the implanted device. As another example, an effective amount of a CBS may enhance the viability of the cells in the device (e.g., number of live cells) and/or increase the level of a therapeutic agent expressed and/or secreted by the cells. An effective amount of a device, composition or component, e.g., afibrotic compound or CBS, may be determined by any technique known in the art or described herein.

An "endogenous nucleic acid" as used herein, is a nucleic acid that occurs naturally in a subject cell.

An "endogenous polypeptide," as used herein, is a polypeptide that occurs naturally in a subject cell.

"Engineered cell," as used herein, is a cell (e.g., an RPE cell) having a non-naturally occurring alteration, and typically comprises a nucleic acid sequence (e.g., DNA or RNA) or a polypeptide not present (or present at a different level than) in an otherwise similar cell under similar conditions that is not engineered (an exogenous nucleic acid sequence). In an embodiment, an engineered cell comprises an exogenous nucleic acid (e.g., a vector or an altered chromosomal sequence). In an embodiment, an engineered cell comprises an exogenous polypeptide. In an embodiment, an engineered cell comprises an exogenous nucleic acid sequence, e.g., a sequence, e.g., DNA or RNA, not present in a similar cell that is not engineered. In an embodiment, the exogenous nucleic acid sequence is chromosomal, e.g., the exogenous nucleic acid sequence is an exogenous sequence disposed in endogenous chromosomal sequence. In an embodiment, the exogenous nucleic acid sequence is chromosomal or extra chromosomal, e.g., a non-integrated vector. In an embodiment, the exogenous nucleic acid sequence comprises an RNA sequence, e.g., an mRNA. In an embodiment, the exogenous nucleic acid sequence comprises a chromosomal or extra-chromosomal exogenous nucleic acid sequence that comprises a sequence which is expressed as RNA, e.g., mRNA or a regulatory RNA. In an embodiment, the exogenous nucleic acid sequence comprises a chromosomal or extra-chromosomal nucleic acid sequence, which comprises a sequence that encodes a polypeptide, or which is expressed as a polypeptide. In an embodiment, the exogenous nucleic acid sequence comprises a first chromosomal or extra-chromosomal exogenous nucleic acid sequence that modulates the conformation or expression of a second nucleic acid sequence, wherein the second amino acid sequence can be exogenous or endogenous. For example, an engineered cell can comprise an exogenous nucleic acid that controls the expression of an endogenous sequence. In an embodiment, an engineered cell comprises a polypeptide present at a level or distribution which differs from the level found in a similar cell that has not been engineered. In an embodiment, an engineered cell comprises an RPE engineered to produce an RNA or a polypeptide. For example, an engineered cell may comprise an exogenous nucleic acid sequence comprising a chromosomal or extra-chromosomal exogenous nucleic acid sequence that comprises a sequence which is expressed as RNA, e.g., mRNA or a regulatory RNA. In an embodiment, an engineered cell (e.g., an RPE cell) comprises an exogenous nucleic acid sequence that comprises a chromosomal or extra-chromosomal nucleic acid sequence comprising a sequence that encodes a polypeptide, or which is expressed as a polypeptide. In an embodiment, the polypeptide is encoded by a codon optimized sequence to achieve higher expression of the polypeptide than a naturally-occurring coding sequence. The codon optimized sequence may be generated using a commercially available algorithm, e.g., GeneOptimizer (ThermoFisher Scientific), OptimumGene^{™} (GenScript, Piscataway, NJ USA), GeneGPS^{®} (ATUM, Newark, CA USA), or Java Codon Adaptation Tool (JCat, www.jcat.de, Grote, A. et al., Nucleic Acids Research, Vol 33, Issue suppl _2, pp. W526-W531 (2005). In an embodiment, an engineered cell (e.g., an RPE cell) comprises an exogenous nucleic acid sequence that modulates the conformation or expression of an endogenous sequence. In an embodiment, an engineered cell (e.g., RPE cell) is cultured from a population of stably-transfected cells, or from a monoclonal cell line.

An "exogenous nucleic acid," as used herein, is a nucleic acid that does not occur naturally in a subject cell.

An "exogenous polypeptide," as used herein, is a polypeptide that does not occur naturally in a subject cell, e.g., engineered cell. Reference to an amino acid position of a specific sequence means the position of said amino acid in a reference amino acid sequence, e.g., sequence of a full-length mature (after signal peptide cleavage) wild-type protein (unless otherwise stated), and does not exclude the presence of variations, e.g., deletions, insertions and/or substitutions at other positions in the reference amino acid sequence.

"Factor VII protein" or "FVII protein" as used herein, means a polypeptide that comprises the amino acid sequence of a naturally occurring factor VII protein or variant thereof that has a FVII biological activity, e.g., promoting blood clotting, as determined by an art-recognized assay, unless otherwise specified. Naturally occurring FVII exists as a single chain zymogen, a zymogen-like two-chain polypeptide and a fully activated two-chain form (FVIIa). In some embodiments, reference to FVII includes single-chain and two-chain forms thereof, including zymogen-like and FVIIa. FVII proteins that may be produced by a device described herein, e.g., a device containing engineered RPE cells, include wild-type primate (e.g., human), porcine, canine, and murine proteins, as well as variants of such wild-type proteins, including fragments, mutants, variants with one or more amino acid substitutions and / or deletions. In some embodiments, a variant FVII protein is capable of being activated to the fully activated two-chain form (Factor VIIa) that has at least 50%, 75%, 90% or more (including >100%) of the activity of wild-type Factor VIIa. Variants of FVII and FVIIa are known, e.g., marzeptacog alfa (activated) (MarzAA) and the variants described in European Patent No. 1373493, US Patent No. 7771996, US Patent No. 9476037 and US published application No. US20080058255.

Factor VII biological activity may be quantified by an art recognized assay, unless otherwise specified. For example, FVII biological activity in a sample of a biological fluid, e.g., plasma, may be quantified by (i) measuring the amount of Factor Xa produced in a system comprising tissue factor (TF) embedded in a lipid membrane and Factor X (Persson et al., J. Biol. Chem. 272:19919-19924, 1997); (ii) measuring Factor X hydrolysis in an aqueous system; (iii) measuring its physical binding to TF using an instrument based on surface plasmon resonance (Persson, FEBS Letts. 413:359-363, 1997); or (iv) measuring hydrolysis of a synthetic substrate; and/or (v) measuring generation of thrombin in a TF-independent in vitro system. In an embodiment, FVII activity is assessed by a commercially available chromogenic assay (BIOPHEN FVII, HYPHEN BioMed Neuville sur Oise, France), in which the biological sample containing FVII is mixed with thromboplastin calcium, Factor X and SXa-11 (a chromogenic substrate specific for Factor Xa.

"Factor VIII protein" or "FVIII protein" as used herein, means a polypeptide that comprises the amino acid sequence of a naturally occurring factor VIII polypeptide or variant thereof that has an FVIII biological activity, e.g., coagulation activity, as determined by an art-recognized assay, unless otherwise specified. FVIII proteins that may be expressed by a device described herein, e.g., a device containing engineered RPE cells, include wild-type primate (e.g., human), porcine, canine, and murine proteins, as well as variants of such wild-type proteins, including fragments, mutants, variants with one or more amino acid substitutions and / or deletions, B-domain deletion (BDD) variants, single chain variants and fusions of any of the foregoing wild-type or variants with a half-life extending polypeptide. In an embodiment, the cells are engineered to encode a precursor factor VIII polypeptide (e.g., with the signal sequence) with a full or partial deletion of the B domain. In an embodiment, the cells are engineered to encode a single chain factor VIII polypeptide which contains a variant FVIII protein preferably has at least 50%, 75%, 90% or more (including >100%) of the coagulation activity of the corresponding wild-type factor VIII. Assays for measuring the coagulation activity of FVIII proteins include the one stage or two stage coagulation assay (Rizza et al., 1982, Coagulation assay of FVIII:C and FIXa in Bloom ed. The Hemophelias. NY Churchill Livingston 1992) or the chromogenic substrate FVIII: C assay (Rosen, S. 1984. Scand J Haematol 33:139-145, suppl.).

A number of FVIII-BDD variants are known, and include, e.g., variants with the full or partial B-domain deletions disclosed in any of the following U.S. Patent Nos: 4,868,112 (e.g., col. 2, line 2 to col. 19, line 21 and table 2); 5,112,950 (e.g., col. 2, lines 55-68, FIG. 2, and example 1); 5,171,844 (e.g., col. 4, line 22 to col. 5, line 36); 5,543,502 (e.g., col. 2, lines 17-46); 5,595,886; 5,610,278; 5,789,203 (e.g., col. 2, lines 26-51 and examples 5-8); 5,972,885 (e.g., col. 1, lines 25 to col. 2, line 40); 6,048,720 (e.g., col. 6, lines 1-22 and example 1); 6,060,447; 6,228,620; 6,316,226 (e.g., col. 4, line 4 to col. 5, line 28 and examples 1-5); 6,346,513; 6,458,563 (e.g., col. 4, lines 25-53) and 7,041,635 (e.g., col. 2, line 1 to col. 3, line 19, col. 3, line 40 to col. 4, line 67, col. 7, line 43 to col. 8, line 26, and col. 11, line 5 to col. 13, line 39).

In some embodiments, a FVIII-BDD protein produced by a device described herein (e.g., expressed by engineered cells contained in the device) has one or more of the following deletions of amino acids in the B-domain: (i) most of the B domain except for amino-terminal B-domain sequences essential for intracellular processing of the primary translation product into two polypeptide chains (WO 91/09122); (ii) a deletion of amino acids 747-1638 (Hoeben R. C., et al. J. Biol. Chem. 265 (13): 7318-7323 (1990)); amino acids 771-1666 or amino acids 868-1562 (Meulien P., et al. Protein Eng. 2(4):301-6 (1988); amino acids 982-1562 or 760-1639 (Toole et al., Proc. Natl. Acad. Sci. U.S.A. 83:5939-5942 (1986)); amino acids 797-1562 (Eaton et al., Biochemistry 25:8343-8347 (1986)); 741-1646 (Kaufman, WO 87/04187)), 747-1560 (Sarver et al., DNA 6:553-564 (1987)); amino acids 741-1648 (Pasek, WO 88/00831)), amino acids 816-1598 or 741-1689 (Lagner (Behring Inst. Mitt. (1988) No 82:16-25, EP 295597); a deletion that includes one or more residues in a furin protease recognition sequence, e.g., LKRHQR (SEQ ID NO: 65) at amino acids 1643-1648, including any of the specific deletions recited in US Patent No. 9,956,269 at col. 10, line 65 to col. 11, line 36.

In other embodiments, a FVIII-BDD protein retains any of the following B-domain amino acids or amino acid sequences: (i) one or more N-linked glycosylation sites in the B-domain, e.g., residues 757, 784, 828, 900, 963, or optionally 943, first 226 amino acids or first 163 amino acids (Miao, H. Z., et al., Blood 103(a): 3412-3419 (2004), Kasuda, A., et al., J. Thromb. Haemost. 6: 1352-1359 (2008), and Pipe, S. W., et al., J. Thromb. Haemost. 9: 2235-2242 (2011).

In some embodiments, the FVIII-BDD protein is a single-chain variant generated by substitution of one or more amino acids in the furin protease recognition sequence (LKRHQR (SEQ ID NO: 65) at amino acids 1643-1648) that prevents proteolytic cleavage at this site, including any of the substitutions at the R1645 and/or R1648 positions described in U.S. Patent Nos. 10,023,628, 9,394,353 and 9,670,267.

In some embodiments, any of the above FVIII-BDD proteins may further comprise one or more of the following variations: a F309S substitution to improve expression of the FVIII-BDD protein (Miao, H. Z., et al., Blood 103(a): 3412-3419 (2004); albumin fusions (WO 2011/020866); and Fc fusions (WO 04/101740).

All FVIII-BDD amino acid positions referenced herein refer to the positions in full-length human FVIII, unless otherwise specified.

"Factor IX protein" or "FIX protein", as used herein, means a polypeptide that comprises the amino acid sequence of a naturally occurring factor IX protein or variant thereof that has a FIX biological activity, e.g., coagulation activity, as determined by an art-recognized assay, unless otherwise specified. FIX is produced as an inactive zymogen, which is converted to an active form by factor XIa excision of the activation peptide to produce a heavy chain and a light chain held together by one or more disulfide bonds. FIX proteins that may be produced by devices described herein (e.g., a device containing engineered RPE cells) include wild-type primate (e.g., human), porcine, canine, and murine proteins, as well as variants of such wild-type proteins, including fragments, mutants, variants with one or more amino acid substitutions and / or deletions and fusions of any of the foregoing wild-type or variant proteins with a half-life extending polypeptide. In an embodiment, cells are engineered to encode a full-length wild-type human factor IX polypeptide (e.g., with the signal sequence) or a functional variant thereof. A variant FIX protein preferably has at least 50%, 75%, 90% or more (including >100%) of the coagulation activity of wild-type factor VIX. Assays for measuring the coagulation activity of FIX proteins include the Biophen Factor IX assay (Hyphen BioMed) and the one stage clotting assay (activated partial thromboplastin time (aPTT), e.g., as described in EP 2 032 607, thrombin generation time assay (TGA) and rotational thromboelastometry, e.g., as described in WO 2012/006624.

A number of functional FIX variants are known and may be expressed by engineered cells encapsulated in a device described herein, including any of the functional FIX variants described in the following international patent publications: WO 02/040544 at page 4, lines 9-30 and page 15, lines 6-31; WO 03/020764 in Tables 2 and 3 at pages 14-24, and at page 12, lines 1-27; WO 2007/149406 at page 4, line 1 to page 19, line 11; WO 2007/149406 A2 at page 19, line 12 to page 20, line 9; WO 08/118507 at page 5, line 14 to page 6, line 5; WO 09/051717 at page 9, line 11 to page 20, line 2; WO 09/137254 at page 2, paragraph [006] to page 5, paragraph [011] and page 16, paragraph [044] to page 24, paragraph [057]; WO 09/130198 A2 at page 4, line 26 to page 12, line 6; WO 09/140015 at page 11, paragraph [0043] to page 13, paragraph [0053]; WO 2012/006624; WO 2015/086406.

In certain embodiments, the FIX polypeptide comprises a wild-type or variant sequence fused to a heterologous polypeptide or non-polypeptide moiety extending the half-life of the FIX protein. Exemplary half-life extending moieties include Fc, albumin, a PAS sequence, transferrin, CTP (28 amino acid C-terminal peptide (CTP) of human chorionic gonadotropin (hCG) with its 4 O-glycans), polyethylene glycol (PEG), hydroxyethyl starch (HES), albumin binding polypeptide, albumin-binding small molecules, or any combination thereof. An exemplary FIX polypeptide is the rFIXFc protein described in WO 2012/006624, which is an FIXFc single chain (FIXFc-sc) and an Fc single chain (Fc-sc) bound together through two disulfide bonds in the hinge region of Fc.

FIX variants also include gain and loss of function variants. An example of a gain of function variant is the "Padua" variant of human FIX, which has a L (leucine) at position 338 of the mature protein instead of an R (arginine) (corresponding to amino acid position 384 of SEQ ID NO:2), and has greater catalytic and coagulant activity compared to wild-type human FIX (Chang et al., J. Biol. Chem., 273:12089-94 (1998)). An example of a loss of function variant is an alanine substituted for lysine in the fifth amino acid position from the beginning of the mature protein, which results in a protein with reduced binding to collagen IV (e.g., loss of function).

"Interleukin-2 protein" or "IL-2 protein", as used herein means a polypeptide comprising the amino acid sequence of a naturally-occurring IL-2 protein or variant thereof that has an IL-2 biological activity, e.g., activate IL-2 receptor signaling in Treg cells, as determined by an art-recognized assay, unless otherwise specified. IL-2 proteins that may be produced by a device described herein, e.g., a device containing engineered RPE cells, include wild-type primate (e.g., human), porcine, canine, and murine proteins, as well as variants of such wild-type proteins. A variant IL-2 protein preferably has at least 50%, 75%, 90% or more (including >100%) of the biological activity of the corresponding wild-type IL-2. Biological activity assays for IL-2 proteins are described in US Patent No. 10,035,836, and include, e.g., measuring the levels of phosphorylated STAT5 protein in Treg cells compared to CD4+CD25-/low T cells or NK cells. Variant IL-2 proteins that may be produced by a device of the present disclosure (e.g., a device containing engineered RPE cells) include proteins with one or more of the following amino acid substitutions: N88R, N88I, N88G, D20H, Q126L, Q126F, and C125S or C125A.

"Islet cell" as used herein means a cell that comprises any naturally occurring or any synthetically created, or modified, cell that is intended to recapitulate, mimic or otherwise express, in part or in whole, the functions, in part or in whole, of the cells of the pancreatic islets of Langerhans. The term "islet cells" includes glucose-responsive, insulin producing cells derived from stem cells, e.g., from an induced pluripotent stem cell line.

"Mannitol", as used herein, refers to D-mannitol unless otherwise explicitly stated.

"Medium molecular weight alginate," or "MMW-Alg" as used herein means an alginate with an approximate molecular weight of 75 kDa to 150 kDa.

"Mesenchymal stem function cell" or "MSFC," as those terms are used herein, refers to a cell derived from, or having at least one characteristic specific to a cell of, mesodermal lineage, and wherein the MSFC is i) not in a terminal state of differentiation and ii) can terminally differentiate into one or more cell types. An MSFC does not comprise a cell of endodermal origin, e.g., a gut cell, or of ectodermal origin, e.g., a cell derived from skin, CNS, or a neural cell. In an embodiment, the MSFC is multipotent. In an embodiment, the MSFC is not totipotent. In an embodiment, an MSFC comprises one or more of the following characteristics:
a) it comprises a mesenchymal stem cell (MSC) or a cell derived therefrom, including a cell derived from a primary cell culture of MSCs, a cell isolated directly (without long term culturing, e.g., less than 5 or 10 passages or rounds of cell division since isolation) from naturally occurring MSCs, e.g., from a human or other mammal, a cell derived from a transformed, a pluripotent, an immortalized, or a long term (e.g., more than 5 or 10 passages or rounds of cell division) MSC culture.
b) it comprises a cell that has been obtained from a less differentiated cell, e.g., a cell developed, programmed, or reprogramed (e.g., in vitro) into an MSC or a cell that is, except for any genetic engineering, substantially similar to one or more of a naturally occurring MSC or a cell from a primary or long term culture of MSCs, or a cell described in a) above. Examples of less differentiated cells from which MSFC can be derived include IPS cells, embryonic stem cells, or other totipotent or pluripotent cells; see, e.g., Chen, Y.S. et al (2012) Stem Cells Transl Med 1(83-95); Frobel, J et al (2014) Stem Cell Reports 3(3):414-422; Zou, L et al (2013) Sci Rep 3:2243;
c) it is multipotent, e.g., as measured by any assay capable of providing information about cell multipotency, e.g., microscopy;
d) it exhibits a characteristic mononuclear ovoid, stellate shape or spindle shape, with a round to oval nucleus. The oval elongate nucleus may have prominent nucleoli and a mix of heterochromatin and euchromatin. An MSFC (e.g., an MSC) may have little cytoplasm, but many thin processes that appear to extend from the nucleus;
e) it is capable of cell division, e.g., as measured any assay capable of providing information about cell division, e.g., microscopy. In an embodiment, an MSFC is capable of cell division in culture (e.g., prior to being encapsulated or incorporated into a device). In an embodiment, it is capable of cell division after being encapsulated, e.g., encapsulated as described herein, or incorporated into a device (e.g., a device described herein). In an embodiment, it is incapable of cell division after reaching confluence;
f) it is capable of differentiating into a mesenchymal cell lineage, e.g., an osteoblast, a chrondoblast, an adipocyte, or a fibroblast;
g) it expresses a mesenchymal cell marker, e.g., one, two, three, four, five or all of CD105, CD106, CD73, CD90, Stro-1, CD49a, CD29, CD44, CD146, CD166, TNAP+, THY-1+, Stro-2, Stro-4, and alkaline phosphatase;
h) it does not express significant levels of one, two, three, or any of CD34, CD31, VE-cadherin, CD45, HLA-DR, CD11b and a glycophorin or leukocyte differentiation antigen, e,g, CD14, CD33, CD3 and CD19;
i) it expresses one, two, or all of CD75, CD90, and CD105 and does not express one, two, or any of CD45, CD34, and CD14;
j) it is anti-inflammatory or immune dampening, e.g., as measured by any method capable of providing information regarding inflammation, e.g., in vivo inhibition of T cell proliferation;
k) it is capable of being adherent, e.g., plastic adherent, e.g., as determined by, e.g., visual inspection; or
l) can grow in three dimensions, e.g., as determined by, e.g., visual inspection.

"Parathyroid hormone" or "PTH" as used herein means a polypeptide or peptide that comprises the amino acid sequence of a naturally occurring parathyroid hormone polypeptide or peptide or variant thereof that has a PTH biological activity, e.g., as determined by an art recognized assay. PTH polypeptides and peptides that may be expressed by encapsulated cells described herein include wild-type primate (e.g., human), porcine, canine, and murine proteins, as well as variants of such wild-type proteins. Such PTH polypeptides and peptides may consist essentially of the wild-type human sequence for pre-pro-PTH polypeptide (115 amino acids), pro-PTH polypeptide (90 amino acids), the mature 84-amino acid peptide (PTH(1-84)), and biologically active variants thereof, such as the truncated variant peptide PTH(1-34). PTH peptide variants with one or more amino acid substitutions in the human wild-type sequence have been described, e.g., in US Patent Nos. 7410948 and 8563513 and in US Patent Application Publication No. 20130217630. A PTH variant preferably has at least 50%, 75%, 90% or more (including >100%) of a biological activity of the corresponding wild-type PTH. An assay to detect certain PTH variants by tandem mass spectrometry is described in US Patent No. 8383417. A biological activity assay for PTH peptide variants - stimulation of adenylate cyclase as determined by measuring cAMP levels - is described in US Patent No. 7410948.

"Poloxamer", as used herein, refers to the standard generic term for a class of nonionic triblock linear copolymers composed of a central hydrophobic chain of polyoxypropylene (poly(propylene oxide)) flanked by two polyoxyethylene (poly(ethylene oxide)) moieties.

"Poloxamer 188" or "P 188", as used herein, refers to a poloxamer with an approximate molecular mass of 1800 g/mole for the polyoxypropylene core and an oxyethylene content of about 80% weight percent, e.g., 79.0 to 83.7 percent. In an embodiment, poloxamer 188 has an average molecular weight of 8350 g/mole. In an embodiment, poloxamer 188 has an average molecular weight of 7680 g/mole to 9510 g/mole, e.g., as determined by size exclusion chromatography, and an oxyethylene content of 81.8 ± 1.9% weight percent. In an embodiment, each polyoxyethylene chain in poloxamer 188 has 75-85 (e.g., 80) ethylene oxide monomers and the polyoxypropylene core has 25-30 (e.g., 27) propylene oxide monomers. In an embodiment, poloxamer 188 used in a process described herein substantially meets the specifications set forth in a poloxamer monograph published by the United States Pharmacopeia-National Formulary (USP-NF) or the European Pharmacopoeia (Ph. Eur.) that is official at the time the process is performed.

"Polymer composition", as used herein, is a composition (e.g., a solution, mixture) comprising one or more polymers. As a class, "polymers' includes homopolymers, heteropolymers, co-polymers, block polymers, block co-polymers and can be both natural and synthetic. Homopolymers contain one type of building block, or monomer, whereas co-polymers contain more than one type of monomer.

"Polypeptide", as used herein, refers to a polymer comprising amino acid residues linked through peptide bonds and having at least two, and in some embodiments, at least 10, 50, 75, 100, 150 or 200 amino acid residues.

"Prevention," "prevent," and "preventing" as used herein refers to a treatment that comprises administering or applying a therapy, e.g., administering a composition of devices encapsulating cells (e.g., as described herein), prior to the onset of a disease, disorder, or condition to preclude the physical manifestation of said disease, disorder, or condition. In some embodiments, "prevention," "prevent," and "preventing" require that signs or symptoms of the disease, disorder, or condition have not yet developed or have not yet been observed. In some embodiments, treatment comprises prevention and in other embodiments it does not.

A "replacement therapy" or "replacement protein" is a therapeutic protein or functional fragment thereof that replaces or augments a beneficial function of a protein that is diminished, present in insufficient quantity, altered (e.g., mutated) or lacking in a subject having a disease or condition related to the diminished, altered or lacking protein. Examples are certain blood clotting factors in certain blood clotting disorders or certain lysosomal enzymes in certain lysosomal storage diseases. In an embodiment, a replacement therapy or replacement protein provides the function of an endogenous protein. In an embodiment, a replacement therapy or replacement protein has the same amino acid sequence of a naturally occurring variant of the replaced protein, e.g., a wild type allele or an allele not associated with a disorder. In an embodiment, or replacement therapy or a replacement protein differs in amino acid sequence from a naturally occurring variant, e.g., a wild type allele or an allele not associated with a disorder, e.g., the allele carried by a subject, at no more than about 1, 2, 3, 4, 5, 10, 15 or 20 % of the amino acid residues.

"Reference device", as used herein with respect to a claimed device (e.g., hydrogel capsule), means a device (e.g., hydrogel capsule) that: (i) lacks a particular feature, e.g., FBR-mitigating means (e.g., a barrier compartment comprising an afibrotic compound (as defined herein) or a CBS (as defined herein) (e.g., an RGD polymer), (ii) encapsulates in the cell-containing compartment about the same quantity of cells of the same cell type(s) as in the claimed device, and (iii) has a substantially similar polymer composition and structure as in the claimed device other than lacking the particular feature (e.g., the afibrotic compound or CBS). In an embodiment, the number of live cells in the cell-containing compartment of a reference device is within 80% to 120%, or within 90% to 110%, of the number of live cells in the cell-containing compartment of the claimed device. In an embodiment, the cells in the reference and claimed devices are obtained from the same cell culture. In an embodiment, a substantially similar polymer composition means all polymers in the reference and claimed device, including the polymer component of any CBP-polymer and afibrotic polymer, as applicable, are of the same chemical and molecular weight class (e.g., an alginate with high G content and the same molecular weight range). For example, in an embodiment, the cell-containing compartment of a CBP-null reference device is formed from the unmodified version of the polymer (e.g., alginate) in the CBP-polymer used to form the cell-containing compartment of the claimed device. In some embodiments in which a claimed two-compartment hydrogel millicapsule has (i) an inner compartment formed from a CBP-polymer encapsulating the plurality of cells and (ii) an outer compartment formed from a mixture of a chemically-modified polymer (e.g., a CM-LMW-alginate as described herein) and an unmodified polymer (e.g., an U-HMW-alginate as described herein), then the outer compartments of the reference and claimed capsules are formed from the same polymer mixture, while the inner compartment of the reference capsule is formed from a suspension of cells in the same polymer mixture used for the outer compartment. In an embodiment, a substantially similar structure means the reference and claimed devices have the same number of compartments (e.g., one, two, three, etc.) and about the same size and shape.

"RPE cell" as used herein refers to a cell having one or more of the following characteristics: a) it comprises a retinal pigment epithelial cell (RPE) (e.g., cultured using the ARPE-19 cell line (ATCC^{®} CRL-2302^{™})) or a cell derived therefrom, e.g., by stably transfecting cells cultured from the ARPE-19 cell line with an exogenous sequence that encodes a therapeutic protein or otherwise engineering such cultured ARPE-19 cells to express an exogenous protein or other exogenous substance, a cell derived from a primary cell culture of RPE cells, a cell isolated directly (without long term culturing, e.g., less than 5 or 10 passages or rounds of cell division since isolation) from naturally occurring RPE cells, e.g., from a human or other mammal, a cell derived from a transformed, an immortalized, or a long term (e.g., more than 5 or 10 passages or rounds of cell division) RPE cell culture; b) a cell that has been obtained from a less differentiated cell, e.g., a cell developed, programmed, or reprogramed (e.g., in vitro) into an RPE cell or a cell that is, except for any genetic engineering, substantially similar to one or more of a naturally occurring RPE cell or a cell from a primary or long term culture of RPE cells (e.g., the cell can be derived from an IPS cell); or c) a cell that has one or more of the following properties: i) it expresses one or more of the biomarkers CRALBP, RPE-65, RLBP, BEST1, or αB-crystallin; ii) it does not express one or more of the biomarkers CRALBP, RPE-65, RLBP, BEST1, or αB-crystallin; iii) it is naturally found in the retina and forms a monolayer above the choroidal blood vessels in the Bruch's membrane; iv) it is responsible for epithelial transport, light absorption, secretion, and immune modulation in the retina; or v) it has been created synthetically, or modified from a naturally occurring cell, to have the same or substantially the same genetic content, and optionally the same or substantially the same epigenetic content, as an immortalized RPE cell line (e.g., the ARPE-19 cell line (ATCC^{®} CRL-2302^{™})). In an embodiment, an RPE cell described herein is engineered, e.g., to have a new property, e.g., the cell is engineered to express the therapeutic agent when encapsulated in the polymer composition comprising a CBP or CBS. In other embodiments, an RPE cell is not engineered.

"Saline solution" as used herein, means normal saline, i.e., water containing 0.9% NaCl, unless otherwise specified.

"Sequence identity" or "percent identical", when used herein to refer to two nucleotide sequences or two amino acid sequences, means the two sequences are the same within a specified region, or have the same nucleotides or amino acids at a specified percentage of nucleotide or amino acid positions within the specified when the two sequences are compared and aligned for maximum correspondence over a comparison window or designated region. Sequence identity may be determined using standard techniques known in the art including, but not limited to, any of the algorithms described in US Patent Application Publication No. 2017/02334455. In an embodiment, the specified percentage of identical nucleotide or amino acid positions is at least about 80%, 85%, 90%, 91%, 92%, 93%, 94%, 95%, 96%, 97%, 98%, 99% or higher.

"Spherical" as used herein, means a device (e.g., a hydrogel capsule or other particle) having a curved surface that forms a sphere (e.g., a completely round ball) or sphere-like shape, which may have waves and undulations, e.g., on the surface. Spheres and sphere-like objects can be mathematically defined by rotation of circles, ellipses, or a combination around each of the three perpendicular axes, a, b, and c. For a sphere, the three axes are the same length. Generally, a sphere-like shape is an ellipsoid (for its averaged surface) with semi-principal axes within 10%, or 5%, or 2.5% of each other. The diameter of a sphere or sphere-like shape is the average diameter, such as the average of the semi-principal axes.

"Spheroid", as that term is used herein to refer to a device (e.g., a hydrogel capsule or other particle), means the device has (i) a perfect or classical oblate spheroid or prolate spheroid shape or (ii) has a surface that roughly forms a spheroid, e.g., may have waves and undulations and/or may be an ellipsoid (for its averaged surface) with semi-principal axes within 100% of each other.

"Subject" as used herein refers to a human or non-human animal. In an embodiment, the subject is a human (i.e., a male or female), e.g., of any age group, a pediatric subject (e.g., infant, child, adolescent) or adult subject (e.g., young adult, middle-aged adult, or senior adult). In an embodiment, the subject is a non-human animal, for example, a mammal (e.g., a mouse, a dog, a primate (e.g., a cynomolgus monkey or a rhesus monkey)). In an embodiment, the subject is a commercially relevant mammal (e.g., a cattle, pig, horse, sheep, goat, cat, or dog) or a bird (e.g., a commercially relevant bird such as a chicken, duck, goose, or turkey). In certain embodiments, the animal is a mammal. The animal may be a male or female and at any stage of development. A non-human animal may be a transgenic animal.

"Total volume," as used herein, refers to a volume within one compartment of a multi-compartment device that includes the space occupied by another compartment. For example, the total volume of the second (e.g., outer) compartment of a two-compartment device refers to a volume within the second compartment that includes space occupied by the first compartment.

"Treatment," "treat," and "treating" as used herein refers to one or more of reducing, reversing, alleviating, delaying the onset of, or inhibiting the progress of one or more of a symptom, manifestation, or underlying cause, of a disease, disorder, or condition. In an embodiment, treating comprises reducing, reversing, alleviating, delaying the onset of, or inhibiting the progress of a symptom of a disease, disorder, or condition. In an embodiment, treating comprises reducing, reversing, alleviating, delaying the onset of, or inhibiting the progress of a manifestation of a disease, disorder, or condition. In an embodiment, treating comprises reducing, reversing, alleviating, reducing, or delaying the onset of, an underlying cause of a disease, disorder, or condition. In some embodiments, "treatment," "treat," and "treating" require that signs or symptoms of the disease, disorder, or condition have developed or have been observed. In other embodiments, treatment may be administered in the absence of signs or symptoms of the disease or condition, e.g., in preventive treatment. For example, treatment may be administered to a susceptible individual prior to the onset of symptoms (e.g., considering a history of symptoms and/or in light of genetic or other susceptibility factors). Treatment may also be continued after symptoms have resolved, for example, to delay or prevent recurrence. In some embodiments, treatment comprises prevention and in other embodiments it does not.

"Von Willebrand factor protein" or "VWF protein", as used herein, means a polypeptide that comprises the amino acid sequence of a naturally occurring VWF polypeptide or variant thereof that has VWF biological activity, e.g., FVIII binding activity, as determined by an art-recognized assay, unless otherwise specified. VWF proteins that may be produced by a device described herein (e.g., expressed by engineered cells contained in the device) include wild-type primate (e.g., human), porcine, canine, and murine proteins, as well as variants of such wild-type proteins. The encapsulated cells may be engineered to encode any of the following VWF polypeptides: precursor VWF of 2813 amino acids, a VWF lacking the signal peptide of 22 amino acids and optionally the prepropeptide of 741 amino acids, mature VWF protein of 2050 amino acids, and truncated variants thereof, such as a VWF fragment sufficient to stabilize endogenous FVIII levels in VWF-deficient mice, e.g, a truncated variant containing the D'D3 region (amino acids 764-1247) or the D1D2D'D3 region; and VWF variants with one or more amino acid substitutions, e.g., in the D'region as described in US Patent No. 9458223. A variant VWF protein preferably has at least 50%, 75%, 90% or more (including >100%) of a biological activity of the corresponding wild-type VWF protein. Art-recognized assays for determining the biological activity of a VWF include ristocetin co-factor activity (Federici A B et al. 2004. Haematologica 89:77-85), binding of VWF to GP Ibα of the platelet glycoprotein complex Ib-V-IX (Sucker et al. 2006. Clin Appl Thromb Hemost. 12:305-310), and collagen binding (Kallas & Talpsep. 2001. Annals of Hematology 80:466-471).

In some embodiments, the VWF protein produced by a device of the disclosure comprises a naturally-occurring or variant VWF amino acid sequence fused to a heterologous polypeptide or non-polypeptide moiety extending the half-life of the VWF protein. Exemplary half-life extending moieties include Fc, albumin, a PAS sequence, transferrin, CTP (28 amino acid C-terminal peptide (CTP) of human chorionic gonadotropin (hCG) with its 4 O-glycans), polyethylene glycol (PEG), hydroxyethyl starch (HES), albumin binding polypeptide, albumin-binding small molecules, or any combination thereof.

### Selected Chemical Definitions

Definitions of specific functional groups and chemical terms are described in more detail below. The chemical elements are identified in accordance with the Periodic Table of the Elements, CAS version, Handbook of Chemistry and Physics, 75th Ed., inside cover, and specific functional groups are generally defined as described therein. Additionally, general principles of organic chemistry, as well as specific functional moieties and reactivity, are described in Thomas Sorrell, Organic Chemistry, University Science Books, Sausalito, 1999; Smith and March, March 's Advanced Organic Chemistry, 5th Edition, John Wiley & Sons, Inc., New York, 2001; Larock, Comprehensive Organic Transformations, VCH Publishers, Inc., New York, 1989; and Carruthers, Some Modern Methods of Organic Synthesis, 3rd Edition, Cambridge University Press, Cambridge, 1987.

The abbreviations used herein have their conventional meaning within the chemical and biological arts. The chemical structures and formulae set forth herein are constructed according to the standard rules of chemical valency known in the chemical arts.

When a range of values is listed, it is intended to encompass each value and sub-range within the range. For example, "C₁-C₆ alkyl" is intended to encompass, C₁, C₂, C₃, C₄, C₅, C₆, C₁-C₆, C₁-C₅, C₁-C₄, C₁-C₃, C₁-C₂, C₂-C₆, C₂-C₅, C₂-C₄, C₂-C₃, C₃-C₆, C₃-C₅, C₃-C₄, C₄-C₆, C₄-C₅, and C₅-C₆ alkyl.

As used herein, "alkyl" refers to a radical of a straight-chain or branched saturated hydrocarbon group having from 1 to 24 carbon atoms ("C₁-C₂₄ alkyl"). In some embodiments, an alkyl group has 1 to 12 carbon atoms ("C₁-C₁₂ alkyl"), 1 to 10 carbon atoms ("C₁-C₁₂ alkyl"), 1 to 8 carbon atoms ("C₁-C₈ alkyl"), 1 to 6 carbon atoms ("C₁-C₆ alkyl"), 1 to 5 carbon atoms ("C₁-C₅ alkyl"), 1 to 4 carbon atoms ("C₁-C₄alkyl"), 1 to 3 carbon atoms ("C₁-C₃ alkyl"), 1 to 2 carbon atoms ("C₁-C₂ alkyl"), or 1 carbon atom ("C₁ alkyl"). In some embodiments, an alkyl group has 2 to 6 carbon atoms ("C₂-C₆alkyl"). Examples of C₁-C₆ alkyl groups include methyl (C₁), ethyl (C₂), n-propyl (C₃), isopropyl (C₃), n-butyl (C₄), tert-butyl (C₄), sec-butyl (C₄), iso-butyl (C₄), n-pentyl (C₅), 3-pentanyl (C₅), amyl (C₅), neopentyl (C₅), 3-methyl-2-butanyl (C₅), tertiary amyl (Cs), and n-hexyl (C₆). Additional examples of alkyl groups include n-heptyl (C₇), n-octyl (C₈) and the like. Each instance of an alkyl group may be independently optionally substituted, i.e., unsubstituted (an "unsubstituted alkyl") or substituted (a "substituted alkyl") with one or more substituents; e.g., for instance from 1 to 5 substituents, 1 to 3 substituents, or 1 substituent.

As used herein, "alkenyl" refers to a radical of a straight-chain or branched hydrocarbon group having from 2 to 24 carbon atoms, one or more carbon-carbon double bonds, and no triple bonds ("C₂-C₂₄ alkenyl"). In some embodiments, an alkenyl group has 2 to 10 carbon atoms ("C₂-C₁₀ alkenyl"), 2 to 8 carbon atoms ("C₂-C₈ alkenyl"), 2 to 6 carbon atoms ("C₂-C₆ alkenyl"), 2 to 5 carbon atoms ("C₂-C₅ alkenyl"), 2 to 4 carbon atoms ("C₂-C₄ alkenyl"), 2 to 3 carbon atoms ("C₂-C₃ alkenyl"), or 2 carbon atoms ("C₂ alkenyl"). The one or more carbon-carbon double bonds can be internal (such as in 2-butenyl) or terminal (such as in 1-butenyl). Examples of C₂-C₄ alkenyl groups include ethenyl (C₂), 1-propenyl (C₃), 2-propenyl (C₃), 1-butenyl (C₄), 2-butenyl (C₄), butadienyl (C₄), and the like. Examples of C₂-C₆ alkenyl groups include the aforementioned C₂₋₄ alkenyl groups as well as pentenyl (C₅), pentadienyl (C₅), hexenyl (C₆), and the like. Each instance of an alkenyl group may be independently optionally substituted, *i.e.,* unsubstituted (an "unsubstituted alkenyl") or substituted (a "substituted alkenyl") with one or more substituents *e.g*., for instance from 1 to 5 substituents, 1 to 3 substituents, or 1 substituent.

As used herein, the term "alkynyl" refers to a radical of a straight-chain or branched hydrocarbon group having from 2 to 24 carbon atoms, one or more carbon-carbon triple bonds ("C₂-C₂₄ alkenyl"). In some embodiments, an alkynyl group has 2 to 10 carbon atoms ("C₂-C₁₀ alkynyl"), 2 to 8 carbon atoms ("C₂-C₈ alkynyl"), 2 to 6 carbon atoms ("C₂-C₆ alkynyl"), 2 to 5 carbon atoms ("C₂-C₅ alkynyl"), 2 to 4 carbon atoms ("C₂-C₄ alkynyl"), 2 to 3 carbon atoms ("C₂-C₃ alkynyl"), or 2 carbon atoms ("C₂ alkynyl"). The one or more carbon-carbon triple bonds can be internal (such as in 2-butynyl) or terminal (such as in 1-butynyl). Examples of C₂-C₄ alkynyl groups include ethynyl (C₂), 1-propynyl (C₃), 2-propynyl (C₃), 1-butynyl (C₄), 2-butynyl (C₄), and the like. Each instance of an alkynyl group may be independently optionally substituted, *i.e.,* unsubstituted (an "unsubstituted alkynyl") or substituted (a "substituted alkynyl") with one or more substituents *e.g*., for instance from 1 to 5 substituents, 1 to 3 substituents, or 1 substituent.

As used herein, the term "heteroalkyl," refers to a non-cyclic stable straight or branched chain, or combinations thereof, including at least one carbon atom and at least one heteroatom selected from the group consisting of O, N, P, Si, and S, and wherein the nitrogen and sulfur atoms may optionally be oxidized, and the nitrogen heteroatom may optionally be quaternized. The heteroatom(s) O, N, P, S, and Si may be placed at any position of the heteroalkyl group. Exemplary heteroalkyl groups include, but are not limited to: -CH₂-CH₂-O-CH₃, -CH₂-CH₂-NH-CH₃, -CH₂-CH₂-N(CH₃)-CH₃, -CH₂-S-CH₂-CH₃, -CH₂-CH₂, -S(O)-CH₃, -CH₂-CH₂-S(O)₂-CH₃, -CH=CH-O-CH₃, -Si(CH₃)₃, -CH₂-CH=N-OCH₃, -CH=CH-N(CH₃)-CH₃, -O-CH₃, and -O-CH₂-CH₃. Up to two or three heteroatoms may be consecutive, such as, for example, -CH₂-NH-OCH₃ and -CH₂-O-Si(CH₃)₃. Where "heteroalkyl" is recited, followed by recitations of specific heteroalkyl groups, such as -CH₂O, -NR^{C}R^{D}, or the like, it will be understood that the terms heteroalkyl and -CH₂O or -NR^{C}R^{D} are not redundant or mutually exclusive. Rather, the specific heteroalkyl groups are recited to add clarity. Thus, the term "heteroalkyl" should not be interpreted herein as excluding specific heteroalkyl groups, such as -CH₂O, -NR^{C}R^{D}, or the like. Each instance of a heteroalkyl group may be independently optionally substituted, *i.e.,* unsubstituted (an "unsubstituted heteroalkyl") or substituted (a "substituted heteroalkyl") with one or more substituents *e*.*g*., for instance from 1 to 5 substituents, 1 to 3 substituents, or 1 substituent.

The terms "alkylene," "alkenylene," "alkynylene," or "heteroalkylene," alone or as part of another substituent, mean, unless otherwise stated, a divalent radical derived from an alkyl, alkenyl, alkynyl, or heteroalkyl, respectively. An alkylene, alkenylene, alkynylene, or heteroalkylene group may be described as, e.g., a C₁-C₆-membered alkylene, C₂-C₆-membered alkenylene, C₂-C₆-membered alkynylene, or C₁-C₆-membered heteroalkylene, wherein the term "membered" refers to the non-hydrogen atoms within the moiety. In the case of heteroalkylene groups, heteroatoms can also occupy either or both chain termini (e.g., alkyleneoxy, alkylenedioxy, alkyleneamino, alkylenediamino, and the like). Still further, for alkylene and heteroalkylene linking groups, no orientation of the linking group is implied by the direction in which the formula of the linking group is written. For example, the formula -C(O)₂R'- may represent both -C(O)₂R'- and -R'C(O)₂-.

As used herein, "aryl" refers to a radical of a monocyclic or polycyclic (*e*.*g*., bicyclic or tricyclic) 4n+2 aromatic ring system (*e*.*g*., having 6, 10, or 14 π electrons shared in a cyclic array) having 6-14 ring carbon atoms and zero heteroatoms provided in the aromatic ring system ("C₆-C₁₄ aryl"). In some embodiments, an aryl group has six ring carbon atoms ("C₆ aryl"; *e.g*., phenyl). In some embodiments, an aryl group has ten ring carbon atoms ("C₁₀ aryl"; *e.g*., naphthyl such as 1-naphthyl and 2-naphthyl). In some embodiments, an aryl group has fourteen ring carbon atoms ("C₁₄ aryl"; *e*.*g*., anthracyl). An aryl group may be described as, *e.g.*, a C₆-C₁₀-membered aryl, wherein the term "membered" refers to the non-hydrogen ring atoms within the moiety. Aryl groups include phenyl, naphthyl, indenyl, and tetrahydronaphthyl. Each instance of an aryl group may be independently optionally substituted, *i.e.,* unsubstituted (an "unsubstituted aryl") or substituted (a "substituted aryl") with one or more substituents.

As used herein, "heteroaryl" refers to a radical of a 5-10 membered monocyclic or bicyclic 4n+2 aromatic ring system (*e.g*., having 6 or 10 π electrons shared in a cyclic array) having ring carbon atoms and 1-4 ring heteroatoms provided in the aromatic ring system, wherein each heteroatom is independently selected from nitrogen, oxygen and sulfur ("5-10 membered heteroaryl"). In heteroaryl groups that contain one or more nitrogen atoms, the point of attachment can be a carbon or nitrogen atom, as valency permits. Heteroaryl bicyclic ring systems can include one or more heteroatoms in one or both rings. "Heteroaryl" also includes ring systems wherein the heteroaryl ring, as defined above, is fused with one or more aryl groups wherein the point of attachment is either on the aryl or heteroaryl ring, and in such instances, the number of ring members designates the number of ring members in the fused (aryl/heteroaryl) ring system. Bicyclic heteroaryl groups wherein one ring does not contain a heteroatom (*e*.*g*., indolyl, quinolinyl, carbazolyl, and the like) the point of attachment can be on either ring, *i.e.,* either the ring bearing a heteroatom (*e.g.,* 2-indolyl) or the ring that does not contain a heteroatom (*e.g., 5-*indolyl). A heteroaryl group may be described as, e.g., a 6-10-membered heteroaryl, wherein the term "membered" refers to the non-hydrogen ring atoms within the moiety.

In some embodiments, a heteroaryl group is a 5-10 membered aromatic ring system having ring carbon atoms and 1-4 ring heteroatoms provided in the aromatic ring system, wherein each heteroatom is independently selected from nitrogen, oxygen, and sulfur ("5-10 membered heteroaryl"). In some embodiments, a heteroaryl group is a 5-8 membered aromatic ring system having ring carbon atoms and 1-4 ring heteroatoms provided in the aromatic ring system, wherein each heteroatom is independently selected from nitrogen, oxygen, and sulfur ("5-8 membered heteroaryl"). In some embodiments, a heteroaryl group is a 5-6 membered aromatic ring system having ring carbon atoms and 1-4 ring heteroatoms provided in the aromatic ring system, wherein each heteroatom is independently selected from nitrogen, oxygen, and sulfur ("5-6 membered heteroaryl"). In some embodiments, the 5-6 membered heteroaryl has 1-3 ring heteroatoms selected from nitrogen, oxygen, and sulfur. In some embodiments, the 5-6 membered heteroaryl has 1-2 ring heteroatoms selected from nitrogen, oxygen, and sulfur. In some embodiments, the 5-6 membered heteroaryl has 1 ring heteroatom selected from nitrogen, oxygen, and sulfur. Each instance of a heteroaryl group may be independently optionally substituted, *i.e.,* unsubstituted (an "unsubstituted heteroaryl") or substituted (a "substituted heteroaryl") with one or more substituents.

Exemplary 5-membered heteroaryl groups containing one heteroatom include, without limitation, pyrrolyl, furanyl and thiophenyl. Exemplary 5-membered heteroaryl groups containing two heteroatoms include, without limitation, imidazolyl, pyrazolyl, oxazolyl, isoxazolyl, thiazolyl, and isothiazolyl. Exemplary 5-membered heteroaryl groups containing three heteroatoms include, without limitation, triazolyl, oxadiazolyl, and thiadiazolyl. Exemplary 5-membered heteroaryl groups containing four heteroatoms include, without limitation, tetrazolyl. Exemplary 6-membered heteroaryl groups containing one heteroatom include, without limitation, pyridinyl. Exemplary 6-membered heteroaryl groups containing two heteroatoms include, without limitation, pyridazinyl, pyrimidinyl, and pyrazinyl. Exemplary 6-membered heteroaryl groups containing three or four heteroatoms include, without limitation, triazinyl and tetrazinyl, respectively. Exemplary 7-membered heteroaryl groups containing one heteroatom include, without limitation, azepinyl, oxepinyl, and thiepinyl. Exemplary 5,6-bicyclic heteroaryl groups include, without limitation, indolyl, isoindolyl, indazolyl, benzotriazolyl, benzothiophenyl, isobenzothiophenyl, benzofuranyl, benzoisofuranyl, benzimidazolyl, benzoxazolyl, benzisoxazolyl, benzoxadiazolyl, benzthiazolyl, benzisothiazolyl, benzthiadiazolyl, indolizinyl, and purinyl. Exemplary 6,6-bicyclic heteroaryl groups include, without limitation, naphthyridinyl, pteridinyl, quinolinyl, isoquinolinyl, cinnolinyl, quinoxalinyl, phthalazinyl, and quinazolinyl. Other exemplary heteroaryl groups include heme and heme derivatives.

As used herein, the terms "arylene" and "heteroarylene," alone or as part of another substituent, mean a divalent radical derived from an aryl and heteroaryl, respectively.

As used herein, "cycloalkyl" refers to a radical of a non-aromatic cyclic hydrocarbon group having from 3 to 10 ring carbon atoms ("C₃-C₁₀ cycloalkyl") and zero heteroatoms in the non-aromatic ring system. In some embodiments, a cycloalkyl group has 3 to 8 ring carbon atoms ("C₃-C₈cycloalkyl"), 3 to 6 ring carbon atoms ("C₃-C₆ cycloalkyl"), or 5 to 10 ring carbon atoms ("C₅-C₁₀ cycloalkyl"). A cycloalkyl group may be described as, e.g., a C₄-C₇-membered cycloalkyl, wherein the term "membered" refers to the non-hydrogen ring atoms within the moiety. Exemplary C₃-C₆ cycloalkyl groups include, without limitation, cyclopropyl (C₃), cyclopropenyl (C₃), cyclobutyl (C₄), cyclobutenyl (C₄), cyclopentyl (C₅), cyclopentenyl (C₅), cyclohexyl (C₆), cyclohexenyl (C₆), cyclohexadienyl (C₆), and the like. Exemplary C₃-C₈ cycloalkyl groups include, without limitation, the aforementioned C₃-C₆ cycloalkyl groups as well as cycloheptyl (C₇), cycloheptenyl (C₇), cycloheptadienyl (C₇), cycloheptatrienyl (C₇), cyclooctyl (C₈), cyclooctenyl (C₈), cubanyl (C₈), bicyclo[1.1.1]pentanyl (C₅), bicyclo[2.2.2]octanyl (C₈), bicyclo[2.1.1]hexanyl (C₆), bicyclo[3.1.1]heptanyl (C₇), and the like. Exemplary C₃-C₁₀ cycloalkyl groups include, without limitation, the aforementioned C₃-C₈ cycloalkyl groups as well as cyclononyl (C₉), cyclononenyl (C₉), cyclodecyl (C₁₀), cyclodecenyl (C₁₀), octahydro-1*H-*indenyl (C₉), decahydronaphthalenyl (C₁₀), spiro [4.5] decanyl (C₁₀), and the like. As the foregoing examples illustrate, in certain embodiments, the cycloalkyl group is either monocyclic ("monocyclic cycloalkyl") or contain a fused, bridged or spiro ring system such as a bicyclic system ("bicyclic cycloalkyl") and can be saturated or can be partially unsaturated. "Cycloalkyl" also includes ring systems wherein the cycloalkyl ring, as defined above, is fused with one or more aryl groups wherein the point of attachment is on the cycloalkyl ring, and in such instances, the number of carbons continue to designate the number of carbons in the cycloalkyl ring system. Each instance of a cycloalkyl group may be independently optionally substituted, *i.e.,* unsubstituted (an "unsubstituted cycloalkyl") or substituted (a "substituted cycloalkyl") with one or more substituents.

"Heterocyclyl" as used herein refers to a radical of a 3- to 10-membered non-aromatic ring system having ring carbon atoms and 1 to 4 ring heteroatoms, wherein each heteroatom is independently selected from nitrogen, oxygen, sulfur, boron, phosphorus, and silicon ("3-10 membered heterocyclyl"). In heterocyclyl groups that contain one or more nitrogen atoms, the point of attachment can be a carbon or nitrogen atom, as valency permits. A heterocyclyl group can either be monocyclic ("monocyclic heterocyclyl") or a fused, bridged or spiro ring system such as a bicyclic system ("bicyclic heterocyclyl"), and can be saturated or can be partially unsaturated. Heterocyclyl bicyclic ring systems can include one or more heteroatoms in one or both rings. "Heterocyclyl" also includes ring systems wherein the heterocyclyl ring, as defined above, is fused with one or more cycloalkyl groups wherein the point of attachment is either on the cycloalkyl or heterocyclyl ring, or ring systems wherein the heterocyclyl ring, as defined above, is fused with one or more aryl or heteroaryl groups, wherein the point of attachment is on the heterocyclyl ring, and in such instances, the number of ring members continue to designate the number of ring members in the heterocyclyl ring system. A heterocyclyl group may be described as, e.g., a 3-7-membered heterocyclyl, wherein the term "membered" refers to the non-hydrogen ring atoms, i.e., carbon, nitrogen, oxygen, sulfur, boron, phosphorus, and silicon, within the moiety. Each instance of heterocyclyl may be independently optionally substituted, *i.e.,* unsubstituted (an "unsubstituted heterocyclyl") or substituted (a "substituted heterocyclyl") with one or more substituents. In certain embodiments, the heterocyclyl group is unsubstituted 3-10 membered heterocyclyl. In certain embodiments, the heterocyclyl group is substituted 3-10 membered heterocyclyl.

In some embodiments, a heterocyclyl group is a 5-10 membered non-aromatic ring system having ring carbon atoms and 1-4 ring heteroatoms, wherein each heteroatom is independently selected from nitrogen, oxygen, sulfur, boron, phosphorus, and silicon ("5-10 membered heterocyclyl"). In some embodiments, a heterocyclyl group is a 5-8 membered non-aromatic ring system having ring carbon atoms and 1-4 ring heteroatoms, wherein each heteroatom is independently selected from nitrogen, oxygen, and sulfur ("5-8 membered heterocyclyl"). In some embodiments, a heterocyclyl group is a 5-6 membered non-aromatic ring system having ring carbon atoms and 1-4 ring heteroatoms, wherein each heteroatom is independently selected from nitrogen, oxygen, and sulfur ("5-6 membered heterocyclyl"). In some embodiments, the 5-6 membered heterocyclyl has 1-3 ring heteroatoms selected from nitrogen, oxygen, and sulfur. In some embodiments, the 5-6 membered heterocyclyl has 1-2 ring heteroatoms selected from nitrogen, oxygen, and sulfur. In some embodiments, the 5-6 membered heterocyclyl has one ring heteroatom selected from nitrogen, oxygen, and sulfur.

Exemplary 3-membered heterocyclyl groups containing one heteroatom include, without limitation, azirdinyl, oxiranyl, thiorenyl. Exemplary 4-membered heterocyclyl groups containing one heteroatom include, without limitation, azetidinyl, oxetanyl and thietanyl. Exemplary 5-membered heterocyclyl groups containing one heteroatom include, without limitation, tetrahydrofuranyl, dihydrofuranyl, tetrahydrothiophenyl, dihydrothiophenyl, pyrrolidinyl, dihydropyrrolyl and pyrrolyl-2,5-dione. Exemplary 5-membered heterocyclyl groups containing two heteroatoms include, without limitation, dioxolanyl, oxasulfuranyl, disulfuranyl, and oxazolidin-2-one. Exemplary 5-membered heterocyclyl groups containing three heteroatoms include, without limitation, triazolinyl, oxadiazolinyl, and thiadiazolinyl. Exemplary 6-membered heterocyclyl groups containing one heteroatom include, without limitation, piperidinyl, piperazinyl, tetrahydropyranyl, dihydropyridinyl, and thianyl. Exemplary 6-membered heterocyclyl groups containing two heteroatoms include, without limitation, piperazinyl, morpholinyl, dithianyl, dioxanyl. Exemplary 6-membered heterocyclyl groups containing two heteroatoms include, without limitation, triazinanyl or thiomorpholinyl-1,1-dioxide. Exemplary 7-membered heterocyclyl groups containing one heteroatom include, without limitation, azepanyl, oxepanyl and thiepanyl. Exemplary 8-membered heterocyclyl groups containing one heteroatom include, without limitation, azocanyl, oxecanyl and thiocanyl. Exemplary 5-membered heterocyclyl groups fused to a C₆ aryl ring (also referred to herein as a 5,6-bicyclic heterocyclic ring) include, without limitation, indolinyl, isoindolinyl, dihydrobenzofuranyl, dihydrobenzothienyl, benzoxazolinonyl, and the like. Exemplary 6-membered heterocyclyl groups fused to an aryl ring (also referred to herein as a 6,6-bicyclic heterocyclic ring) include, without limitation, tetrahydroquinolinyl, tetrahydroisoquinolinyl, and the like.

"Amino" as used herein refers to the radical -NR⁷⁰R⁷¹, wherein R⁷⁰ and R⁷¹ are each independently hydrogen, C₁-C₈ alkyl, C₃-C₁₀ cycloalkyl, C₄-C₁₀ heterocyclyl, C₆-C₁₀ aryl, and C₅-C₁₀ heteroaryl. In some embodiments, amino refers to NH₂.

As used herein, "cyano" refers to the radical -CN.

As used herein, "halo" or "halogen," independently or as part of another substituent, mean, unless otherwise stated, a fluorine (F), chlorine (Cl), bromine (Br), or iodine (I) atom.

As used herein, "hydroxy" refers to the radical -OH.

Alkyl, alkenyl, alkynyl, heteroalkyl, cycloalkyl, heterocyclyl, aryl, and heteroaryl groups, as defined herein, are optionally substituted (*e*.*g*., "substituted" or "unsubstituted" alkyl, "substituted" or "unsubstituted" alkenyl, "substituted" or "unsubstituted" alkynyl, "substituted" or "unsubstituted" heteroalkyl, "substituted" or "unsubstituted" cycloalkyl, "substituted" or "unsubstituted" heterocyclyl, "substituted" or "unsubstituted" aryl or "substituted" or "unsubstituted" heteroaryl group). In general, the term "substituted", whether preceded by the term "optionally" or not, means that at least one hydrogen present on a group (*e.g*., a carbon or nitrogen atom) is replaced with a permissible substituent, *e*.*g*., a substituent which upon substitution results in a stable compound, *e*.*g*., a compound which does not spontaneously undergo transformation such as by rearrangement, cyclization, elimination, or other reaction. Unless otherwise indicated, a "substituted" group has a substituent at one or more substitutable positions of the group, and when more than one position in any given structure is substituted, the substituent is either the same or different at each position. The term "substituted" is contemplated to include substitution with all permissible substituents of organic compounds, such as any of the substituents described herein that result in the formation of a stable compound. The present disclosure contemplates any and all such combinations to arrive at a stable compound. For purposes of this disclosure, heteroatoms such as nitrogen may have hydrogen substituents and/or any suitable substituent as described herein which satisfy the valencies of the heteroatoms and results in the formation of a stable moiety.

Two or more substituents may optionally be joined to form aryl, heteroaryl, cycloalkyl, or heterocyclyl groups. Such so-called ring-forming substituents are typically, though not necessarily, found attached to a cyclic base structure. In one embodiment, the ring-forming substituents are attached to adjacent members of the base structure. For example, two ring-forming substituents attached to adjacent members of a cyclic base structure create a fused ring structure. In another embodiment, the ring-forming substituents are attached to a single member of the base structure. For example, two ring-forming substituents attached to a single member of a cyclic base structure create a spirocyclic structure. In yet another embodiment, the ring-forming substituents are attached to non-adjacent members of the base structure.

Compounds of Formula (I) described herein can comprise one or more asymmetric centers, and thus can exist in various isomeric forms, e.g., enantiomers and/or diastereomers. For example, the compounds described herein can be in the form of an individual enantiomer, diastereomer or geometric isomer, or can be in the form of a mixture of stereoisomers, including racemic mixtures and mixtures enriched in one or more stereoisomer. Isomers can be isolated from mixtures by methods known to those skilled in the art, including chiral high-pressure liquid chromatography (HPLC) and the formation and crystallization of chiral salts; or preferred isomers can be prepared by asymmetric syntheses. See, for example, Jacques et al., Enantiomers, Racemates and Resolutions (Wiley Interscience, New York, 1981); Wilen et al., Tetrahedron 33:2725 (1977); Eliel, Stereochemistry of Carbon Compounds (McGraw-Hill, NY, 1962); and Wilen, Tables of Resolving Agents and Optical Resolutions p. 268 (E.L. Eliel, Ed., Univ. of Notre Dame Press, Notre Dame, IN 1972). The disclosure additionally encompasses compounds described herein as individual isomers substantially free of other isomers, and alternatively, as mixtures of various isomers.

As used herein, a pure enantiomeric compound is substantially free from other enantiomers or stereoisomers of the compound (i.e., in enantiomeric excess). In other words, an "S" form of the compound is substantially free from the "R" form of the compound and is, thus, in enantiomeric excess of the "R" form. The term "enantiomerically pure" or "pure enantiomer" denotes that the compound comprises more than 75% by weight, more than 80% by weight, more than 85% by weight, more than 90% by weight, more than 91% by weight, more than 92% by weight, more than 93% by weight, more than 94% by weight, more than 95% by weight, more than 96% by weight, more than 97% by weight, more than 98% by weight, more than 99% by weight, more than 99.5% by weight, or more than 99.9% by weight, of the enantiomer. In certain embodiments, the weights are based upon total weight of all enantiomers or stereoisomers of the compound.

Compounds of Formula (I) described herein may also comprise one or more isotopic substitutions. For example, H may be in any isotopic form, including ¹H, ²H (D or deuterium), and ³H (T or tritium); C may be in any isotopic form, including ¹²C, ¹³C, and ¹⁴C; O may be in any isotopic form, including ¹⁶O and ¹⁸O; and the like.

The term "pharmaceutically acceptable salt" is meant to include salts of the active compounds that are prepared with relatively nontoxic acids or bases, depending on the particular substituents found on the compounds described herein. When compounds of Formula (I) used to prepare devices of the present disclosure contain relatively acidic functionalities, base addition salts can be obtained by contacting the neutral form of such compounds with a sufficient amount of the desired base, either neat or in a suitable inert solvent. Examples of pharmaceutically acceptable base addition salts include sodium, potassium, calcium, ammonium, organic amino, or magnesium salt, or a similar salt. When compounds used in the present disclosure contain relatively basic functionalities, acid addition salts can be obtained by contacting the neutral form of such compounds with a sufficient amount of the desired acid, either neat or in a suitable inert solvent. Examples of pharmaceutically acceptable acid addition salts include those derived from inorganic acids like hydrochloric, hydrobromic, nitric, carbonic, monohydrogencarbonic, phosphoric, monohydrogenphosphoric, dihydrogenphosphoric, sulfuric, monohydrogensulfuric, hydriodic, or phosphorous acids and the like, as well as the salts derived from organic acids like acetic, propionic, isobutyric, maleic, malonic, benzoic, succinic, suberic, fumaric, lactic, mandelic, phthalic, benzenesulfonic, p-tolylsulfonic, citric, tartaric, methanesulfonic, and the like. Also included are salts of amino acids such as arginate and the like, and salts of organic acids like glucuronic or galacturonic acids and the like (see, e.g., Berge et al, Journal of Pharmaceutical Science 66: 1-19 (1977)). Certain specific compounds used in the devices of the present disclosure (e.g., a particle, a hydrogel capsule) contain both basic and acidic functionalities that allow the compounds to be converted into either base or acid addition salts. These salts may be prepared by methods known to those skilled in the art. Other pharmaceutically acceptable carriers known to those of skill in the art are suitable for use in the present disclosure.

Devices of the present disclosure may contain a compound of Formula (I) in a prodrug form. Prodrugs are those compounds that readily undergo chemical changes under physiological conditions to provide the compounds useful for preparing devices in the present disclosure. Additionally, prodrugs can be converted to useful compounds of Formula (I) by chemical or biochemical methods in an *ex vivo* environment.

Certain compounds of Formula (I) described herein can exist in unsolvated forms as well as solvated forms, including hydrated forms. In general, the solvated forms are equivalent to unsolvated forms and are encompassed within the scope of the present disclosure. Certain compounds of Formula (I) described herein may exist in multiple crystalline or amorphous forms. In general, all physical forms are equivalent for the uses contemplated by the present disclosure and are intended to be within the scope of the present disclosure.

The term "solvate" refers to forms of the compound that are associated with a solvent, usually by a solvolysis reaction. This physical association may include hydrogen bonding. Conventional solvents include water, methanol, ethanol, acetic acid, DMSO, THF, diethyl ether, and the like. The compounds described herein may be prepared, *e.g.,* in crystalline form, and may be solvated. Suitable solvates include pharmaceutically acceptable solvates and further include both stoichiometric solvates and non-stoichiometric solvates.

The term "hydrate" refers to a compound which is associated with water. Typically, the number of the water molecules contained in a hydrate of a compound is in a definite ratio to the number of the compound molecules in the hydrate. Therefore, a hydrate of a compound may be represented, for example, by the general formula R·x H₂O, wherein R is the compound and wherein x is a number greater than 0.

The term "tautomer" as used herein refers to compounds that are interchangeable forms of a compound structure, and that vary in the displacement of hydrogen atoms and electrons. Thus, two structures may be in equilibrium through the movement of π electrons and an atom (usually H). For example, enols and ketones are tautomers because they are rapidly interconverted by treatment with either acid or base. Tautomeric forms may be relevant to the attainment of the optimal chemical reactivity and biological activity of a compound of interest.

The symbol " 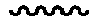 " as used herein refers to a connection to an entity, e.g., a polymer (e.g., hydrogel-forming polymer such as alginate) or surface of an implantable device, e.g., a particle, a hydrogel capsule. The connection represented by " 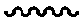 " may refer to direct attachment to the entity, e.g., a polymer or an implantable element, may refer to linkage to the entity through an attachment group. An "attachment group," as described herein, refers to a moiety for linkage of a compound of Formula (I) to an entity (e.g., a polymer or an implantable element (e.g., a device) as described herein), and may comprise any attachment chemistry known in the art. A listing of exemplary attachment groups is outlined in Bioconjugate Techniques (3rd ed, Greg T. Hermanson, Waltham, MA: Elsevier, Inc, 2013), which is incorporated herein by reference in its entirety. In some embodiments, an attachment group comprises alkyl, alkenyl, alkynyl, heteroalkyl, cycloalkyl, heterocyclyl, aryl, heteroaryl, -C(O)-, -OC(O)-, -N(R^{C})-, -N(R^{C})C(O)-, - C(O)N(R^{C})-, -N(R^{C})N(R^{D})-, -NCN-, -C(=N(R^{C})(R^{D}))O-, -S-, -S(O)ₓ-, -OS(O)ₓ-, - N(R^{C})S(O)ₓ-, -S(O)ₓN(R^{C})-, -P(R^{F})_{y}-, -Si(OR^{A})₂ -, -Si(R^{G})(OR^{A})-, -B(OR^{A})-, or a metal, wherein each of R^{A}, R^{C}, R^{D}, R^{F}, R^{G}, x and y is independently as described herein. In some embodiments, an attachment group comprises an amine, ketone, ester, amide, alkyl, alkenyl, alkynyl, or thiol. In some embodiments, an attachment group is a cross-linker. In some embodiments, the attachment group is -C(O)(C₁-C₆-alkylene)-, wherein alkylene is substituted with R¹, and R¹ is as described herein. In some embodiments, the attachment group is -C(O)(C₁-C₆-alkylene)-, wherein alkylene is substituted with 1-2 alkyl groups (e.g., 1-2 methyl groups). In some embodiments, the attachment group is -C(O)C(CH₃)₂-. In some embodiments, the attachment group is -C(O)(methylene)-, wherein alkylene is substituted with 1-2 alkyl groups (e.g., 1-2 methyl groups). In some embodiments, the attachment group is -C(O)CH(CH₃)-. In some embodiments, the attachment group is -C(O)C(CH₃)-.

### Features of Devices

The present disclosure features an implantable device comprising a plurality of cells (e.g., live cells) which are encapsulated in a first cell-containing compartment by a polymer composition that comprises at least one cell binding substance (CBS) (as defined herein). The cells are capable of expressing a therapeutic agent upon implant of the device into a subject, e.g., a human or other mammalian subject. In addition, the device comprises at least one means for mitigating the FBR (as defined herein).

In an embodiment, the CBS in the cell-containing compartment is present in an amount effective to increase viability of the cells and / or increase productivity of the cells at a timepoint after the device is implanted into an immune-compromised or immune-competent animal, e.g., immune-competent mice (e.g., the C57BL/6J mouse strain available from the Jackson Laboratory, Bar Harbor, ME USA) as compared to a CBS-null reference device, as defined above herein. In an embodiment, the increase in cell viability and / or productivity is detectable at a desired timepoint after implant, e.g., at one or more of 1 day, 3 days, 5 days, 1 week, 2 weeks, 4 weeks, 8 weeks, 12 weeks, 24 weeks, 36 weeks and 48 weeks. In an embodiment, the effective amount of the CBS results in an increase in one or both of (i) cell viability by at least 10%, 25%, 50% or 100% when measured at 1 week, 2 weeks, 4 weeks or 12 weeks after implant and (ii) increases cell productivity by at least 1.25-fold, 1.5-fold, 2-fold, 5-fold, 8-fold or 10-fold when measured at 1 week, 2 weeks, 4 weeks or 12 weeks after implant. In an embodiment, the effective amount of the CBS in the cell-containing compartment falls within a range between the minimally effective amount and a higher amount at which the cell viability and/or productivity are reduced compared to a CBS-null reference device or compared to the a device containing a maximally-effective amount, e.g., the optimal amount, of the CBS in the cell-containing compartment. In an embodiment, the amount of the CBS in the cell-containing compartment is no more than 50%, 25%, 10% or 5% above or below the optimal amount, e.g., the amount that results in the greatest increase in cell viability and/or productivity as compared to the CBS-null reference device. In an embodiment, the effective amount of a CBP to achieve improved productivity after implant is determined for the desired combination of cells, CBS, therapeutic protein expressed by the cells, e.g., engineered ARPE-19 cells, specific polypeptide (e.g., Factor VIII, Factor IX, Factor VII) and optionally including the particular coding sequence for the therapeutic protein.

The number of viable (and optionally dead) cells in a device described herein may be estimated using any technique known in the art, including an assay that differentially labels live and dead cells with two fluorescent dyes followed by detection, and optionally quantification, of labeled cells using fluorescent microscopy. Cell viability may also be evaluated by assessing other cell viability indicators, including measuring esterase activity, or quantitating the amount of ATP in the cells.

In an embodiment, the post-implant increase in cell productivity is detected by assaying for the level of the therapeutic agent expressed by the cells *in vivo* or *ex vivo* (e.g., cell expression after the device has been retrieved from the animal. Depending on the nature of the therapeutic agent, its expression may be measured intracellularly, extracellularly but inside the device, and/or outside of the device, e.g., in a tissue sample removed from an animal (e.g., a non-human animal) treated with a device or device preparation described herein. In an embodiment, the cell productivity is expressed as the measured amount of the therapeutic agent or agent activity divided by the number of administered devices (e.g., number of capsules placed in the animal) and/or by the number of administered cells (e.g., approximate number of cells per capsule in the administered capsule preparation, e.g., determined as described in the Examples herein below). In an embodiment, the increase in cell productivity is further normalized by dividing the determined amount (e.g., of therapeutic agent or amount of activity) by the time between two time points of interest, e.g., between administration and measurement, e.g., number of hours, days or weeks. In an embodiment, the therapeutic agent is a protein, and the increase in productivity is determined by measuring the amount and/or activity of the protein in a tissue sample removed from the animal (e.g., plasma separated from a blood sample collected from the animal), dividing the measured amount and/or activity by the number of administered devices (e.g, number of implanted 2-compartment capsules), and optionally further dividing the result by the number of days between administration and tissue sample removal.

The CBS may comprise a polypeptide polymer (e.g., a CBPP such as fibronectin or laminin) that is within the encapsulating polymer composition or it may be a CBP that is covalently attached to an unrelated amino acid polymer (e.g., a silk polymer), or to a naturally occurring polymer (e.g., an alginate, chitin, cotton) or to a synthetic polymer (e.g., a polymer in any of the following classes: acrylic, polyester, polyethylene, polypropylene polyacetonitrile, polyethylene teraphthalate, nylon, polyamide, polyurethane, polybutester). In addition to the CBS, the polymer composition in the cell-containing compartment may contain one or more unmodified naturally-occurring or synthetic polymers of any of the above-recited polymer classes to help provide structural integrity to the compartment and / or help provide a scaffold for supporting the cells.

The CBS recited in the present claims comprises an alginate covalently modified with one or more CBPs, e.g., RGD (SEQ ID NO: 43), or RGDSP (SEQ ID NO: 59).In embodiments not recited in the present claims, the CBS comprises an alginate covalently modified with one or more CBPs, e.g., DGEA (SEQ ID NO: 39), FYFDLR (SEQ ID NO: 40), PHSRN (SEQ ID NO: 46), YIGSR (SEQ ID NO: 50), a peptide comprising or consisting essentially of any of the cell binding sequences listed in Table 1 herein, or a mixture of 2, 3 or more of these CBPs (e.g., RGD + DGEA (SEQ ID NOs: 43 and 39, respectively), RGD + PHSRN (SEQ ID NOs: 43 and 46, respectively), RGD + DGEA + PHSRN (SEQ ID NOs: 43, 39, and 46, respectively)). Alginate is a polysaccharide made up of β-D-mannuronic acid (M) and α-L-guluronic acid (G). In some embodiments, the alginate is a high guluronic acid (G) alginate, and comprises greater than about 50%, 55%, 60%, 65%, 70%, 75%, 80%, 85%, 90%, or more guluronic acid (G). In some embodiments, the ratio of G:M is at least 1.3, 1.5 or greater than 1.5. In some embodiments, the alginate is a high mannuronic acid (M) alginate, and comprises greater than about 50%, 55%, 60%, 65%, 70%, 75%, 80%, 85%, 90%, or more mannuronic acid (M). In some embodiments, the ratio of M:G is about 1. In some embodiments, the ratio of M:G is less than 1.

Each CBP recited in the present claims is attached to the alginate via an amino acid linker consisting essentially of one, two or three glycine residues, or one, two or three beta-alanine residues. In an embodiment, the linker is a single glycine residue attached to the N-terminus of the CBP. In an embodiment, the first CBS is a polymer covalently modified with an RGD peptide. In an embodiment, the first CBS is an alginate with a molecular weight of 75-150 kDa and a G:M ratio of greater than or equal to 1.5 and is covalently modified with a linker-CBP that consists essentially of GRGD (SEQ ID NO: 62) or GRGDSP (SEQ ID NO: 60).

In an embodiment, each CBP may further comprise a terminal capping group or protecting group on the free terminus of the CBP, e.g, the non-linker terminus of the CBP. A terminal capping group or protecting group may prevent the terminal amino acid residue from unwanted or unnecessary reactions and/or reduce or prevent degradation or modification of the CBP. Exemplary capping groups include alkyl groups, ethers, amides, and the like. In an embodiment, the linker-CBP comprises GRGD or GRGDSP and a terminal capping group. In an embodiment, the linker-CBP comprises GRGD or GRGDSP and does not comprise a terminal capping group.

In an embodiment, the encapsulating polymer composition in the cell-containing compartment may comprise one or more additional cell-binding substances, which may be a CBPP or a polymer covalently modified with a CBP. In an embodiment, the polymer composition also comprises a second CBP-alginate, e.g., an alginate covalently modified with a linker-CBP selected from the group consisting of G₍₁₋₃₎DGEA (SEQ ID NO: 66), G₍₁₋₃₎PHRSN (SEQ ID NO: 67), or G₍₁₋₃₎REDV (SEQ ID NO: 68). In an embodiment, the alginate in the second CBP-alginate also has a molecular weight of 75-150 kDa and a G:M ratio of greater than or equal to 1.5.

As described in the Examples herein below, it has been surprisingly discovered that when an RGD-modified alginate is used to encapsulate engineered RPE cells in the inner compartment of a two-compartment hydrogel capsule, the productivity of the encapsulated cells can be significantly impacted by the density of the RGD peptide in the modified alginate. Thus, in some embodiments, the density of a cell-binding peptide (e.g., RGD (SEQ ID NO: 43) or RGDSP (SEQ ID NO: 59)) in the CBP-polymer used to form the cell-containing compartment is (a) 0.10 % nitrogen (N) to 1.00 % N, about 0.20 % N to about 0.80 % N, about 0.30 % N to about 0.60% N, about 0.30% to about 0.50%, or 0.33 % N to 0.46% N, or (b) about 0.1 to about 1.0 micromoles of the CBP per g of the CBP-polymer (e.g., a MMW-alginate covalently modified with RGD (SEQ ID NO: 43) or RGDSP (SEQ ID NO: 59)) in solution as determined by a quantitative peptide conjugation assay, e.g., an assay described herein. In an embodiment, the encapsulated cells are engineered ARPE-19 cells. In an embodiment, the engineered ARPE-19 cells are stably-transfected cells or a monoclonal cell line derived from ATCC (CRL-2302). In an embodiment, the engineered ARPE-19 cells express a Factor VIII or a Factor IX protein. In some embodiments, in which the device comprises a mixture of different CBP-polymers in a cell-containing compartment, the total density of cell-binding peptide in the cell-containing compartment is 0.1 % nitrogen (N) to 1.00 % N, about 0.20 % N to about 0.80 % N, about 0.30 % N to about 0.60% N or about 0.30% N to about 0.50% N or about 0.1 to about 1.0 micromoles of the CBP per g of the CBP-polymer in solution as determined by a quantitative peptide conjugation assay, e.g., an assay described herein.

In an embodiment, the quantitative peptide conjugation assay includes subjecting a sample of a CPB-polymer to acid hydrolysis to generate individual amino acids from the conjugated peptide (and any residual unconjugated peptide in the CBP-polymer), quantitating the individual amino acids, averaging the molar concentration of each amino acid, and calculating the total peptide concentration in the sample. In an embodiment, the quantitative peptide conjugation assay is performed substantially similar to the process described in Example 25 herein. In an embodiment, the quantitative peptide conjugation assay also includes subtracting the concentration of any residual unconjugated peptide in the sample from the total peptide concentration. The concentration of unconjugated peptide in a CBP-polymer composition may be determined using any suitable assay known in the art, e.g., by LC-MS as described in Example 26 herein. Typically, the quantitative peptide conjugation assay is performed on a sample of a saline solution of the CBP-polymer that is used to prepare the device, but may also be performed on a lyophilized sample of the CBP-polymer.

Polymers may be covalently modified with a CBP using any of a variety of methods known in the art, see, e.g., Jeon O, et al., Biodegradable, photocrosslinked alginate hydrogels with independently tailorable physical properties and cell adhesivity. Tissue Eng. Part A 16:2915-2925 (2010); Rowley, J.A. et al., Biomaterials 20:45-53 (1999). For example, when the polymer to be modified is an alginate, the N-terminus of a peptide that consists essentially of an amino acid linker sequence (of one to several amino acids) and a cell binding amino acid sequence can be covalently attached to the alginate using an approach similar to that described in Example 1B herein below.

In addition to the polymer composition in the cell-containing compartment, the device (e.g., macrodevice, particle, hydrogel capsule) may comprise or be formed from materials such as metals, metallic alloys, ceramics, polymers, fibers, inert materials, and combinations thereof. A device may be completely made up of one type of material, or may comprise other materials within the cell-containing compartment and any other compartments.

The means for mitigating the FBR in the devices of the disclosure may comprise one or more of a variety of approaches known in the art.

For example, the means for mitigating the FBR in devices disclosed herein can comprise surrounding the cells with a semi-permeable biocompatible membrane having a pore size that is selected to allow oxygen and other molecules important to cell survival and function to move through the semi-permeable membrane while preventing immune cells from traversing through the pores. In an embodiment, the semi-permeable membrane has a molecular weight cutoff of less than 1000 kD or between 50-700 kD, 70-300 kD, or between 70-150 kD, or between 70 and 130 kD.

Another FBR-mitigating means comprises surrounding the cell-containing compartment with a barrier compartment formed from a cell-free biocompatible material, such as the core-shell microcapsules described in Ma, M et al., Adv. Healthc Mater., 2(5):667-672 (2012). Such a barrier compartment could be used with or without the semi-permeable member means. FBR-mitigating means can comprise disposing on or within the device an anti-inflammatory drug that is released from the implanted device to inhibit FBR, e.g., as described in US Patent No. 9,867,781. Other FBR-mitigating means employ a CSF-1R inhibitor that is disposed on the device surface or encapsulated within the device, as described in WO 2017/176792 and WO 2017/176804. Other FBR-mitigating means employ configuring the device in a spherical shape with a diameter of greater than 1 mm, as described in Veiseh, O., et al., Nature Materials 14:643-652 (2015). In some embodiments, the means for mitigating the FBR comprises disposing an afibrotic compound on the exterior surface of the device and / or within a barrier compartment surrounding the cell-containing compartment. Exemplary afibrotic compounds are described herein below. In some embodiments, the device can comprise combinations of two or more of the above FBR-mitigating means.

The hydrogel capsule recited in the present claims has a spherical shape. In other embodiments not recited in the present claims, the device (e.g., particle) can have any configuration and shape appropriate for supporting the viability and productivity of the encapsulated cells after implant into the intended target location. As non-limiting examples, device shapes not recited in the present claims are cylinders, rectangles, disks, ovoids, or stellates. The device can be comprised of a mesh-like or nested structure. In some embodiments, a device is capable of preventing materials over a certain size from passing through a pore or opening. In some embodiments, a device (e.g., particle) is capable of preventing materials greater than 50 kD, 75 kD, 100 kD, 125 kD, 150 kD, 175 kD, 200 kD, 250 kD, 300 kD, 400 kD, 500 kD, 750 kD, or 1,000 kD from passing through.

In an embodiment, the device is a macroencapsulation device. Nonlimiting examples of macrodevices are described in: US Patent Numbers 9,526,880, 9724430 and 8278106; European Patent No. EP742818B1, and Sang, S. and Roy, S., Biotechnol. Bioeng. 113(7):1381-1402 (2016).

In some embodiments, a device (e.g., particle) has a largest linear dimension (LLD), e.g., mean diameter, or size that is at least about 0.5 millimeter (mm), preferably about 1.0 mm, about 1.5 mm or greater. In some embodiments not recited in the present claims, a device can be as large as 10 mm in diameter or size. For example, a device or particle described herein is in a size range of 0.5 mm to 10 mm, 1 mm to 10 mm, 1 mm to 8 mm, 1 mm to 6 mm, 1 mm to 5 mm, 1 mm to 4 mm, 1 mm to 3 mm, 1 mm to 2 mm, 1 mm to 1.5 mm, 1.5 mm to 8 mm, 1.5 mm to 6 mm, 1.5 mm to 5 mm, 1.5 mm to 4 mm, 1.5 mm to 3 mm, 1.5 mm to 2 mm, 2 mm to 8 mm, 2 mm to 7 mm, 2 mm to 6 mm, 2 mm to 5 mm, 2 mm to 4 mm, 2 mm to 3 mm, 2.5 mm to 8 mm, 2.5 mm to 7 mm, 2.5 mm to 6 mm, 2.5 mm to 5 mm, 2.5 mm to 4 mm, 2.5 mm to 3 mm, 3 mm to 8 mm, 3 mm to 7 mm, 3 mm to 6 mm, 3 mm to 5 mm, 3 mm to 4 mm, 3.5 mm to 8 mm, 3.5 mm to 7 mm, 3.5 mm to 6 mm, 3.5 mm to 5 mm, 3.5 mm to 4 mm, 4 mm to 8 mm, 4 mm to 7 mm, 4 mm to 6 mm, 4 mm to 5 mm, 4.5 mm to 8 mm, 4.5 mm to 7 mm, 4.5 mm to 6 mm, 4.5 mm to 5 mm, 5 mm to 8 mm, 5 mm to 7 mm, 5 mm to 6 mm, 5.5 mm to 8 mm, 5.5 mm to 7 mm, 5.5 mm to 6 mm, 6 mm to 8 mm, 6 mm to 7 mm, 6.5 mm to 8 mm, 6.5 mm to 7 mm, 7 mm to 8 mm, or 7.5 mm to 8 mm.

The device may form part of a plurality of substantially the same devices in a preparation (e.g., composition). In some embodiments not recited in the present claims, the devices (e.g. particles, hydrogel capsules) in the preparation have a mean diameter or size between about 0.5 mm to about 8 mm. In some embodiments not recited in the present claims, the mean diameter or size of devices in the preparation is between about 0.5 mm to about 4 mm or between about 0.5 mm to about 2 mm. The devices in the preparation are two-compartment hydrogel capsules and have a mean diameter or size of about 0.7 mm to about 3 mm.

In some embodiments, the device has two hydrogel compartments, in which the inner, cell-containing compartment is completely surrounded by the second, outer (e.g., barrier) compartment. In an embodiment, the inner boundary of the second compartment forms an interface with the outer boundary of the first compartment, e.g., as illustrated in FIG. 1. In such embodiments, the thickness of the second (outer) compartment means the average distance between the outer boundary of the second compartment and the interface between the two compartments, e.g., the average of the distances measured at each of the thinnest and thickest points visually observed in the outer compartment. In some embodiments (e.g., the device is about 1.5 mm in diameter), the thinnest and thickest distances for the outer compartment are between 25 and 110 micrometers (µm) and between 270 and 480 µm, respectively. In some embodiments, the thickness of the outer compartment is greater than about 10 nanometers (nm), preferably 100 nm or greater and can be as large as 1 millimeter (mm). For example, the thickness (e.g., average distance) of the outer compartment in a hydrogel capsule device described herein may be 10 nm to 1 mm, 100 nm to 1mm, 500 nm to 1 millimeter, 1 micrometer (µm) to 1 mm, 1 µm to 1 mm, 1 µm to 500 µm, 1 µm to 250 µm, 1 µm to 1 mm, 5 µm to 500 µm, 5 µm to 250 µm, 10 µm to 1 mm, 10 µm to 500 µm, or 10 µm to 250 µm. In some embodiments, the thickness (e.g., average distance) of the outer compartment is 100 nm to 1 mm, between 1 µm and 1 mm, between 1 µm and 500 µm or between 5 µm and 1 mm. In some embodiments, the thickness (e.g., average distance) of the outer compartment is between about 50 µm and about 100 µm. In some embodiments (e.g., the device is about 1.5 mm in diameter), the thickness of the outer compartment (e.g., average distance) is between about 180 µm and 260 µm or between about 310 µm and 440 µm.

In some embodiments, a device of the disclosure (e.g., particle, capsule) comprises at least one pore or opening, e.g., to allow for the free flow of materials. In some embodiments, the mean pore size of a device is between about 0.1 µm to about 10 µm. For example, the mean pore size may be between 0.1 µm to 10 µm, 0.1 µm to 5 µm, 0.1 µm to 2 µm, 0.15 µm to 10 µm, 0.15 µm to 5 µm, 0.15 µm to 2 µm, 0.2 µm to 10 µm, 0.2 µm to 5 µm, 0.25 µm to 10 µm, 0.25 µm to 5 µm, 0.5 µm to 10 µm, 0.75 µm to 10 µm, 1 µm to 10 µm, 1 µm to 5 µm, 1 µm to 2 µm, 2 µm to 10 µm, 2 µm to 5 µm, or 5 µm to 10 µm. In some embodiments, the mean pore size of a device is between about 0.1 µm to 10 µm. In some embodiments, the mean pore size of a device is between about 0.1 µm to 5 µm. In some embodiments, the mean pore size of a device is between about 0.1 µm to 1 µm. In some embodiments of a two-compartment hydrogel capsule device, the mean pore size of the cell-containing inner compartment and the outer compartment is substantially the same. In some embodiments, the mean pore size of the inner compartment and the second compartment differ by about 1.5%, 2%, 5%, 7.5%, 10%, 15%, 20%, 25%, 30%, 35%, 40%, 45%, 50%, 55%, 60%, 65%, 70%, 75%, or more. In some embodiments, the mean pore size of the device (e.g., mean pore size of the first compartment and/or mean pore size of the second compartment) is dependent on a number of factors, such as the material(s) within each compartment and the presence and density of a compound of Formula (I).

In some embodiments, the device comprises a metal or a metallic alloy. In an embodiment, one or more of the compartments in the device (e.g., the first compartment, the second compartment, or all compartments) comprises a metal or a metallic alloy. Exemplary metallic or metallic alloys include comprising titanium and titanium group alloys (e.g., nitinol, nickel titanium alloys, thermo-memory alloy materials), platinum, platinum group alloys, stainless steel, tantalum, palladium, zirconium, niobium, molybdenum, nickel-chrome, chromium molybdenum alloys, or certain cobalt alloys (e.g., cobalt-chromium and cobalt-chromium-nickel alloys, e.g., ELGILOY^{®} and PHYNOX^{®}). For example, a metallic material may be stainless steel grade 316 (SS 316L) (comprised of Fe, <0.3% C, 16-18.5% Cr, 10-14% Ni, 2-3% Mo, <2% Mn, <1% Si, <0.45% P, and <0.03% S). In metal-containing devices, the amount of metal (e.g., by % weight, actual weight) can be at least 5%, e.g., at least 5%, 10%, 20%, 30%, 40%, 50%, 60%, 70%, 80%, 90%, 95%, 99%, or more, e.g., w/w; less than 20%, e.g., less than 20%, 15%, 10%, 5%, 1%, 0.5%, 0.1%, or less.

In some embodiments, the device comprises a ceramic. In an embodiment, one or more of the compartments in the device (e.g., the first compartment, the second compartment, or all compartments) comprises a ceramic. Exemplary ceramic materials include oxides, carbides, or nitrides of the transition elements, such as titanium oxides, hafnium oxides, iridium oxides, chromium oxides, aluminum oxides, and zirconium oxides. Silicon based materials, such as silica, may also be used. In ceramic-containing devices, the amount of ceramic (e.g., by % weight, actual weight) can be at least 5%, e.g., at least 5%, 10%, 20%, 30%, 40%, 50%, 60%, 70%, 80%, 90%, 95%, 99%, or more, e.g., w/w; less than 20%, e.g., less than 20%, 15%, 10%, 5%, 1%, 0.5%, 0.1%, or less.

In some embodiments, one or more compartments in a device comprises an afibrotic polymer, e.g., an afibrotic compound of Formula (I) covalently attached to a polymer that is the same or different than the polymer in the CBP-polymer. In an embodiment, some or all the monomers in the afibrotic polymer are modified with the same compound of Formula (I). In some embodiments, some or all the monomers in the afibrotic polymer are modified with different compounds of Formula (I). In some embodiments in which the device is a 2-compartment hydrogel capsule, the afibrotic polymer is present only in the outer, barrier compartment.

One or more compartments in a device may comprise an unmodified polymer that is the same or different than the polymer in the CBP-polymer and in any afibrotic polymer that is present in the device. In an embodiment, the first compartment, second compartment or all compartments in the device comprises the unmodified polymer.

Each of the modified and unmodified polymers in the device may be a linear, branched, or cross-linked polymer, or a polymer of selected molecular weight ranges, degree of polymerization, viscosity or melt flow rate. Branched polymers can include one or more of the following types: star polymers, comb polymers, brush polymers, dendronized polymers, ladders, and dendrimers. A polymer may be a thermoresponsive polymer, e.g., gel (e.g., becomes a solid or liquid upon exposure to heat or a certain temperature) or a photocrosslinkable polymer. Exemplary polymers include polystyrene, polyethylene, polypropylene, polyacetylene, poly(vinyl chloride) (PVC), polyolefin copolymers, poly(urethane)s, polyacrylates and polymethacrylates, polyacrylamides and polymethacrylamides, poly(methyl methacrylate), poly(2-hydroxyethyl methacrylate), polyesters, polysiloxanes, polydimethylsiloxane (PDMS), polyethers, poly(orthoester), poly(carbonates), poly(hydroxyalkanoate)s, polyfluorocarbons, PEEK^{®}, Teflon^{®} (polytetrafluoroethylene, PTFE), PEEK, silicones, epoxy resins, Kevlar^{®}, Dacron^{®} (a condensation polymer obtained from ethylene glycol and terephthalic acid), polyethylene glycol, nylon, polyalkenes, phenolic resins, natural and synthetic elastomers, adhesives and sealants, polyolefins, polysulfones, polyacrylonitrile, biopolymers such as polysaccharides and natural latex, collagen, cellulosic polymers (e.g., alkyl celluloses, etc.), polyethylene glycol and 2-hydroxyethyl methacrylate (HEMA), polysaccharides, poly(glycolic acid), poly(L-lactic acid) (PLLA), poly(lactic glycolic acid) (PLGA), a polydioxanone (PDA), or racemic poly(lactic acid), polycarbonates, (e.g., polyamides (e.g., nylon)), fluoroplastics, carbon fiber, agarose, alginate, chitosan, and blends or copolymers thereof. In polymer-containing devices, the amount of a polymer (e.g., by % weight of the device, actual weight of the polymer) can be at least 5%, e.g., at least 5%, 10%, 20%, 30%, 40%, 50%, 60%, 70%, 80%, 90%, 95%, 99%, or more, e.g., w/w; less than 20%, e.g., less than 20%, 15%, 10%, 5%, 1%, 0.5%, 0.1%, or less.

In some embodiments, one or more of the modified and unmodified polymers in the device comprises a polyethylene. Exemplary polyethylenes include ultra-low-density polyethylene (ULDPE) (e.g., with polymers with densities ranging from 0.890 to 0.905 g/cm³, containing comonomer); very-low-density polyethylene (VLDPE) (e.g., with polymers with densities ranging from 0.905 to 0.915 g/cm³, containing comonomer); linear low-density polyethylene (LLDPE) (e.g., with polymers with densities ranging from 0.915 to 0.935 g/cm³, contains comonomer); low-density polyethylene (LDPE) (e.g., with polymers with densities ranging from about 0.915 to 0.935 g/m³); medium density polyethylene (MDPE) (e.g., with polymers with densities ranging from 0.926 to 0.940 g/cm³, may or may not contain comonomer); high-density polyethylene (HDPE) (e.g., with polymers with densities ranging from 0.940 to 0.970 g/cm³, may or may not contain comonomer) and polyethylene glycol.

In some embodiments, one or more of the modified and unmodified polymers in the device comprises a polypropylene. Exemplary polypropylenes include homopolymers, random copolymers (homophasic copolymers), and impact copolymers (heterophasic copolymers), e.g., as described in McKeen, *Handbook of Polymer Applications in Medicine and Medical Devices, 3-*Plastics Used in Medical Devices, (2014):21-53, which is incorporated herein by reference in its entirety.

In some embodiments, one or more of the modified and unmodified polymers in the device comprises a polypropylene. Exemplary polystyrenes include general purpose or crystal (PS or GPPS), high impact (HIPS), and syndiotactic (SPS) polystyrene.

In some embodiments, one or more of the modified and unmodified polymers comprises a comprises a thermoplastic elastomer (TPE). Exemplary TPEs include (i) TPA-polyamide TPE, comprising a block copolymer of alternating hard and soft segments with amide chemical linkages in the hard blocks and ether and/or ester linkages in the soft blocks; (ii) TPC-co-polyester TPE, consisting of a block copolymer of alternating hard segments and soft segments, the chemical linkages in the main chain being ester and/or ether; (iii) TPO-olefinic TPE, consisting of a blend of a polyolefin and a conventional rubber, the rubber phase in the blend having little or no cross-linking; (iv) TPS-styrenic TPE, consisting of at least a triblock copolymer of styrene and a specific diene, where the two end blocks (hard blocks) are polystyrene and the internal block (soft block or blocks) is a polydiene or hydrogenated polydiene; (v) TPU-urethane TPE, consisting of a block copolymer of alternating hard and soft segments with urethane chemical linkages in the hard blocks and ether, ester or carbonate linkages or mixtures of them in the soft blocks; (vi) TPV-thermoplastic rubber vulcanizate consisting of a blend of a thermoplastic material and a conventional rubber in which the rubber has been cross-linked by the process of dynamic vulcanization during the blending and mixing step; and (vii) TPZ-unclassified TPE comprising any composition or structure other than those grouped in TPA, TPC, TPO, TPS, TPU, and TPV.

In some embodiments, the unmodified polymer is an unmodified alginate. In some embodiments, the alginate is a high guluronic acid (G) alginate, and comprises greater than about 50%, 55%, 60%, 65%, 70%, 75%, 80%, 85%, 90%, or more guluronic acid (G). In some embodiments, the alginate is a high mannuronic acid (M) alginate, and comprises greater than about 50%, 55%, 60%, 65%, 70%, 75%, 80%, 85%, 90%, or more mannuronic acid (M). In some embodiments, the ratio of M:G is about 1. In some embodiments, the ratio of M:G is less than 1. In some embodiments, the ratio of M:G is greater than 1. In an embodiment, the unmodified alginate has a molecular weight of 150 kDa - 250 kDa and a G:M ratio of ≥ 1.5.

In some embodiments, the afibrotic polymer comprises an alginate chemically modified with a Compound of Formula (I). The alginate in the afibrotic polymer may be the same or different than any unmodified alginate that is present in the device. In an embodiment, the density of the Compound of Formula (I) in the afibrotic alginate (e.g., amount of conjugation) is between about 4.0% and about 8.0%, between about 5.0% and about 7.0 %, or between about 6.0% and about 7.0 % nitrogen (e.g., as determined by combustion analysis for percent nitrogen). In an embodiment, the amount of Compound 101 produces an increase in % N (as compared with the unmodified alginate) of about 0.5% to 2% 2% to 4% N, about 4% to 6% N, about 6% to 8%, or about 8% to 10% N), where % N is determined by combustion analysis and corresponds to the amount of Compound 101 in the modified alginate.

In other embodiments, the density (e.g., concentration) of the Compound of Formula (I) (e.g., Compound 101) in the afibrotic alginate is defined as the % w/w, e.g., % of weight of amine / weight of afibrotic alginate in solution (e.g., saline) as determined by a suitable quantitative amine conjugation assay (e.g. by an assay described herein), and in certain embodiments, the density of a Compound of Formula (I) (e.g., Compound 101) is between about 1.0 % w/w and about 3.0 % w/w, between about 1.3 % w/w and about 2.5 % w/w or between about 1.5 % w/w and 2.2 % w/w. In an embodiment, the quantitative amine conjugation assay includes subjecting a sample of a chemically-modified polymer (e.g., an alginate modified with a Compound of Formula (I), e.g., CM-LMW-Alg-101) to acid hydrolysis to generate free amine and quantitating the total free amine in the sample. In an embodiment, the quantitative amine conjugation assay also includes subtracting the concentration of unconjugated amine (e.g., Compound of Formula (I)) in an unhydrolyzed sample from the total amine concentration. The quantitative amine conjugation assay is typically performed on a sample of a saline solution of the chemically-modified alginate used to prepare the device, but may also be performed on a lyophilized sample of the chemically-modified alginate. In an embodiment, the quantitative amine conjugation assay is performed substantially similar to the process described in Example 27 herein. In an embodiment, the Compound of Formula (I) is Compound 101 shown in Table 4.

In alginate-containing devices, the amount of modified and unmodified alginates (e.g., by % weight of the device, actual weight of the alginate) can be at least 5%, e.g., at least 5%, 10%, 20%, 30%, 40%, 50%, 60%, 70%, 80%, 90%, 95%, 99%, or more, e.g., w/w; less than 20%, e.g., less than 20%, 15%, 10%, 5%, 1%, 0.5%, 0.1%, or less.

The alginate in an afibrotic polymer can be chemically modified with a compound of Formula (I) using any suitable method known in the art. For example, the alginate carboxylic acid moiety can be activated for coupling to one or more amine-functionalized compounds to achieve an alginate modified with a compound of Formula (I). The alginate polymer may be dissolved in water (30 mL/gram polymer) and treated with 2-chloro-4,6-dimethoxy-1,3,5-triazine (0.5 eq) and N-methylmorpholine (1 eq). To this mixture may be added a solution of the compound of Formula (I) in acetonitrile (0.3M). The reaction may be warmed to 55 °C for 16h, then cooled to room temperature and gently concentrated via rotary evaporation, then the residue may be dissolved, e.g., in water. The mixture may then be filtered, e.g., through a bed of cyano-modified silica gel (Silicycle) and the filter cake washed with water. The resulting solution may then be dialyzed (10,000 MWCO membrane) against water for 24 hours, e.g., replacing the water twice. The resulting solution can be concentrated, e.g., via lyophilization, to afford the desired chemically modified alginate.

Each device described herein comprises at least one cell-containing compartment. In an embodiment, the device contains two, three, four or more cell-containing compartments. Each cell-containing compartment comprises a plurality of cells (e.g., live cells) and the cells in at least one of the compartments are capable of expressing a therapeutic agent when the device is implanted into a subject. In some embodiments, the therapeutic agent is a protein or polypeptide, e.g., an antibody, protein, enzyme, or growth factor).

In an embodiment, all the cells in a cell-containing compartment are derived from a single parental cell-type or a mixture of at least two different parental cell types. In an embodiment, all of the cells in a cell-containing compartment are derived from the same parental cell type, but a first plurality of the derived cells are engineered to express a first therapeutic agent, and a second plurality of the derived cells are engineered to express a second therapeutic agent. In devices with two or more cell-containing compartments, the cells and the therapeutic agent produced thereby may be the same or different in each cell-containing compartment. In some embodiments, all of the cell-containing compartments are surrounded by a single barrier compartment. In some embodiments, the barrier compartment is substantially cell-free.

In addition to therapeutic agent(s) expressed by the encapsulated cells, a device (e.g., capsule, particle) may comprise one or more exogenous agents that are not expressed by the cells, and may include, e.g., a nucleic acid (e.g., an RNA or DNA molecule), a protein (e.g., a hormone, an enzyme (e.g., glucose oxidase, kinase, phosphatase, oxygenase, hydrogenase, reductase) antibody, antibody fragment, antigen, or epitope)), small molecule, lipid, drug, vaccine, or any derivative thereof, a small-molecule, an active or inactive fragment of a protein or polypeptide. In an embodiment, the device is configured to release such an exogenous agent.

A device described herein may be provided as a preparation or composition for implantation or administration to a subject, i.e., a device preparation or device composition. In some embodiments, a device preparation or device composition comprises at least 2, 4, 8, 16, 32, 64 or more devices, and at least 20%, 30%, 35%, 40%, 45%, 50%, 55%, 60%, 65%, 70%, 75%, 80%, 85%, 90%, 95% or 100% of the devices in the preparation or composition have a characteristic as described herein, e.g., mean diameter or mean pore size or cell density.

In some embodiments, a device, device preparation or device composition targets or is designed for a certain system of the body, e.g. the nervous system (e.g., peripheral nervous system (PNS) or central nervous system (CNS)), vascular system, skeletal system, respiratory system, endocrine system, lymph system, reproductive system, or gastrointestinal tract. In some embodiments, a device, preparation or composition is targeted to the CNS. In some embodiments, a device, device preparation or device composition targets or is designed for a certain part of the body, e.g., blood, eye, brain, skin, lung, stomach, mouth, ear, leg, foot, hand, liver, heart, kidney, bone, pancreas, spleen, large intestine, small intestine, spinal cord, muscle, ovary, uterus, vagina, or penis.

A device, device preparation or device composition may be configured for implantation, or implanted or disposed into or onto any site of the body. In some embodiments, a device, device preparation or device composition is configured for implantation, implanted or disposed into the omentum of a subject, into the subcutaneous fat of a subject, or into the muscle tissue of a subject. A device, device composition or device preparation can be configured for implantation, or implanted, or disposed on or in the skin; a mucosal surface, a body cavity, the peritoneal cavity (e.g., the lesser sac); the central nervous system, e.g., the brain or spinal cord; an organ, e.g., the heart, liver, kidney, spleen, lung, lymphatic system, vasculature, the oral cavity, the nasal cavity, the teeth, the gums, the GI tract; bone; hip; fat tissue; muscle tissue; circulating blood; the eye (e.g., intraocular); breast, vagina; uterus, a joint, e.g., the knee or hip joint, or the spine.

In some embodiments, the device, device preparation or device composition is configured for implantation or implanted or disposed into the peritoneal cavity (e.g., the omentum). In some embodiments, the device is configured for implantation or implanted or disposed into or onto the lesser sac, also known as the omental bursa or bursalis omentum. The lesser sac refers to a cavity located in the abdomen formed by the omentum, and is in close proximity to, for example, the greater omentum, lesser omentum, stomach, small intestine, large intestine, liver, spleen, gastrosplenic ligament, adrenal glands, and pancreas. Typically, the lesser sac is connected to the greater sac via the omental foramen (i.e., the Foramen of Winslow). In some embodiments, the lesser sac comprises a high concentration of adipose tissue. A device, device preparation or device composition may be implanted in the peritoneal cavity (e.g., the omentum, e.g., the lesser sac) or disposed on a surface within the peritoneal cavity (e.g., omentum, e.g., lesser sac) via injection or catheter. Additional considerations for implantation or disposition of a device, device preparation or device composition into the omentum (e.g., the lesser sac) are provided in M. Pellicciaro et al. (2017) CellR4 5(3):e2410, which is incorporated herein by reference in its entirety.

In some embodiments, the device, device preparation or device composition is configured for implantation or implanted or disposed into the central nervous system (CNS), e.g., the brain or spinal cord and their corresponding tissues and cavities. In vertebrates, the CNS is contained within the dorsal body cavity, including the cranial cavity and the spinal canal. In some embodiments, the device, device composition or device preparation is configured for implantation or implanted or disposed into an intracerebral space, e.g., the intraparenchymal space, the intraventricular space, or the subdural space. A device, device composition or device preparation may be implanted in the CNS or disposed on a surface within the CNS through a hole made in the skull and delivered via injection or catheter.

In some embodiments, a device, device composition or device preparation is configured for implantation or implanted in or disposed into the eye. Exemplary regions suitable for implantation or disposition of the device include any surface or cavity within the eye, such as the retina, cornea, epithelium, aqueous humor, or vitreal space. In some embodiments, the device, device composition or device preparation is configured for implantation or implanted or disposed into the vitreal space. A device, device composition or devise preparation may be implanted in the eye or disposed on a surface within the eye through incision and/or injection.

In some embodiments, a device or device preparation is easily retrievable from a subject, e.g., without causing injury to the subject or without causing significant disruption of the surrounding tissue. In an embodiment, the device or device preparation can be retrieved with minimal or no surgical separation of the device(s) from surrounding tissue, e.g., via minimally invasive surgical approach, extraction, or resection.

A device, device composition or device preparation can be configured for a variety of exposures after implant into a mammalian recipient, including: limited exposure (e.g., less than 2 days, e.g., less than 2 days, 1 day, 24 hours, 20 hours, 16 hours, 12 hours, 10 hours, 8 hours, 6 hours, 5 hours, 4 hours, 3 hours, 2 hours, 1 hour or less); prolonged exposure (e.g., at least 2 days, 3 days, 4 days, 5 days, 6 days, 7 days, 1 week, 2 weeks, 3 weeks, 4 weeks, 5 weeks, 1 month, 2 months, 3 months, 4 months, 5 months, 6 months, 7 months, 8 months, 9 months, 10 months, 11 months, 12 months, 13 months, 14 months, 15 months, 16 months, 17 months, 18 months, 19 months, 20 months, 21 months, 22 months, 23 months, 24 months, 1 year, 1.5 years, 2 years, 2.5 years, 3 years, 3.5 years, 4 years or more) and permanent exposure (e.g., at least 6 months, 7 months, 8 months, 9 months, 10 months, 11 months, 12 months, 13 months, 14 months, 15 months, 16 months, 17 months, 18 months, 19 months, 20 months, 21 months, 22 months, 23 months, 24 months, 1 year, 1.5 years, 2 years, 2.5 years, 3 years, 3.5 years, 4 years or more).

In some embodiments, the device is not any capsule, device, implant or other object disclosed in any of WO2012/112982, WO2012/167223, WO2014/153126, WO2016/019391, WO2016/187225, US2012-0213708, US 2016-0030359, and US 2016-0030360.

### Small Molecule Compounds

In some embodiments, the devices described herein comprise at least one compound of Formula (I): 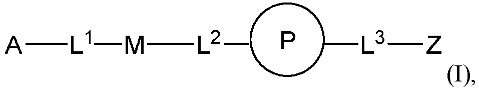 or a pharmaceutically acceptable salt thereof, wherein:
A is alkyl, alkenyl, alkynyl, heteroalkyl, cycloalkyl, heterocyclyl, aryl, heteroaryl, -O-, - C(O)O-, -C(O)-, -OC(O)-, -N(R^{C})-, -N(R^{C})C(O)-, -C(O)N(R^{C})-, -N(R^{C})C(O)(C₁-C₆-alkylene)-, -N(R^{C})C(O)(C₁-C₆-alkenylene)-, -N(R^{C})N(R^{D})-, -NCN-, -C(=N(R^{C})(R^{D}))O-, -S-, -S(O)ₓ-, -OS(O)ₓ-, -N(R^{C})S(O)ₓ-, -S(O)ₓN(R^{C})-, -P(R^{F})_{y}-, -Si(OR^{A})₂ -, -Si(R^{G})(OR^{A})-, - B(OR^{A})-, or a metal, each of which is optionally linked to an attachment group (e.g., an attachment group described herein) and is optionally substituted by one or more R¹;
each of L¹ and L³ is independently a bond, alkyl, or heteroalkyl, wherein each alkyl and heteroalkyl is optionally substituted by one or more R²;
L² is a bond;
M is absent, alkyl, heteroalkyl, cycloalkyl, heterocyclyl, aryl, or heteroaryl, each of which is optionally substituted by one or more R³;
P is absent, cycloalkyl, heterocycyl, or heteroaryl, each of which is optionally substituted by one or more R⁴;
Z is hydrogen, alkyl, alkenyl, alkynyl, heteroalkyl, -OR^{A}, -C(O)R^{A}, -C(O)OR^{A}, - C(O)N(R^{C})(R^{D}), -N(R^{C})C(O)R^{A}, cycloalkyl, heterocyclyl, aryl, or heteroaryl, wherein each alkyl, alkenyl, alkynyl, heteroalkyl, cycloalkyl, heterocyclyl, aryl, and heteroaryl is optionally substituted by one or more R⁵;
each R^{A}, R^{B}, R^{C}, R^{D}, R^{E}, R^{F}, and R^{G} is independently hydrogen, alkyl, alkenyl, alkynyl, heteroalkyl, halogen, azido, cycloalkyl, heterocyclyl, aryl, or heteroaryl, wherein each alkyl, alkenyl, alkynyl, heteroalkyl, cycloalkyl, heterocyclyl, aryl, and heteroaryl is optionally substituted with one or more R⁶;
or R^{C} and R^{D}, taken together with the nitrogen atom to which they are attached, form a ring (e.g., a 5-7 membered ring), optionally substituted with one or more R⁶;
each R¹, R², R³, R⁴, R⁵, and R⁶ is independently alkyl, alkenyl, alkynyl, heteroalkyl, halogen, cyano, azido, oxo, -OR^{A1}, -C(O)OR^{A1}, -C(O)R^{B1},-OC(O)R^{B1}, -N(R^{C1})(R^{D1}), ^{_}N(R^{C1})C(O)R^{B1}, -C(O)N(R^{C1}), SR^{E1}, S(O)ₓR^{E1}, -OS(O)ₓR^{E1}, -N(R^{C1})S(O)ₓR^{E1}, - S(O)ₓN(R^{C1})(R^{D1}), -P(R^{F1})_{y}, cycloalkyl, heterocyclyl, aryl, heteroaryl, wherein each alkyl, alkenyl, alkynyl, heteroalkyl, cycloalkyl, heterocyclyl, aryl, and heteroaryl is optionally substituted by one or more R⁷;
each R^{A1}, R^{B1}, R^{C1}, R^{D1}, R^{E1}, and R^{F1} is independently hydrogen, alkyl, alkenyl, alkynyl, heteroalkyl, cycloalkyl, heterocyclyl, aryl, or heteroaryl, wherein each alkyl, alkenyl, alkynyl, heteroalkyl, cycloalkyl, heterocyclyl, aryl, heteroaryl is optionally substituted by one or more R⁷;
each R⁷ is independently alkyl, alkenyl, alkynyl, heteroalkyl, halogen, cyano, oxo, hydroxyl, cycloalkyl, or heterocyclyl;
x is 1 or 2; and
y is 2, 3, or 4.
In some embodiments, the compound of Formula (I) is a compound of Formula (I-a): 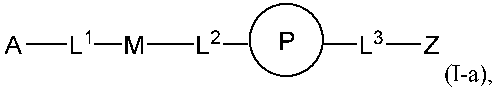 or a pharmaceutically acceptable salt thereof, wherein:
A is alkyl, alkenyl, alkynyl, heteroalkyl, cycloalkyl, heterocyclyl, aryl, heteroaryl, -O-, - C(O)O-, -C(O)-, -OC(O)-, -N(R^{C})-, -N(R^{C})C(O)-, -C(O)N(R^{C})-, -N(R^{C})N(R^{D})-, N(R^{C})C(O)(C₁-C₆- alkylene)-, -N(R^{C})C(O)(C₁-C₆-alkenylene)-, -NCN-, -C(=N(R^{C})(R^{D}))O-, - S-, -S(O)ₓ-, -OS(O)ₓ-, -N(R^{C})S(O)ₓ-, -S(O)ₓN(R^{C})-, -P(R^{F})_{y}-, -Si(OR^{A})₂ -, -Si(R^{G})(OR^{A})-, -B(OR^{A})-, or a metal, each of which is optionally linked to an attachment group (e.g., an attachment group described herein) and optionally substituted by one or more R¹;
each of L¹ and L³ is independently a bond, alkyl, or heteroalkyl, wherein each alkyl and heteroalkyl is optionally substituted by one or more R²;
L² is a bond;
M is absent, alkyl, heteroalkyl, cycloalkyl, heterocyclyl, aryl, or heteroaryl, each of which is optionally substituted by one or more R³;
P is heteroaryl optionally substituted by one or more R⁴;
Z is alkyl, alkenyl, alkynyl, heteroalkyl, cycloalkyl, heterocyclyl, aryl, or heteroaryl, each of which is optionally substituted by one or more R⁵;
each R^{A}, R^{B} , R^{C}, R^{D}, R^{E} , R^{F}, and R^{G} is independently hydrogen, alkyl, alkenyl, alkynyl, heteroalkyl, halogen, azido, cycloalkyl, heterocyclyl, aryl, or heteroaryl, wherein each alkyl, alkenyl, alkynyl, heteroalkyl, cycloalkyl, heterocyclyl, aryl, and heteroaryl is optionally substituted with one or more R⁶;
or R^{C} and R^{D}, taken together with the nitrogen atom to which they are attached, form a ring (e.g., a 5-7 membered ring), optionally substituted with one or more R⁶;
each R¹, R², R³, R⁴, R⁵, and R⁶ is independently alkyl, alkenyl, alkynyl, heteroalkyl, halogen, cyano, azido, oxo, -OR^{A1}, -C(O)OR^{A1}, -C(O)R^{B1},-OC(O)R^{B1}, -N(R^{C1})(R^{D1}), -N(R^{C1})C(O)R^{B1}, -C(O)N(R^{C1}), SR^{E1}, S(O)ₓR^{E1}, -OS(O)ₓR^{E1}, -N(R^{C1})S(O)ₓR^{E1}, - S(O)ₓN(R^{C1})(R^{D1}), -P(R^{F1})_{y}, cycloalkyl, heterocyclyl, aryl, heteroaryl, wherein each alkyl, alkenyl, alkynyl, heteroalkyl, cycloalkyl, heterocyclyl, aryl, and heteroaryl is optionally substituted by one or more R⁷;
each R^{A1}, R^{B1}, R^{C1}, R^{D1}, R^{E1}, and R^{F1} is independently hydrogen, alkyl, alkenyl, alkynyl, heteroalkyl, cycloalkyl, heterocyclyl, aryl, or heteroaryl, wherein each alkyl, alkenyl, alkynyl, heteroalkyl, cycloalkyl, heterocyclyl, aryl, heteroaryl is optionally substituted by one or more R⁷;
each R⁷ is independently alkyl, alkenyl, alkynyl, heteroalkyl, halogen, cyano, oxo, hydroxyl, cycloalkyl, or heterocyclyl;
x is 1 or 2; and
y is 2, 3, or 4.

In some embodiments, for Formulas (I) and (I-a), A is alkyl, alkenyl, alkynyl, heteroalkyl, cycloalkyl, heterocyclyl, aryl, heteroaryl, -O-, -C(O)O-, -C(O)-, -OC(O) -, -N(R^{C})C(O)-, - N(R^{C})C(O)(C₁-C₆-alkylene)-, -N(R^{C})C(O)(C₁-C₆-alkenylene)-, or -N(R^{C})-. In some embodiments, A is alkyl, alkenyl, alkynyl, heteroalkyl, cycloalkyl, heterocyclyl, aryl, heteroaryl, -O-, -C(O)O-, -C(O)-, -OC(O) -, or -N(R^{C})-. In some embodiments, A is alkyl, alkenyl, alkynyl, heteroalkyl,-O-, -C(O)O-, -C(O)-,-OC(O) -, or -N(R^{C})-. In some embodiments, A is alkyl, -O-, -C(O)O-, -C(O)-, -OC(O), or -N(R^{C})-. In some embodiments, A is -N(R^{C})C(O)-, -N(R^{C})C(O)(C₁-C₆-alkylene)-, or -N(R^{C})C(O)(C₁-C₆-alkenylene)-. In some embodiments, A is -N(R^{C})-. In some embodiments, A is -N(R^{C})-, and R^{C} an R^{D} is independently hydrogen or alkyl. In some embodiments, A is -NH-. In some embodiments, A is -N(R^{C})C(O)(C₁-C₆-alkylene)-, wherein alkylene is substituted with R¹. In some embodiments, A is -N(R^{C})C(O)(C₁-C₆-alkylene)-, and R¹ is alkyl (e.g., methyl). In some embodiments, A is -NHC(O)C(CH₃)₂-. In some embodiments, A is -N(R^{C})C(O)(methylene)-, and R¹ is alkyl (e.g., methyl). In some embodiments, A is -NHC(O)CH(CH₃)-. In some embodiments, A is -NHC(O)C(CH₃)-.

In some embodiments, for Formulas (I) and (I-a), L¹ is a bond, alkyl, or heteroalkyl. In some embodiments, L¹ is a bond or alkyl. In some embodiments, L¹ is a bond. In some embodiments, L¹ is alkyl. In some embodiments, L¹ is C₁-C₆ alkyl. I n some embodiments, L¹ is -CH₂-, - CH(CH₃)-, -CH₂CH₂CH₂, or -CH₂CH₂-. In some embodiments, L¹ is -CH₂-or -CH₂CH₂-.

In some embodiments, for Formulas (I) and (I-a), L³ is a bond, alkyl, or heteroalkyl. In some embodiments, L³ is a bond. In some embodiments, L³ is alkyl. In some embodiments, L³ is C₁-C₁₂ alkyl. In some embodiments, L³ is C₁-C₆ alkyl. In some embodiments, L³ is -CH₂-. In some embodiments, L³ is heteroalkyl. In some embodiments, L³ is C₁-C₁₂ heteroalkyl, optionally substituted with one or more R² (e.g., oxo). In some embodiments, L³ is C₁-C₆ heteroalkyl, optionally substituted with one or more R² (e.g., oxo). In some embodiments, L³ is -C(O)OCH₂-, -CH₂(OCH₂CH₂)₂-, -CH₂(OCH₂CH₂)₃-, CH₂CH₂O-, or -CH₂O-. In some embodiments, L³ is -CH₂O-.

In some embodiments, for Formulas (I) and (I-a), M is absent, alkyl, heteroalkyl, aryl, or heteroaryl. In some embodiments, M is heteroalkyl, aryl, or heteroaryl. In some embodiments, M is absent. In some embodiments, M is alkyl (e.g., C₁-C₆ alkyl). In some embodiments, M is - CH₂-. In some embodiments, M is heteroalkyl (e.g., C₁-C₆ heteroalkyl). In some embodiments, M is (-OCH₂CH₂-)z, wherein z is an integer selected from 1 to 10. In some embodiments, z is an integer selected from 1 to 5. In some embodiments, M is -OCH₂CH₂-, (-OCH₂CH₂-)₂, (-OCH₂CH₂-)₃, (-OCH₂CH₂-)₄, or (-OCH₂CH₂-)₅. In some embodiments, M is -OCH₂CH₂-, (-OCH₂CH₂-)₂, (-OCH₂CH₂-)₃, or (-OCH₂CH₂-)₄. In some embodiments, M is (-OCH₂CH₂-)₃. In some embodiments, M is aryl. In some embodiments, M is phenyl. In some embodiments, M is unsubstituted phenyl. In some embodiments, M is 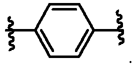 In some embodiments, M is phenyl substituted with R⁷ (e.g., 1 R⁷). In some embodiments, M is 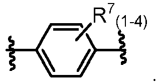 In some embodiments, R⁷ is CF₃.

In some embodiments, for Formulas (I) and (I-a), P is absent, heterocyclyl, or heteroaryl. In some embodiments, P is absent. In some embodiments, for Formulas (I) and (I-a), P is a tricyclic, bicyclic, or monocyclic heteroaryl. In some embodiments, P is a monocyclic heteroaryl. In some embodiments, P is a nitrogen-containing heteroaryl. In some embodiments, P is a monocyclic, nitrogen-containing heteroaryl. In some embodiments, P is a 5-membered heteroaryl. In some embodiments, P is a 5-membered nitrogen-containing heteroaryl. In some embodiments, P is tetrazolyl, imidazolyl, pyrazolyl, or triazolyl, pyrrolyl, oxazolyl, or thiazolyl. In some embodiments, P is tetrazolyl, imidazolyl, pyrazolyl, or triazolyl, or pyrrolyl. In some embodiments, P is imidazolyl. In some embodiments, P is 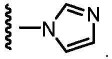 In some embodiments, P is triazolyl. In some embodiments, P is 1,2,3-triazolyl. In some embodiments, P is 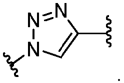 In some embodiments, P is heterocyclyl. In some embodiments, P is a 5-membered heterocyclyl or a 6-membered heterocyclyl. In some embodiments, P is imidazolidinonyl. In some embodiments, P is 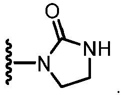 In some embodiments, P is thiomorpholinyl-1,1-dioxidyl. In some embodiments, P is 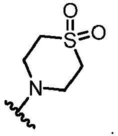 In some embodiments, for Formulas (I) and (I-a), Z is alkyl, heteroalkyl, cycloalkyl, heterocyclyl, aryl, or heteroaryl. In some embodiments, Z is heterocyclyl. In some embodiments, Z is monocyclic or bicyclic heterocyclyl. In some embodiments, Z is an oxygen-containing heterocyclyl. In some embodiments, Z is a 4-membered heterocyclyl, 5-membered heterocyclyl, or 6-membered heterocyclyl. In some embodiments, Z is a 6-membered heterocyclyl. In some embodiments, Z is a 6-membered oxygen-containing heterocyclyl. In some embodiments, Z is tetrahydropyranyl. In some embodiments, Z is 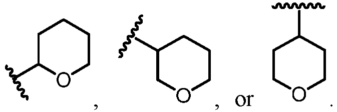 In some embodiments, Z is a 4-membered oxygen-containing heterocyclyl. In some embodiments, Z is

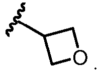

In some embodiments, Z is a bicyclic oxygen-containing heterocyclyl. In some embodiments, Z is phthalic anhydridyl. In some embodiments, Z is a sulfur-containing heterocyclyl. In some embodiments, Z is a 6-membered sulfur-containing heterocyclyl. In some embodiments, Z is a 6-membered heterocyclyl containing a nitrogen atom and a sulfur atom. In some embodiments, Z is thiomorpholinyl-1,1-dioxidyl. In some embodiments, Z is 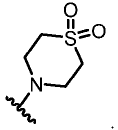 In some embodiments, Z is a nitrogen-containing heterocyclyl. In some embodiments, Z is a 6-membered nitrogen-containing heterocyclyl. In some embodiments, Z is

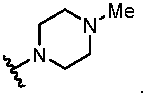

In some embodiments, Z is a bicyclic heterocyclyl. In some embodiments, Z is a bicyclic nitrogen-containing heterocyclyl, optionally substituted with one or more R⁵. In some embodiments, Z is 2-oxa-7-azaspiro[3.5]nonanyl. In some embodiments, Z is 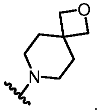 In some embodiments, Z is 1-oxa-3,8-diazaspiro[4.5]decan-2-one. In some embodiments, Z is

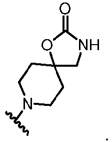

In some embodiments, for Formulas (I) and (I-a), Z is aryl. In some embodiments, Z is monocyclic aryl. In some embodiments, Z is phenyl. In some embodiments, Z is monosubstituted phenyl (e.g., with 1 R⁵). In some embodiments, Z is monosubstituted phenyl, wherein the 1 R⁵ is a nitrogen-containing group. In some embodiments, Z is monosubstituted phenyl, wherein the 1 R⁵ is NH₂. In some embodiments, Z is monosubstituted phenyl, wherein the 1 R⁵ is an oxygen-containing group. In some embodiments, Z is monosubstituted phenyl, wherein the 1 R⁵ is an oxygen-containing heteroalkyl. In some embodiments, Z is monosubstituted phenyl, wherein the 1 R⁵ is OCH₃. In some embodiments, Z is monosubstituted phenyl, wherein the 1 R⁵ is in the ortho position. In some embodiments, Z is monosubstituted phenyl, wherein the 1 R⁵ is in the meta position. In some embodiments, Z is monosubstituted phenyl, wherein the 1 R⁵ is in the para position.

In some embodiments, for Formulas (I) and (I-a), Z is alkyl. In some embodiments, Z is C₁-C₁₂ alkyl. In some embodiments, Z is C₁-C₁₀ alkyl. In some embodiments, Z is C₁-C₈ alkyl. In some embodiments, Z is C₁-C₈ alkyl substituted with 1-5 R⁵. In some embodiments, Z is C₁-C₈ alkyl substituted with 1 R⁵. In some embodiments, Z is C₁-C₈ alkyl substituted with 1 R⁵, wherein R⁵ is alkyl, heteroalkyl, halogen, oxo, -OR^{A1}, -C(O)OR^{A1}, -C(O)R^{B1},-OC(O)R^{B1}, or -N(R^{C1})(R^{D1}). In some embodiments, Z is C₁-C₈ alkyl substituted with 1 R⁵, wherein R⁵ is -OR^{A1} or -C(O)OR^{A1}. In some embodiments, Z is C₁-C₈ alkyl substituted with 1 R⁵, wherein R⁵ is -OR^{A1} or -C(O)OH. In some embodiments, Z is -CH₃.

In some embodiments, for Formulas (I) and (I-a), Z is heteroalkyl. I n some embodiments, Z is C₁-C₁₂ heteroalkyl. I n some embodiments, Z is C₁-C₁₀ heteroalkyl. In some embodiments, Z is C₁-C₈ heteroalkyl. I n some embodiments, Z is C₁-C₆ heteroalkyl. In some embodiments, Z is a nitrogen-containing heteroalkyl optionally substituted with one or more R⁵. I n some embodiments, Z is a nitrogen and sulfur-containing heteroalkyl substituted with 1-5 R⁵. In some embodiments, Z is N-methyl-2-(methylsulfonyl)ethan-1-aminyl.

In some embodiments, Z is -OR^{A} or -C(O)OR^{A}. In some embodiments, Z is -OR^{A} (e.g., -OH or -OCH₃). In some embodiments, Z is -OCH₃. In some embodiments, Z is -C(O)OR^{A} (e.g., -C(O)OH).

In some embodiments, Z is hydrogen.

In some embodiments, L² is a bond and P and L³ are independently absent. In some embodiments, L² is a bond, P is heteroaryl, L³ is a bond, and Z is hydrogen. In some embodiments, P is heteroaryl, L³ is heteroalkyl, and Z is alkyl.

In some embodiments, the compound of Formula (I) is a compound of Formula (I-b): 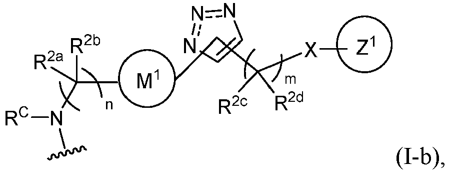 or a pharmaceutically acceptable salt thereof, wherein Ring M¹ is cycloalkyl, heterocyclyl, aryl, or heteroaryl, each of which is optionally substituted with 1-5 R³; Ring Z¹ is cycloalkyl, heterocyclyl, aryl or heteroaryl, optionally substituted with 1-5 R⁵; each of R^{2a}, R^{2b}, R^{2c}, and R^{2d} is independently hydrogen, alkyl, alkenyl, alkynyl, heteroalkyl, halo, cyano, nitro, amino, cycloalkyl, heterocyclyl, aryl, or heteroaryl, or each of R^{2a} and R^{2b} or R^{2c} and R^{2d} is taken together to form an oxo group; X is absent, N(R¹⁰)(R¹¹), O, or S; R^{C} is hydrogen, alkyl, alkenyl, alkynyl, heteroalkyl, cycloalkyl, heterocyclyl, aryl, or heteroaryl, wherein each of alkyl, alkenyl, alkynyl, heteroalkyl, cycloalkyl, heterocyclyl, aryl, or heteroaryl is optionally substituted with 1-6 R⁶; each R³, R⁵, and R⁶ is independently alkyl, alkenyl, alkynyl, heteroalkyl, halogen, cyano, azido, oxo, -OR^{A1}, -C(O)OR^{A1}, -C(O)R^{B1},-OC(O)R^{B1}, -N(R^{C1})(R^{D1}), -N(R^{C1})C(O)R^{B1}, - C(O)N(R^{C1}), SR^{E1}, cycloalkyl, heterocyclyl, aryl, or heteroaryl; each of R¹⁰ and R¹¹ is independently hydrogen, alkyl, alkenyl, alkynyl, heteroalkyl, -C(O)OR^{A1}, -C(O)R^{B1},-OC(O)R^{B1}, -C(O)N(R^{C1}), cycloalkyl, heterocyclyl, aryl, or heteroaryl; each R^{A1}, R^{B1}, R^{C1}, R^{D1} and R^{E1} is independently hydrogen, alkyl, alkenyl, alkynyl, heteroalkyl, cycloalkyl, heterocyclyl, aryl, heteroaryl, wherein each of alkyl, alkenyl, alkynyl, heteroalkyl, cycloalkyl, heterocyclyl, aryl, heteroaryl is optionally substituted with 1-6 R⁷; each R⁷ is independently alkyl, alkenyl, alkynyl, heteroalkyl, halogen, cyano, oxo, hydroxyl, cycloalkyl, or heterocyclyl; each m and n is independently 1, 2, 3, 4, 5, or 6; and " 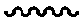 " refers to a connection to an attachment group or a polymer described herein. In some embodiments, for each R³ and R⁵, each alkyl, alkenyl, alkynyl, heteroalkyl, cycloalkyl, heterocyclyl, aryl, or heteroaryl is optionally and independently substituted with halogen, oxo, cyano, cycloalkyl, or heterocyclyl.

In some embodiments, the compound of Formula (I-b) is a compound of Formula (I-b-i): 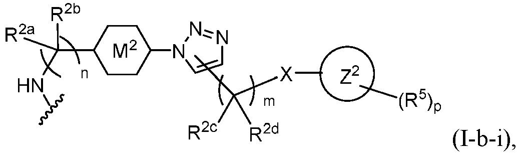 or a pharmaceutically acceptable salt thereof, wherein Ring M² is aryl or heteroaryl optionally substituted with one or more R³; Ring Z² is cycloalkyl, heterocyclyl, aryl, or heteroaryl; each of R^{2a}, R^{2b}, R^{2c}, and R^{2d} is independently hydrogen, alkyl, or heteroalkyl, or each of R^{2a} and R^{2b} or R^{2c} and R^{2d} is taken together to form an oxo group; X is absent, O, or S; each R³ and R⁵ is independently alkyl, heteroalkyl, halogen, oxo, -OR^{A1}, -C(O)OR^{A1}, or -C(O)R^{B1}, wherein each alkyl and heteroalkyl is optionally substituted with halogen; or two R⁵ are taken together to form a 5-6 membered ring fused to Ring Z²; each R^{A1} and R^{B1} is independently hydrogen, alkyl, or heteroalkyl; m and n are each independently 1, 2, 3, 4, 5, or 6; p is 0, 1, 2, 3, 4, 5, or 6; and " 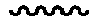 " refers to a connection to an attachment group or a polymer described herein.

In some embodiments, the compound of Formula (I-b-i) is a compound of Formula (I-b-ii): 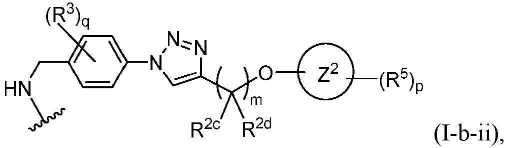 or a pharmaceutically acceptable salt thereof, wherein Ring Z² is cycloalkyl, heterocyclyl, aryl or heteroaryl; each of R^{2c} and R^{2d} is independently hydrogen, alkyl, or heteroalkyl, or R^{2c} and R^{2d} and taken together to form an oxo group; each R³ and R⁵ is independently alkyl, heteroalkyl, halogen, oxo, -OR^{A1}, -C(O)OR^{A1}, or -C(O)R^{B1}, wherein each alkyl and heteroalkyl is optionally substituted with halogen; each R^{A1} and R^{B1} is independently hydrogen, alkyl, or heteroalkyl; each of p and q is independently 0, 1, 2, 3, 4, 5, or 6; and " 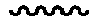 " refers to a connection to an attachment group or a polymer described herein.

In some embodiments, the compound of Formula (I) is a compound of Formula (I-c): 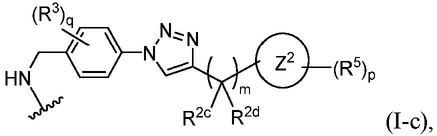 or a pharmaceutically acceptable salt thereof, wherein Ring Z² is cycloalkyl, heterocyclyl, aryl or heteroaryl; each of R^{2c} and R^{2d} is independently hydrogen, alkyl, or heteroalkyl, or R^{2c} and R^{2d} is taken together to form an oxo group; each R³ and R⁵ is independently alkyl, heteroalkyl, halogen, oxo, -OR^{A1}, -C(O)OR^{A1}, or -C(O)R^{B1}, wherein each alkyl and heteroalkyl is optionally substituted with halogen; each R^{A1} and R^{B1} is independently hydrogen, alkyl, or heteroalkyl; m is 1, 2, 3, 4, 5, or 6; each of p and q is independently 0, 1, 2, 3, 4, 5, or 6; and " 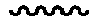 " refers to a connection to an attachment group or a polymer described herein.

In some embodiments, the compound of Formula (I) is a compound of Formula (I-d): 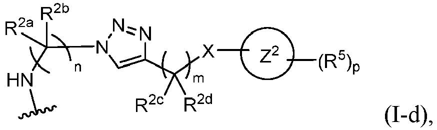 or a pharmaceutically acceptable salt thereof, wherein Ring Z² is cycloalkyl, heterocyclyl, aryl or heteroaryl; X is absent, O, or S; each of R^{2a}, R^{2b}, R^{2c}, and R^{2d} is independently hydrogen, alkyl, or heteroalkyl, or each of R^{2a} and R^{2b} or R^{2c} and R^{2d} is taken together to form an oxo group; each R⁵ is independently alkyl, heteroalkyl, halogen, oxo, -OR^{A1}, -C(O)OR^{A1}, or -C(O)R^{B1}, wherein each alkyl and heteroalkyl is optionally substituted with halogen; each R^{A1} and R^{B1} is independently hydrogen, alkyl, or heteroalkyl; each of m and n is independently 1, 2, 3, 4, 5, or 6; p is 0, 1, 2, 3, 4, 5, or 6; and " 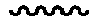 " refers to a connection to an attachment group or a polymer described herein.

In some embodiments, the compound of Formula (I) is a compound of Formula (I-e): 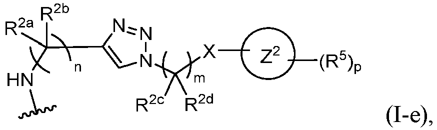 or a pharmaceutically acceptable salt thereof, wherein Ring Z² is cycloalkyl, heterocyclyl, aryl or heteroaryl; X is absent, O, or S; each of R^{2a}, R^{2b}, R^{2c}, and R^{2d} is independently hydrogen, alkyl, or heteroalkyl, or each of R^{2a} and R^{2b} or R^{2c} and R^{2d} is taken together to form an oxo group; each R⁵ is independently alkyl, heteroalkyl, halogen, oxo, -OR^{A1}, -C(O)OR^{A1}, or -C(O)R^{B1}; each R^{A1} and R^{B1} is independently hydrogen, alkyl, or heteroalkyl; each of m and n is independently 1, 2, 3, 4, 5, or 6; p is 0, 1, 2, 3, 4, 5, or 6; and " 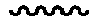 " refers to a connection to an attachment group or a polymer described herein.

In some embodiments, the compound of Formula (I) is a compound of Formula (I-f): 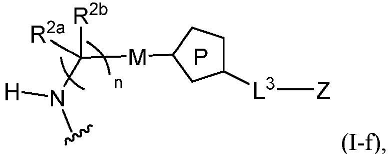 or a pharmaceutically acceptable salt thereof, wherein M is alkyl optionally substituted with one or more R³; Ring P is heteroaryl optionally substituted with one or more R⁴; L³ is alkyl or heteroalkyl optionally substituted with one or more R²; Z is alkyl, heteroalkyl, cycloalkyl, heterocyclyl, aryl, or heteroaryl, each of which is optionally substituted with one or more R⁵; each of R^{2a} and R^{2b} is independently hydrogen, alkyl, or heteroalkyl, or R^{2a} and R^{2b} is taken together to form an oxo group; each R², R³, R⁴, and R⁵ is independently alkyl, heteroalkyl, halogen, oxo, -OR^{A1}, -C(O)OR^{A1}, or -C(O)R^{B1}; each R^{A1} and R^{B1} is independently hydrogen, alkyl, or heteroalkyl; n is independently 1, 2, 3, 4, 5, or 6; and " 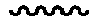 " refers to a connection to an attachment group or a polymer described herein.

In some embodiments, the compound of Formula (I) is a compound of Formula (II): 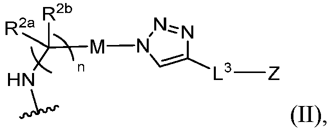 or a pharmaceutically acceptable salt thereof, wherein M is a bond, alkyl or aryl, wherein alkyl and aryl is optionally substituted with one or more R³; L³ is alkyl or heteroalkyl optionally substituted with one or more R²; Z is hydrogen, alkyl, heteroalkyl, cycloalkyl, heterocyclyl, aryl, heteroaryl or -OR^{A}, wherein alkyl, heteroalkyl, cycloalkyl, heterocyclyl, aryl, and heteroaryl is optionally substituted with one or more R⁵; R^{A} is hydrogen; each of R^{2a} and R^{2b} is independently hydrogen, alkyl, or heteroalkyl, or R^{2a} and R^{2b} is taken together to form an oxo group; each R², R³, and R⁵ is independently alkyl, heteroalkyl, halogen, oxo, -OR^{A1}, -C(O)OR^{A1}, or -C(O)R^{B1}; each R^{A1} and R^{B1} is independently hydrogen, alkyl, or heteroalkyl; n is independently 1, 2, 3, 4, 5, or 6; and " 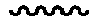 " refers to a connection to an attachment group or a polymer described herein.

In some embodiments, the compound of Formula (II) is a compound of Formula (II-a): 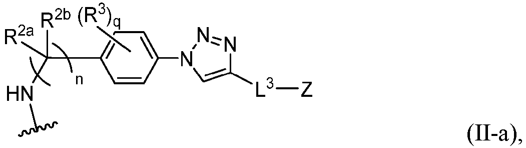 or a pharmaceutically acceptable salt thereof, wherein L³ is alkyl or heteroalkyl, each of which is optionally substituted with one or more R²; Z is hydrogen, alkyl, heteroalkyl, or -OR^{A}, wherein alkyl and heteroalkyl are optionally substituted with one or more R⁵; each of R^{2a} and R^{2b} is independently hydrogen, alkyl, or heteroalkyl, or R^{2a} and R^{2b} is taken together to form an oxo group; each R², R³, and R⁵ is independently alkyl, heteroalkyl, halogen, oxo, -OR^{A1}, - C(O)OR^{A1}, or -C(O)R^{B1}; R^{A} is hydrogen; each R^{A1} and R^{B1} is independently hydrogen, alkyl, or heteroalkyl; n is independently 1, 2, 3, 4, 5, or 6; and " 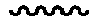 " refers to a connection to an attachment group or a polymer described herein.

In some embodiments, the compound of Formula (I) is a compound of Formula (III): 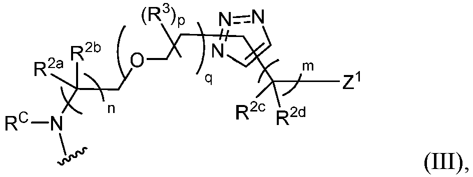 or a pharmaceutically acceptable salt thereof, wherein Z¹ is alkyl, alkenyl, alkynyl, heteroalkyl, cycloalkyl, heterocyclyl, aryl, or heteroaryl, each of which is optionally substituted with 1-5 R⁵; each of R^{2a} , R^{2b}, R^{2c}, and R^{2d} is independently hydrogen, alkyl, alkenyl, alkynyl, heteroalkyl, halo, cyano, nitro, amino, cycloalkyl, heterocyclyl, aryl, or heteroaryl; or R^{2a} and R^{2b} or R^{2c} and R^{2d} are taken together to form an oxo group; R^{C} is hydrogen, alkyl, alkenyl, alkynyl, or heteroalkyl, wherein each of alkyl, alkenyl, alkynyl, or heteroalkyl is optionally substituted with 1-6 R⁶; each of R³, R⁵, and R⁶ is independently alkyl, heteroalkyl, halogen, oxo, -OR^{A1}, - C(O)OR^{A1}, or -C(O)R^{B1}; each R^{A1} and R^{B1} is independently hydrogen, alkyl, or heteroalkyl; m and n are each independently 1, 2, 3, 4, 5, or 6; q is an integer from 0 to 25; and " 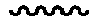 " refers to a connection to an attachment group or a polymer described herein.

In some embodiments, the compound of Formula (III) is a compound of Formula (III-a): 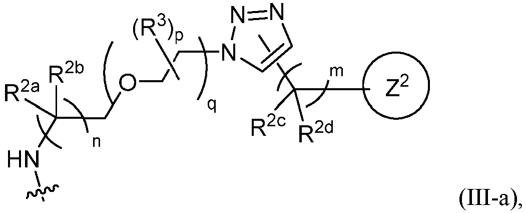 or a pharmaceutically acceptable salt thereof, wherein Ring Z² is cycloalkyl, heterocyclyl, aryl, or heteroaryl, each of which is optionally substituted with 1-5 R⁵; each of R^{2a} , R^{2b}, R^{2c}, and R^{2d} is independently hydrogen, alkyl, heteroalkyl, halo; or R^{2a} and R^{2b} or R^{2c} and R^{2d} are taken together to form an oxo group; each of R³ and R⁵ is independently alkyl, heteroalkyl, halogen, oxo, -OR^{A1}, -C(O)OR^{A1}, or -C(O)R^{B1}; each R^{A1} and R^{B1} is independently hydrogen, alkyl, or heteroalkyl; m and n are each independently 1, 2, 3, 4, 5, or 6; o and p are each independently 0, 1, 2, 3, 4, or 5; q is an integer from 0 to 25; and " 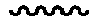 " refers to a connection to an attachment group or a polymer described herein.

In some embodiments, the compound of Formula (III-a) is a compound of Formula (III-b): 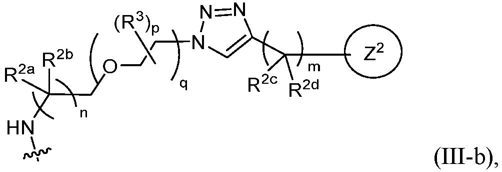 or a pharmaceutically acceptable salt thereof, wherein Ring Z² is cycloalkyl, heterocyclyl, aryl, or heteroaryl, each of which is optionally substituted with 1-5 R⁵; each of R^{2a} , R^{2b}, R^{2c}, and R^{2d} is independently hydrogen, alkyl, heteroalkyl, halo; or R^{2a} and R^{2b} or R^{2c} and R^{2d} are taken together to form an oxo group; each of R³ and R⁵ is independently alkyl, heteroalkyl, halogen, oxo, -OR^{A1}, -C(O)OR^{A1}, or -C(O)R^{B1}; each R^{A1} and R^{B1} is independently hydrogen, alkyl, or heteroalkyl; m and n are each independently 1, 2, 3, 4, 5, or 6; o and p are each independently 0, 1, 2, 3, 4, or 5; q is an integer from 0 to 25; and " 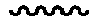 " refers to a connection to an attachment group or a polymer described herein.

In some embodiments, the compound of Formula (III-a) is a compound of Formula (III-c): 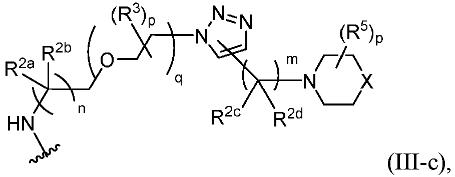 or a pharmaceutically acceptable salt thereof, wherein X is C(R')(R"), N(R'), or S(O)ₓ; each of R' and R" is independently hydrogen, alkyl, halogen, or cycloalkyl; each of R^{2a} , R^{2b}, R^{2c}, and R^{2d} is independently hydrogen, alkyl, heteroalkyl, or halo; or R^{2a} and R^{2b} or R^{2c} and R^{2d} are taken together to form an oxo group; each of R³ and R⁵ is independently alkyl, heteroalkyl, halogen, oxo, -OR^{A1}, -C(O)OR^{A1}, or -C(O)R^{B1}; each R^{A1} and R^{B1} is independently hydrogen, alkyl, or heteroalkyl; m and n are each independently 1, 2, 3, 4, 5, or 6; p is 0, 1, 2, 3, 4, or 5; q is an integer from 0 to 25; x is 0, 1, or 2; and " 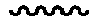 " refers to a connection to an attachment group or a polymer described herein.

In some embodiments, the compound of Formula (III-c) is a compound of Formula (III-d): 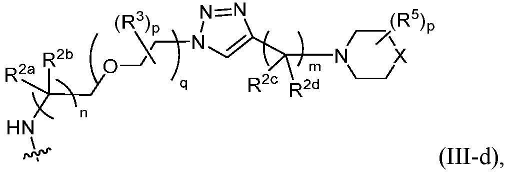 or a pharmaceutically acceptable salt thereof, wherein X is C(R')(R"), N(R'), or S(O)ₓ; each of R' and R" is independently hydrogen, alkyl, halogen, or cycloalkyl; each of R^{2a}, R^{2b}, R^{2c}, and R^{2d} is independently hydrogen, alkyl, heteroalkyl, or halo; or R^{2a} and R^{2b} or R^{2c} and R^{2d} are taken together to form an oxo group; each of R³ and R⁵ is independently alkyl, heteroalkyl, halogen, oxo, -OR^{A1}, -C(O)OR^{A1}, or -C(O)R^{B1}; each R^{A1} and R^{B1} is independently hydrogen, alkyl, or heteroalkyl; m and n are each independently 1, 2, 3, 4, 5, or 6; p is 0, 1, 2, 3, 4, or 5; q is an integer from 0 to 25; x is 0, 1, or 2; and " 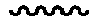 " refers to a connection to an attachment group or a polymer described herein.

In some embodiments, the compound is a compound of Formula (I). In some embodiments, L² is a bond and P and L³ are independently absent.

In some embodiments, the compound is a compound of Formula (I-a). In some embodiments of Formula (II-a), L² is a bond, P is heteroaryl, L³ is a bond, and Z is hydrogen. In some embodiments, P is heteroaryl, L³ is heteroalkyl, and Z is alkyl. In some embodiments, L² is a bond and P and L³ are independently absent. In some embodiments, L² is a bond, P is heteroaryl, L³ is a bond, and Z is hydrogen. In some embodiments, P is heteroaryl, L³ is heteroalkyl, and Z is alkyl.

In some embodiments, the compound is a compound of Formula (I-b). In some embodiments, P is absent, L¹ is -NHCH₂, L² is a bond, M is aryl (e.g., phenyl), L³ is -CH₂O, and Z is heterocyclyl (e.g., a nitrogen-containing heterocyclyl, e.g., thiomorpholinyl-1,1-dioxide). In some embodiments, the compound of Formula (I-b) is Compound 116.

In some embodiments of Formula (I-b), P is absent, L¹ is -NHCH₂, L² is a bond, M is absent, L³ is a bond, and Z is heterocyclyl (e.g., an oxygen-containing heterocyclyl, e.g., tetrahydropyranyl, tetrahydrofuranyl, oxetanyl, or oxiranyl). In some embodiments, the compound of Formula (I-b) is Compound 105.

In some embodiments, the compound is a compound of Formula (I-b-i). In some embodiments of Formula (I-b-i), each of R^{2a} and R^{2b} is independently hydrogen or CH₃, each of R^{2c} and R^{2d} is independently hydrogen, m is 1 or 2, n is 1, X is O, p is 0, M² is phenyl optionally substituted with one or more R³, R³ is -CF₃, and Z² is heterocyclyl (e.g., an oxygen-containing heterocyclyl, e.g., tetrahydropyranyl, tetrahydrofuranyl, oxetanyl, or oxiranyl). In some embodiments, the compound of Formula (I-b-i) is Compound 100, Compound 106, Compound 107, Compound 108, Compound 109, or Compound 111.

In some embodiments, the compound is a compound of Formula (I-b-ii). In some embodiments of Formula (I-b-ii), each of R^{2a}, R^{2b}, R^{2c}, and R^{2d} is independently hydrogen, q is 0, p is 0, m is 1, and Z² is heterocyclyl (e.g., an oxygen-containing heterocyclyl, e.g., tetrahydropyranyl). In some embodiments, the compound of Formula (I-b-ii) is Compound 100.

In some embodiments, the compound is a compound of Formula (I-c). In some embodiments of Formula (I-c), each of R^{2c} and R^{2d} is independently hydrogen, m is 1, p is 1, q is 0, R⁵ is -CH₃, and Z is heterocyclyl (e.g., a nitrogen-containing heterocyclyl, e.g., piperazinyl). In some embodiments, the compound of Formula (I-c) is Compound 113.

In some embodiments, the compound is a compound of Formula (I-d). In some embodiments of Formula (I-d), each of R^{2a}, R^{2b}, R^{2c}, and R^{2d} is independently hydrogen, m is 1, n is 3, X is O, p is 0, and Z is heterocyclyl (e.g., an oxygen-containing heterocyclyl, e.g., tetrahydropyranyl, tetrahydrofuranyl, oxetanyl, or oxiranyl). In some embodiments, the compound of Formula (I-d) is Compound 110 or Compound 114.

In some embodiments, the compound is a compound of Formula (I-f). In some embodiments of Formula (I-f), each of R^{2a} and R^{2b} is independently hydrogen, n is 1, M is -CH₂-, P is a nitrogen-containing heteroaryl (e.g., imidazolyl), L³ is -C(O)OCH₂-, and Z is CH₃. In some embodiments, the compound of Formula (I-f) is Compound 115.

In some embodiments, the compound is a compound of Formula (II-a). In some embodiments of Formula (II-a), each of R^{2a} and R^{2b} is independently hydrogen, n is 1, q is 0, L³ is -CH₂(OCH₂CH₂)₂, and Z is -OCH₃. In some embodiments, the compound of Formula (II-a) is Compound 112.

In some embodiments of Formula (II-a), each of R^{2a} and R^{2b} is independently hydrogen, n is 1, L³ is a bond or -CH₂, and Z is hydrogen or OH. In some embodiments, the compound of Formula (II-a) is Compound 103 or Compound 104.

In some embodiments, the compound is a compound of Formula (III). In some embodiments of Formula (III), each of R^{2a}, R^{2b}, R^{2c}, and R^{2d} is independently hydrogen, m is 1, n is 2, q is 3, p is 0, R^{C} is hydrogen, and Z¹ is heteroalkyl optionally substituted with R⁵ (e.g., - N(CH₃)(CH₂CH₂)S(O)₂CH₃). In some embodiments, the compound of Formula (III) is Compound 120.

In some embodiments, the compound is a compound of Formula (III-b). In some embodiments of Formula (III-b), each of R^{2a}, R^{2b}, R^{2c}, and R^{2d} is independently hydrogen, m is 0, n is 2, q is 3, p is 0, and Z² is aryl (e.g., phenyl) substituted with 1 R⁵ (e.g., -NH₂). In some embodiments, the compound of Formula (III-b) is Compound 102.

In some embodiments, the compound is a compound of Formula (III-b). In some embodiments of Formula (III-b), each of R^{2a}, R^{2b}, R^{2c}, and R^{2d} is independently hydrogen, m is 1, n is 2, q is 3, p is 0, R^{C} is hydrogen, and Z² is heterocyclyl (e.g., an nitrogen-containing heterocyclyl, e.g., a nitrogen-containing spiro heterocyclyl, e.g., 2-oxa-7-azaspiro[3.5]nonanyl). In some embodiments, the compound of Formula (III-b) is Compound 121.

In some embodiments, the compound is a compound of Formula (III-d). In some embodiments of Formula (III-d), each of R^{2a}, R^{2b}, R^{2c}, and R^{2d} is independently hydrogen, m is 1, n is 2, q is 1, 2, 3, or 4, p is 0, and X is S(O)₂. In some embodiments of Formula (III-d), each of R^{2a} and R^{2b} is independently hydrogen, m is 1, n is 2, q is 1, 2, 3, or 4, p is 0, and X is S(O)₂. In some embodiments, the compound of Formula (III-d) is Compound 101, Compound 117, Compound 118, or Compound 119.

In some embodiments, the compound is a compound of Formula (I-b), (I-d), or (I-e). In some embodiments, the compound is a compound of Formula (I-b), (I-d), or (II). In some embodiments, the compound is a compound of Formula (I-b), (I-d), or (I-f). In some embodiments, the compound is a compound of Formula (I-b), (I-d), or (III).

In some embodiments, the compound of Formula (I) is not a compound disclosed in WO2012/112982, WO2012/167223, WO2014/153126, WO2016/019391, WO 2017/075630, US2012-0213708, US 2016-0030359 or US 2016-0030360.

In some embodiments, the compound of Formula (I) comprises a compound shown in Table 4, or a pharmaceutically acceptable salt thereof. In some embodiments, the exterior surface and / or one or more compartments within a device described herein comprises a small molecule compound shown in Table 4, or a pharmaceutically acceptable salt thereof.

**Table 4: Exemplary small molecule compounds**

| **Compound No.** | **Structure** |
|---|---|
| 100 | 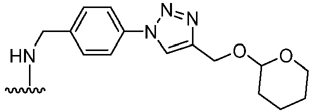 |
| 101 | 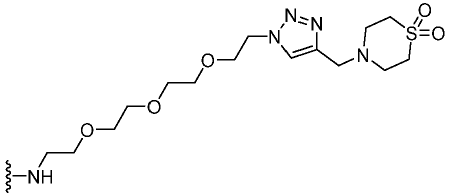 |
| 102 | 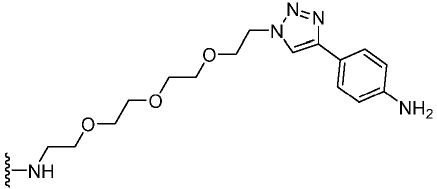 |
| 103 | 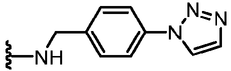 |
| 104 | 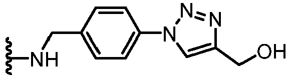 |
| 105 | 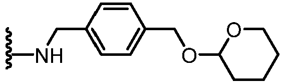 |
| 106 | 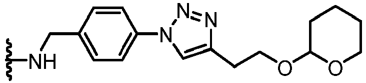 |
| 107 | 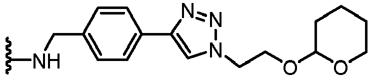 |
| 108 | 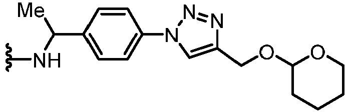 |
| 109 | 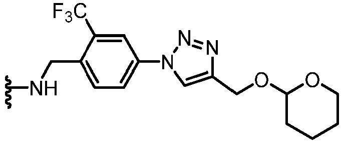 |
| 110 | 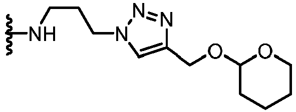 |
| 111 | 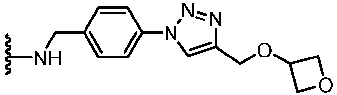 |
| 112 | 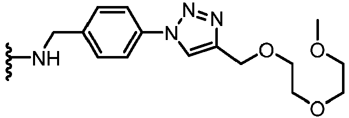 |
| 113 | 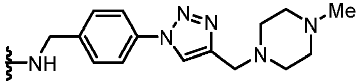 |
| 114 | 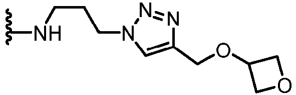 |
| 115 | 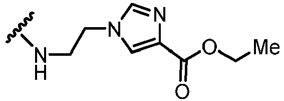 |
| 116 | 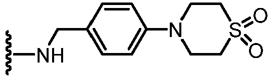 |
| 117 | 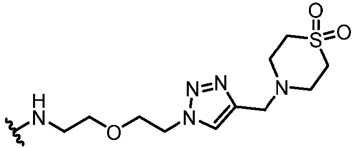 |
| 118 | 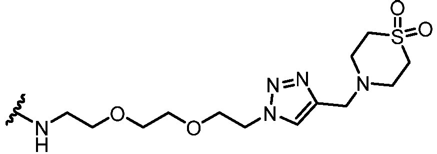 |
| 119 | 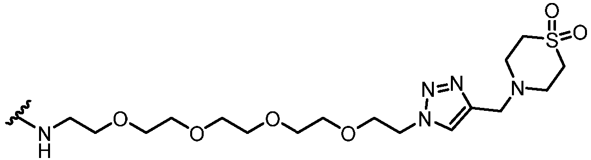 |
| 120 | 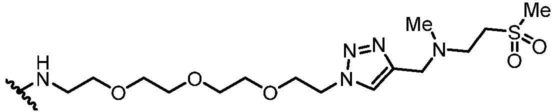 |
| 121 | 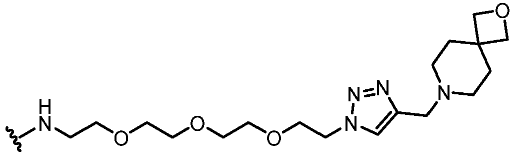 |

In some embodiments, the compound is a compound of Formula (I) (e.g., Formulas (I-a), (I-b), (I-c), (I-d), (I-e), (I-f), (II), (II-a), (III), (III-a), (III-b), (III-c), or (III-d)), or a pharmaceutically acceptable salt thereof and is selected from: 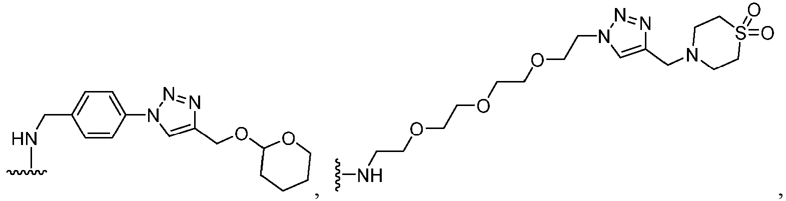 and 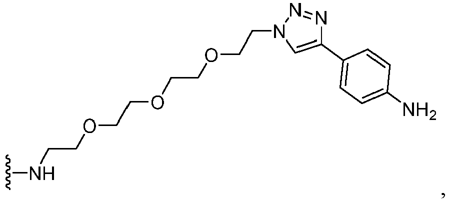 or a pharmaceutically acceptable salt thereof.

In some embodiments, the device described herein comprises the compound of 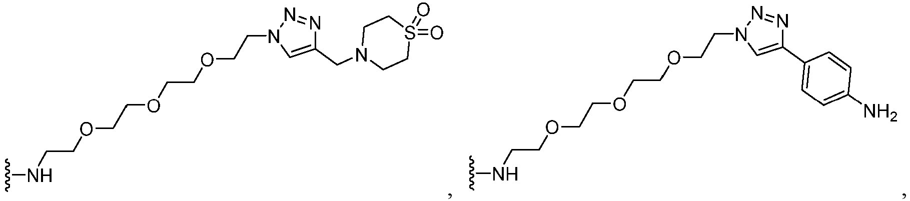 or a pharmaceutically acceptable salt of either compound.

In some embodiments, a compound of Formula (I) (e.g., Compound 101 in Table 4) is covalently attached to an alginate (e.g., an alginate with approximate MW < 75 kDa, G:M ratio ≥ 1.5) at a conjugation density of at least 2.0 % and less than 9.0 %, or 3.0 % to 8.0 %, 4.0-7.0, 5.0 to 7.0, or 6.0 to 7.0 or about 6.8 as determined by combustion analysis for percent nitrogen as described in the Examples below.

### Cells

The devices of the present disclosure comprise at least one cell-containing compartment that comprises a plurality of cells (e.g., live cells). The cells may be a variety of different cell types (e.g., human cells), including epithelial cells, endothelial cells, fibroblast cells, mesenchymal stem cells, keratinocyte cells and islet cells. Exemplary cell types include the cell types recited in WO 2017/075631. In some embodiments, the cells are derived from a cell-line shown in Table 5 below.

**Table 5: Exemplary cell lines**

| **Cell Line** | **Cell Type** | **Germ Layer** | **Commercial Source** |
|---|---|---|---|
| ARPE-19 | Epithelial (Retinal) | Ectoderm | ATCC (CRL-2302) |
| BJ | Fibroblast (Foreskin) | Ectoderm | ATCC (CRL-2522) |
| CCD-841-CoN | Epithelial (Colon) | Endoderm | ATCC (CRL-1790) |
| HaCat | Keratinocyte | Ectoderm | Addexbio (T0020001) |
| HHSEC | Endothelial (Hepatic Sinusoidal) | Endoderm | Sciencellonline.com (#5000) |
| Huv-EC-C | Endothelial (Embryonic umbilical) | Mesoderm | ATCC (CRL-1730) |
| MCF-10A | Epithelial (Mammary Gland) | Ectoderm | ATCC (CRL-10317) |
| MRC-5 | Fibroblast (Lung) | Mesoderm | ATCC (CCL-171) |
| MSC, human | Mesenchyme (Bone Marrow) | Mesoderm | ATCC (PCS-500-012) |
| MSC, mouse | Mesenchyme (Bone Marrow) | Mesoderm | Cyagen (MU BMX-01001) |
| WS-1 | Fibroblast (Skin) | Ectoderm | ATCC (CRL-1502) |
| 293F | Epithelial (Embryonic Kidney) | Mesoderm | Thermo Fisher (R790007) |

In some embodiments, the device does not comprise any islet cells, as defined herein. In an embodiment, cells contained in a device of the disclosure, e.g., RPE cells, MSFCs, including engineered RPE cells and MSFCs, have one or more of the following characteristics: (i) are not capable of producing insulin (e.g., insulin A-chain, insulin B-chain, or proinsulin) in an amount effective to treat diabetes or another disease or condition that may be treated with insulin; (ii) not capable of producing insulin in a glucose-responsive manner; or (iii) not derived from an induced pluripotent stem cell that was engineered or differentiated into insulin-producing pancreatic beta cells.

In an embodiment, the plurality of cells is in the form of a cell suspension prior to being encapsulated within a device described herein, e.g., a hydrogel capsule. The cells in the suspension may take the form of single cells (e.g., from a monolayer cell culture), or provided in another form, e.g., disposed on a microcarrier (e.g., a bead or matrix) or as a three-dimensional aggregate of cells (e.g., a cell cluster or spheroid). The cell suspension can comprise multiple cell clusters (e.g., as spheroids) or microcarriers.

In some embodiments, the cells in the plurality have been engineered to produce a therapeutic agent. In an embodiment, the therapeutic agent is for the prevention or treatment of a disease, disorder, or condition, e.g., those described in WO 2017/075631. The therapeutic agent may be any biological substance, such as a nucleic acid (e.g., a nucleotide, DNA, or RNA), a polypeptide, a lipid, a sugar (e.g., a monosaccharide, disaccharide, oligosaccharide, or polysaccharide), or a small molecule. Exemplary therapeutic agents include the agents listed in WO 2017/075631.

In some embodiments, the therapeutic agent is a peptide or polypeptide (e.g., a protein), such as a hormone, enzyme, cytokine (e.g., a pro-inflammatory cytokine or an anti-inflammatory cytokine), growth factor, clotting factor, or lipoprotein. A peptide or polypeptide (e.g., a protein, e.g., a hormone, growth factor, clotting factor or coagulation factor, antibody molecule, enzyme, cytokine, cytokine receptor, or a chimeric protein including cytokines or a cytokine receptor) produced by an engineered cell can have a naturally occurring amino acid sequence, or may contain a variant of the naturally occurring sequence. The variant can be a naturally occurring or non-naturally occurring amino acid substitution, mutation, deletion or addition relative to the reference sequence, e.g., a naturally occurring sequence. The naturally occurring amino acid sequence may be a polymorphic variant. The naturally occurring amino acid sequence can be a human or a non-human amino acid sequence. In some embodiments, the naturally occurring amino acid sequence or naturally occurring variant thereof is a human sequence. In addition, a peptide or polypeptide (e.g., a protein) for use with the present disclosure may be modified in some way, e.g., via chemical or enzymatic modification (e.g., glycosylation, phosphorylation). In some embodiments, the peptide has about 2, 3, 4, 5, 6, 7, 8, 9, 10, 12, 14, 16, 18, 20, 25, 30, 35, 40, 45, or less than 50 amino acids. In some embodiments, the protein has an average molecular weight of 5 kD, 10 kD, 25 kD, 50 kD, 100 kD, 150 kD, 200 kD, 250 kD, 500 kD, or more.

In some embodiments, the protein is a hormone. Exemplary hormones include anti-diuretic hormone (ADH), oxytocin, growth hormone (GH), prolactin, growth hormone-releasing hormone (GHRH), thyroid stimulating hormone (TSH), thyrotropin-release hormone (TRH), adrenocorticotropic hormone (ACTH), follicle-stimulating hormone (FSH), luteinizing hormone (LH), luteinizing hormone-releasing hormone (LHRH), thyroxine, calcitonin, parathyroid hormone (PTH), aldosterone, cortisol, epinephrine, glucagon, insulin, estrogen, progesterone, and testosterone. In some embodiments, the protein is insulin (e.g., insulin A-chain, insulin B-chain, or proinsulin). In some embodiments, the protein is a growth hormone, such as human growth hormone (hGH), recombinant human growth hormone (rhGH), bovine growth hormone, methionine-human growth hormone, des-phenylalanine human growth hormone, and porcine growth hormone.

In some embodiments, the protein is a growth factor, e.g., vascular endothelial growth factor (VEGF), nerve growth factor (NGF), platelet-derived growth factor (PDGF), fibroblast growth factor (FGF), epidermal growth factor (EGF), transforming growth factor (TGF), and insulin-like growth factor-I and -II (IGF-I and IGF-II).

In some embodiments, the protein is a clotting factor or a coagulation factor, e.g., a blood clotting factor or a blood coagulation factor. In some embodiments, the protein is a protein involved in coagulation, i.e., the process by which blood is converted from a liquid to solid or gel. Exemplary clotting factors and coagulation factors include Factor I (e.g., fibrinogen), Factor II (e.g., prothrombin), Factor III (e.g., tissue factor), Factor V (e.g., proaccelerin, labile factor), Factor VI, Factor VII (e.g., stable factor, proconvertin), Factor VIII (e.g., antihemophilic factor A), Factor VIIIC, Factor IX (e.g., antihemophilic factor B), Factor X (e.g., Stuart-Prower factor), Factor XI (e.g., plasma thromboplastin antecedent), Factor XII (e.g., Hagerman factor), Factor XIII (e.g., fibrin-stabilizing factor), von Willebrand factor (vWF), prekallikrein, heparin cofactor II, high molecular weight kininogen (e.g., Fitzgerald factor), antithrombin III, and fibronectin. In some embodiments, the protein is an anti-clotting factor, such as Protein C.

In some embodiments, the protein is an antibody molecule. As used herein, the term "antibody molecule" refers to a protein, e.g., an immunoglobulin chain or fragment thereof, comprising at least one immunoglobulin variable domain sequence. The term "antibody molecule" includes, for example, a monoclonal antibody (including a full-length antibody which has an immunoglobulin Fc region). In an embodiment, an antibody molecule comprises a full-length antibody, or a full-length immunoglobulin chain. In an embodiment, an antibody molecule comprises an antigen binding or functional fragment of a full-length antibody, or a full-length immunoglobulin chain. In an embodiment, an antibody molecule is a monospecific antibody molecule and binds a single epitope, e.g., a monospecific antibody molecule having a plurality of immunoglobulin variable domain sequences, each of which binds the same epitope. In an embodiment, an antibody molecule is a multispecific antibody molecule, e.g., it comprises a plurality of immunoglobulin variable domains sequences, wherein a first immunoglobulin variable domain sequence of the plurality has binding specificity for a first epitope and a second immunoglobulin variable domain sequence of the plurality has binding specificity for a second epitope. In an embodiment, the first and second epitopes are on the same antigen, e.g., the same protein (or subunit of a multimeric protein). In an embodiment, a multispecific antibody molecule comprises a third, fourth or fifth immunoglobulin variable domain. In an embodiment, a multispecific antibody molecule is a bispecific antibody molecule, a trispecific antibody molecule, or tetraspecific antibody molecule.

Various types of antibody molecules may be produced by the encapsulated engineered cells, including whole immunoglobulins of any class, fragments thereof, and synthetic proteins containing at least the antigen binding variable domain of an antibody. The antibody molecule can be an antibody, e.g., an IgG antibody, such as IgG₁, IgG₂, IgG₃, or IgG₄. An antibody molecule can be in the form of an antigen binding fragment including a Fab fragment, F(ab')2 fragment, a single chain variable region, and the like. Antibodies can be polyclonal or monoclonal (mAb). Monoclonal antibodies may include "chimeric" antibodies in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they specifically bind the target antigen and/or exhibit the desired biological activity. In some embodiments, the antibody molecule is a single-domain antibody (e.g., a nanobody). The described antibodies can also be modified by recombinant means, for example by deletions, additions or substitutions of amino acids, to increase efficacy of the antibody in mediating the desired function. Exemplary antibodies include anti-beta-galactosidase, anti-collagen, anti-CD14, anti-CD20, anti-CD40, anti-HER2, anti-IL-1, anti-IL-4, anti-IL6, anti-IL-13, anti-IL17, anti-IL18, anti-IL-23, anti-IL-28, anti-IL-29, anti-IL-33, anti-EGFR, anti-VEGF, anti-CDF, anti-flagellin, anti-IFN-α, anti-IFN-β, anti-IFN-γ, anti-mannose receptor, anti-VEGFA, anti-TLR1, anti-TLR2, anti-TLR3, anti-TLR4, anti-TLR5, anti-TLR6, anti-TLR9, anti-PDF, anti-PD1 (also known as anti-PD-1), anti-PD-L1, or anti-nerve growth factor antibody. In some embodiments, the antibody is an anti-nerve growth factor antibody (e.g., fulranumab, fasinumab, tanezumab).

In some embodiments, the protein is a cytokine or a cytokine receptor, or a chimeric protein including cytokines or their receptors, including, for example tumor necrosis factor alpha and beta, their receptors and their derivatives, renin; lipoproteins; colchicine; corticotrophin; vasopressin; somatostatin; lypressin; pancreozymin; leuprolide; alpha-1-antitrypsin; atrial natriuretic factor; lung surfactant; a plasminogen activator other than a tissue-type plasminogen activator (t-PA), for example a urokinase; bombesin; thrombin; enkephalinase; RANTES (regulated on activation normally T-cell expressed and secreted); human macrophage inflammatory protein (MIP-1-alpha); a serum albumin such as human serum albumin; mullerian-inhibiting substance; relaxin A-chain; relaxin B-chain; prorelaxin; mouse gonadotropin-associated peptide; chorionic gonadotropin; a microbial protein, such as beta-lactamase; DNase; inhibin; activin; receptors for hormones or growth factors; integrin; protein A or D; rheumatoid factors; platelet-derived growth factor (PDGF); epidermal growth factor (EGF); transforming growth factor (TGF) such as TGF-α and TGF-β, including TGF-β1, TGF-β2, TGF-β3, TGF-β4, or TGF-β5; insulin-like growth factor-I and -II (IGF-I and IGF-II); des(1-3)-IGF-I (brain IGF-I), insulin-like growth factor binding proteins; CD proteins such as CD-3, CD-4, CD-8, and CD-19; erythropoietin; osteoinductive factors; immunotoxins; an interferon such as interferon-alpha (e.g., interferon.alpha.2A), -beta, - gamma, -lambda and consensus interferon; colony stimulating factors (CSFs), e.g., M-CSF, GM-CSF, and G-CSF; interleukins (ILs), e.g., IL-1, IL-2 to IL-10; superoxide dismutase; T-cell receptors; surface membrane proteins; decay accelerating factor; transport proteins; homing receptors; addressins; fertility inhibitors such as the prostaglandins; fertility promoters; regulatory proteins; antibodies (including fragments thereof) and chimeric proteins, such as immunoadhesins; precursors, derivatives, prodrugs and analogues of these compounds, and pharmaceutically acceptable salts of these compounds, or their precursors, derivatives, prodrugs and analogues. Suitable proteins or peptides may be native or recombinant and include, e.g., fusion proteins.

Examples of a polypeptide (e.g., a protein) produced by devices described herein also include CCL1, CCL2 (MCP-1), CCL3 (MIP-1α), CCL4 (MIP-1β), CCL5 (RANTES), CCL6, CCL7, CCL8, CCL9 (CCL10), CCL11, CCL12, CCL13, CCL14, CCL15, CCL16, CCL17, CCL18, CCL19, CCL20, CCL21, CCL22, CCL23, CCL24, CCL25, CCL26, CCL27, CCL28, CXCL1 (KC), CXCL2 (SDF1a), CXCL3, CXCL4, CXCL5, CXCL6, CXCL7, CXCL8 (IL8), CXCL9, CXCL10, CXCL11, CXCL12, CXCL13, CXCL14, CXCL15, CXCL16, CXCL17, CX3CL1, XCL1, XCL2, TNFA, TNFB (LTA), TNFC (LTB), TNFSF4, TNFSF5 (CD40LG), TNFSF6, TNFSF7, TNFSF8, TNFSF9, TNFSF10, TNFSF11, TNFSF13B, EDA, IL2, IL15, IL4, IL13, IL7, IL9, IL21, IL3, IL5, IL6, IL11, IL27, IL30, IL31, OSM, LIF, CNTF, CTF1, IL12a, IL12b, IL23, IL27, IL35, IL14, IL16, IL32, IL34, IL10, IL22, IL19, IL20, IL24, IL26, IL29, IFNL1, IFNL2, IFNL3, IL28, IFNA1, IFNA2, IFNA4, IFNA5, IFNA6, IFNA7, IFNA8, IFNA10, IFNA13, IFNA14, IFNA16, IFNA17, IFNA21, IFNB1, IFNK, IFNW1, IFNG, IL1A (IL1F1), IL1B (IL1F2), IL1Ra (IL1F3), IL1F5 (IL36RN), IL1F6 (IL36A), IL1F7 (IL37), IL1F8 (IL36B), IL1F9 (IL36G), IL1F10 (IL38), IL33 (IL1F11), IL18 (IL1G), IL17, KITLG, IL25 (IL17E), CSF1 (M-CSF), CSF2 (GM-CSF), CSF3 (G-CSF), SPP1, TGFB1, TGFB2, TGFB3, CCL3L1, CCL3L2, CCL3L3, CCL4L1, CCL4L2, IL17B, IL17C, IL17D, IL17F, AIMP1 (SCYE1), MIF, Areg, BC096441, Bmp1, Bmp10, Bmp15, Bmp2, Bmp3, Bmp4, Bmp5, Bmp6, Bmp7, Bmp8a, Bmp8b, C1qtnf4, Ccl21a, Ccl27a, Cd70, Cer1, Cklf, Clcf1, Cmtm2a, Cmtm2b, Cmtm3, Cmtm4, Cmtm5, Cmtm6, Cmtm7, Cmtm8, Crlf1, Ctf2, Ebi3, Edn1, Fam3b, Fasl, Fgf2, Flt3l, Gdf10, Gdf11, Gdf15, Gdf2, Gdf3, Gdf5, Gdf6, Gdf7, Gdf9, Gm12597, Gm13271, Gm13275, Gm13276, Gm13280, Gm13283, Gm2564, Gpi1, Grem1, Grem2, Grn, Hmgb1, Ifna11, Ifna12, Ifna9, Ifnab, Ifne, Il17a, Il23a, Il25, Il31, Iltifb,Inhba, Lefty1, Lefty2, Mstn, Nampt, Ndp, Nodal, Pf4, Pglyrp1, Prl7d1, Scg2, Scgb3a1, Slurp1, Spp1, Thpo, Tnfsf10, Tnfsf11, Tnfsf12, Tnfsf13, Tnfsf13b, Tnfsf14, Tnfsf15, Tnfsf18, Tnfsf4, Tnfsf8, Tnfsf9, Tslp, Vegfa, Wnt1, Wnt2, Wnt5a, Wnt7a, Xcl1, epinephrine, melatonin, triiodothyronine, a prostaglandin, a leukotriene, prostacyclin, thromboxane, islet amyloid polypeptide, millerian inhibiting factor or hormone, adiponectin, corticotropin, angiotensin, vasopressin, arginine vasopressin, atriopeptin, brain natriuretic peptide, calcitonin, cholecystokinin, cortistatin, enkephalin, endothelin, erythropoietin, follicle-stimulating hormone, galanin, gastric inhibitory polypeptide, gastrin, ghrelin, glucagon, glucagon-like peptide-1, gonadotropin-releasing hormone, hepcidin, human chorionic gonadotropin, human placental lactogen, inhibin, somatomedin, leptin, lipotropin, melanocyte stimulating hormone, motilin, orexin, oxytocin, pancreatic polypeptide, pituitary adenylate cyclase-activating peptide, relaxin, renin, secretin, somatostatin, thrombopoietin, thyrotropin, thyrotropin-releasing hormone, vasoactive intestinal peptide, androgen, alpha-glucosidase (also known as acid maltase), glycogen phosphorylase, glycogen debrancher enzyme, phosphofructokinase, phosphoglycerate kinase, phosphoglycerate mutase, lactate dehydrogenase, carnitine palymityl transferase, carnitine, and myoadenylate deaminase.

In some embodiments, the protein is a replacement therapy or a replacement protein. In some embodiments, the replacement therapy or replacement protein is a clotting factor or a coagulation factor, e.g., Factor VIII (e.g., comprises a naturally occurring human Factor VIII amino acid sequence or a variant thereof) or Factor IX (e.g., comprises a naturally occurring human Factor IX amino acid sequence or a variant thereof).

In some embodiments, the plurality of cells are engineered to express a human Factor VIII protein, e.g., a recombinant Factor VIII. In some embodiments, the recombinant Factor VIII is a B-domain-deleted recombinant Factor VIII (FVIII-BDD). In some embodiments, the FVIII-BDD protein is a FVIII-BDD protein shown in Figure 20. In an embodiment, the FVIII-BDD protein comprises, consists essentially of, or consists of SEQ ID NO: 1.

In some embodiments, the cells are engineered to express a Factor IX, e.g., a human Factor IX (FIX) protein. In some embodiments, the FIX protein is a FIX-padua protein and comprises, consists essentially of, or consists of SEQ ID NO:36.

In some embodiments, the encapsulated cells are derived from a human RPE cell line and comprise an exogenous nucleic acid sequence which comprises a promoter sequence (e.g., nucleotides 337-2069 of SEQ ID NO:26 or SEQ ID NO:23) operably linked to a coding sequence for a polypeptide. In an embodiment, the coding sequence is a codon-optimized FVIII-BDD coding sequence shown in FIG. 24 (SEQ ID NO: 9, 10, 11, 12, 13, 14, 15, 16 or 17) or a codon-optimized FIX-padua coding sequence shown in FIG. 24 (SEQ ID NO:19, 20 or 21).

In some embodiments, the replacement therapy or replacement protein is an enzyme, e.g., alpha-galactosidase A (GLA), alpha-L-iduronidase (IDUA), glucocerebrosidase, N-sulfoglucosamine sulfohydrolase (SGSH), N-acetyl-alpha-d-glucosaminidase, N-acetylglucosamine 4-sulphatase, urate oxidase, phenyalanine hydroxylase, or phenylalanine ammonia lyase. In some embodiments, the replacement therapy or replacement protein is a cytokine or an antibody.

In some embodiments not recited in the present claims, the therapeutic agent is a sugar, e.g., monosaccharide, disaccharide, oligosaccharide, or polysaccharide. In some embodiments, a sugar comprises a triose, tetrose, pentose, hexose, or heptose moiety. In some embodiments, the sugar comprises a linear monosaccharide or a cyclized monosaccharide. In some embodiments, the sugar comprises a glucose, galactose, fructose, rhamnose, mannose, arabinose, glucosamine, galactosamine, sialic acid, mannosamine, glucuronic acid, galactosuronic acid, mannuronic acid, or guluronic acid moiety. In some embodiments, the sugar is attached to a protein (e.g., an N-linked glycan or an O-linked glycan). Exemplary sugars include glucose, galactose, fructose, mannose, rhamnose, sucrose, ribose, xylose, sialic acid, maltose, amylose, inulin, a fructooligosaccharide, galactooligosaccharide, a mannan, a lectin, a pectin, a starch, cellulose, heparin, hyaluronic acid, chitin, amylopectin, or glycogen. In some embodiments, the therapeutic agent is a sugar alcohol.

In some embodiments not recited in the present claims, the therapeutic agent is a lipid. A lipid may be hydrophobic or amphiphilic, and may form a tertiary structure such as a liposome, vesicle, or membrane or insert into a liposome, vesicle, or membrane. A lipid may comprise a fatty acid, glycerolipid, glycerophospholipid, sterol lipid, prenol lipid, sphingolipid, saccharolipid, polyketide, or sphingolipid. Examples of lipids produced by the encapsulated cells include anandamide, docosahexaenoic acid, a prostaglandin, a leukotriene, a thromboxane, an eicosanoid, a triglyceride, a cannabinoid, phosphatidylcholine, phosphatidylethanolamine, a phosphatidylinositol, a phosohatidic acid, a ceramide, a sphingomyelin, a cerebroside, a ganglioside, estrogen, androsterone, testosterone, cholesterol, a carotenoid, a quinone, a hydroquinone, or a ubiquinone.

In some embodiments not recited in the present claims, the therapeutic agent is a small molecule. A small molecule may include a natural product produced by a cell. In some embodiments, the small molecule has poor availability or does not comply with the Lipinski rule of five (a set of guidelines used to estimate whether a small molecule will likely be an orally active drug in a human; see, e.g., Lipinski, C.A. et al (2001) Adv Drug Deliv 46:2-36). Exemplary small molecule natural products include an anti-bacterial drug (e.g., carumonam, daptomycin, fidaxomicin, fosfomycin, ispamicin, micronomicin sulfate, miocamycin, mupiocin, netilmicin sulfate, teicoplanin, thienamycin, rifamycin, erythromycin, vancomycin), an anti-parasitic drug (e.g., artemisinin, ivermectin), an anticancer drug (e.g., doxorubicin, aclarubicin, aminolaevulinic acid, arglabin, omacetaxine mepesuccinate, paclitaxel, pentostatin, peplomycin, romidepsin, trabectdin, actinomycin D, bleomycin, chromomycin A, daunorubicin, leucovorin, neocarzinostatin, streptozocin, trabectedin, vinblastine, vincristine), anti-diabetic drug (e.g., voglibose), a central nervous system drug (e.g., L-dopa, galantamine, zicontide), a statin (e.g., mevastatin), an anti-fungal drug (e.g., fumagillin, cyclosporin), 1-deoxynojirimycin, and theophylline, sterols (cholesterol, estrogen, testerone). Additional small molecule natural products are described in Newman, D.J. and Cragg, M. (2016) J Nat Prod 79:629-661 and Butler, M.S. et al (2014) Nat Prod Rep 31:1612-1661, which are incorporated herein by reference in their entirety.

In some embodiments not recited in the present claims, the cells are engineered to synthesize a non-protein or non-peptide small molecule. For example, in an embodiment an engineered cell can produce a statin (e.g., taurostatin, pravastatin, fluvastatin, or atorvastatin).

In some embodiments, the therapeutic agent is an antigen (e.g., a viral antigen, a bacterial antigen, a fungal antigen, a plant antigen, an environmental antigen, or a tumor antigen). An antigen is recognized by those skilled in the art as being immunostimulatory, i.e., capable of stimulating an immune response or providing effective immunity to the organism or molecule from which it derives. An antigen may be a nucleic acid, peptide, protein, sugar, lipid, or a combination thereof.

The cells in the devices described herein may produce a single therapeutic agent or a plurality of therapeutic agents. The plurality of therapeutic agents may be related or may form a complex. In some embodiments, the therapeutic agent secreted or released from a device is in an active form. In some embodiments, the therapeutic agent is secreted or released from a device in an inactive form, e.g., as a prodrug. In the latter instance, the therapeutic agent may be activated by a downstream agent, such as an enzyme.

### Methods of Treatment

Described herein are methods for preventing or treating a disease, disorder, or condition in a subject through administration or implantation of a device or preparation of devices described herein that produces a therapeutic agent that treats the disease, disorder or condition. The methods comprise administering or implanting a device or device preparation comprising a plurality of the device, wherein the device comprises (i) at least one cell-containing compartment which comprises a polymer composition comprising a first cell-binding substance (CBS) and encapsulating a plurality of cells (e.g., live cells) capable of expressing the therapeutic agent when the device or device preparation is implanted into the subject, and (ii) means for mitigating the FBR when the device or device preparation is implanted in the subject. In an embodiment, the means comprises a barrier compartment surrounding the cell-containing compartment and comprising a compound of Formula (I) (e.g., as described herein). In some embodiments, the methods described herein directly or indirectly reduce or alleviate at least one symptom of the disease, disorder, or condition. In some embodiments, the methods described herein prevent or slow the onset of the disease, disorder, or condition. In some embodiments, the subject is a human.

In some embodiments, the disease, disorder, or condition affects a system of the body, e.g. the nervous system (e.g., peripheral nervous system (PNS) or central nervous system (CNS)), vascular system, skeletal system, respiratory system, endocrine system, lymph system, reproductive system, or gastrointestinal tract. In some embodiments, the disease, disorder, or condition affects a part of the body, e.g., blood, eye, brain, skin, lung, stomach, mouth, ear, leg, foot, hand, liver, heart, kidney, bone, pancreas, spleen, large intestine, small intestine, spinal cord, muscle, ovary, uterus, vagina, or penis.

In some embodiments, the disease, disorder or condition is a neurodegenerative disease, diabetes, a heart disease, an autoimmune disease, a cancer, a liver disease, a lysosomal storage disease, a blood clotting disorder or a coagulation disorder, an orthopedic condition, an amino acid metabolism disorder.

In some embodiments, the disease, disorder or condition is a neurodegenerative disease. Exemplary neurodegenerative diseases include Alzheimer's disease, Huntington's disease, Parkinson's disease (PD) amyotrophic lateral sclerosis (ALS), multiple sclerosis (MS) and cerebral palsy (CP), dentatorubro-pallidoluysian atrophy (DRPLA), neuronal intranuclear hyaline inclusion disease (NIHID), dementia with Lewy bodies, Down's syndrome, Hallervorden-Spatz disease, prion diseases, argyrophilic grain dementia, cortocobasal degeneration, dementia pugilistica, diffuse neurofibrillary tangles, Gerstmann-Straussler-Scheinker disease, Jakob-Creutzfeldt disease, Niemann-Pick disease type 3, progressive supranuclear palsy, subacute sclerosing panencephalitis, spinocerebellar ataxias, Pick's disease, and dentatorubral-pallidoluysian atrophy.

In some embodiments, the disease, disorder, or condition is an autoimmune disease, e.g., scleroderma, multiple sclerosis, lupus, or allergies.

In some embodiments, the disease is a liver disease, e.g., hepatitis B, hepatitis C, cirrhosis, NASH.

In some embodiments, the disease, disorder, or condition is cancer. Exemplary cancers include leukemia, lymphoma, melanoma, lung cancer, brain cancer (e.g., glioblastoma), sarcoma, pancreatic cancer, renal cancer, liver cancer, testicular cancer, prostate cancer, or uterine cancer.

In some embodiments, the disease, disorder, or condition is an orthopedic condition. Exemplary orthopedic conditions include osteoporosis, osteonecrosis, Paget's disease, or a fracture.

In some embodiments, the disease, disorder or condition is a lysosomal storage disease. Exemplary lysosomal storage diseases include Gaucher disease (e.g., Type I, Type II, Type III), Tay-Sachs disease, Fabry disease, Farber disease, Hurler syndrome (also known as mucopolysaccharidosis type I (MPS I)), Hunter syndrome, lysosomal acid lipase deficiency, Niemann-Pick disease, Salla disease, Sanfilippo syndrome (also known as mucopolysaccharidosis type IIIA (MPS3A)), multiple sulfatase deficiency, Maroteaux-Lamy syndrome, metachromatic leukodystrophy, Krabbe disease, Scheie syndrome, Hurler-Scheie syndrome, Sly syndrome, hyaluronidase deficiency, Pompe disease, Danon disease, gangliosidosis, or Morquio syndrome.

In some embodiments, the disease, disorder, or condition is a blood clotting disorder or a coagulation disorder. Exemplary blood clotting disorders or coagulation disorders include hemophilia (e.g., hemophilia A or hemophilia B), Von Willebrand disease, thrombocytopenia, uremia, Bernard-Soulier syndrome, Factor XII deficiency, vitamin K deficiency, or congenital afibrinogenimia.

In some embodiments, the disease, disorder, or condition is an amino acid metabolism disorder, e.g., phenylketonuria, tyrosinemia (e.g., Type 1 or Type 2), alkaptonuria, homocystinuria, hyperhomocysteinemia, maple syrup urine disease.

In some embodiments, the disease, disorder, or condition is a fatty acid metabolism disorder, e.g., hyperlipidemia, hypercholesterolemia, galactosemia.

In some embodiments, the disease, disorder, or condition is a purine or pyrimidine metabolism disorder, e.g., Lesch-Nyhan syndrome.

In some embodiments, the disease, disorder, or condition is diabetes (e.g., Type I or Type II diabetes).

The present disclosure further comprises methods for identifying a subject having or suspected of having a disease, disorder, or condition described herein, and upon such identification, administering to the subject a device comprising an inner compartment comprising an alginate covalently modified with a cell-binding peptide and an outer compartment comprising a compound of Formula (I) (e.g., as described herein), or a composition comprising such a plurality of such a device. In an embodiment, the subject is a human.

### Pharmaceutical Compositions, Kits, and Administration

The present disclosure further comprises pharmaceutical compositions comprising the devices described herein, as well as kits thereof.

In some embodiments, a pharmaceutical composition comprises a device, or a plurality of the device, wherein the device comprises at least one cell-containing compartment which comprises a polymer composition comprising a cell-binding substance and encapsulating a plurality of cells (e.g., live cells), means for mitigating the foreign body response (FBR), and a pharmaceutically acceptable excipient. The live cells express a therapeutic agent when the composition is administered to (e.g., implanted into) a subject. In some embodiments, the pharmaceutical composition comprises an effective amount of the device (e.g., a quantity of hydrogel capsules, e.g., 100, 200, 400, 800, 2,000 or more capsules. In some embodiments, the effective amount is a therapeutically effective amount. In some embodiments, the effective amount is a prophylactically effective amount.

Pharmaceutical compositions described herein can be prepared by any method known in the art of pharmacology. In general, such preparatory methods include the steps of bringing the devices as the "active ingredient" (e.g., hydrogel capsules, particles) into association with a carrier and/or one or more other accessory ingredients, and then, if necessary and/or desirable, shaping and/or packaging the product into a desired single- or multi-dose unit.

Pharmaceutical compositions can be prepared, packaged, and/or sold in bulk, as a single unit dose, and/or as a plurality of single unit doses. As used herein, a "unit dose" is a discrete amount of the pharmaceutical composition comprising a predetermined amount of the active ingredient (i.e., number of hydrogel capsules, particles). The predetermined amount is generally equal to the dosage of the active ingredient which would be administered to a subject and/or a convenient fraction of such a dosage such as, for example, one-half or one-third of such a dosage.

Relative amounts of the active ingredient, the pharmaceutically acceptable excipient, and/or any additional ingredients in a pharmaceutical composition of the disclosure will vary, depending upon the identity, size, and/or condition of the subject treated and further depending upon the route by which the composition is to be administered. By way of example, the composition may comprise between 0.1% and 100% (w/w) active ingredient.

The term "pharmaceutically acceptable excipient" refers to a non-toxic carrier, adjuvant, diluent, or vehicle that does not destroy the pharmacological activity of the device with which it is formulated. Pharmaceutically acceptable excipients useful in the manufacture of the pharmaceutical compositions of the disclosure are any of those that are well known in the art of pharmaceutical formulation and include inert diluents, dispersing and/or granulating agents, surface active agents and/or emulsifiers, disintegrating agents, binding agents, preservatives, buffering agents, lubricating agents, and/or oils. Pharmaceutically acceptable excipients useful in the manufacture of the pharmaceutical compositions of the disclosure include, but are not limited to, ion exchangers, alumina, aluminum stearate, lecithin, serum proteins, such as human serum albumin, buffer substances such as phosphates, glycine, sorbic acid, potassium sorbate, partial glyceride mixtures of saturated vegetable fatty acids, water, salts or electrolytes, such as protamine sulfate, disodium hydrogen phosphate, potassium hydrogen phosphate, sodium chloride, zinc salts, colloidal silica, magnesium trisilicate, polyvinyl pyrrolidone, cellulose-based substances, polyethylene glycol, sodium carboxymethylcellulose, polyacrylates, waxes, polyethylene-polyoxypropylene-block polymers, polyethylene glycol and wool fat.

The devices and related compositions described herein may be administered orally, parenterally (including subcutaneous, intramuscular, and intradermal), topically, rectally, nasally, intratumorally, intrathecally, intraocularlly, intravitreally, buccally, vaginally or via an implanted reservoir. In some embodiments, provided devices or compositions are administrable subcutaneously or by implant.

In some embodiments, the devices and related compositions described herein may be administered to or implanted in or on a certain region of the body, such as a mucosal surface or a body cavity. Exemplary sites of administration or implantation include the peritoneal cavity (e.g., lesser sac), adipose tissue, heart, eye (e.g., retina), muscle, spleen, lymph node, esophagus, nose, sinus, teeth, gums, tongue, mouth, throat, small intestine, large intestine, thyroid, bone (e.g. hip or a joint), breast, cartilage, vagina, uterus, fallopian tube, ovary, penis, testicles, blood vessel, liver, kidney, central nervous system (e.g., brain, spinal cord, nerve), or ear (e.g., cochlea).

In some embodiments, the devices and related compositions described herein are administered to or implanted at a site other than the central nervous system, e.g., the brain, spinal cord, nerve. In some embodiments, the devices and related compositions described herein are administered or implanted at a site other than the eye.

Sterile injectable forms of the compositions of this disclosure may be aqueous or oleaginous suspension. These suspensions may be formulated according to techniques known in the art using suitable dispersing or wetting agents and suspending agents. The sterile injectable preparation may also be a sterile injectable solution or suspension in a non-toxic parenterally acceptable diluent or solvent, for example as a solution in 1,3-butanediol. Among the acceptable vehicles and solvents that may be employed are water, Ringer's solution and isotonic sodium chloride solution. In addition, sterile, fixed oils are conventionally employed as a solvent or suspending medium.

For ophthalmic use, provided pharmaceutically acceptable compositions may be formulated as micronized suspensions or in an ointment such as petrolatum.

Although the descriptions of pharmaceutical compositions provided herein are principally directed to pharmaceutical compositions which are suitable for administration to humans, it will be understood by the skilled artisan that such compositions are generally suitable for administration to subjects of all sorts. Modification of pharmaceutical compositions suitable for administration to humans in order to render the compositions suitable for administration to various animals is well understood, and the ordinarily skilled veterinary pharmacologist can design and/or perform such modification with ordinary experimentation.

The devices and related compositions described herein may be formulated in dosage unit form, e.g., single unit dosage form, for ease of administration and uniformity of dosage. It will be understood, however, that the total dosage and usage regimens of the compositions of the present disclosure will be decided by the attending physician within the scope of sound medical judgment. The specific therapeutically effective dose level for any particular subject or organism will depend upon a variety of factors including the disease being treated and the severity of the disorder; the productivity of the specific devices employed and the activity of the therapeutic agent produced by the devices; the specific composition employed; the age, body weight, general health, sex and diet of the subject; the time of administration, route of administration, and half-life of the specific therapeutic agent secreted by the administered device(s); the duration of the treatment; drugs used in combination or coincidental with the specific therapeutic agent expressed by the device; and like factors well known in the medical arts.

The exact amount of a treatment required to achieve an effective amount will vary from subject to subject, depending, for example, on species, age, and general condition of a subject, severity of the side effects or disorder, identity of the particular device(s), mode of administration, and the like. The desired dosage can be delivered three times a day, two times a day, once a day, every other day, every third day, every week, every two weeks, every three weeks, every four weeks. In some embodiments, the desired dosage is delivered once every two, three, four, six, nine or twelve months or once every one, two, three or more years. In certain embodiments, the desired dosage can be delivered using multiple administrations (e.g., two, three, four, five, six, seven, eight, nine, ten, eleven, twelve, thirteen, fourteen, or more administrations). In some embodiments, each succeeding administration or implant of devices or related composition is performed following removal of the preceding administration or implant.

It will be appreciated that the devices and related compositions described herein can be administered in combination with one or more additional pharmaceutical agents. Such pharmaceutical agents may improve bioavailability or the therapeutic agent, reduce and/or modify metabolism of the therapeutic agent, inhibit the FBR of the implanted devices, and/or modify distribution of the therapeutic agent within the body. It will also be appreciated that the therapy employed may achieve a desired effect for the same disorder, and/or it may achieve different effects.

Also encompassed by the disclosure are kits (e.g., pharmaceutical packs). The inventive kits may be useful for preventing and/or treating any of the diseases, disorders or conditions described herein. The kits provided may comprise a pharmaceutical composition (e.g., composition of devices) or a device as described herein and a container (e.g., a vial, ampule, bottle, syringe, and/or dispenser package, or other suitable container). In some embodiments, provided kits may optionally further include a second container comprising a pharmaceutical excipient for dilution or suspension of a pharmaceutical composition or device described herein. In some embodiments, the pharmaceutical composition or device provided in the container and the second container are combined to form one unit dosage form.

### Methods of Making Devices

The present disclosure further comprises methods for making a device described herein, e.g., a cell-containing compartment comprising a plurality of cells encapsulated in a polymer composition comprising a cell-binding substance and means for mitigating the FBR.

In embodiments where the device comprises a preparation of single compartment hydrogel capsules, the device may be made as follows. Briefly, a volume of hydrogel-forming polymer solution that comprises an afibrotic polymer (e.g., an alginate modified with a Compound of Formula (I)) may be loaded into a syringe, e.g., a syringe that is then capped with a blunt tipped needle. The syringe may then be placed into a syringe pump oriented vertically above a vessel containing an aqueous cross-linking solution which comprises a cross-linking agent, a buffer, an osmolarity-adjusting agent. A high voltage power generator may then be connected to the needle. The syringe pump and power generator can then be used to extrude the polymer solution through the syringe with settings determined to achieve a desired droplet rate of polymer solution into a cross-linking solution. After exhausting the volume of polymer solution, the droplets are allowed to cross-link in the cross-linking solution for several minutes, e.g., about five minutes. In an embodiment, the number of non-capsule debris on the surface of the cross-linking solution is determined. Hydrogel capsules that have fallen to the bottom of the cross-linking vessel may then be collected, e.g., by transferring cross-linking solution containing the hydrogel capsules to a separate container, leaving behind any non-capsular debris on the solution surface in the original cross-linking vessel. The removed hydrogel capsules may then be allowed to settle, the cross-linking solution can be removed, and the capsules may then be washed one or more times with a buffer (e.g., a HEPES buffer). In an embodiment, one or more aliquots of the resulting hydrogel capsule composition is inspected by microscopy to assess the quality of the composition, e.g., the number of capsule defects and satellite capsules.

In some embodiments where the device comprises a hydrogel capsule with two hydrogel compartments or a preparation of two-compartment hydrogel capsules, the method of making the device comprises contacting a plurality of droplets comprising first and second polymer solutions (e.g., each solution comprising a hydrogel-forming polymer) with an aqueous cross-linking solution. The droplets can be formed using any technique known in the art.

Briefly, in performing a method of making a composition of two-compartment hydrogel capsules, a volume of a first polymer solution (e.g., comprising a plurality of cells (e.g., live cells) suspended in a polymer modified with a cell-binding peptide and optionally blended with an unmodified polymer) is loaded into a first syringe connected to the inner lumen of a coaxial needle. The first syringe may then be connected to a syringe pump oriented vertically above a vessel containing an aqueous cross-linking solution which comprises a cross-linking agent, a buffer, and an osmolarity-adjusting agent. A volume of the second polymer solution (e.g., comprising an unmodified polymer, a polymer modified with a compound of Formula (I), or a blend thereof) is loaded into a second syringe connected to the outer lumen of the coaxial needle. The second syringe may then be connected to a syringe pump oriented horizontally with respect to the vessel containing the cross-linking solution. A high voltage power generator may then be connected to the needle. The syringe pumps and power generator can then be used to extrude the first and second polymer solutions through the syringes with settings determined to achieve a desired droplet rate of polymer solution into the cross-linking solution. After exhausting the first and second volumes of polymer solution, the droplets may be allowed to cross-link in the cross-linking solution for certain amount of time, e.g., about five minutes. In an embodiment, the number of non-capsular debris on the surface of the cross-linking solution is determined. Capsules that have fallen to the bottom of the cross-linking vessel may then be collected, e.g., by transferring cross-linking solution containing the capsules to a separate container, leaving behind any non-capsular debris on the solution surface in the original cross-linking vessel. The removed capsules may then be allowed to settle, the cross-linking solution can be removed, and the capsules may then be washed one or more times with a buffer (e.g., a HEPES buffer). In an embodiment, one or more aliquots of the resulting device composition is inspected by microscopy to assess the quality of the composition, e.g., the number of capsule defects and capsules.

In some embodiments, the cross-linking solution used in a process for making a single-compartment or two-compartment capsules further comprises a process additive (e.g., a hydrophilic, non-ionic surfactant). The process additive may reduce surface tension of the cross-linking solution. Agents useful as the process additive in the present disclosure include polysorbate-type surfactants, copolymer of polyethyleneoxide (PEO) and polypropyleneoxide (PPO), poly(ethylene oxide)-poly(propylene oxide)-poly(ethylene oxide) (PEO-PPO-PEO) triblock copolymers, and non-ionic surfactants, such as Tween^{®} 20, Tween^{®} 80, Triton^{™} X-100, IGEPAL^{®} CA-630, poloxamer 188, or poloxamer 407. In some embodiments, the process additive is a non-ionic surfactant. In an embodiment, the process additive comprises more than one surfactant, e.g., more than one hydrophilic surfactant. In some embodiments, the process additive does not contain Tween^{®} 20 or Triton^{™} X-100. In an embodiment, the process additive is IGEPAL^{®} CA-630. In some embodiments, the process additive is poloxamer 188.

In some embodiments, the process additive (e.g., surfactant) is present in the cross-linking solution at a concentration of at least 0.0001% or more. In some embodiments, the cross-linking solution comprises at least 0.001%, 0.01%, or 0.1% of the process additive. In some embodiments, the process additive is present at a concentration selected from about 0.001% to about 0.1%, about 0.005% to about 0.05%, about 0.005% to about 0.01%, and about 0.01% to about 0.5%. In an embodiment, the process additive is a surfactant and is present at a concentration that is below the critical micelle concentration for the surfactant.

In some embodiments, the cross-linking agent comprises divalent cations of a single type or a mixture of different types, e.g., one or more of Ba²⁺, Ca²⁺, Sr²⁺. In some embodiments, the cross-linking agent is BaCl₂, e.g., at a concentration of 1 mM to 100 mM, 2.5 mM to 75 mM, 5 mM to 50 mM or 7.5 mM to 25 mM, or about 20 mM. In some embodiments, the cross-linking agent is CaCl₂, e.g., at a concentration of 50 mM to 150 mM or about 100 mM. In some embodiments, the cross-linking agent is SrCl₂, e.g., at a concentration of 37.5 mM to 100 mM. In some embodiments, the cross-linking agent is a mixture of BaCl₂ (e.g., 5 mM to 20 mM) and CaCl₂ (e.g., 37.5 mM to 12.5 mM) or a mixture of BaCl₂ (e.g., 5 mM to 20 mM) and SrCl₂ (e.g., 37.5 mM to 12.5 mM).

The type and concentration of buffer in the aqueous cross-linking solution is selected to be compatible with the cells to be encapsulated and to maintain the solution pH at approximately neutral, e.g., from about 6.5 to about 7.5, about 7.0 to about 7.5, or about 7.0. In some embodiments, the buffer in the aqueous cross-linking solution comprises HEPES (4-(2-hydroxyethyl)-1-piperazineethanesulfonic acid).

The osmolarity-adjusting agent in the aqueous cross-linking solution is selected to maintain the solution osmolarity at a value similar to the osmolarity of the first polymer solution comprising a suspension of cells (e.g., live cells), e.g., an osmolarity that has a higher or lower variance of up to 20%, 10% or 5%. In some embodiments, the osmolarity agent is mannitol at a concentration of 0.1 M to 0.3 M.

In an embodiment, the process additive is poloxamer 188. In an embodiment, the poloxamer 188 is present in the capsule composition in a detectable amount after the wash steps. Poloxamer 188 may be detected by any technique known in the art, e.g., by removing an aliquot of the capsules, adding a sodium sulfate solution to partially or completely dissolve the capsules in the aliquot, and analyzing the resulting solution by LC/MS.

Reduction in the surface tension of the cross-linking solution may be assessed by any method known in the art, for example, through the use of a contact angle goniometer or a tensiometer, e.g., via the du Nouy ring method (see, e.g., Davarci et al (2017) Food Hydrocolloids 62:119-127).

### EXAMPLES

In order that the disclosure described herein may be more fully understood, the following examples are set forth. The examples described in this application are offered to illustrate the devices (e.g., capsules, particles, chemical modifications, compositions and methods provided herein and are not to be construed in any way as limiting their scope.

### Example 1: Preparation of exemplary modified polymers

1A. *Chemically-modified Polymer.* A polymeric material may be chemically modified with a compound of Formula (I) (or pharmaceutically acceptable salt thereof) prior to formation of a device described herein (e.g., a hydrogel capsule described herein) using methods known in the art.

For example, in the case of alginate, the alginate carboxylic acid is activated for coupling to one or more amine-functionalized compounds to achieve an alginate modified with an afibrotic compound, e.g., a compound of Formula (I). The alginate polymer is dissolved in water (30 mL/gram polymer) and treated with 2-chloro-4,6-dimethoxy-1,3,5-triazine (0.5 eq) and N-methylmorpholine (1 eq). To this mixture is added a solution of the compound of interest (e.g., Compound 101 shown in Table 4) in acetonitrile (0.3M). A compound of interest may be prepared using methods known in the art, for example, as described in WO2018/067615, which is incorporated herein by reference in its entirety.

The amounts of the compound and coupling reagent added depends on the desired concentration of the compound bound to the alginate, e.g., conjugation density. To prepare a CM-LMW-Alg-101-Medium polymer solution, the dissolved unmodified low molecular weight alginate (approximate MW < 75 kDa, G:M ratio ≥ 1.5) is treated with 2-chloro-4,6-dimethoxy-1,3,5-triazine (5.1 mmol/g alginate) and N-methylmorpholine (10.2 mmol/ g alginate) and Compound 101 (5.4 mmol/ g alginate). To prepare a CM-LMW-Alg-101-High polymer solution, the dissolved unmodified low-molecular weight alginate (approximate MW < 75 kDa, G:M ratio ≥ 1.5) is treated with 2-chloro-4,6-dimethoxy-1,3,5-triazine (5.1 mmol/g alginate) and N-methylmorpholine (10.2 mmol/ g alginate) and Compound 101 (5.4 mmol/ g alginate).

The reaction is warmed to 55°C for 16h, then cooled to room temperature and gently concentrated via rotary evaporation, then the residue is dissolved in water. The mixture is filtered through a bed of cyano-modified silica gel (Silicycle) and the filter cake is washed with water. The resulting solution is then extensively dialyzed (10,000 MWCO membrane) and the alginate solution is concentrated via lyophilization to provide the desired chemically-modified alginate as a solid or is concentrated using any technique suitable to produce a chemically modified alginate solution with a viscosity of 25 cP to 35 cP.

In an embodiment, the conjugation density of a chemically modified alginate is measured by combustion analysis for percent nitrogen. The sample is prepared by dialyzing a solution of the chemically modified alginate against water (10,000 MWCO membrane) for 24 hours, replacing the water twice followed by lyophilization to a constant weight.

In another embodiment, the conjugation density of a chemically modified alginate is determined using a quantitative amine assay as described in Example 27 below.

For use in generating the hydrogel capsules described in the Examples below, chemically modified alginate polymers were prepared with Compound 101 (shown in Table 4) conjugated to a low molecular weight alginate (approximate MW < 75 kDa, G:M ratio ≥ 1.5) at medium (2% to 5% N) or high (5.1% to 8% N) densities, as determined by combustion analysis for percent nitrogen, and are referred to herein as CM-LMW-Alg-101-Medium and CM-LMW-Alg-101-High. Unless otherwise specified, the chemically modified alginate in the capsules made in the Examples below is CM-LMW-Alg-101-Medium.

1B. *CBP-Alginates.* A polymeric material may be covalently modified with a peptide prior to formation of a device described herein (e.g., a hydrogel capsule described herein) using methods known in the art, see, e.g., Jeon O, et al., Tissue Eng Part A. 16:2915-2925 (2010) and Rowley, J.A. et al., Biomaterials 20:45-53 (1999).

For example, in the case of alginate, an alginate solution (1%, w/v) is prepared with 50mM of 2-(N-morpholino)-ethanesulfonic acid hydrate buffer solution containing 0.5M NaCl at pH 6.5, and sequentially mixed with N-hydroxysuccinimide and 1-ethyl-3-[3-(dimethylamino)propyl] carbodiimide (EDC). The molar ratio of N-hydroxysuccinimide to EDC is 0.5:1.0. The peptide of interest is added to the alginate solution. The amounts of peptide and coupling reagent added depends on the desired concentration of the peptide bound to the alginate, e.g., peptide conjugation density. By increasing the amount of peptide and coupling reagent, higher conjugation density can be obtained. After reacting for 24 h, the reaction is purified by dialysis against ultrapure deionized water (diH2O) (MWCO 3500) for 3 days, treated with activated charcoal for 30 min, filtered (0.22 mm filter), and concentrated to the desired viscosity.

In an embodiment, the conjugation density of a peptide-modified alginate is measured by combustion analysis for percent nitrogen. The sample is prepared by dialyzing a solution of the peptide-modified alginate against water (10,000 MWCO membrane) for 24 hours, replacing the water twice followed by lyophilization to a constant weight.

In another embodiment, the conjugation density of a peptide-modified alginate is measured using a quantitative peptide-conjugation assay as described in Examples 25 and optionally 26.

### Example 2: Preparation of exemplary alginate solutions for making hydrogel capsules

*70:30 mixture of chemically-modified and unmodified alginate.* A low molecular weight alginate (PRONOVA^{™} VLVG alginate, NovaMatrix^{®} Cat. #4200506, approximate MW < 75 kDa, G:M ratio ≥ 1.5) was chemically modified with Compound 101 in Table 4 to produce a chemically modified low molecular weight alginate solution with a viscosity of 25 cP to 35 cP (CM-LMW-Alg-101). A solution of high molecular weight unmodified alginate (U-HMW-Alg) was prepared by dissolving unmodified alginate (PRONOVA^{™} SLG100, NovaMatrix, Sandvika, Norway, cat. #4202106, approximate MW of 150 kDa - 250 kDa, G:M ratio ≥ 1.5) at 3% weight to volume in 0.9% saline. The CM-LMW-Alg solution was blended with the U-HMW-Alg solution at a volume ratio of 70% CM-LMW-Alg to 30% U-HMW-Alg (referred to herein as a 70:30 CM-LMW-Alg:U-HMW-Alg solution).

*60:40 mixture of chemically-modified and unmodified alginate.* This solution was prepared using the same procedure as for the above 70:30 mixture, except that the CM-LMW-Alg-101 solution was blended with the U-HMW-Alg solution at a volume ratio of 60% CM-LMW-Alg-101 to 40% U-HMW-Alg (referred to herein as a 60:40 CM-LMW-Alg-101:U-HMW-Alg solution).

*Unmodified alginate solution.* An unmodified medium molecular weight alginate (SLG20, NovaMatrix, Sandvika, Norway, cat. #4202006, approximate molecular weight of 75-150 kDa), was dissolved at 1.4% weight to volume in 0.9% saline to prepare a U-MMW-Alg solution.

*Alginate Solutions Comprising Cell Binding Sites.* Various alginate solutions containing a CBP-alginate or a mixture of a cell-binding protein and an unmodified alginate were prepared as summarized in Table 6 below.

**Table 6. Experimental Alginate Solutions Containing Cell-Binding Sites Used To Form Cell-Containing Compartments**

| Solution Name* | %N | Cell Binding Peptide (CBP) | Linker | Modified Alginate Source Alginate MW; G:M Ratio | Solution Composition** |
|---|---|---|---|---|---|
| VLVG-4GRGDSP | 0.7 | RGDSP (SEQ ID NO: 59) | GGGG (SEQ ID NO: 61) | NovaMatrix 4270519 | 5 w/v% of CBP-alginate blended at 70:30 volume ratio with 3% SLG100 in saline |
| | | | | MW = < 75 kDa; | |
| | | | | G:M ≥ 1.5 | |
| MVG-GRGDSP | 0.95 | RGDSP (SEQ ID NO: 59) | G | NovaMatrix 4270129 | 1.4 w/v% of CBP-alginate in saline |
| | | | | MW = > 200 kDa; G:M ≥ 1.5 | |
| 1G-MVG: 0.71, 143 cP | 0.71 | RGDSP (SEQ ID NO: 59) | G | Prepared in Examples 1, 2 | Viscosity = 143 cP |
| 1G-MVG: 0.71, 79 cP | | | | | Viscosity = 79 cP |
| 1G-MVG: 0.71, 97 cP | | | | | Viscosity = 97 cP |
| 1G-MVG: 0.71, 202 cP | | | | | Viscosity = 202 cP |
| 1G-SLG100: 0.65 | 0.65 | RGDSP (SEQ ID NO: 59) | G | Prepared in Examples 1, 2 | Viscosity = 98 cP |
| 1G-RGD: 0.22 | 0.22 | | | | Viscosity = 118 cP |
| 1G-RGD: 0.41 | 0.41 | | | | Viscosity = 57 cP |
| 1G-RGD: 0.33 | 0.33 | | | | Viscosity = 113 cP |
| 1G-RGD: 0.72 | 0.72 | | | | Viscosity = 133 cP |
| 1G-RGD: 1.47 | 1.47 | RGDSP (SEQ ID NO: 59) | G or GGGG (SEQ ID NO: 61) | Prepared in Examples 1, 2 | Viscosity = 141 cP |
| 4G-RGD: 0.53 | 0.53 | | | | Viscosity = 70 cP |
| 4G-RGD: 0.96 | 0.96 | | | | Viscosity = 58 cP |
| 4G-RGD: 1.77 | 1.77 | | | | Viscosity = 102 cP |
| 4G-RGD: 2.95 | 2.95 | | | | Viscosity = 123 cP |
| 1G-RGD: 6.04 | 6.04 | RGDSP (SEQ ID NO: 59) | G | Prepared in Examples 1, 2 | Viscosity = 102 cP |
| 1G-RGD: 6.04, 1:55 | 0.11 | | | | Blended 1:55 with 1.4 w/v% SLG20 |
| 1G-RGD: 6.04, 1:30 | 0.20 | | | | Blended 1:30 with 1.4 w/v% SLG20 |
| 1G-RGD: 6.04, 1:15 | 0.40 | | | | Blended 1:15 with 1.4 w/v% SLG20 |
| 4G-DGEA: 0.15 | 0.15 | DGEA (SEQ ID NO: 39) | GGGG (SEQ ID NO: 61) | Prepared in Examples 1, 2 | Viscosity = 133 cP |
| 4G-PHSRN: 0.68 | 0.68 | PHSRN (SEQ ID NO: 46) | GGGG (SEQ ID NO: 61) | Prepared in Examples 1,2 | Viscosity = 133 cP |
| G-PHSRN: 0.83 | 0.83 | PHSRN (SEQ ID NO: 46) | G | Prepared in Examples 1, 2 | Viscosity = 108 cP |
| PHSRN/RGD, same polymer | 0.87 | RGDSP (SEQ ID NO: 59) and PHSRN (SEQ ID NO: 46) | G | Prepared in Examples 1, 2 | Viscosity = 98 cP |
| G-HAVDI: 0.38 | 0.38 | HAVDI (SEQ ID NO: 38) | G | Prepared in Examples 1, 2 | Viscosity = 132 cP |
| Collagen I | N/A | Full-length protein | None | Not Applicable | 0.36 mg/ml Collagen I in 1.4 w/v% SLG20 in saline |
| Hyaluronic Acid (HA) | N/A | Full-length glycosaminoglycan | None | Not Applicable | 0.15 w/v% HA in 1.4 w/v% SLG20 in saline |

| | | | | | |
|---|---|---|---|---|---|
| * Where known, the solution names incorporate the peptide conjugation density as percent nitrogen determined by combustion analysis as described in Example 1B. ** When present, the type and concentration of cells are noted in the Examples below. | | | | | |

All unmodified and peptide-modified alginates were purchased from NovaMatrix^{®}, Sandvika Norway unless otherwise stated. Collagen I was purchased from Corning Life Sciences, Cat No. 354236 (Collagen Type I, Rat Tail), Oneonta, NY. HA was purchased from Lifecore Biomedical, Cat. No. HA700K (MW = 500 kDa-749 kDa), Chaska MN).

### Example 3: Preparation of exemplary cells for encapsulation in one-compartment or two-compartment hydrogel millicapsules

### 3A. Engineered ARPE-19 cells for encapsulation as single cells.

ARPE-19 cells engineered to express a therapeutic agent, e.g., a blood clotting factor (e.g., a FVIII or FIX protein) may be cultured according to any method known in the art, such as according to the following protocol.

Engineered ARPE-19 cells in a 75 cm² culture flask were aspirated to remove culture medium, and the cell layer was briefly rinsed with 0.05% (w/v) trypsin/ 0.53 mM EDTA solution ("TrypsinEDTA") to remove all traces of serum containing a trypsin inhibitor. Two to three mL of Trypsin/EDTA solution was added to the flask, and the cells were observed under an inverted microscope until the cell layer was dispersed, usually between 5-15 minutes. To avoid clumping, cells were handled with care and hitting or shaking the flask during the dispersion period was minimized. If the cells did not detach, the flasks were placed at 37 °C to facilitate dispersal. Once the cells dispersed, 6-8 mL complete growth medium was added and the cells were aspirated by gentle pipetting. The cell suspension was transferred to a centrifuge tube and spun down at approximately 125 x g for 5-10 minutes to remove TrypsinEDTA. The supernatant was discarded, and the cells were re-suspended in fresh growth medium. Appropriate aliquots of cell suspension was added to new culture vessels, which were incubated at 37 °C. The medium was renewed 2-3 times weekly.

### 3B. ARPE-19 cells for encapsulation as clusters

Spheroid clusters of exemplary cells (e.g., engineered ARPE-19 cells) are prepared using AggreWell^{™} spheroid plates (STEMCELL Technologies) and the protocol outlined herein. On Day 1, rinsing solution (4 mL) is added to each plate, and the plates is spun down for 5 minutes at 3,000 RPM in a large centrifuge. The rinsing solution is removed by pipet, and 4 mL of the complete growth medium is added. The engineered ARPE-19 cells are seeded into the plates at the desired cell density and pipetted immediately to prevent aggregation, with the general rule of thumb that 3.9 million cells per well will generate 150 µm diameter clusters. The plate is spun down for 3 minutes at 800 RPM, and the plate is placed into an incubator overnight at 37°C.

On Day 2, the plate is removed from incubation. Using wide bore pipet tips, the cells are gently pipetted to dislodge the spheroid clusters. The clusters are filtered through a 40 µm or 80 µm cell strainer to remove extraneous detached single cells and then spun down in a centrifuge for 2 x 1 minute. The clusters are resuspended gently using wide bore pipet tips and are gently stirred to distribute them throughout the medium or another material (e.g., alginate).

Alternatively, ARPE-19 spheroids are prepared using the following protocol. On Day 1, AggreWell^{™} plates are removed from the packaging in a sterile tissue culture hood. 2 mL of Aggrewell^{™} Rinsing solution is added to each well. The plate is centrifuged at 2,000 g for 5 minutes to remove air bubbles, and the AggreWell^{™} Rinsing Solution is removed from the wells. Each well is rinsed with 2 mL of the complete growth medium, and 2 million engineered ARPE-19 cells in 3.9 mL of the complete growth medium is added to each well. The plate is centrifuged at 100 g for 3 minutes, then the cells are incubated the cells at 37° C for 48 hours. On Day 3, the same protocol described above is used to dislodge the spheroid clusters.

Alternatively, ARPE19 spheroids are prepared using a PBS MINI bioreactor (PBS Biotec, Inc., Camarillo CA, USA) with the following protocol. Cell culture media and 220 million ARPE19 cells are added into a PBS 0.1 L or PBS 0.5 L vessel which is then inserted into the base unit which is placed in an incubator. The PBS MINI speed adjust dial is set at 40 rpm and the vessel is incubated at 37°C for at least 48 hours prior to collection of spheroids as described above.

### Example 4: Preparation of hydrogel capsules used in the Examples below

*Capsules encapsulating RPE cells as single cells.* Immediately before encapsulation, single ARPE-19 cells, engineered to express a therapeutic protein were centrifuged at 1,400 r.p.m. for 1 min and washed with calcium-free Krebs-Henseleit (KH) Buffer (4.7 mM KCl, 25 mM HEPES, 1.2 mM KH₂PO₄, 1.2 mM MgSO₄ × 7H₂O, 135 mM NaCl, pH ≈ 7.4, ≈290 mOsm). After washing, the cells were centrifuged again and all of the supernatant was aspirated. In some experiments, the cell pellet was then resuspended in the 70:30 CM-LMW-Alg:U-HMW-Alg solution described in Example 2 (control capsules) or one of the modified alginate solutions described in Table 6 of Example 2 at the desired density of suspended single cells per ml alginate solution.

Prior to fabrication of one-compartment and two-compartment hydrogel capsules, buffers and alginate solutions were sterilized by filtration through a 0.2-µm filter using aseptic processes.

For fabrication of one-compartment hydrogel capsules of about 1.5 mm diameter encapsulating cells, the desired number of cells were suspended in the desired alginate solution (e.g., the 70:30 CM-LMW-Alg:U-HMW-Alg solution used in Control capsules or one of the experimental alginate solutions described in Example 2 and Table 6) the resulting cell suspension was loaded into a syringe and capped with an 18-gauge blunt tipped needle (SAI Infusion Technologies). The syringe was placed onto a syringe pump oriented vertically above a dish containing a cross-linking solution. A high voltage power generator was connected to the needle and grounded to the biosafety cabinet. The syringe pump and power generator were turned on to extrude the alginate solution through the needle with a flow-rate of 0.16 mL/min or 10 mL/hr and adjusting the voltage in a range of 5-9 kV until there was a droplet rate of 12 droplets per 10 seconds.

To prepare devices configured as two-compartment hydrogel millicapsules of about 1.5 mm diameter, an electrostatic droplet generator was set up as follows: an ES series 0-100-kV, 20-watt high-voltage power generator (EQ series, Matsusada, NC, USA) was connected to the top and bottom of a coaxial needle (inner lumen of 22G, outer lumen of 18G, Ramé-Hart Instrument Co., Succasunna, NJ, USA). The inner lumen was attached to a first BD disposable 5-ml syringe with BD Luer-Lok^{™} tip (BD (Cat. No. 309646), Franklin Lakes, NJ, USA), which was connected to a syringe pump (Pump 11 Pico Plus, Harvard Apparatus, Holliston, MA, USA) that was oriented vertically. The outer lumen was connected via a luer coupling to a second 5-ml Luer-lock syringe which was connected to a second syringe pump (Pump 11 Pico Plus) that was oriented horizontally. To encapsulate cells only in the first (inner) compartment, a first alginate solution (70:30 CM-Alg-101:UM-Alg solution (as a control) or one of the experimental alginate solutions described in Example 2 and Table 6) comprising the cells (as single cell suspension) was placed in the first syringe and a second cell-free alginate solution comprising an afibrotic compound (e.g., a mixture of CM-LMW-Alg-101 and an U-HMW-Alg) was placed in the second syringe. For control 2-compartment hydrogel capsules in the Examples below, the second (outer) compartment was formed using the 70:30 CM-LMW-Alg-101:U-HMW-Alg solution. The two syringe pumps move the first and second alginate solutions from the syringes through both lumens of the coaxial needle and single droplets containing both alginate solutions are extruded from the needle into a glass dish containing a cross-linking solution. The settings of each Pico Plus syringe pump were 12.06 mm diameter and the flow rates of each pump were adjusted to achieve a flow rate ratio of 1:1 for the two alginate solutions. Thus, with the total flow rate set at 10ml/h, the flow rate for each alginate solution was about 5 mL/h.

For fabrication of both the two-compartment and one-compartment millicapsules, after extrusion of the desired volumes of alginate solutions, the alginate droplets were crosslinked for five minutes in a cross-linking solution which contained 25mM HEPES, 20 mM BaCl₂, 0.2M mannitol, and poloxamer 188. Capsules that had fallen to the bottom of the crosslinking vessel were collected by pipetting into a conical tube. After the capsules settled in the tube, the crosslinking buffer was removed, and capsules were washed. Capsules without cells were washed four times with HEPES buffer (NaCl 15.428 g, KCl 0.70 g, MgCl₂·6H₂O 0.488 g, 50 ml of HEPES (1 M) buffer solution (Gibco, Life Technologies, California, USA) in 2 liters of deionized water) and stored at 4 °C until use. Capsules encapsulating cells were washed four times in HEPES buffer, two times in 0.9% saline, and two times in culture media and stored in an incubator at 37°C.

### Example 5: Effect of incorporating cell binding sites within a single compartment alginate capsule encapsulating cells on in vitro cell productivity and viability

Compositions containing single-compartment hydrogel millicapsules (about 1.5mm in diameter) were prepared essentially as described in Example 4, using alginate solutions described in Example 2, which lacked or contained cell binding sites.

ARPE-19 cells engineered to express a FVIII-BDD protein (SEQ ID NO:1) (ARPE-19:FVIII-BDD cells) were suspended at 3.3 million cells/ml alginate in the control alginate solution (70:30 CM-LMW-Alg-101:U-HMW-Alg) or one of the experimental alginate solutions described in Example 2 and Table 6. After preparation, one capsule per well of each capsule composition was placed into a 96-well plate. The supernatants were collected 24h later and the concentration of FVIII in each supernatant was measured by ELISA. Also, at this time, the number of viable cells present in two aliquots of one capsule per well for each capsule composition was measured by a CellTiter Glo^{®} viability assay (Promega Corporation, Madison, WI USA), Productivity (pg/cell/day) was calculated by dividing the pg of protein in the supernatant by the cell number in each aliquot. The results of this analyses are shown in FIG 2.

As shown in FIG. 2, cells encapsulated in an alginate solution covalently modified with a cell-binding peptide (e.g., a CBP-alginate) did not show increased productivity compared to the control (e.g., alginate lacking a CBP). Additionally, alginate blends with Collagen I and HA also did not improve productivity of the encapsulated cells. These results demonstrate that the *in vitro* productivity of encapsulated cells is not affected by the presence of cell binding sites in the encapsulating alginate polymer.

### Example 6: Encapsulating ARPE19:FVIII-BDD cells in an RGD-modified alginate in the inner capsule compartment increases FVIII-BDD plasma levels after capsule implant

Compositions of control and RGD-modified two-compartment hydrogel millicapsules encapsulating ARPE19:FVIII-BDD cells were prepared by extruding first and second alginate solutions through a coaxial needle as described in Example 4. The solution for the second (outer) compartment was the cell-free 70:30 CM-LMW-Alg-101:U-HMW-Alg solution and the solution for the first (inner) compartment was a suspension of ARPE19:FVIII-BDD cells in the 70:30 CM-LMW-Alg-101:U-HMW-Alg solution (control) or in one of the RGD-modified alginate solutions described in Example 2 (VLVG-4GRGD and 1G-RGD: 0.41). ARPE19:FVIII-BDD cells were suspended at 100 million cells/ml in the first alginate solution (inner compartment solution).

A 0.5 mL aliquot of each capsule composition was implanted into the intraperitoneal (IP) space of nude mice (4 mice per composition). At 2 weeks post-implantation, blood samples were collected from each mouse via an interim bleed. Plasma levels of FVIII-BDD in these samples were measured by ELISA and the results are shown in FIG 3.

Plasma FVIII-BDD levels were higher for both RGD-modified alginate capsules compared to the control, indicating that the presence of an RGD -alginate within the first compartment can increase FVIII-BDD plasma levels in *vivo.*

### Example 7: Encapsulating cells in a polymer comprising cell binding sites in the inner capsule compartment does not affect in vivo cell proliferation or ex vivo capsule mechanical properties

Compositions of two-compartment control and capsules containing a CBP and encapsulating ARPE19:FVIII-BDD cells in the first (inner) compartment were prepared as described in Example 6. Similar two-compartment capsules were also prepared in which the outer compartment was the same, but the inner compartments were formed from the Collagen I or HA alginate solutions described in Example 2 and Table 6 above. For all control and test capsules, the concentration of suspended cells in the solution used to form the inner compartment was 100 million cells/ml.

A 0.5 mL aliquot of each capsule composition was implanted into the IP space of nude mice (4 mice per composition). At 4 weeks post-implantation, the large majority of the capsules were retrieved from the mice and capsule cell numbers (one capsule in duplicate for each mouse) was measured using a CellTiter Glo^{®} 3D Cell Viability Assay (Promega Corporation, Madison, WI USA). Briefly, one capsule per well was analyzed in duplicate and compared to a standard curve of plated cells. 100µl of the CellTiter Glo^{®} 3D reagent was added to the each well containing 100µl of medium, the plate was placed onto a shaker at 400rpm for 15 minutes and then luminescence was read on a plate reader. Also, a texture analyzer was used to measure the mechanical strength (initial fracture) of the capsules in aliquots of each composition at pre-implantation and upon retrieval after the 1-month implantation period. The cell number and capsule strength results are shown in FIG. 4A and FIG. 4B, respectively. There was some variability observed due to experimental error.

In the control and CBP capsules, some cell growth during the one-month implantation period was observed, but the cell number in the retrieved capsules was similar for the control and CBP-alginate groups. Cell numbers in the Collagen and HA capsules did not significantly change during the one month implantation period. Although the magnitude of the proliferation varies across groups, other data (not shown) suggests that cell numbers plateau around ~50,000 cells/capsule, as shown in FIG. 4A, regardless of starting cell concentration. Thus, the data in FIG. 4A suggest that the GRGDSP (SEQ ID NO: 60) peptide is not stimulating significant proliferation *in vivo* beyond what is observed in control capsules.

The initial fracture measured at pre-implantation varied across the capsule compositions: a first (inner) compartment comprised of SLG20 (i.e., 1G-RGD, HA, Collagen I) created a stronger capsule compared to the Control and VLVG-4GRGD capsules. However, the initial fracture strength determined at retrieval after the one-month implantation was similar for capsules in the Control and all test capsule compositions. These data suggest that the presence of cell binding domains within the capsule does not affect mechanical integrity of the capsules.

### Example 8: Encapsulating ARPE19-FVIII-BDD cells in a G-RGD-alginate in the inner capsule compartment increases FVIII-BDD plasma levels after capsule implant

Compositions of two-compartment hydrogel millicapsules encapsulating ARPE19:FVIII-BDD cells were prepared by extruding first and second alginate solutions through a coaxial needle as described in Example 4. The second (outer) compartment was prepared using the 70:30 CM-LMW-Alg-101:U-LMW-Alg solution and the inner compartment contained the 70:30 CM-LMW-Alg-101:U-LMW-Alg solution (Control) or one of the following CBP-modified alginate solutions described in Example 2 and Table 6: MVG-GRGDSP, 1G-RGD:0.41, 4G-RGD:0.53. For all control and test capsules, the concentration of cells in the alginate solution used to form the inner compartment was 100 million cells/ml.

A 0.5 mL aliquot of each of capsule composition was implanted into the IP space of nude mice (4 mice per composition). At 14 days post-implantation, animals were sacrificed and blood samples were collected from each mouse. Plasma levels of FVIII-BDD in these samples was measured by ELISA; the results are shown in FIG. 5.

Plasma FVIII-BDD levels were higher in mice implanted with capsules containing an alginate covalently modified with an RGD peptide via a single G linker than in mice implanted with control capsules or capsules containing an alginate covalently modified with the same RGD peptide via a 4 G linker. There was no significant difference between two RGD alginates with the same RGD peptide, but different MW alginates (MVG-GRGDSP and 1G-RGD:0.41) or between a mixture of the MVG-GRGDSP alginate and M REDV alginate. Surprisingly, the 4G-RGD:0.53 alginate, which was modified with the same RGD peptide (RGDSP (SEQ ID NO: 59)) as in the 1G-RGD:0.41 alginate, but with a 4 G linker instead of a single G linker, appeared to have a negative impact on cell productivity: plasma FVIII-BDD was undetectable in mice implanted with the 4G-RGD:0.53 capsules. These data indicate that plasma levels of a therapeutic protein expressed by implanted engineered RPE cells in an animal model can be increased by implanting the cells via a device (e.g., a hydrogel millicapsule) that encapsulates the engineered RPE cells in a polymer (e.g., alginate) conjugated to an RGD cell binding peptide via a single G linker.

### Example 9: Presence of an RGD-alginate in the inner capsule compartment does not impact fibrosis of implanted capsules

This experiment examined whether the presence of a cell-binding peptide in the inner compartment and medium or high density of an afibrotic compound in the outer compartment affected the level of fibrosis after capsule implant into an animal model. The capsule compositions used in this experiment had the 2-compartment capsule configurations described below.
(1) Control: inner compartment formed from a solution comprising a suspension of ARPE19-FVIII-BDD cells in the 70:30 CM-LMW-Alg-101-Medium:U-HMW-Alg mixture, outer compartment formed from 70:30 CM-LMW-Alg-101-Medium:U-HMW-Alg mixture;
(2) RGD Inner / Medium Outer: inner compartment formed from a solution comprising a suspension of ARPE19-FVIII-BDD cells in 1G-RGD:0.41 (Table 6), outer compartment formed from 70:30 CM-LMW-Alg-101-Medium:U-HMW-Alg mixture;
(3) RGD Inner / High Outer: inner compartment formed from a solution comprising a suspension of ARPE19-FVIII-BDD cells in 1G-RGD:0.41 (Table 6), outer compartment formed from 70:30 CM-LMW-Alg-101-High:U-HMW-Alg mixture;
(4) RGD Inner / High (60:40) Outer: inner compartment formed from a solution comprising a suspension of ARPE19-FVIII-BDD cells in 1G-RGD:0.41 (Table 6), outer compartment formed from 60:40 CM-LMW-Alg-101-High:U-HMW-Alg mixture; and
(5) RGD, Empty: Inner / Medium Outer: inner compartment formed from 1G-RGD:0.41 (Table 6), outer compartment formed from 70:30 CM-LMW-Alg-101-Medium:U-HMW-Alg mixture, but no cells were encapsulated.

A 0.5 mL aliquot of each capsule composition was implanted into the IP space of C57/BL6 mice (4 mice per composition), and retrieved after 11 days. The retrieved capsules were imaged by brightfield microscopy, and the results are shown in FIG. 6.

There was no difference in fibrosis for capsules that had medium density afibrotic compound in the second (outer) compartment and a first compartment encapsulating cells in an afibrotic alginate:unmodified alginate mixture (Control) or an RGD-alginate. As expected, implanted capsules without cells (RGD (empty)) exhibited less fibrosis in mice than capsules containing the xenogenic RPE cells. Varying the density of the afibrotic compound in the second (outer) compartment by using high density CM-LMW Alginate decreased fibrosis, while changing the CM-LMW:U-HMW blend ratio from 70:30 to 60:40 increased fibrosis, as expected. Therefore, the presence of a cell binding peptide within the first (inner) compartment does not impact the afibrotic property of the capsules.

### Example 10: Encapsulating ARPE19:FIX-Padua cells in a G-RGD-alginate in inner capsule

### compartment increases plasma FIXpadua levels after implant

Compositions of two-compartment hydrogel millicapsules encapsulating ARPE19:FIX-Padua cells were prepared by extruding first and second alginate solutions through a coaxial needle as described in Example 4. The second (outer) compartment was formed from the 70:30 CM-LMW-Alg-101-Medium:U-HMW-Alg solution and the inner compartment was formed from one of the following alginate solutions, each of which contained 100 million ARPE19:FIX-Padua cells/ml:
(1) Control: 70:30 CM-LMW-Alg-101-Medium:U-HMW-Alg mixture;
(2) 1G-RGD: 0.41
(3) 4G-RGD: 0.53
(4) 4G-RGD: 0.96
(5) 4G-RGD: 1.77
(6) RGD-4G: 2.95
A 0.5 mL aliquot of each capsule composition was implanted into the IP space of nude mice (4 mice per composition). At 12 days post-implantation, animals were sacrificed and blood samples were collected from each mouse. Plasma levels of FIX in these samples was measured by ELISA.

As shown in FIG. 7, post-implant plasma FIX levels were significantly higher for capsules containing the 1G-RGD:0.41 alginate or the 4G-RGD:0.53 alginate in the inner compartment compared to the control and capsules with the higher density 4G-RGD alginate in the inner compartment. Surprisingly, implant of the 1G-RGD:0.41 capsules resulted in significantly higher plasma FIX levels than implant of the 4G-RGD:0.53capsules, demonstrating that the composition of the RGD linker can affect cell productivity with respect to the amount of therapeutic protein secreted from the capsules. Finally, capsules prepared using the alginate polymers with higher concentrations of the 4G linker peptide (SEQ ID NO: 61) all had similar FIX levels to the control. Together, these data demonstrate that implanting 2-compartment hydrogel capsules comprising RPE cells engineered to express a FIX protein and encapsulated in an RGD-alginate within the first (inner) compartment can significantly increase plasma FIX levels in an animal model, and suggest that some combinations of RGD density and linker size may not work as well as an alginate modified with 1G-RGDSP at about 0.41% N.

### Example 11. Post-implant plasma FIX levels are higher using a blend of an RGD-alginate and a second CBP-alginate to encapsulate ARPE19:FIX-Padua cells in the inner capsule compartment

Compositions of two-compartment hydrogel millicapsules encapsulating ARPE19:FIX-Padua cells were prepared by extruding first and second alginate solutions through a coaxial needle as described in Example 4. The second (outer) compartment was formed from the 70:30 CM-LMW-Alg-101-Medium:U-HMW-Alg solution and the inner compartment was formed from one of the following alginate solutions, each of which contained 100 million ARPE19:FIX-Padua cells/ml.
(1) Control: 70:30 CM-LMW-Alg-101-Medium:U-HMW-Alg mixture;
(2) 1G-RGD: 0.41 (Table 6)
(3) 4G-DGEA (Table 6)
(4) 1G-RGD/4G-DGEA: a 1:1 blend of the 1G-RGD: 0.41 solution and the 4G-DGEA solution
(5) 1G-RGD/4G-PHSRN: a 1:1 blend of the 1G-RGD: 0.41 solution and the 4G-PHSRN solution
(6) 1G-RGD/4G-DGEA/4G-PHSRN: a 1:1:1 blend of the 1G-RGD: 0.41, 4G-DGEA and PHSRN solutions.
A 0.5mL aliquot of each capsule composition was implanted into the IP space of nude mice (4 mice per composition). At 2 weeks post-implantation, animals were sacrificed and blood samples were collected from each mouse. Plasma levels of FIX in these samples was measured by ELISA.

As shown in FIG. 8, the plasma levels of FIX were higher for the capsules containing the 1G-RGD-alginate or DGEA-alginate compared to the control, with the higher levels in the RGD capsules reaching statistical significance with a one-way ANOVA. Although it did not reach statistical significance, the 1G-RGD alginate was still substantially higher than the 4G-DGEA capsules. The FIX plasma levels were highest for the three groups of capsules containing the RGD alginate blended with another CBP-alginate. These data demonstrates that capsules containing ARPE19-FIX-Padua cells and combinations of multiple CBP-alginates can improve the FIX levels compared to capsules created with a single CBP-alginate (RGDSP (SEQ ID NO: 59) or DGEA (SEQ ID NO: 39)).

### Example 12. Density of an RGD peptide in the inner compartment encapsulating ARPE19:FIX-Padua cells affects FIX plasma levels produced by implanted hydrogel capsules

Compositions of two-compartment hydrogel millicapsules encapsulating ARPE19:FIX-Padua cells were prepared by extruding first and second alginate solutions through a coaxial needle as described in Example 4. The second (outer) compartment was formed from the 70:30 CM-LMW-Alg-101-Medium:U-HMW-Alg solution and the inner compartment was formed from one of the following alginate solutions described in Table 6, each of which contained 100 million ARPE19:FIX-Padua cells/ml:
(1) Control: 70:30 CM-LMW-Alg-101-Medium:U-HMW-Alg mixture;
(2) 1G-RGD: 0.22
(3) 1G-RGD: 0.33
(4) 1G-RGD: 0.72
(5) 1G-RGD: 1.47
A 0.5mL aliquot of each capsule composition was implanted into the IP space of nude mice. At 2 weeks post-implantation, animals were sacrificed and blood samples were collected from each mouse. Plasma levels of FIX in these samples was measured by ELISA.

As shown in FIG. 9, the amount of RGD conjugation to the first (inner) compartment polymer affected the levels of FIX detected in the plasma. FIX levels increased as the RGD density increased from 0 to 1G-RGD: 53 but, surprisingly, the FIX levels were successively lower following implant of the 1G-RGD: 0.72 and 1G-RGD: 1.47 capsules. Together, these data demonstrate that the conjugation density of the RGD peptide can have a significant effect on FIX levels produced by hydrogel capsules, and that further, for capsules configured as desribed in this Example, capsules prepared with 1G-RGD: 0.33 in the inner compartment may be optimal for achieving the highest plasma FIX-Padua levels.

### Example 13. Encapsulating ARPE19:PTH cells in a 1G-RGD-alginate in inner capsule compartment increases plasma PTH levels after implant

Compositions of control and RGD-modified two-compartment hydrogel millicapsules encapsulating ARPE19:PTH cells were prepared by extruding first and second alginate solutions through a coaxial needle as described in Example 4. The alginate solution for the second (outer) compartment was the cell-free 70:30 CM-LMW-Alg-101:U-HMW-Alg solution and the alginate solution for the first (inner) compartment was a suspension of ARPE19:FVIII-PTH cells at 100 million cells/ml in the 70:30 CM-LMW-Alg-101:U-HMW-Alg solution (control) or an RGD-modified alginate solution (1G-RGD: 0.72%) described in Table 6.

An aliquot of 0.5mL of each capsule composition was implanted into the intraperitoneal (IP) space of nude mice (4 mice per composition). At 1 week post-implantation, animals were sacrificed and blood samples were collected from each mouse. Plasma levels of PTH in these samples were measured by ELISA and the results are shown in FIG 10.

Plasma PTH levels were approximately two-times higher for the RGD-modified alginate capsules compared to the control, indicating that the presence of an RGD -alginate within the first compartment can increase PTH plasma levels in *vivo.*

### Example 14: Capsules containing ARPE19:FVIII cells within a G-RGD-alginate in inner capsule compartment have stability comparable to a no RGD control

Compositions of two-compartment hydrogel millicapsules encapsulating ARPE19:FVIII cells were prepared by extruding first and second alginate solutions through a coaxial needle as described in Example 4. Compositions of capsules were prepared with the following combinations of first and second compartment solutions: (1) first and second: CM-LMW-Alg-101-Medium:U-HMW-Alg solution (the control condition), (2) first: 1G-RGD: 0.41, second: CM-LMW-Alg-101-Medium:U-HMW-Alg solution, (3) first: 1G-RGD: 0.41, second: CM-LMW-Alg-101-High:U-HMW-Alg solution, (4) first: 1G-RGD: 0.41, second: CM-LMW-Alg-101-High:U-HMW-Alg solution prepared at a 60:40 volume ratio of CM-LMW-ALG-101 to High:U-HMW, and (5) first: 1G-RGD: 0.41, second: CM-LMW-Alg-101-Medium:U-HMW-Alg solution. The ARPE19:FIX cells were encapsulated in the first (inner) compartment at 100 million cells/ml for conditions (1-4) above. A 0.5mL aliquot of each capsule composition was implanted into the IP space of nude mice. At 2 months post-implantation, animals were sacrificed and the capsules were collected from each mouse. Capsules were imaged and a live/dead stain was performed. The data are shown in FIG. 11.

All capsules were retrieved intact and viable cells were detected. There were visible differences in densities of cells within the first compartment for control compared to GRGDSP-containing capsules (SEQ ID NO: 60), but the total number of viable cells was similar (data not shown). Together, these data demonstrate that the incorporation of RGD within the first compartment does not negatively affect capsule integrity.

### Example 15: Capsules containing Balb/3T3 cells within a G-RGD-alginate in inner capsule compartment are equally afibrotic to no RGD control through 2 month implantation in C57/BL6

Compositions of two-compartment hydrogel millicapsules encapsulating ARPE19:FVIII cells were prepared by extruding first and second alginate solutions through a coaxial needle as described in Example 4. Compositions of capsules were prepared with the following combinations of first and second compartment solutions as described in Example 14. The Balb/3T3 cells were encapsulated in the first (inner) compartment at 20 million cells/ml for conditions (1-4) above. A 0.5mL aliquot of each capsule composition was implanted into the IP space of C57/BL6 mice. At 2 months post-implantation, capsules were retrieved and imaged by brightfield microscopy for the presence of fibrosis.

All capsules were retrieved intact and were largely lacking fibrosis, as shown in FIG. 12 where the empty, clear outer layer is not covered in opaque, fibrotic deposition. More fibrosis was observed on the capsules prepared with the 60:40 blend of CM-LMW-Alg-101-High:U-HMW-Alg compared to the 70:30 blend, as expected. Together, these data demonstrate that the incorporation of RGD within the first compartment does impact the afibrotic nature of the capsules within an allogeneic context.

### Example 16: Identity of a G-RGD-modified polymer in the inner compartment encapsulating ARPE19:FIX-Padua cells affects FIX plasma levels produced by implanted hydrogel capsules

Compositions of two-compartment hydrogel millicapsules encapsulating ARPE19:FIX-Padua cells were prepared by extruding first and second alginate solutions through a coaxial needle as described in Example 4. The second (outer) compartment was formed from the 70:30 CM-LMW-Alg-101-Medium:U-HMW-Alg solution and the inner compartment was formed from one of the following alginate solutions using different initial polymers as described in Table 6, each of which contained 100 million ARPE19:FIX-Padua cells/ml:
(1) 1G-RGD: 0.72
(2) 1G-MVG: 0.71, 79 cP
(3) 1G-MVG: 0.71, 97 cP
(4) 1G-MVG: 0.71, 143 cP
(5) 1G-MVG: 0.71, 202 cP
(6) 1G-SLG100: 0.65

A 0.5mL aliquot of each capsule composition was implanted into the IP space of nude mice. At 4 weeks post-implantation, animals were sacrificed and capsules and blood samples were collected from each mouse. Plasma levels of FIX in these samples was measured by ELISA. The initial fracture of the capsules at pre-implantation and ex-vivo retrieval were measured using a texture analyzer.

The initial fracture of capsules varied with polymer identity and solution viscosity prior to implantation. All capsules were retrieved intact with similar ex vivo mechanical strength as shown in FIG. 13A. As shown in FIG. 13B, the plasma levels of FIX-padua were highest for the capsules containing the G-RGD: 0.72 compared to the G-SLG100: 0.65 or the G-MVG: 0.71 at any of the four viscosities (79, 97, 143, 202 cP). Together, these data demonstrate that the identity of the polymer with conjugated RGD peptide can have a significant effect on FIX levels produced by hydrogel capsules, and that further, for capsules configured as desribed in this Example, the SLG20 polymer may be optimal for achieving the highest plasma FIX levels.

### Example 17: Encapsulating ARPE19:FIX cells in a 1G-HAVDI-alginate in inner capsule compartment increases plasma FIX levels after implant

Compositions of control and HAVDI-modified (SEQ ID NO: 38) two-compartment hydrogel millicapsules encapsulating ARPE19:FIX cells were prepared by extruding first and second alginate solutions through a coaxial needle as described in Example 4. The alginate solution for the second (outer) compartment was the cell-free 70:30 CM-LMW-Alg-101:U-HMW-Alg solution and the alginate solution for the first (inner) compartment was a suspension of ARPE19:FIX cells at 100 million cells/ml in the 70:30 CM-LMW-Alg-101:U-HMW-Alg solution (control) or an HAVDI-modified alginate solution (G-HAVDI: 0.38%) described in Table 6.

An aliquot of 0.5mL of each capsule composition was implanted into the intraperitoneal (IP) space of nude mice (4 mice per composition). At 2 week post-implantation, samples were collected from an interim bleed on each mouse. Plasma levels of FIX in these samples were measured by ELISA and the results are shown in FIG 14.

Plasma FIX levels were approximately two-times higher for the HAVDI-modified alginate capsules compared to the control, indicating that the presence of an alginate modified with a CBP derived from a cadherin (e.g., G-HAVDI) within the first compartment can increase FIX plasma levels in *vivo.*

### Example 18: A G-RGD-modified polymer in the inner compartment encapsulating ARPE19:FIX-Padua cells increases FIX levels in plasma from IP and SubQ sites

Compositions of two-compartment hydrogel millicapsules encapsulating ARPE19:FIX-Padua cells were prepared by extruding first and second alginate solutions through a coaxial needle as described in Example 4. The second (outer) compartment was formed from the 70:30 CM-LMW-Alg-101-Medium:U-HMW-Alg solution and the inner compartment was formed from G-RGD: 0.33 as described in Table 6, each of which contained 100 million ARPE19:FIX-Padua cells/ml.

Capsules were implanted into IP and SubQ sites of nude mice at 0.2mL capsules/animal. Animals were sacrificed after 10 days when plasma samples and capsules were collected. FIX levels in the plasma were determined by ELISA and number of viable cells per capsule was determined by Cell Titer Glo.

As shown in FIG. 15A, FIX was detected in the plasma from all conditions, but levels were higher for the animals with capsules implanted IP rather than SubQ. The FIX levels were higher for the G-RGD containing capsules compared to the control for both the IP and SubQ site. The number of viable cells per capsule was slightly higher for capsules retrieved from IP compared to SubQ, as shown in FIG. 15B. Together, this demonstrates that CBP-containing capsules can increase serum levels of a therapeutic protein from multiple routes of administration.

### Example 19: A G-RGD-modified polymer in the inner compartment encapsulating ARPE19:FVII cells causes detectable FVII from the IP and SubQ sites

Capsules encapsulating ARPE19:FVII cells were prepared with 1G-RGD: 0.33 and implanted as described in Example 18. FIG. 16 shows that FVII was detectable in the plasma from both the IP and SubQ sites, demonstrating that capsules containing 1G-RGD: 0.33 can be used for delivery of FVII and that capsules can be implanted either IP or SubQ.

### Example 20: Capsules prepared with a blend of highly conjugated G-RGD alginate with unmodified alginate achieves FIX similar levels to the 1G-RGD: 0.33 alginate.

Compositions of two-compartment hydrogel millicapsules encapsulating ARPE19:FIX-Padua cells were prepared by extruding first and second alginate solutions through a coaxial needle as described in Example 4. The second (outer) compartment was formed from the 70:30 CM-LMW-Alg-101-Medium:U-HMW-Alg solution and the inner compartment was formed from one of the following alginate solutions using different initial polymers as described in Table 6, each of which contained 100 million ARPE19:FIX-Padua cells/ml:
(1) 1G-RGD: 0.33
(2) 1G-RGD: 6.04, 1:55
(3) 1G-RGD: 6.04, 1:30
(4) 1G-RGD: 6.04, 1:15

A 0.5mL aliquot of each capsule composition was implanted into the IP space of nude mice. At 2 weeks post-implantation, animals were sacrificed and capsules and blood samples were collected from each mouse. Plasma levels of FIX in these samples was measured by ELISA.

As shown in FIG. 17, the plasma levels of FIX-padua were highest for the capsules containing the 1G-RGD: 0.33 and the G-RGD: 6.04, 1:30 (0.20% N) compared to the G-RGD: 6.04, 1:55 and G-RGD: 6.04: 1:15. Together, these data demonstrate that the optimal RGD conjugation levels can be mimicked by blending a highly conjugated polymer with an unconjugated polymer to the approximately the same levels. Additionally, with the blended solutions described in this Example, there is a maximum FIX level at an intermediate conjugation level similar to that described in Example 12.

### Example 21: 1G-RGD must be contained within the first compartment to prevent fibrosis

Compositions of two-compartment hydrogel millicapsules encapsulating ARPE19:FIX-Padua were prepared by extruding first and second alginate solutions through a coaxial needle as described in Example 4. Compositions of capsules were prepared with the following combinations of first and second compartment solutions and encapsulated 20 million ARPE19:FIX cells/ml in the first compartment: (1) first: unmodified 1.4 w/v% SLG20, second: CM-LMW-Alg-101-High:U-HMW-Alg , (2): first: 1G-RGD: 0.33, second: CM-LMW-Alg-101-High:U-HMW-Alg, and (3): first: unmodified 1.4w/v% SLG20 blended with unconjugated G-RGD peptide to achieve 0.33% N in the solution, and (4): first: unmodified 1.4w/v% SLG20, second: 1G-RGD: 0.33.

Capsules were implanted at 0.5mL/animal into the IP space of C57/BL6 mice for 2 weeks. At sacrifice, the capsules were collected. Retrieved capsules were stained for mouse macrophages with anti-F4/80 and imaged by fluorescent microscopy for the presence of fibrosis.

As shown in FIG. 18, similar macrophage adhesion was observed for capsules with a control, conjugated 1G-RGD: 0.33 or unconjugated 0.33% N G-RGD peptide in the first compartment. Notably, the capsules containing the unconjugated 0.33% N G-RGD within the first compartment did not have increased macrophage adhesion compared to the control, demonstrating that unconjugated G-RGD does not drive a fibrotic response. Importantly, there was increased macrophage adhesion for the capsules where the G-RGD: 0.33 alginate was used for the second (outer) compartment, demonstrating that the CBP-alginate must be contained within the first (inner) compartment of the capsule to prevent fibrosis.

### Example 22: Encapsulating ARPE19:FIX cells with G-RGD and G-PHSRN either on two polymers blended together or as conjugated to the same polymer result in similar plasma FIX levels after implant

Compositions of two-compartment hydrogel millicapsules encapsulating ARPE19:FIX-Padua cells were prepared by extruding first and second alginate solutions through a coaxial needle as described in Example 4. The second (outer) compartment was formed from the 70:30 CM-LMW-Alg-101-Medium:U-HMW-Alg solution and the inner compartment was formed from one of the following alginate solutions, using different initial polymers as described in Table 6, each of which contained 100 million ARPE19:FIX-Padua cells/ml:
(1) 1:1 blend of 1G-RGD: 0.72 with G-PHSRN: 0.83; and
(2) PHSRN/RGD, same polymer (0.87% N overall).

A 0.5mL aliquot of each capsule composition was implanted into the IP space of nude mice. At 2 weeks post-implantation, animals were sacrificed and capsules and blood samples were collected from each mouse. Plasma levels of FIX in these samples was measured by ELISA.

As shown in FIG. 19, the plasma levels of FIX were similar for both compositions of capsules containing both G-RGD and G-PHSRN.

### Example 23: Evaluation of linker composition on productivity of ARPE19-FIX cells encapsulated in RGDSP-conjugated alginate

Compositions of two-compartment hydrogel millicapsules encapsulating ARPE19:FIX cells were prepared by extruding first and second alginate solutions through a coaxial needle as described in Example 4. The second (outer) compartment was formed from the 70:30 CM-LMW-Alg-101-High:U-HMW-Alg solution and the inner compartment was formed from RGD-alginate solutions with the RGDSP peptide (SEQ ID NO: 59) conjugated to the alginate with no linker or with a linker consisting of 1, 2, 3, 4 or 6 glycine residues (1G, 2G, 3G, 4G (SEQ ID NO: 61), 6G (SEQ ID NO: 69)), or a linker consisting of one β-alanine residue. The peptide conjugation level was determined by elemental analysis after lyophilization for percent nitrogen content to afford: 0.19% N for no linker, 0.30% N for a 1G linker, 0.32% N for a 2G linker, 0.29% N for a 3G linker, 0.48% N for a 4G linker (SEQ ID NO: 61), 0.43% N for a 6G linker, and 0.28% N for the 1 β-alanine linked RGDSP peptide (SEQ ID NO: 59). The ARPE19:FIX cells were encapsulated in the first (inner) compartment at 100 million cells/mL. A 0.25 mL aliquot of each capsule composition was implanted into the IP space of nude mice (n=3/group). At 12 days post-implantation, animals were sacrificed and plasma collected from each mouse. Plasma levels of FIX in these samples was measured by ELISA.

As shown in FIG.22, the choice of linker used to conjugate the RGDSP peptide (SEQ ID NO: 59) to the first (inner) compartment polymer affected the levels of FIX detected in the plasma. Plasma FIX levels produced by the capsules dropped significantly as the number of glycine linker residues in the peptide-modified alginate increased from 3 to 4, and from 4 to 6. In contrast, similar levels of plasma FIX levels were produced by capsules with alginate in which the peptide RGDSP (SEQ ID NO: 59) was attached via no linker, or via any of the 1G, 2G, 3G or 1-β-alanine linkers.

### Example 24: Low to moderate viscosity of GRDGSP-conjugated alginate maximizes FIX levels

Compositions of two-compartment hydrogel millicapsules encapsulating ARPE19:FIX cells were prepared by extruding first and second alginate solutions through a coaxial needle as described in Example 4. The second (outer) compartment was formed from the 70:30 CM-LMW-Alg-101-High:U-HMW-Alg solution and the inner compartment was formed from RGD-alginate solutions with the GRGDSP peptide (SEQ ID NO: 60) conjugated to the alginate. The RGD-alginate solution was formulated to different solution viscosities: 91, 112, 142, 228, 366, 453 cP. The peptide conjugation level was determined by elemental analysis after lyophilization for percent nitrogen content to afford: 0.38% N for the 91 cP, 142 cP, 228 cP samples and 0.32% N for the 112 cP, 366 cP, and 453 cP samples, respectively. These RGD-alginate solutions were used to encapsulate ARPE19:FIX cells in the first (inner) compartment at 100 million cells/mL. A 0.25mL aliquot of each capsule composition was implanted into the IP space of nude mice (n=4/group). At 18 days post-implantation, animals were sacrificed and plasma collected from each mouse. Plasma levels of FIX in these samples was measured by ELISA.

As shown in FIG. 23, the viscosity of the first (inner) compartment polymer solution affected the levels of FIX detected in the plasma. ARPE19:FX cells produced higher plasma FIX levels when encapsulated in the low to intermediate viscosity solutions (91 cP to 228 cP) than when encapsulated in the higher viscosity solutions (366 cP and 453 cP).

### Example 25: Exemplary quantitative peptide conjugation assay.

This assay determines the amount of peptide in a CBP-polymer by subjecting a sample of the CBP-polymer to acid hydrolysis, which cleaves off the CBP as individual amino acids. The individual amino acids in the hydrolyzed sample are separated and quantitated using amino acid references by pre-column on-line derivatization and reverse-phase liquid chromatograpy-Ultra-Violet-Fluoscense (LC-UV-FLR) (adapted from Agilent Biocolumns Amino Acid Analysis "How-To" Guide, Agilent Technologies, Inc., 5991-7694EN, published March 1, 2018). Primary AAs (e.g., all but proline of the 20 standard L-alpha amino acids) are derivatized with Ortho-phthaladehyde (OPA) and secondary AAs (e.g., proline) are derivatized with 9-Fluorenylmethyl chloroformate (FMOC). The molar concentration of each amino acid is then averaged to calculate the concentration of the total peptide in the sample. This concentration can be corrected for the presence of any residual unconjugated CBP in the CBP-polymer by determining the amount of peptide in an unhydrolyzed sample of the CBP-polymer using any suitable analytical technique, e.g., as described in Example 26, and subtracting that amount from the total peptide amount.

The assay is further described below as applied to determining peptide conjugation density in a GRGDSP-alginate; however, the skilled artisan can readily modify the assay to determine peptide concentration in a GRGDSP-alginate or other peptide-modified polymers, provided the unmodified polymer does not contain any amino acids. Also, the skilled person can readily substitute any equipment, material or chemical specified below with a different equipment, material or chemical that can perform or provide substantially the same function or role in the assay.

### DEFINITIONS

| **Abbreviation** | **Definition** |
|---|---|
| LC-UV-FLR | Liquid Chromatography-Ultra-Violet-Fluorescence |
| LCMS | Liquid Chromatography-Mass Spectroscopy |
| SLG20 | Pronova Ultrapure SLG20 sterile sodium alginate |
| RT | Retention Time |
| ACN | Acetonitrile |
| MeOH | Methanol |
| SST | System Suitability |
| RSD | Relative Standard Deviation |
| TBD | To Be Determined |
| NMT | No More Than |
| NLT | No Less Than |
| RSQ | Coefficient of Determination |
| AA | Amino acid |
| OPA | Ortho-phthaladehyde |
| FMOC | 9-Fluorenylmethyl chloroformate |
| G | Glycine |
| R | Arginine |
| D | Aspartic acid |
| S | Serine |
| P | Proline |
| iSTD | Internal standard |
| PPE | Personal Protective Equipment |
| SDS | Safety Data Sheet |
| MW | Molecular Weight |
| PTFE | Polytetrafluoroethylene |
| RPM | Revolutions per Minute |
| min | Minutes |
| s | Seconds |
| mL | Millilitre |
| µL | Microlitre |
| nm | Nanometre |

### EQUIPMENT, MATERIALS AND CHEMICALS

### EQUIPMENT

- Agilent 1260 LC system
- Agilent diode array detector (G1315D): 13 µL/10 mm flow cell
- Agilent Fluorescence detector (G1321B)
- ChemStation software
- Hot/stir plate with heating blocks, IKA RCT BS001
- Analytical balance, Mettler Toledo XPE105
- pH meter, Mettler Toledo S220 SevenCompact
- Centrifuge, Beckman Coulter Allegra 25R
- Vortex mixer, VWR 97043-562
- Nitrogen evaporator with heating (acid resistant), Organomation 1156Y80

### MATERIALS

- AdvanceBio AA LC column, 2.7 µm, 4.6x100 mm, Agilent 655950-80
- AdvanceBio AAA guard column, 2.7 µm, 4.6x5 mm, Agilent 820750-931
- Microwave reaction vials with PTFE lined caps, Chemglass CG-4920-02
- 50 mL Centrifuge tubes, VWR 10160-140
- Crimper, Chemglass CG-4930-20
- Decapper, VWR 10806-372
- Graduated cylinders, class A
- Volumetric transfer pipets (to deliver) class A
- Volumetric flasks, class A
- Transfer pipette (200 µL variable volume), Eppendorf 3123000055
- Transfer pipette (1000 µL variable volume), Eppendorf 3123000063
- Transfer pipette (10 mL variable volume), Eppendorf 3123000080
- Disposable transfer pipet, VWR 16001-180
- Stir bar, VWR 58948-078
- LC vial (2 mL), Agilent 5182-0716
- LC vial cap (non-preslit), Agilent 5182-0721
- LC vial insert (250 µL), Agilent 5183-2085
- Various lab glassware

### CHEMICALS

| Name | CAS # | Supplier & PN | Storage |
|---|---|---|---|
| Sodium monophosphate (Na₂HPO₄·7H₂O) | 7782-85-6 | BDH 92960 | ambient |
| Sodium tetraborate (Na₂B₄O₇) | 1330-43-4 | Acros Organics 206291000 | ambient |
| AA standard (17 AA): 25 pmol/µL | n/a | Agilent 5061-3333 | 2-8 °C |
| AA standard (17 AA): 100 pmol/µL | n/a | Agilent 5061-3332 | 2-8 °C |
| AA standard (17 AA): 250 pmol/µL | n/a | Agilent 5061-3331 | 2-8 °C |
| L-Norvaline | 6600-40-4 | Sigma Aldrich N7627 | ambient |
| Sarcosine | 107-97-1 | TCI M0332 | ambient |
| Glycine | 56-40-6 | (L-amino acids kit) Sigma-Aldrich LAA21-1KT | 2-8 °C |
| L-Arginine HCl | 1119-34-2 | | 2-8 °C |
| L-Aspartic acid | 56-84-8 | | 2-8 °C |
| L-Serine | 56-45-1 | | 2-8 °C |
| L-Proline | 147-85-3 | | 2-8 °C |
| Borate buffer | n/a | Agilent 5061-3339 | 2-8 °C |
| FMOC reagent | n/a | Agilent 5061-3337 | 2-8 °C |
| OPA reagent | n/a | Agilent 5061-3335 | 2-8 °C |
| 6N HCl | n/a | Ricca 3750-32 | ambient |
| 0.1N HCl | n/a | Ricca 3600-16 | ambient |
| Phosphoric acid 85% | n/a | BDH 153155E | ambient |
| Acetronitrile (LCMS grade) | 75-05-8 | Fisher Chemical A955-4 | ambient |
| Methanol (LCMS grade) | 67-56-1 | Fisher Chemical A454-4 | ambient |
| Water (LCMS grade) | 7732-18-5 | Fisher Chemical W6-4 | ambient |
| Nitrogen (high purity > 99.99%) | 7727-37-9 | Airgas NI HP200 | ambient |

### PROCEDURE

### Reagent Preparation

**NOTE:** the volumes can be scaled proportionally to make a smaller or larger batch

### 10mM Na₂HPO₄ / Na₂B₄O₇ / pH 8.2 (aqueous mobile phase)

- Using a 1-L graduated cylinder, measure and transfer 1 L of LCMS grade water to a mobile phase bottle
- Weigh 2.64 ± 0.10 g of Na₂HPO₄·7H₂O
- Weigh 2.00 ± 0.10 g of Na₂B₄O₇
- Add both salts in the same bottle containing 1 L of water
- Pipet 2.4 mL of 6N HCl into the same bottle
- Stir on the stir plate at ambient temperature until the solid completely dissolves
- Adjust the final pH to 8.20 ± 0.05 with 6N HCl
   Expiration date/storage condition (1 week at ambient; 1 month at 2-8 °C)

### 45/45/10 ACN/MeOH/water (organic mobile phase)

- Using a 500-mL graduated cylinder, measure and transfer 450 mL of LCMS grade ACN to a mobile phase bottle.
- Using a 500-mL graduated cylinder, measure and transfer 450 mL of LCMS grade MeOH to the same bottle.
- Using a 100-mL graduated cylinder, measure and transfer 100 mL of LCMS grade water to the same bottle.
- Mix well by inverting and shaking manually.
- Expiration date/storage condition (1 month at ambient)

### Injection diluent

- Using a 100-mL graduated cylinder, measure and transfer 100 mL of the aqueous mobile phase to a mobile phase bottle
- Pipet 0.4 mL of concentrated phosphoric acid into the same bottle
- Mix well by inverting and shaking manually
- Expiration date/storage condition (1 month at 2-8 °C)
- Transfer 1.5 mL of the injection diluent to an LC vial and place the vial at location "4" in the Agilent LC system for instrument analysis

### Derivatization reagent: Borate buffer

- Transfer 1.5 mL to an LC vial and place at location "1" in the Agilent LC system for instrument analysis

### Derivatization reagents: OPA and FMOC

- Break open an ampoule of each of the OPA reagent and the FMOC reagent
- Aliquot (NLT) 100 µL of the OPA reagent or the FMOC reagent into an LC vial with an LC vial insert and place the LC vial containing the OPA reagent in location "2" and place the LC vial containing the FMOC reagent into location "3" (FMOC) in the Agilent LC system for instrument analysis

### Acid hydrolysis of a sample of a lyophilized peptide-alginate conjugate

Weigh 12~16 mg of the lyophilized conjugate into a microwave reaction vial, make sure the sample stays on the bottom
Hydrolyze according to steps 3.3.2-3.3.19 below

### Acid hydrolysis of a sample of a peptide-alginate conjugate in saline solution

Weigh 1000 ± 50 mg of the conjugate solution in saline into a microwave reaction vial
Add 10.0 mL of 6N HCl to the sample using a 10-mL transfer pipet or volumetric pipet
Add one stir bar
Seal the PTFE lined cap with a crimper
Place each sample vial in the matching heat block on a hot/stir plate
Heat at 120⁰ C, stir at 400 rpm, for 6 hours
Remove from heat and let cool to ambient temperature
Remove cap and transfer the entire solution from the reaction vial to a 20-mL volumetric flask with a disposable transfer pipet
Pipet 2 mL of LCMS grade water into the empty reaction vial, rinse the inner wall thoroughly with the same disposable transfer pipet, and transfer the rinseate completely to the same 20-mL volumetric flask
Repeat the above step twice
Bring to mark of the volumetric flask with LCMS grade water
Cap and invert the flask multiple times to mix well
Transfer completely to a 50-mL centrifuge tube
Centrifuge at 5000 rpm for 10 minutes
Pipet accurately 1 mL of the supernatant to an LC vial and store at 2-8 °C until drying (e.g., the next day) (store the remaining supernatant at 2-8⁰ C for any repeat testing if needed)
Dry the 1 mL supernatant completely under nitrogen at 60⁰ C, make sure the needle does not touch the sample but is low enough for fast drying of the sample Into the vial with the dried sample, pipet 0.25 mL of 0.1 µmol/mL internal standard mixture
Vortex thoroughly
Transfer with a pipet to an LC vial with a LC vial insert
Store at 2-8⁰ C until HPLC analysis (step 3.6)

### Standard preparation

### AA standard stock solutions: 10 µmol/mL

• For each AA, calculate the weight needed to prepare a 10 µmol/mL stock solution based on the MW
• See an example below:

| **Letter name** | **Full name** | **MW (g/mol)** | **Actual weight (mg)** | **Volume (mL)** | **Purity (%)** | **Actual Concentration (µmol/mL)** |
|---|---|---|---|---|---|---|
| D | Aspartic acid | 133.10 | 66.38 | 50 | 100 | 9.97 |
| S | Serine | 105.09 | 52.55 | 50 | 100 | 10.00 |
| G | Glycine | 75.07 | 38.56 | 50 | 100 | 10.27 |
| R | Arginine HCl | 210.66 | 102.67 | 50 | 100 | 9.75 |
| N-iSTD | Norvaline | 117.15 | 58.76 | 50 | 100 | 10.03 |
| S-iSTD | Sarcosine | 89.09 | 44.00 | 50 | 98.4 | 9.72 |
| P | Proline | 115.13 | 57.74 | 50 | 100 | 10.03 |

• Weigh the calculated weight into a 50-mL volumetric flask
• Dissolve and bring to mark with 0.1N HCl
• Mix well by capping and inverting or vortexing
• Store at 2-8 °C until HPLC analysis (step 3.6)

### Internal standard mixture: 1 µmol/mL

- Into the same 10-mL volumetric flask, pipet accurately 1.0 mL of Norvaline stock (10 µmol/mL) and 1.0 mL of Sarcosine stock (10 µmol/mL) solutions
- Bring to mark with 0.1N HCl
- Mix well by capping and inverting or vortexing
- Store at 2-8 °C until HPLC analysis (step 3.6)

### Internal standard mixture: 0.1 µmol/mL

NOTE: this solution is for reconstituting samples after drying
- Into a 10-mL volumetric flask, pipet accurately 1.0 mL of the internal standard mixture (1 µmol/mL)
- Bring to mark with 0.1N HCl
- Mix well by capping and inverting or vortexing
- Store at 2-8 °C until HPLC analysis (step 3.6)

### 5 AA mixture (+ iSTD 0.1): 0.025/0.1/0.25 µmol/mL

- Into the same 10-mL volumetric flask, pipet accurately xx µL (see table below) of D, S, G, R, N-iSTD, S-iSTD, and P (10 µmol/mL) solutions
- Bring to mark with 0.1N HCl
- Mix well by capping and inverting or vortexing
- Store at 2-8 °C until HPLC analysis (step 3.6)

| **D/S/G/R/P STD (µmol/mL)** | **AA STD (xx µL)** | **N-iSTD or S-iSTD (µmol/mL)** | **N-iSTD or S-iSTD (xx µL)** | **Total (mL)** | **Final µmol/mL (D/S/G/R/P)** | **Final µmol/mL (iSTD)** |
|---|---|---|---|---|---|---|
| 10 | 25 | 10 | 100 | 10 | 0.025 | 0.1 |
| 10 | 100 | 10 | 100 | 10 | 0.1 | 0.1 |
| 10 | 250 | 10 | 100 | 10 | 0.25 | 0.1 |

### 17 AA standard (+ iSTD 0.1) mixture: 0.1 µmol/mL

- Break open an ampoule of the 0.1 µmol/mL 17 AA standard solution
- Accurately pipet 0.9 mL of the AA standard mixture into an LC vial
- Into the same LC vial, pipet accurately 100 µL of the iSTD mixture (1 µmol/mL)
- Mix well by vortexing
- Aliquot NLT 100 µL into an LC vial with an LC vial insert
- Store at 2-8 °C until HPLC analysis (step 3.6)

### HPLC conditions

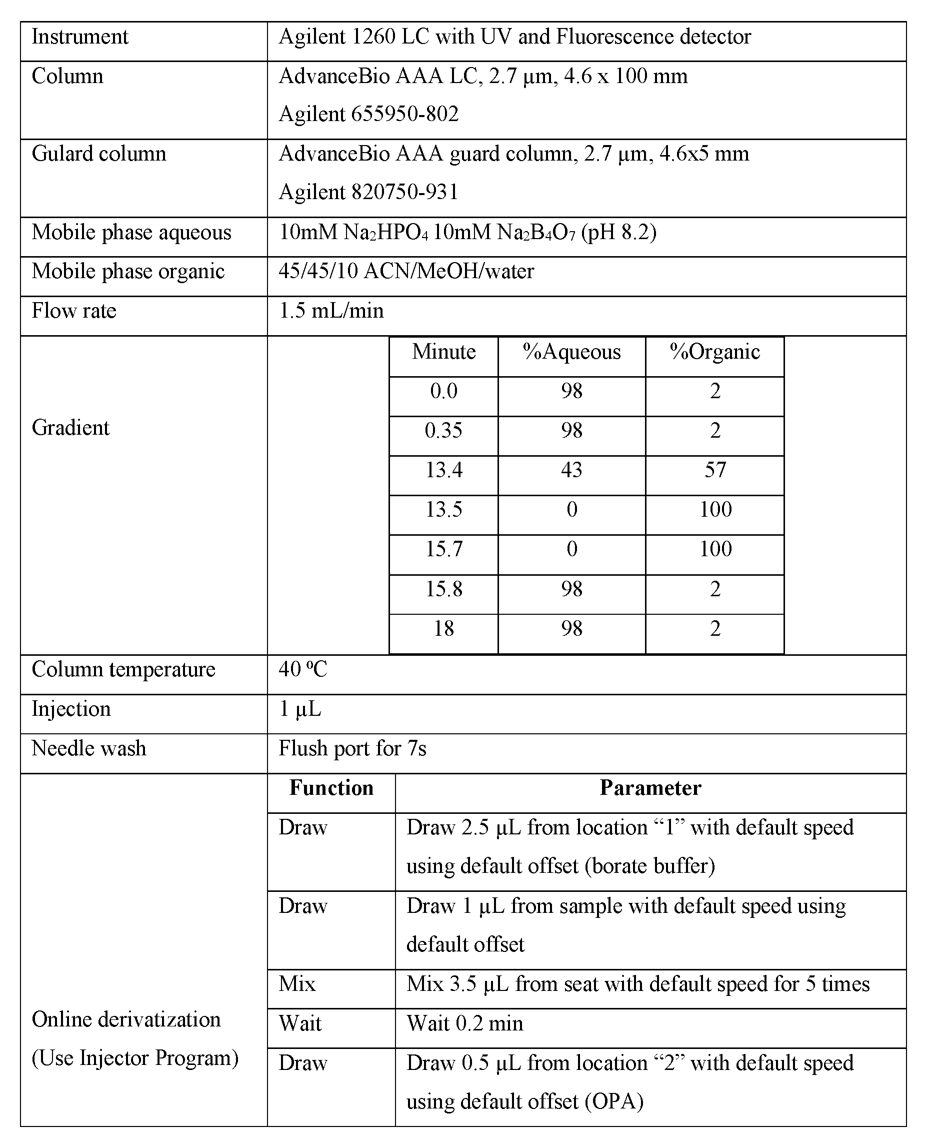

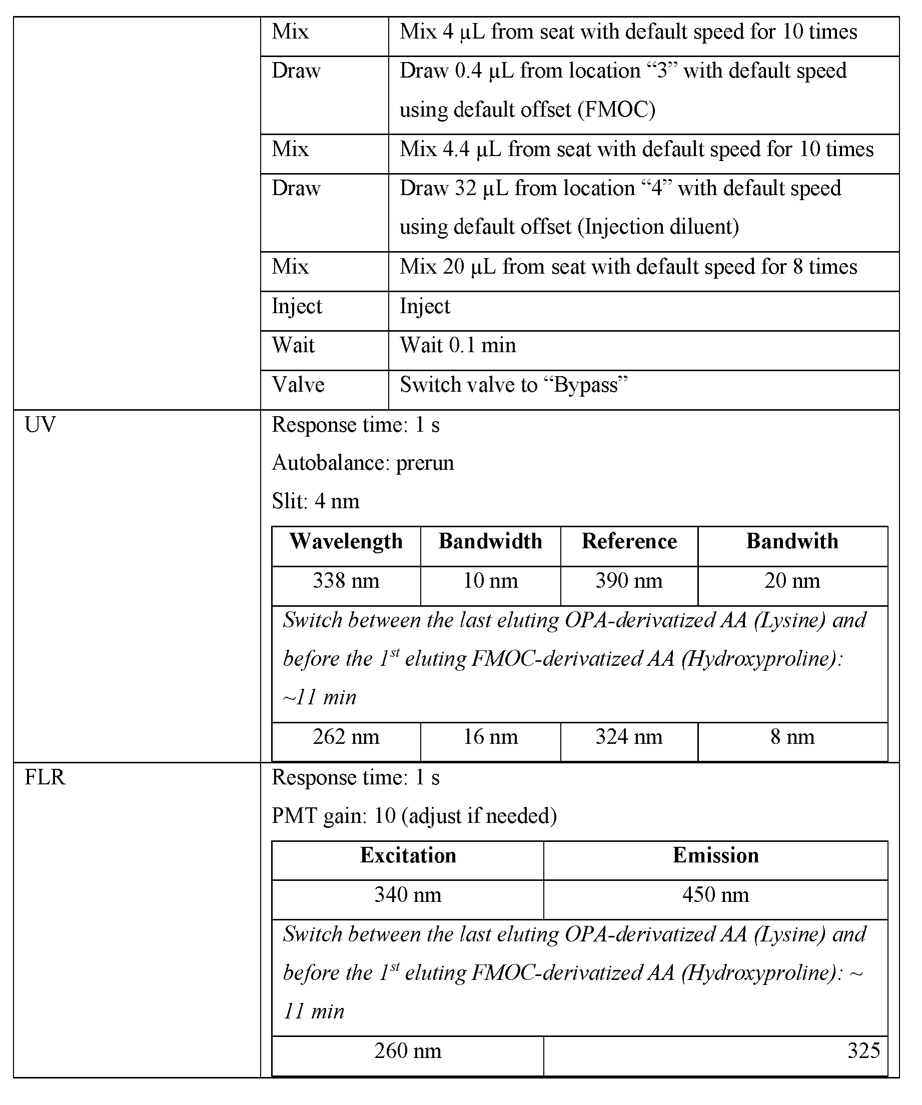

### HPLC analysis

### (Example sequence for sample analysis)

| **Vial** # | **Replicates** | **Sample description** |
|---|---|---|
| P1-A1 | 5 | Blank: 0.1N HCl |
| P1-A2 | 3* | 0.025 µmol/mL 17AA (+iSTD): initial |
| P1-A3 | 3* | 0.1 µmol/mL 17AA (+iSTD): initial |
| P1-A4 | 3* | 0.25 µmol/mL 17AA (+iSTD): initial |
| P1-A1 | 2 | Blank: 0.1N HCl |
| P1-A5 | 1 | 0.1 µmol/mL 5AA (+iSTD): RT and check standard |
| P1-A1 | 2 | Blank: 0.1N HCl |
| P1-B1 | 1 | Conjugate sample A: acid hydrolyzed |
| P1-B2 | 1 | Conjugate sample B: acid hydrolyzed |
| ... | 1 | ... |
| Bracket with 0.1 µmol/mL STD (+iSTD) injection between every 10 sample injections | | |
| P1-A1 | 2 | Blank: 0.1N HCl |
| P1-A2 | 1 | 0.025 µmol/mL STD (+iSTD): end |
| P1-A3 | 1 | 0.1 µmol/mL STD (+iSTD): end |
| P1-A4 | 1 | 0.25 µmol/mL STD (+iSTD): end |
| 5 | 1 | • Water 100 µL (needle cleaning) |
| | | • Column flushing with organic mobile phase |

| | | |
|---|---|---|
| * For information only analysis, run only one injection as initial standard and end standard. | | |

### System suitability criteria

Analyze retention time and peak area for each AA of interest used in quantitation (D/S/G/R) and internal standards (Norvaline and Sarcosine) in standard injections (both UV and FLR).

| **Parameter** | **Criteria** | |
|---|---|---|
| Blanks | No significant interference in UV and FLR | |
| %RSD (Retention time), initial 3 | NMT 5% | |
| %RSD (Area), initial 3 | NMT 20% | |
| %RSD (Relative Area), initial 3 | NMT 20% | |
| %RSD (Retention time), all | NMT 5% | |
| %RSD (Area), all | NMT 20% | |
| %RSD (Relative Area), all | NMT 20% | |
| Resolution: | | Baseline separation |
| | • Glycine from other AAs | |
| | • Norvaline (iSTD) from other AAs | |
| | • Sarcosine (iSTD) vs. Proline | |
| Linearity | | RSQ NLT 0.99 |
| Check standard | | Quantitated result: within 80%~120% of theoretical |

### Data Analysis - Analyze samples only when system suitability passes Integration guidelines

- Only integrate the AA of interest
- Start from "automatic integration"
- Manually adjust the integraton as necessary

### Identification of AA of interest

- RT of AA of interest and internal standards in the 17AA (+iSTD) standard mixture should match the RT of the 5AA (+iSTD) standard mixture
- RT of the AA in each sample should match the RT of the standard (UV/FLR)
   **Relative area (D, S, G, R)** = Area (D, S, G, R) / Area (Norvaline)
   **Relative area (P)** = Area (P) / Area (Sarcosine)
   **Standard calibration curve**
- Calculate concentrations of standard solutions using the reported value on certificate of analysis or actual weights adjusted by dilution factor during the standard preparation.
- Plot the average area or average relative area vs. concentration for each AA of interest from the standard injections: 0.025, 0.1, and 0.25 µmol/mL. Perform linear regression.
   **Conc** = m * Area or Relative Area + b
   Where, m is the slope of the linear fitted curve and b is the Y-intercept of the linear fitted curve.
- The RSQ should be no less than 0.99.
- If the linearity fails, prepare fresh derivatization reagents/standard solutions, and troubleshoot system malfunction and repeat the test.

### Sample analysis

- Quantitation can be done by both UV and FLR
- Quantitation is done using relative area (area ratio relative to the internal standard)
- Calculate concentration of AA: G, R, D, S in each sample using the linear fitted standard curve:
   **Conc, AA** (µmol/mL) = (m * Area or Relative Area + b)
- Calculate concentration of the total GRGDSP (SEQ ID NO: 60) by averaging the concentration of each AA:
   **Concentration, total GRGDSP** (SEQ ID NO: 60) (µmol/mL) = (Conc, G/2 + Conc, R + Conc, D + Conc, S + Conc, P) / 5
   **µmol (Total GRGDSP)** (SEQ ID NO: 60) /**g (conjugate)** = Conc, total GRGDSP (SEQ ID NO: 60) (µmol/mL) x 0.25 mL/1.0 mL x 20 mL / Weight (g)
   **µmol (conjugated GRGDSP** (SEQ ID NO: 60)) / **g (conjugate)** = µmol (Total GRGDSP (SEQ ID NO: 60)) / g (conjugate) - µmol (Residual free GRGDSP (SEQ ID NO: 60)) / g (conjugate)

### Example 26: Exemplary assay to determine residual free peptide in a CBP-polymer composition

This assay uses liquid chromatography - mass spectroscopy (LC-MS) to determine the amount of residual, unconjugated peptide in a composition containing a peptide-polymer conjugate, e.g., typically after one or more purufication steps have been performed to remove a substantial portion, e.g., greater than 95%, 98%, 99% or more, of unconjugated peptide. In brief, a sample of the conjugate in saline solution is added to a molecular weight cut-off (MWCO) tube that has a MWCO higher than the molecular weight of the peptide, the tube is centrifuged to separate the residual peptide from the conjugate, and the amount of peptide is quantitated by LC-MS using as a standard a reference composition containing a known concentration of the same peptide.

### Example 27: Exemplary quantitative amine assay to determine amine-conjugation density in an afibrotic polymer modified with a compound of Formula (I).

This assay determines the amount of an amine-containing compound (e.g., a compound of Formula I, e.g., Compound 101 in Table 4) in a polymer chemically modified with the amine compound. A sample of the chemically-modified polymer is subjected to acid hydrolysis, which cleaves off the conjugated amine and the weight % of total amine in the hydrolyzed sample is quantitated by reverse-phase, liquid chromatorgraphy with ultraviolet detection (LC-UV) using the unconjugated amine compound as a standrard. The identity of the LC peak can be further confirmed by mass spectrometry. The weight % of total amine can be used as the % conjugation of the amine-compound in the chemically-modified polymer. A more precise result can be obtained by determining the amount of any residual unconjugated amine compound in an unhydrolyzed sample of the chemically-modified polymer using any suitable method (e.g., as described below) and subtracting that amount from the total peptide amount.

The assay is further described below as applied to determining % conjugation density in an alginate chemically modified with Compound 101 (i.e., CM-LMW-Alg-101); however, the skilled artisan can readily modify the assay to determine the conjugation density of any Formula I compound used to chemically-modify a polysaccharide (e.g., an alginate) or another polymer that does not contain amines. Also, the skilled person can readily substitute any equipment, material or chemical specified below with a different equipment, material or chemical that can perform or provide substantially the same function or role in the assay.

### DEFINITIONS

| **Abbreviation** | **Definition** |
|---|---|
| LC-UV-MS | Liquid Chromatography-Ultra-Violet-Mass Spectrometry |
| VLVG | Pronova Ultrapure VLVG sodium alginate |
| SLG100 | Pronova Ultrapure Sterile Alginate |
| TIC | Total Ion Chromatogram |
| RT | Retention Time |
| ACN | Acetonitrile |
| SST | System Suitability |
| RSD | Relative Standard Deviation |
| TBD | To Be Determined |
| NMT | No More Than |
| m/z | Mass charge ratio |

### EQUIPMENT AND MATERIALS

### EQUIPMENT

- Agilent 1260 LC system (DAD: 13 µL/10 mm flow cell)
- Agilent SQ MS detector (G1956B)
- ChemStation software
- Hot/stir plate with heating blocks, IKA RCT BS001
- Analytical balance, Mettler Toledo XPE105
- Centrifuge, Beckman Coulter Allegra 25R
- Vortex mixer, VWR 97043-562

### MATERIALS

- XBridge C18, 2.5 µm, 4.6 x 50 mm, Waters 186006037
- SG-SM0001 reference material (>98.0% purity)
- 2N HCl (ACS grade), BDH 7203-1
- Ammonia solution 28%-30% (ACS grade), MilliPore Sigma 1.05423.1000
- Acetronitrile (LCMS grade), Fisher Chemical A955-4
- Water (LCMS grade), Fisher Chemical W6-4
- 0.9% saline, ROBio SAL-090
- Microwave reaction vials with PTFE lined caps, Chemglass CG-4920-02
- Vivaspin 20 3kDa MWCO concentrator, GE Healthcare 28-9323-58
- 50 mL Centrifuge tubes, VWR 10160-140
- Crimper, Chemglass CG-4930-20
- Decapper, VWR 10806-372
- Graduated cylinders, class A
- Volumetric transfer pipets (to deliver) class A
- Volumetric flasks, class A
- Gastight syringe 1 mL, Hamilton 81316
- Transfer pipette (200 µL variable volume), Eppendorf 3123000055
- Transfer pipette (1000 µL variable volume), Eppendorf 3123000063
- Transfer pipette (10 mL variable volume), Eppendorf 3123000080
- Disposable transfer pipet, VWR 16001-180
- Stir bar, VWR 58948-078
- LC vial and cap, VWR 46610-724
- Assortment of lab glassware

### PROCEDURE

### Reagent Preparation

NOTE: the volumes can be scaled proportionally to make a smaller or larger batch

### 0.1% ammonia in water (aqueous mobile phase)

- Using a 1-L graduated cylinder, measure and transfer 1 L of LCMS grade water to a mobile phase bottle.
- Using a 1-mL volumetric transfer pipet or Hamilton syringe, measure 1.0 mL of the concentrated ammonia and transfer to the same mobile phase bottle.
- Mix well by inverting and shaking manually.
- Expiration/storage condition - 1 month at ambient

### 0.1% ammonia in ACN (organic mobile phase)

- Using a 1-L graduated cylinder, measure and transfer 1 L of LCMS grade ACN to a mobile phase bottle.
- Using a 1-mL volumetric transfer pipet or Hamilton syringe, measure 1.0 mL of the concentrated ammonia and transfer to the same mobile phase bottle.
- Mix well by inverting and shaking manually.
- Expiration/storage condition -1 month at ambient

### Acid hydrolysis of solid conjugate sample

Weigh 50 ± 5 mg of the lyophilized conjugate solid, into a microwave reaction vial, make sure the conjugate stays on the bottom of the vial
Add 10.0 mL of 2N HCl using a 10-mL transfer pipet or volumetric pipet
Add one stir bar
Seal the PTFE lined cap with a crimper
Place each sample vial in the matching heat block on a hot/stir plate
Heat at 120 °C, stir at 400 rpm, for 120 minutes
Remove from heat and let cool to ambient temperature
Transfer the entire solution from the reaction vial to a 25-mL volumetric flask with a disposable transfer pipet
Pipet 5 mL of LCMS grade water into the empty reaction vial, rinse the inner wall thoroughly with the same disposable transfer pipet, and transfer the rinseate completely to the same 25-mL volumetric flask
Repeat the above step twice
Bring to mark of the volumetric flask with LCMS grade water
Transfer completely to a 50-mL centrifuge tube
Centrifuge at 3000 rpm for 10 minutes
Take supernatant for HPLC analysis
Store at 2-8 °C

### Acid hydrolysis of conjugate in saline sample

Weigh 1000 ± 50 mg of the conjugate solution in saline into a microwave reaction vial
Add 10.0 mL of 2N HCl using a 10-mL transfer pipet or volumetric pipet
Add one stir bar
Seal the PTFE lined cap with a crimper
Place each sample vial in the matching heat block on a hot/stir plate
Heat at 120 °C, stir at 400 rpm, for 120 minutes
Remove from heat and let cool to ambient temperature
Transfer the entire solution from the reaction vial to a 25-mL volumetric flask with a disposable transfer pipet
Pipet 5 mL of LCMS grade water into the empty reaction vial, rinse the inner wall thoroughly with the same disposable transfer pipet, and transfer the rinseate completely to the same 25-mL volumetric flask
Repeat the above step twice
Bring to mark of the volumetric flask with LCMS grade water
Transfer completely to a 50-mL centrifuge tube
Centrifuge at 3000 rpm for 10 minutes
Take supernatant for HPLC analysis
Store at 2-8 ⁰C

### Sample preparation for residual free amine in solid conjugate

Weigh 50 ± 5 mg of the lyophilized conjugate solid, into a scintillation vial
Pipet 5.0 mL of saline into the scintillation vial
Dissolve completely by shaking and vortexing for 10 minutes
Transfer completely to a MWCO tube
Centrifuge at 5000 rpm for 60 minutes (Position the flat side of membrane in parallel to the rotor arm)
Remove the top portion of the MWCO tube and discard
Transfer the sample in the bottom portion completely to a 5 mL volumetric flask
Bring to mark with water or saline and invert to mix well
Transfer to a scintillation vial for storage at 2-8 °C
Transfer an aliquot for HPLC analysis

### Sample preparation for residual free amine in conjugate in saline

Weigh 1000 ± 50 mg of the conjugate (or blend with unmodified alginate) in saline, into a MWCO tube
Pipet 4.0 mL of saline into the MWCO tube
Invert and vortex the tube 5 times or until the solution is mixed well to fully extract free amine
Centrifuge at 5000 rpm for 90 minutes
Remove the top portion of the MWCO tube and discard
Transfer the sample in the bottom portion completely to a 5 mL volumetric flask
Bring to mark with water and invert to mix well
Transfer to a scintillation vial for storage at 2-8 °C
Transfer an aliquot for HPLC analysis

### Standard preparation

### Standard solution: 1 mg/mL

- Weigh 50.00 ± 5.00 mg of SG-SM0001 standard into a scintillation vial
- Add ~ 10 mL of LCMS-grade water, dissolve the solid completely by shaking and vortexing
- Transfer completely to a 50-mL volumetric flask by rinsing the scintillation vial twice with LCMS-grade water, using a disposable transfer pipet
- Bring to volume with LCMS-grade water, mix well
- Store at 2-8 ⁰C

### Standard solution: 0.01 mg/mL

- Pipet 100 µL of the 1 mg/mL solution to a 10-mL volumetric flask
- Bring to volume with LCMS-grade water
- Mix well by inverting
- Store at 2-8 ⁰C

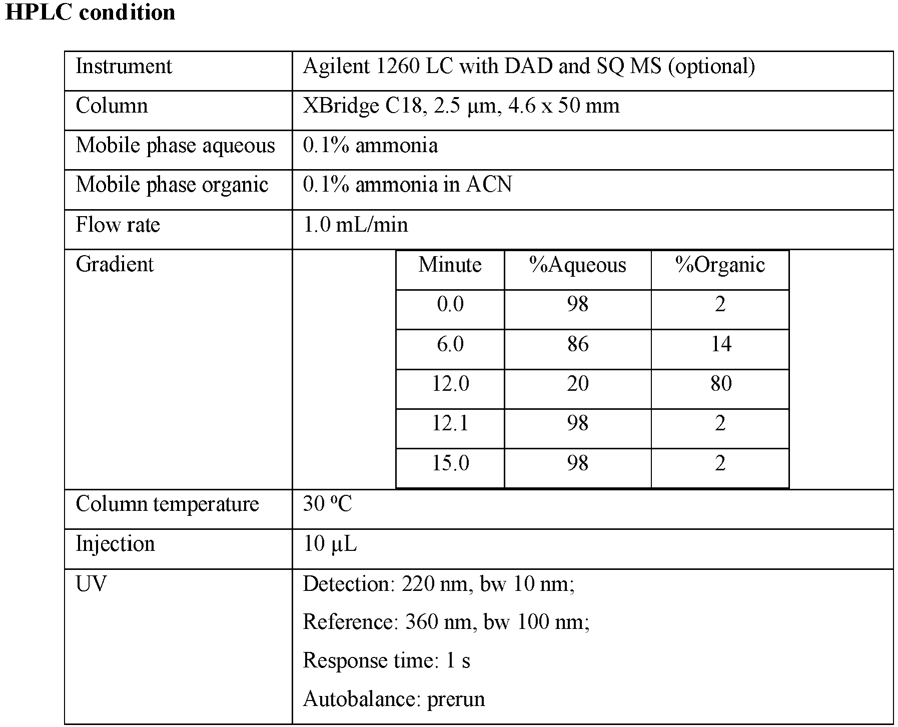

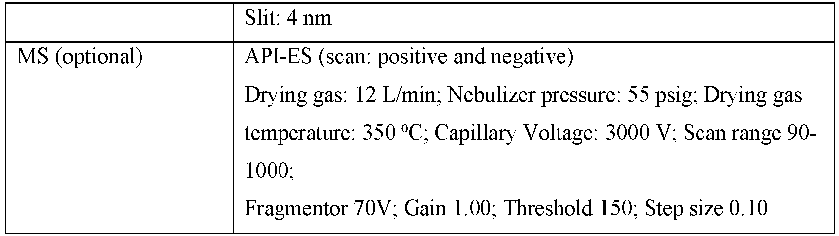

### HPLC analysis

### Example sequence for sample analysis

| **Vial #** | **Replicates** | **Sample description** |
|---|---|---|
| 1 | 2 | Blank: water |
| 2 | 5* | 0.01 mg/mL standard: initial |
| 3 | 1 | Conjugate sample A: residual free amine |
| 4 | 1 | Conjugate sample B: residual free amine |
| ... | 1 | ... |
| Bracket with standard injection between every 10 sample injections | | |
| 1 | 1 | Blank: water |
| 2 | 1 | 0.01 mg/mL standard: end |
| 11 | 5* | 1.0 mg/mL standard: initial |
| 12 | 1 | Conjugate sample A: acid hydrolyzed |
| 13 | 1 | Conjugate sample B: acid hydrolyzed |
| ... | 1 | ... |
| Bracket with standard injection between every 10 sample injections | | |
| 11 | 1 | 1.0 mg/mL standard: end |
| 1 | 1 | Flush column with 50:50 H₂O:ACN |

| | | |
|---|---|---|
| * For information only analysis, run only one injection as initial standard and end standard. | | |

### System suitability criteria

| **Parameter** | **Criteria** |
|---|---|
| Blanks | No significant interference in UV and TIC (optional) |
| %RSD (Retention time), initial 5 | NMT 2% |
| SG-SM0001 | |
| %RSD (area), initial 5 | NMT 10% |
| SG-SM0001 | |
| %RSD (Retention time), initial 5 and all bracketing | NMT 2% |
| SG-SM0001 | |
| %RSD (area), initial 5 and all bracketing | NMT 10% |
| SG-SM0001 | |
| m/z: amine peak (optional) | 392.1 ± 0.5 |

### Data Analysis - Analyze samples only when system suitability passes

### Integration guidelines

- Only integrate the amine peak
- Start from "automatic integration"
- Manually adjust the integraton as necessary

### Identification of amine

- (optional) m/z of the amine peak in each sample should be within 392.1 ± 0.5.
- RT of the amine peak in UV in each sample matches the RT of the standard.

### Concentration, standard (mg/mL) =

Weight (mg) / 50 mL / dilution factor,
Where dilution factor = 1 for 1.0 mg/mL standard;
Where dilution factor = 100 for 0.01 mg/mL standard

### Concentration, free amine (mg/mL) =

Area, non-hydrolyzed sample / Area, 0.01 standard x Concentration, 0.01 standard

### % Residual free amine =

Concentration, free amine (mg/mL) x 5 mL /
weight, non-hydrolyzed conjugate (mg) x 100

### Concentration, total amine (mg/mL) =

Area, hydrolyzed sample / Area, 1.0 standard x Concentration, 1.0 standard

### % Total amine =

Concentration, total amine (mg/mL) x 25 mL / weight, hydrolyzed conjugate (mg) x 100

### EQUIVALENTS AND SCOPE

This application refers to various issued patents, published patent applications, journal articles, and other publications. If there is a conflict between any of the references and the instant specification, the specification shall control.

Those skilled in the art will recognize or be able to ascertain using no more than routine experimentation many equivalents to the specific embodiments described herein. The scope of the present embodiments described herein is not intended to be limited to the above Description, Figures, or Examples but rather is as set forth in the appended claims. Those of ordinary skill in the art will appreciate that various changes and modifications to this description may be made without departing from the spirit or scope of the present disclosure, as defined in the following claims.

## Claims

1. A hydrogel capsule comprising:
(a) a cell-containing compartment which comprises a plurality of engineered RPE cells encapsulated in a first polymer composition which comprises an alginate covalently modified with a cell-binding peptide (CBP) via a linker and
(b) a barrier compartment surrounding the cell-binding compartment and comprising a second polymer composition which comprises an alginate covalently modified with at least one compound selected from the group consisting of Compound 101, Compound 100, Compound 110, Compound 112, Compound 113 and Compound 114 shown in Table 4,
wherein the hydrogel capsule has a spherical shape and a diameter of 0.7 millimeter to 3.0 millimeters,
wherein the cell-containing compartment is substantially free of any afibrotic compound and the barrier compartment is substantially free of cells and substantially free of the CBP;
wherein the engineered RPE cells comprise an exogenous nucleotide sequence encoding a therapeutic polypeptide, and
wherein the CBP peptide is less than 6 amino acids in length and comprises RGD (SEQ ID NO: 43) or RGDSP (SEQ ID NO: 59) and the linker consists essentially of one glycine residue, two glycine residues, three glycine residues, one beta-alanine residue, two beta-alanine residues or three beta-alanine residues.

2. The hydrogel capsule of claim 1, wherein the density of the CBP on the covalently modified alginate is an amount effective to increase the production of the therapeutic polypeptide *in vivo* by at least 1.25-fold, 1.5 fold, 2-fold, or 5-fold at 1 week, 2 weeks, 4 weeks, 8 weeks or 12 weeks after implant of the capsule into a mouse as compared to a CBP-null reference hydrogel capsule.

3. The hydrogel capsule of claim 1 or 2, wherein the CBP is RGD (SEQ ID NO: 43) or RGDSP (SEQ ID NO: 59) and the linker consists essentially of one glycine residue joined to the amine terminus of the CBP.

4. The hydrogel capsule of any one of claims 1 to 3, wherein the alginate in the first polymer composition has a molecular weight of 75 kDa to 150 kDa and a G:M ratio of greater than or equal to 1.5.

5. The hydrogel capsule of any one of claims 1 to 4, wherein the alginate in the covalently modified alginate in the barrier compartment has a molecular weight of less than 75 kDa and a G:M ratio of greater than or equal to 1.5, and the second polymer composition further comprises an unmodified alginate which has a molecular weight of 150 kDa - 250 kDa and a G:M ratio of greater than or equal to 1.5.

6. The hydrogel capsule of any one of claims 1 to 5, wherein Compound 101 shown in Table 4 is the compound in the covalently modified alginate in the second polymer composition.

7. The hydrogel capsule of any one of claims 1 to 6, wherein the therapeutic polypeptide is selected from the group consisting of a Factor VIII polypeptide, a Factor VII polypeptide and a FIX polypeptide.

8. The hydrogel capsule of any one of claims 1 to 7, wherein the engineered RPE cells are derived from ARPE-19 cells and the ARPE-19 cells are present as a concentration of about 25 million to 35 million, about 35 million to 45 million or about 40 million cells per milliliter of the first-polymer composition, the alginate in the covalently modified alginate in the cell-containing compartment has a molecular weight of 75 kDa to 150 kDa, a G:M ratio of greater than or equal to 1.5, and is covalently modified with GRGDSP, and wherein the density of GRGDSP on the alginate, as determined by a quantitative peptide conjugation assay, is about 0.1 to about 1.0 micromoles of GRGDSP per gram of the GRGDSP-alginate in a saline solution with a viscosity of 80-120 cP.

9. The hydrogel capsule of any one of claims 1 to 8, wherein the therapeutic polypeptide is a Factor VIII-BDD polypeptide, the exogenous nucleotide sequence comprises SEQ ID NO: 15, the alginate in the first polymer composition has a molecular weight of 75 kDa to 150 kDa and a G:M ratio of greater than or equal to 1.5, and the density of GRGDSP on the alginate, as determined by a quantitative peptide conjugation assay, is about 0.3 to about 0.6 micromoles of GRGDSP per gram of the GRGDSP-alginate in a saline solution with a viscosity of 80-120 cP.

10. The hydrogel capsule of any one of claims 1 to 9, wherein:
the second polymer composition in the barrier compartment further comprises an unmodified alginate with a molecular weight of 150 kDa - 250 kDa and a G:M ratio of greater than or equal to 1.5,
the alginate in the covalently modified alginate in the barrier compartment has a molecular weight of less than 75 kDa, a G:M ratio of greater than or equal to 1.5 and the compound shown in Table 4 is Compound 101, and
the conjugation density of Compound 101 in the covalently modified alginate, as determined by combustion analysis for percent nitrogen, is at least 2.0 % nitrogen and less than 9.0 % nitrogen, or is 3.0 % to 8.0 '%, 4.0 % to 7.0%, 5.0 % to 7.0 %, or 6.0 % to 7.0 % nitrogen.

11. The hydrogel capsule of any one of claims 1 to 10, wherein the hydrogel capsule is about 1.5 mm in diameter and the thickness of the barrier compartment is between about 180 µm and 260 µm or between about 310 µm and 440 µm.

12. A preparation of hydrogel capsules, wherein each capsule in the preparation is a hydrogel capsule of any one of claims 1 to 11.

13. The preparation of hydrogel capsules of claim 12, wherein each capsule in the preparation has a diameter of 1.4 to 1.6 millimeters.

14. The preparation of hydrogel capsules of claim 12 or 13, which is a pharmaceutically acceptable composition suitable for placing in or implanting into the peritoneal cavity of a subject.

15. A composition for use in a method of treating a subject in need of a therapeutic agent, the method comprising:
providing a preparation of hydrogel capsules of any one of claims 12 to 14, and
implanting the preparation into a body cavity of a subject.

16. The composition for use of claim 15, wherein the therapeutic agent is a blood clotting factor.

17. The composition for use of claim 16, wherein the blood clotting factor is a Factor VIII-BDD protein, which consists essentially of SEQ ID NO:1.

18. The composition for use of claim 16, wherein the blood clotting factor is a Factor IX-padua protein, which comprises SEQ ID NO:36.

## Patentansprüche

1. Hydrogelkapsel, umfassend:
(a) ein zellhaltiges Kompartiment, das mehrere konstruierte RPE-Zellen umfasst, die in einer ersten Polymerzusammensetzung eingekapselt sind, die ein Alginat umfasst, das über einen Linker kovalent mit einem zellbindenden Peptid (CBP) modifiziert ist, und
(b) ein Barrierenkompartiment, das das zellbindende Kompartiment umgibt und eine zweite Polymerzusammensetzung umfasst, die ein mit mindestens einer Verbindung, die aus der Gruppe bestehend aus Verbindung 101, Verbindung 100, Verbindung 110, Verbindung 112, Verbindung 113 und Verbindung 114 gemäß Tabelle 4 ausgewählt ist, kovalent modifiziertes Alginat umfasst,
wobei die Hydrogelkapsel in Kugelform vorliegt und einen Durchmesser von 0,7 Millimeter bis 3,0 Millimeter aufweist,
wobei das zellhaltige Kompartiment weitgehend frei von jeglicher afibrotischen Verbindung ist und das Barrierenkompartiment weitgehend frei von Zellen sowie weitgehend frei vom CBP ist;
wobei die konstruierten RPE-Zellen eine exogene Nukleotidsequenz umfassen, die ein therapeutisches Polypeptid codiert, und
wobei das CBP-Peptid eine Länge von weniger als 6 Aminosäuren aufweist und RGD (SEQ ID NO: 43) oder RGDSP (SEQ ID NO: 59) umfasst und der Linker im Wesentlichen aus einem Glycinrest, zwei Glycinresten, drei Glycinresten, einem Beta-Alaninrest, zwei Beta-Alaninresten oder drei Beta-Alaninresten besteht.

2. Hydrogelkapsel nach Anspruch 1, wobei die Dichte des CBP auf dem kovalent modifizierten Alginat eine wirksame Menge zur Steigerung der Produktion des therapeutischen Polypeptids *in vivo* um mindestens das 1,25-, 1,5-, 2- oder 5-fache 1 Woche, 2 Wochen, 4 Wochen, 8 Wochen oder 12 Wochen nach Implantation der Kapsel in eine Maus im Vergleich zu einer CBP-Null-Referenz-Hydrogelkapsel darstellt.

3. Hydrogelkapsel nach Anspruch 1 oder 2, wobei es sich bei dem CBP um RGD (SEQ ID NO: 43) oder RGDSP (SEQ ID NO: 59) handelt und der Linker im Wesentlichen aus einem Glycinrest besteht, der mit dem Aminterminus des CBP verbunden ist.

4. Hydrogelkapsel nach einem der Ansprüche 1 bis 3, wobei das Alginat in der ersten Polymerzusammensetzung ein Molekulargewicht von 75 kDa bis 150 kDa und ein G:M-Verhältnis größer oder gleich 1,5 aufweist.

5. Hydrogelkapsel nach einem der Ansprüche 1 bis 4, wobei das Alginat im kovalent modifizierten Alginat im Barrierenkompartiment ein Molekulargewicht kleiner 75 kDa und ein G:M-Verhältnis größer oder gleich 1,5 aufweist und die zweite Polymerzusammensetzung ferner ein unmodifiziertes Alginat umfasst, das ein Molekulargewicht von 150 kDa - 250 kDa und ein G:M-Verhältnis größer oder gleich 1,5 aufweist.

6. Hydrogelkapsel nach einem der Ansprüche 1 bis 5, wobei Verbindung 101 gemäß Tabelle 4 die Verbindung im kovalent modifizierten Alginat in der zweiten Polymerzusammensetzung ist.

7. Hydrogelkapsel nach einem der Ansprüche 1 bis 6, wobei das therapeutische Polypeptid aus der Gruppe bestehend aus einem Faktor-VIII-Polypeptid, einem Faktor-VII-Polypeptid und einem FIX-Polypeptid ausgewählt ist.

8. Hydrogelkapsel nach einem der Ansprüche 1 bis 7, wobei die konstruierten RPE-Zellen von ARPE-19-Zellen abgeleitet sind und die ARPE-19-Zellen als eine Konzentration von etwa 25 Millionen bis 35 Millionen, etwa 35 Millionen bis 45 Millionen oder etwa 40 Millionen Zellen pro Milliliter der Erstpolymerzusammensetzung vorliegen, das Alginat im kovalent modifizierten Alginat im zellhaltigen Kompartiment ein Molekulargewicht von 75 kDa bis 150 kDa aufweist, ein G:M-Verhältnis größer oder gleich 1,5 aufweist und kovalent mit GRGDSP modifiziert ist und wobei die Dichte von GRGDSP auf dem Alginat, wie mit einem quantitativen Peptidkonjugations-Assay bestimmt, etwa 0,1 bis etwa 1,0 Mikromol GRGDSP pro Gramm des GRGDSP-Alginats in einer Salzlösung mit einer Viskosität von 80-120 cP beträgt.

9. Hydrogelkapsel nach einem der Ansprüche 1 bis 8, wobei es sich bei dem therapeutischen Polypeptid um ein Faktor-VIII-BDD-Polypeptid handelt, die exogene Nukleotidsequenz SEQ ID NO:15 umfasst, das Alginat in der ersten Polymerzusammensetzung ein Molekulargewicht von 75 kDa bis 150 kDa und ein G:M-Verhältnis größer oder gleich 1,5 aufweist und die Dichte von GRGDSP auf dem Alginat, wie mit einem quantitativen Peptidkonjugations-Assay bestimmt, etwa 0,3 bis etwa 0,6 Mikromol GRGDSP pro Gramm des GRGDSP-Alginats in einer Salzlösung mit einer Viskosität von 80-120 cP beträgt.

10. Hydrogelkapsel nach einem der Ansprüche 1 bis 9, wobei:
die zweite Polymerzusammensetzung im Barrierenkompartiment ferner ein unmodifiziertes Alginat mit einem Molekulargewicht von 150 kDa - 250 kDa und einem G:M-Verhältnis größer oder gleich 1,5 umfasst,
das Alginat im kovalent modifizierten Alginat im Barrierenkompartiment ein Molekulargewicht kleiner 75 kDa, ein G:M-Verhältnis größer oder gleich 1,5 aufweist und es sich bei der Verbindung gemäß Tabelle 4 um Verbindung 101 handelt und
die Konjugationsdichte von Verbindung 101 im kovalent modifizierten Alginat, wie durch Verbrennungsanalyse für Prozent Stickstoff bestimmt, mindestens 2,0 % Stickstoff und weniger als 9,0 % Stickstoff beträgt oder 3,0 % bis 8,0 %, 4,0 % bis 7,0 %, 5,0 % bis 7,0 % oder 6,0 % bis 7,0 % Stickstoff beträgt.

11. Hydrogelkapsel nach einem der Ansprüche 1 bis 10, wobei die Hydrogelkapsel einen Durchmesser von etwa 1,5 mm aufweist und die Dicke des Barrierenkompartiments zwischen etwa 180 µm und 260 µm oder zwischen etwa 310 µm und 440 µm beträgt.

12. Präparat von Hydrogelkapseln, wobei es sich bei den Kapseln im Präparat jeweils um eine Hydrogelkapsel nach einem der Ansprüche 1 bis 11 handelt.

13. Präparat von Hydrogelkapseln nach Anspruch 12, wobei die Kapseln im Präparat jeweils einen Durchmesser von 1,4 bis 1,6 Millimeter aufweisen.

14. Präparat von Hydrogelkapseln nach Anspruch 12 oder 13, bei der es sich um eine pharmazeutisch unbedenkliche Zusammensetzung handelt, die zum Einsetzen oder Implantieren in die Bauchhöhle eines Individuums geeignet ist.

15. Zusammensetzung zur Verwendung bei einem Verfahren zur Behandlung eines Individuums, das ein Therapeutikum benötigt, wobei das Verfahren Folgendes umfasst:
Bereitstellen eines Präparats von Hydrogelkapseln nach einem der Ansprüche 12 bis 14 und
Implantieren des Präparats in eine Körperhöhle eines Individuums.

16. Zusammensetzung zur Verwendung nach Anspruch 15, wobei es sich bei dem Therapeutikum um einen Blutgerinnungsfaktor handelt.

17. Zusammensetzung zur Verwendung nach Anspruch 16, wobei es sich bei dem Blutgerinnungsfaktor um ein Faktor-VIII-BDD-Protein, das im Wesentlichen aus SEQ ID NO:1 besteht, handelt.

18. Zusammensetzung zur Verwendung nach Anspruch 16, wobei es sich bei dem Blutgerinnungsfaktor um ein Faktor-IX-padua-Protein, das SEQ ID NO:36 umfasst, handelt.

## Revendications

1. Capsule d'hydrogel comprenant :
(a) un compartiment contenant des cellules qui comprennent une pluralité de cellules RPE modifiées, encapsulées dans une première composition polymère contenant un alginate modifié de manière covalente par un peptide de liaison cellulaire (CBP) via un lieur et
(b) un compartiment barrière entourant le compartiment de liaison cellulaire et comprenant une deuxième composition polymère qui comprend un alginate modifié de manière covalente par au moins un composé choisi dans le groupe constitué par le composé 101, le composé 100, le composé 110, le composé 112, le composé 113 et le composé 114, présentés dans le tableau 4,
la capsule d'hydrogel ayant une forme sphérique et un diamètre de 0,7 millimètre à 3,0 millimètres,
le compartiment contenant des cellules étant sensiblement exempt de composé afibrotique et le compartiment barrière étant sensiblement exempt de cellules et sensiblement exempt de CBP ;
les cellules RPE modifiées comprenant une séquence nucléotidique exogène codant pour un polypeptide thérapeutique, et
le peptide CBP ayant une longueur inférieure à 6 acides aminés et comprenant RGD (SEQ ID NO : 43) ou RGDSP (SEQ ID NO : 59) et le lieur étant essentiellement constitué d'un résidu glycine, deux résidus glycine, trois résidus glycine, un résidu bêta-alanine, deux résidus bêta-alanine ou trois résidus bêta-alanine.

2. Capsule d'hydrogel selon la revendication 1, dans laquelle la densité du CBP sur l'alginate modifié de manière covalente est une valeur suffisante pour augmenter la production du polypeptide thérapeutique *in vivo* d'au moins 1,25 fois, 1,5 fois, 2 fois ou 5 fois à 1 semaine, 2 semaines, 4 semaines, 8 semaines ou 12 semaines après l'implantation de la capsule chez une souris, par rapport à une capsule d'hydrogel de référence sans CBP.

3. Capsule d'hydrogel selon la revendication 1 ou 2, dans laquelle le CBP est RGD (SEQ ID NO : 43) ou RGDSP (SEQ ID NO : 59) et le lieur est essentiellement constitué d'un résidu glycine lié à l'extrémité amino du CBP.

4. Capsule d'hydrogel selon l'une quelconque des revendications 1 à 3, dans laquelle l'alginate de la première composition polymère a une masse moléculaire de 75 kDa à 150 kDa et un rapport G:M supérieur ou égal à 1,5.

5. Capsule d'hydrogel selon l'une quelconque des revendications 1 à 4, dans laquelle l'alginate présent dans l'alginate modifié de manière covalente du compartiment barrière a une masse moléculaire inférieure à 75 kDa et un rapport G:M supérieur ou égal à 1,5, et la deuxième composition polymère comprend en outre un alginate non modifié qui a une masse moléculaire de 150 kDa à 250 kDa et un rapport G:M supérieur ou égal à 1,5.

6. Capsule d'hydrogel selon l'une quelconque des revendications 1 à 5, dans laquelle le composé 101 décrit dans le tableau 4 est le composé présent dans l'alginate modifié de manière covalente dans la deuxième composition polymère.

7. Capsule d'hydrogel selon l'une quelconque des revendications 1 à 6, dans laquelle le polypeptide thérapeutique est choisi dans le groupe constitué par un polypeptide du facteur VIII, un polypeptide du facteur VII et un polypeptide FIX.

8. Capsule d'hydrogel selon l'une quelconque des revendications 1 à 7, dans laquelle les cellules RPE modifiées sont dérivées de cellules ARPE-19 et les cellules ARPE-19 sont présentes à une concentration d'environ 25 millions à 35 millions, d'environ 35 millions à 45 millions ou d'environ 40 millions de cellules par millilitre de la première composition polymère, l'alginate présent dans l'alginate modifié de manière covalente dans le compartiment contenant des cellules présente une masse moléculaire de 75 kDa à 150 kDa, un rapport G:M supérieur ou égal à 1,5 et est modifié de manière covalente par GRGDSP, et dans laquelle la densité de GRGDSP sur l'alginate, déterminée par un dosage quantitatif de conjugaison peptidique, est d'environ 0,1 à environ 1,0 micromole de GRGDSP par gramme de GRGDSP-alginate dans une solution saline ayant une viscosité de 80 à 120 cP.

9. Capsule d'hydrogel selon l'une quelconque des revendications 1 à 8, dans laquelle le polypeptide thérapeutique est un polypeptide du facteur VIII-BDD, la séquence nucléotidique exogène comprend SEQ ID NO : 15, l'alginate présent dans la première composition polymère a une masse moléculaire de 75 kDa à 150 kDa et un rapport G:M supérieur ou égal à 1,5, et la densité de GRGDSP sur l'alginate, déterminée par un dosage quantitatif de conjugaison peptidique, est d'environ 0,3 à environ 0,6 micromole de GRGDSP par gramme de GRGDSP-alginate dans une solution saline ayant une viscosité de 80 à 120 cP.

10. Capsule d'hydrogel selon l'une quelconque des revendications 1 à 9, dans laquelle :
la deuxième composition polymère dans le compartiment barrière comprend en outre un alginate non modifié ayant une masse moléculaire de 150 kDa à 250 kDa et un rapport G/M supérieur ou égal à 1,5,
l'alginate dans l'alginate modifié de manière covalente dans le compartiment barrière a une masse moléculaire inférieure à 75 kDa, un rapport G:M supérieur ou égal à 1,5 et le composé décrit dans le tableau 4 est le composé 101, et
la densité de conjugaison du composé 101 dans l'alginate modifié de manière covalente, déterminée par analyse de combustion pour le pourcentage d'azote, est d'au moins 2,0 % d'azote et inférieure à 9,0 % d'azote, ou est de 3,0 % à 8,0 %, de 4,0 % à 7,0 %, de 5,0 % à 7,0 %, ou de 6,0 % à 7,0 % d'azote.

11. Capsule d'hydrogel selon l'une quelconque des revendications 1 à 10, la capsule d'hydrogel ayant un diamètre d'environ 1,5 mm et l'épaisseur du compartiment barrière étant comprise entre environ 180 µm et 260 µm ou entre environ 310 µm et 440 µm.

12. Préparation de capsules d'hydrogel, chaque capsule dans la préparation étant une capsule d'hydrogel selon l'une quelconque des revendications 1 à 11.

13. Préparation de capsules d'hydrogel selon la revendication 12, chaque capsule dans la préparation ayant un diamètre de 1,4 à 1,6 millimètre.

14. Préparation de capsules d'hydrogel selon la revendication 12 ou 13, qui est une composition pharmaceutiquement acceptable, adaptée pour la mise en place ou l'implantation dans la cavité péritonéale d'un sujet.

15. Composition destinée à être utilisée dans une méthode de traitement d'un sujet ayant besoin d'un agent thérapeutique, la méthode comprenant :
la fourniture d'une préparation de capsules d'hydrogel selon l'une quelconque des revendications 12 à 14, et
l'implantation de la préparation dans une cavité corporelle d'un sujet.

16. Composition selon la revendication 15, dans laquelle l'agent thérapeutique est un facteur de coagulation sanguine.

17. Composition selon la revendication 16, dans laquelle le facteur de coagulation sanguine est une protéine de facteur VIII-BDD, qui est essentiellement constituée de SEQ ID NO : 1.

18. Composition selon la revendication 16, dans laquelle le facteur de coagulation sanguine est une protéine de facteur IX-padua, qui comprend SEQ ID NO : 36.
